(19) Europäisches Patentamt
European Patent Office
Office européen des brevets

(11) EP 4 582 106 A1

(12) **EUROPEAN PATENT APPLICATION**
published in accordance with Art. 153(4) EPC

(43) Date of publication:
**09.07.2025 Bulletin 2025/28**

(21) Application number: **23860252.8**

(22) Date of filing: **28.08.2023**

(51) International Patent Classification (IPC):
***A61K 47/68*** (2017.01) ***A61K 31/7064*** (2006.01)
***A61K 31/708*** (2006.01) ***A61K 39/395*** (2006.01)
***A61K 45/00*** (2006.01) ***A61P 35/00*** (2006.01)
***A61P 43/00*** (2006.01) ***C07K 16/28*** (2006.01)
***C12N 15/13*** (2006.01)

(52) Cooperative Patent Classification (CPC):
**A61K 31/7064; A61K 31/708; A61K 39/395; A61K 45/00; A61K 47/68; A61P 35/00; A61P 43/00; C07K 16/00; C07K 16/28**

(86) International application number:
**PCT/JP2023/030872**

(87) International publication number:
**WO 2024/048490 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.08.2022 JP 2022135598**

(71) Applicant: **Daiichi Sankyo Company, Limited**
**Tokyo 103-8426 (JP)**

(72) Inventors:
- **HARA, Kyoko**
  **Tokyo 103-8426 (JP)**
- **CHIHARA, Masataka**
  **Tokyo 103-8426 (JP)**
- **SHINOZAKI, Naoya**
  **Tokyo 103-8426 (JP)**
- **NAGAOKA, Nobumi**
  **Tokyo 103-8426 (JP)**
- **TSUDA, Masashi**
  **Tokyo 103-8426 (JP)**
- **KADOHIRA, Mariko**
  **Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) ANTIBODY-DRUG CONJUGATE INCLUDING MUTANT FC REGION

(57) [Problem] It is desirable to develop a molecule having excellent safety while maintaining anti-tumor activity. It is also desirable to develop an antibody-drug conjugate capable of being systemically administered and delivering a STING agonist specifically to a target cell or organ (for example, a tumor lesion), and a therapeutic agent and/or therapeutic method using the antibody-drug conjugate for diseases related to STING agonist activity, for example, diseases (for example, cancers) to which immunostimulation therapy can be applied. [Solution] An antibody-drug conjugate obtained by conjugating a CDN derivative, which is characterized by the presence of a fused tricyclic substituent, and a specific antibody comprising a mutated Fc region or a functional fragment of the antibody via a linker, and an antibody comprising a mutated Fc region, are provided.

EP 4 582 106 A1

## Description

Technical Field

The present invention relates to a molecule having excellent safety while maintaining anti-tumor activity, an antibody-drug conjugate in which a cyclic dinucleotide derivative having STING agonist activity is conjugated with an antibody comprising a mutated Fc region or a functional fragment of the antibody via a linker, a pharmaceutical composition comprising the antibody-drug conjugate, an antibody comprising a mutated Fc region, a method for producing an Fc-containing molecule having an N297-linked glycan including a glycan donor molecule-derived glycan, and so on.

BACKGROUND ART

Fc regions impart a long half-life in blood or effector functions such as antibody-dependent cellularcytotoxicity (ADCC) or antibody dependent cellular phagocytosis (ADCP) to antibodies. In the case where an Fc region is derived from a subclass called IgG which is commonly used in antibody medicines, the effector functions depend on the binding of the Fc region to a receptor family called Fcγ receptor (FcγR). However, the FcγR-binding activity is often regarded as an undesirable characteristic for antibody medicines because it has been suggested to be involved in safety risks such as, for example, infusion reactions (Non-Patent Literature 1).

Various amino acid substitutions, in the Fc regions of IgG-type antibodies, which attenuate binding activity against human FcγR have previously been reported (for example, Patent Literatures 1 to 6 and Non-Patent Literatures 2 and 3).

However, it has been reported that in an antibody-drug conjugate in which a drug having immune cell stimulating activity (immunostimulator) is conjugated with an antibody, a pharmaceutical effect is decreased when the effector functions of the antibody are decreased (Non-Patent Literatures 4 to 6 and Patent Literature 36).

STING (Stimulator of Interferon Genes) is a transmembrane adaptor protein localized in the endoplasmic reticulum (Non-Patent Literature 7). STING functions as a central molecule for the activation of innate immunity in mammals, and is at the forefront of defense against the entry of pathogens such as bacteria and viruses. The activation of STING is known to be triggered by a signal(s) from multiple cytoplasmic DNA sensors at the time of detecting exogenous or endogenous DNA. Among the cytoplasmic DNA sensors, cGAS (Cyclic GMP-AMP Synthase) is considered to be an important DNA sensor. When cGAS senses DNA, a cyclic dinucleotide (2',3'-cGAMP) is produced, and this 2',3'-cGAMP binds directly to STING and activates STING (Non-Patent Literature 8). Activated STING translocates to the Golgi apparatus, where autophosphorylation of TBK1 (Tank-binding kinase 1) is promoted. Autophosphorylated and activated TBK1 activates both the IRF3 (Interferon regulatory factor 3) transcriptional pathway (Non-Patent Literature 9) and the NFκB transcriptional pathway (Non-Patent Literature 10), and increases the production of inflammatory proteins called interferons and cytokines (type I IFN (Interferon), IL-6 (Interleukin-6), TNF-α (Tumor Necrosis Factor-α)). These proteins trigger the adaptive immune system including T cells, which destroy pathogens and cancer cells through a complex cascade.

Recent studies have shown that STING promotes not only host defenses against microbes but also anti-tumor immunity. For example, when immunogenic tumor cells are transplanted into STING-deficient mice, the tumor grows more rapidly than in wild-type mice or in TRIF (Toll/Interleukin-1 (IL-1) receptor domain containing adaptor-inducing interferon-β)-deficient mice. In addition, unlike in TLR (Toll-like receptor), MyD88 (Myeloid differentiation primary response 88), or MAVS (Mitochondrial antiviral-signaling protein)-deficient mice, spontaneous priming of $CD8^+$ T cells to tumors was also lost in the STING-deficient mice. These results suggest that the STING pathway, which is initiated by detecting cytoplasmic DNA, is involved in the regulation of tumor growth (Non-Patent Literature 11). Other studies have also shown that STING is necessary for the anti-tumor effects of radiation therapy (Non-Patent Literature 12) and for anti-CD47 antibody therapy (Non-Patent Literature 13). DNA derived from dead tumor cells after treatment with radiation or anti-CD47 antibody translocates to the cytoplasm of dendritic cells, activates the cGAS-STING pathway, and induces IFN production to activate adaptive immunity through innate immunity. These studies suggest that cross-priming mediated by dendritic cells activated by the STING pathway is critical for triggering adaptive immunity against tumors.

DMXAA, a flavonoid-based small molecule compound known as a vascular disrupting agent, induces type I IFN in macrophages and has been shown to have potent anti-tumor activity in a mouse tumor model (Non-Patent Literature 14). DMXAA was expected to be an immunotherapeutic drug for non-small cell lung carcinoma due to its excellent anti-tumor effect in preclinical studies, but failed in clinical trials (Non-Patent Literature 15). Recent studies have revealed that DMXAA is a mouse-specific STING agonist and cannot bind to human STING due to lack of cross-species reactivity (Non-Patent Literature 16). Although DMXAA was ineffective in humans, studies in the mouse model suggest that small molecule drugs can effectively prime $CD8^+$ T cells and enhance anti-tumor immunity via STING.

Another small molecule compound, cyclic dinucleotide (CDN), has been shown to enhance a STING-mediated anti-tumor immune response, markedly inhibit tumor growth, and improve the survival rate in tumor-bearing mice (Non-Patent Literature 17). CDNs are grouped into bacterial CDN with two canonical 3'-5' phosphate bonds (cyclic-di-GMP, cyclic-di-AMP, 3',3'-cGAMP), and a mixed-linkage CDN with two non-canonical 2'-5' phosphate bonds and produced by mammalian cGAS (2',3'-cGAMP). Recent studies have demonstrated that mixed-linkage CDNs are more capable of universally activating a variety of STINGs than canonical CDNs (Non-Patent Literature 18).

Natural CDNs, like most nucleic acid molecules, are rapidly degraded by nucleases in blood, and cannot thus be administered as they are. In view of this, synthetic small molecule compounds with *in vivo* STING agonist activity have been developed (e.g., Patent Literatures 8 to 33).

The STING agonist MIW-815 (also called ADU-S100, ML RR-S2 CDA, or ML-RR-CDA·$2Na^+$), which is currently undergoing a clinical trial as an anti-tumor agent, is administered directly into a tumor. In the method for directly administering a STING agonist to a tumor, the drug can only be delivered to a limited area in the tumor. In addition, it is difficult to directly administer the drug to all distant metastatic tumors. Unfortunately, this limits the type of tumors that can be treated. Non-Patent Literature 19 describes the anti-tumor effect upon administration of ML RR-S2 CDA, but only by intratumor administration, not by systemic administration (e.g., intravenous administration). Non-Patent Literature 20 discloses that intravenous administration of SB11285, a STING agonist, to a mouse tumor model elicited anti-tumor effects. However, the specific structure of the compound SB11285 has not been indicated. Patent Literature 21 describes a conjugate containing an immunostimulant, an antibody construct, and a linker. However, no specific examples of the conjugate using a STING agonist as the immunostimulant have been disclosed. Patent Literature 33 describes a conjugate in which a CDN with a specific structure is conjugated via a linker to an antibody. However, there are no examples of *in vivo* administration or anti-tumor effects of the conjugate. Patent Literatures 34, 35, 37, and 38 describe a conjugate in which a STING agonist used as the immunostimulant is conjugated via a linker to an antibody and describe examples of *in vivo* administration of the conjugate.

Meanwhile, transglycosylation reaction using an enzyme is known as a method for preparing a homogeneous glycan to be added to a medical antibody or a glycoprotein molecule comprising an antibody Fc region. In particular, a one-pot method is known which directly transfers a glycan to a GlcNAc acceptor using two types of ENGase.

Citation List

Patent Literature

Patent Literature 1: International Publication No. WO 1988/07089
Patent Literature 2: International Publication No. WO 1999/51642
Patent Literature 3: International Publication No. WO 2000/42072
Patent Literature 4: International Publication No. WO 2013/092001
Patent Literature 5: International Publication No. WO 2015/109131
Patent Literature 6: International Publication No. WO 2020/086776
Patent Literature 7: International Publication No. WO 2013/118858
Patent Literature 8: International Publication No. WO 2014/099824
Patent Literature 9: International Publication No. WO 2014/179335
Patent Literature 10: International Publication No. WO 2014/189805
Patent Literature 11: International Publication No. WO 2014/189806
Patent Literature 12: International Publication No. WO 2015/074145
Patent Literature 13: International Publication No. WO 2015/185565
Patent Literature 14: International Publication No. WO 2016/096714
Patent Literature 15: International Publication No. WO 2016/012305
Patent Literature 16: International Publication No. WO 2016/145102
Patent Literature 17: International Publication No. WO 2017/027646
Patent Literature 18: International Publication No. WO 2017/027645
Patent Literature 19: International Publication No. WO 2017/075477
Patent Literature 20: International Publication No. WO 2017/093933
Patent Literature 21: International Publication No. WO 2017/100305
Patent Literature 22: International Publication No. WO 2017/123669
Patent Literature 23: International Publication No. WO 2017/161349
Patent Literature 24: International Publication No. WO 2017/175147
Patent Literature 25: International Publication No. WO 2017/175156
Patent Literature 26: International Publication No. WO 2018/009466
Patent Literature 27: International Publication No. WO 2018/045204

Patent Literature 28: International Publication No. WO 2018/060323
Patent Literature 29: International Publication No. WO 2018/067423
Patent Literature 30: International Publication No. WO 2018/065360
Patent Literature 31: International Publication No. WO 2014/093936
Patent Literature 32: International Publication No. WO 2018/009648
Patent Literature 33: International Publication No. WO 2018/100558
Patent Literature 34: International Publication No. WO 2021/202984
Patent Literature 35: International Publication No. WO 2022/097117
Patent Literature 36: International Publication No. WO 2019/084060
Patent Literature 37: International Publication No. WO 2020/050406
Patent Literature 38: International Publication No. WO 2021/177438

Non Patent Literature

Non-Patent Literature 1: J Immnotoxicol; 5(1): 11-5 (2008)
Non Patent Literature 2: Protein Eng Des Sel.; 29 (10): 457-66 (2016).
Non Patent Literature 3: J Biol Chem; 292 (5): 1865-75 (2017)
Non Patent Literature 4: Nature Cancer 2021, 2, 18-33
Non Patent Literature 5: AACR 2021 Poster#1773
Non Patent Literature 6: Sean W. Smith, "SBT6050, a HER2-Directed TLR8 ImmunoTAC™ Therapeutic, is a Potent Human Myeloid Cell Agonist with Tumor-Localized Activity", World ADC 2020
Non Patent Literature 7: Nature 2008, 455, 674-678
Non Patent Literature 8: Mol. Cell, 2013, 51, 226-235
Non Patent Literature 9: Science 2015a, 347, aaa2630
Non Patent Literature 10: J. Virol. 2014, 88, 5328-5341
Non Patent Literature 11: Immunity 2014, 41, 830-842
Non Patent Literature 12: Immunity 2014, 41, 843-852
Non Patent Literature 13: Nat. Med. 2015, 21, 1209-1215
Non Patent Literature 14: J. Immunol. 1994, 153, 4684-4693
Non Patent Literature 15: J. Clin. Oncol. 2011, 29, 2965-2971
Non Patent Literature 16: J. Immunol. 2013, 190, 5216-5225
Non Patent Literature 17: Sci. Rep. 2016, 6, 19049
Non Patent Literature 18: Mol. Cell, 2015, 59, 891-903
Non Patent Literature 19: Cell Rep. 2015, 11, 1018-1030
Non Patent Literature 20: AACR Tumor Immunology and Immunotherapy, 2017, Poster#A25

SUMMARY OF INVENTION

Problem to be resolved by the Invention

A molecule having excellent safety while maintaining anti-tumor activity should be developed. The following should be also developed: an antibody-drug conjugate that can be systemically administered and can specifically deliver a STING agonist to a target cell(s) or organ(s) (e.g., a tumor site), and a therapeutic agent and/or method for treating a STING agonist-related disease (e.g., a disease (e.g., cancer) that can be treated by immunostimulants) by using the antibody-drug conjugate. Further, a novel method for producing an Fc-containing molecule (particularly, an antibody) having a homogeneous glycan structure should be established, the method having, in particular, an improved rate of transglycosylation.

Means of solving the Problem

The present inventors have conducted diligent studies to address the above problems, and have consequently discovered an antibody-drug conjugate obtained by conjugating a CDN derivative, which is characterized by the presence of a fused tricyclic substituent, and a specific antibody comprising a mutated Fc region or a functional fragment of the antibody via a linker. It has also been found that systemic administration of the antibody-drug conjugate elicits anti-tumor effects in an antigen-expressing tumor and further has excellent safety, thus completing the present invention. It has been further found that the specific antibody comprising a mutated Fc region has excellent safety. A method for producing an

improved Fc-containing molecule (particularly, an antibody) by use of a one-pot method has been also discovered, thus completing the present invention.

Specifically, the invention of the present application relates to the following, but is not limited thereto.

[1] An antibody-drug conjugate represented by the following formula (II):

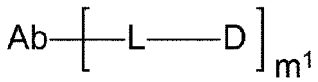

(II)

wherein $m^1$ ranges from 1 to 10;

Ab represents an antibody or a functional fragment of the antibody, wherein the antibody or the functional fragment of the antibody optionally has a remodeled glycan, and the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody;

L represents a linker linking Ab and D, wherein

Ab may directly bond to L from an amino acid residue of Ab or may bond to L via the glycan or remodeled glycan of Ab;

D represents a compound represented by the following formula (I) :

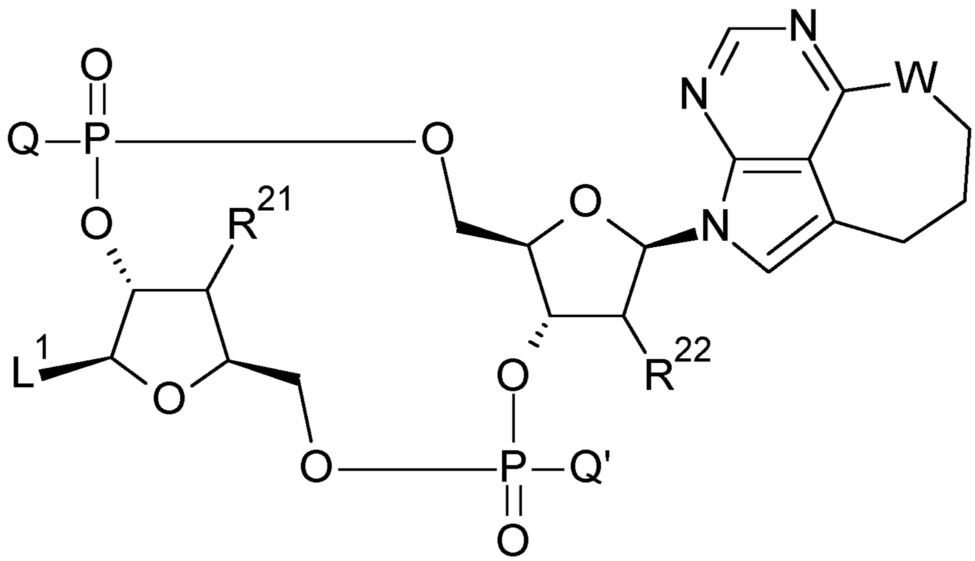

wherein

L bonds to a hydroxy group included in $L^1$,

$L^1$ represents a group having the following formula:

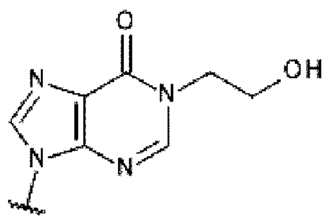

wherein the wavy line represents the position of substitution,

Q and Q' each independently represent a hydroxy group or a thiol group,

$R^{21}$ and $R^{22}$ each independently represent a hydroxy group or a fluorine atom, and

W represents -NH- or a sulfur atom.

[2] The antibody-drug conjugate according to [1], wherein D is represented by any one of the following two formulae:

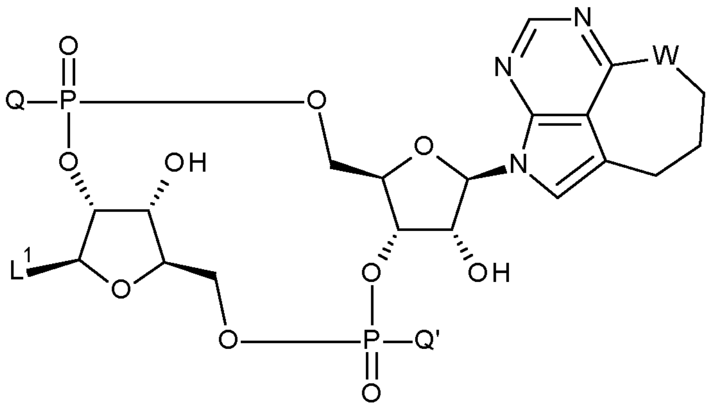

or

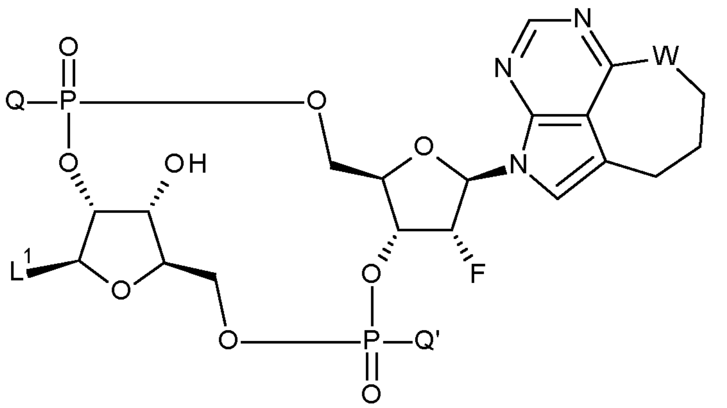

wherein $L^1$, Q, Q', and W are as defined above.

[3] The antibody-drug conjugate according to [1] or [2], wherein D is represented by any one of the following two formulae:

or

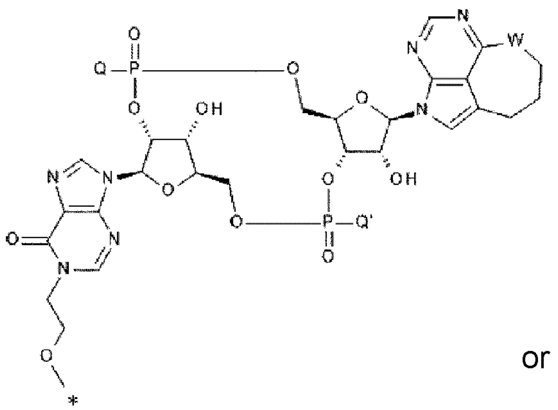

or

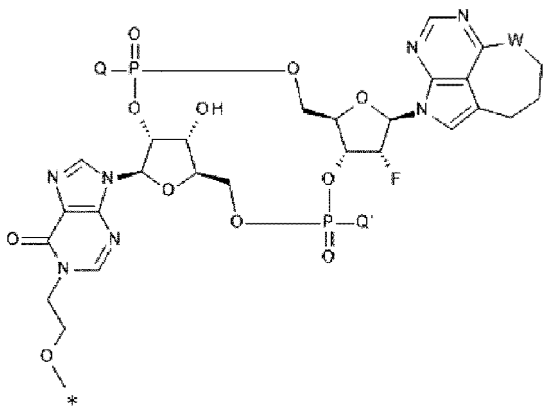

wherein the asterisk represents bonding to L, and Q, Q', and W are as defined above.

[4] The antibody-drug conjugate according to any one of [1] to [3], wherein D is represented by any one of the following three formulae:

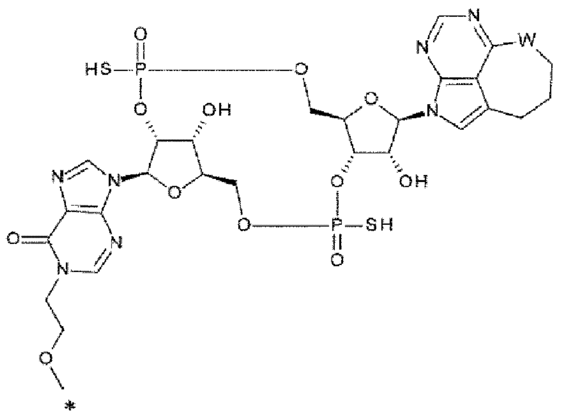

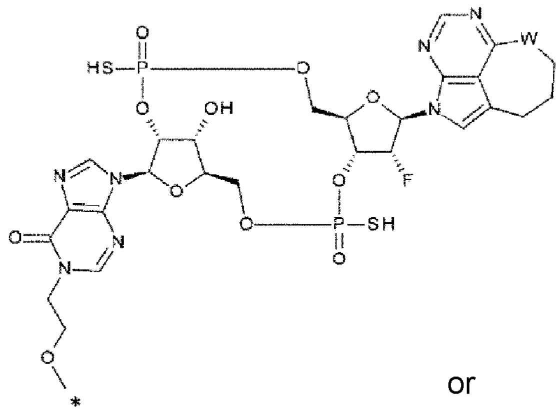

or

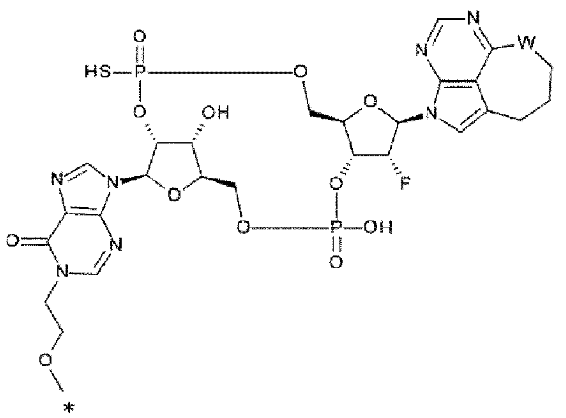

wherein the asterisk represents bonding to L, and W is as defined above.

[5] The antibody-drug conjugate according to any one of [1] to [4], wherein D is represented by any one of the following three formulae:

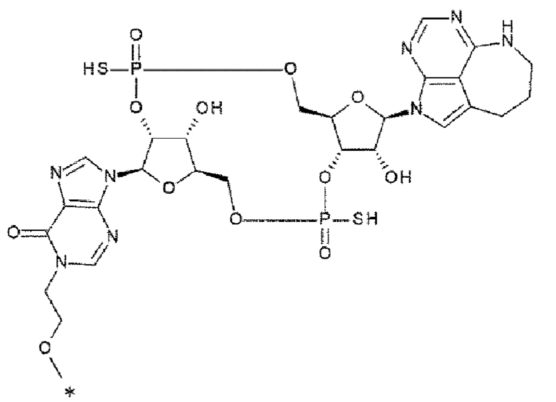

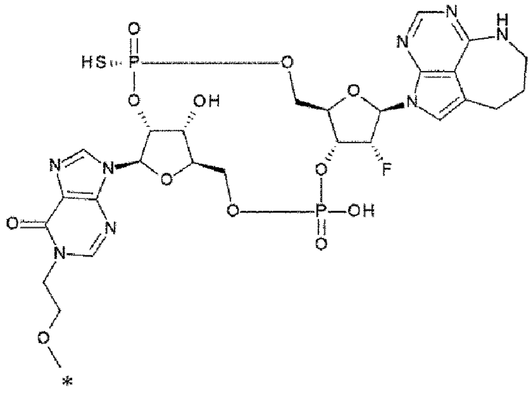

or

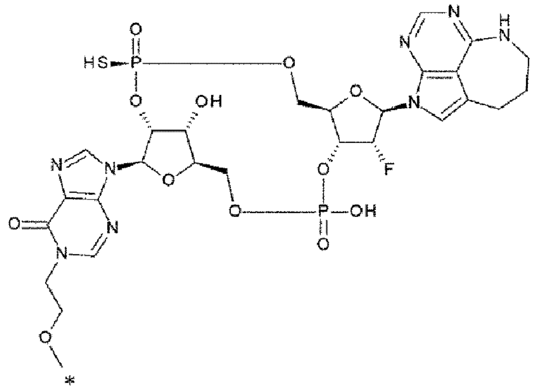

wherein the asterisk represents bonding to L.

[6] The antibody-drug conjugate according to any one of [1] to [4], wherein D is represented by any one of the following four formulae:

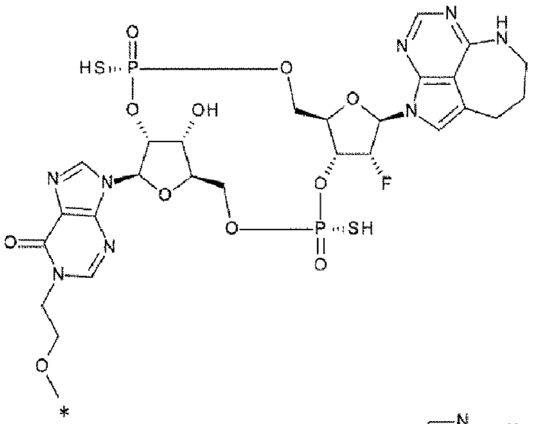

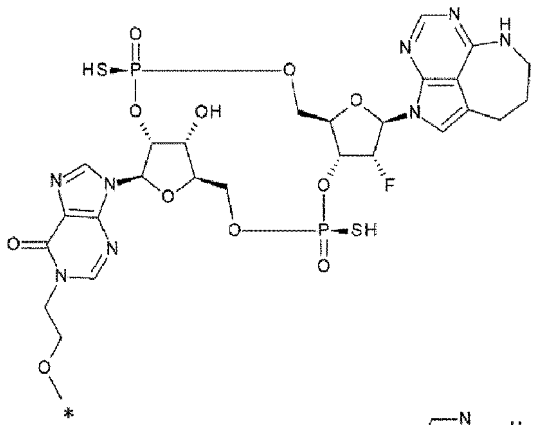

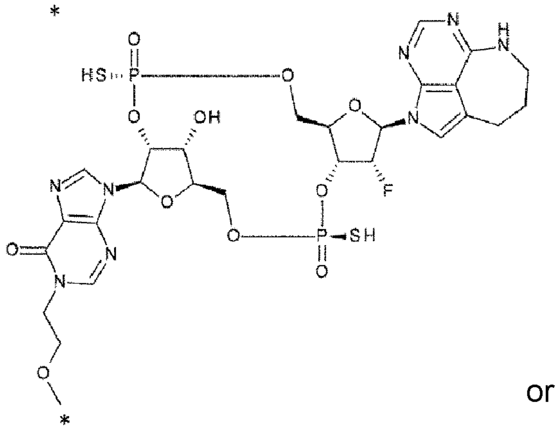

or

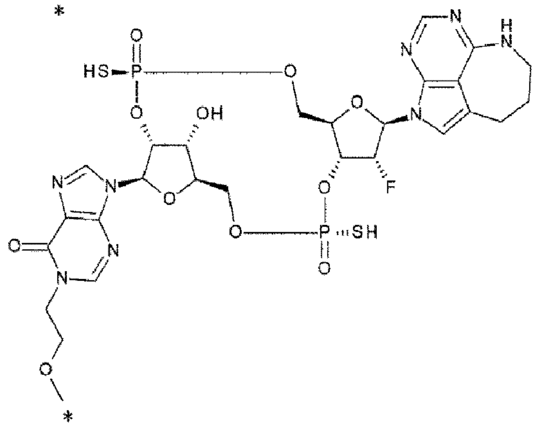

wherein the asterisk represents bonding to L.

[7] The antibody-drug conjugate according to any one of [1] to [4] and [6], wherein D is represented by the following formula:

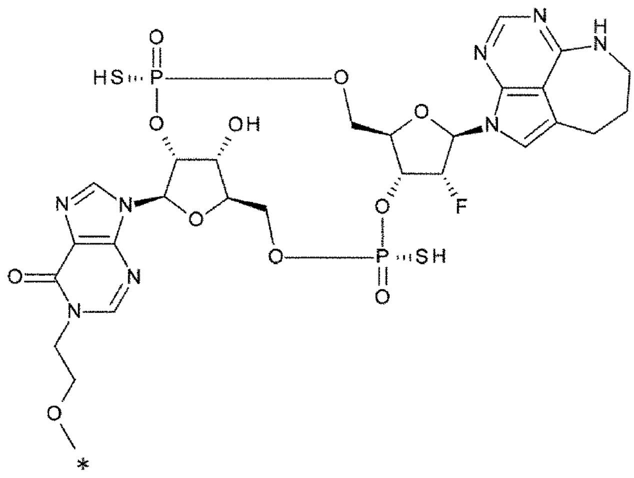

wherein the asterisk represents bonding to L.

[8] The antibody-drug conjugate according to any one of [1] to [7], wherein the linker L is represented by -Lb-La-Lp-Lc-*

wherein the asterisk represents bonding to the drug D;
Lp represents a linker consisting of an amino acid sequence cleavable in a target cell or is absent;
La represents any one selected from the group consisting of the following:

$-C(=O)-(CH_2CH_2)n^2-C(=O)-$,

$-C(=O)-(CH_2CH_2)n^2-CH_2-C(=O)-$,

$-C(=O)-(CH_2CH_2)n^2-C(=O)-NH-(CH_2CH_2)n^3-C(=O)-$,

$-C(=O)-(CH_2CH_2)n^2-C(=O)-NH-(CH_2CH_2)n^3-CH_2-C(=O)$

$-C(=O)-(CH_2CH_2)n^2-C(=O)-NH-(CH_2CH_2O)n^3-CH_2-C(=O)-$,

$-(CH_2)n^4-O-C(=O)-$,

and

$-(CH_2)n^9-C(=O)-$,

where $n^2$ represents an integer of 1 to 3, $n^3$ represents an integer of 1 to 5, $n^4$ represents an integer of 0 to 2, and $n^9$ represents an integer of 2 to 7;
Lb represents a spacer for bonding La to the glycan or remodeled glycan of Ab or a spacer for bonding La to a cysteine residue of Ab; and
Lc represents $-NH-CH_2-$, $-NH-phenyl-CH_2-O(C=O)-$, or $-NH-$ heteroaryl$-CH_2-O(C=O)-$, or is absent.

[9] The antibody-drug conjugate according to [8], wherein Lc is $-NH-CH_2-$.

[10] The antibody-drug conjugate according to [8] or [9], wherein Lp is any one of -GGFG-, -GGPI-, -GGVA-, -GGFM-, -GGVCit-, - GGFCit-, -GGICit-, -GGPL-, -GGAQ-, or -GGPP-.

[11] The antibody-drug conjugate according to [10], wherein Lp is -GGFG- or -GGPI-.

[12] The antibody-drug conjugate according to any one of [8] to [11], wherein La represents any one selected from the group consisting of the following:

$-C(=O)-CH_2CH_2-C(=O)-$,

$-C(=O)-CH_2CH_2-C(=O)-NH-(CH_2CH_2O)_3-CH_2-C(=O)-$,

$-C(=O)-CH_2CH_2-C(=O)-NH-(CH_2CH_2O)_4-CH_2-C(=O)-$,

and

$$-(CH_2)_5-C(=O)-.$$

[13] The antibody-drug conjugate according to any one of [8] to [12], wherein Lb is represented by any one of the following formulae:

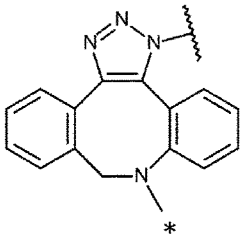

or

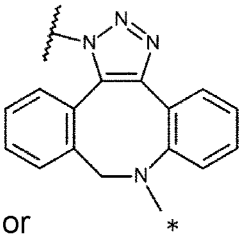

Or

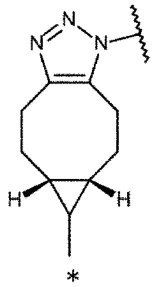

or

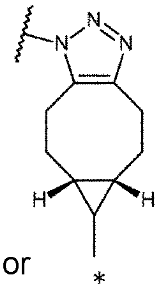

wherein, in the structural formulae of Lb shown above, the asterisk represents bonding to La, and the wavy line represents bonding to the glycan or remodeled glycan of Ab.

[14] The antibody-drug conjugate according to any one of [8] to [12], wherein Lb represents -(succinimid-3-yl-N)-, wherein - (succinimid-3-yl-N)- is represented by the following structural formula:

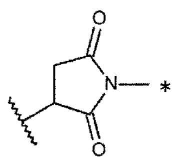

wherein the asterisk represents bonding to La, and the wavy line represents bonding to a side chain of a cysteine residue of the antibody by forming a thioether.

[15] The antibody-drug conjugate according to any one of [8] to [13], wherein the linker L is represented by -Lb-La-Lp-Lc-*

wherein the asterisk represents bonding to the drug D;
Lp is -GGFG- or -GGPI-;
La represents $-C(=O)-CH_2CH_2-C(=O)-$;
Lb represents the following formula:

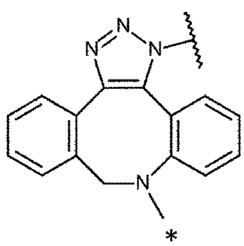

or

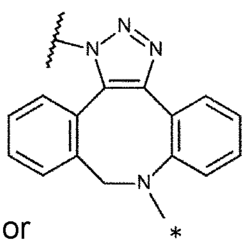

wherein, in the structural formulae of Lb shown above, the asterisk represents bonding to La, and the wavy line represents bonding to the glycan or remodeled glycan of Ab; and
Lc represents $-NH-CH_2-$.

[16] The antibody-drug conjugate according to any one of [1] to [15], wherein the average number of the conjugated drug molecules per antibody molecule in the antibody-drug conjugate ranges from 1 to 10.
[17] The antibody-drug conjugate according to [16], wherein the average number of the conjugated drug molecules per antibody molecule in the antibody-drug conjugate ranges from 1 to 5.
[18] The antibody-drug conjugate according to [17], wherein the average number of the conjugated drug molecules per antibody molecule in the antibody-drug conjugate ranges from 1 to 3 or from 3 to 5.
[19] The antibody-drug conjugate according to any one of [1] to [18], wherein the antibody bonds to L via a glycan bonding to Asn297 of the antibody (N297glycan).
[20] The antibody-drug conjugate according to [19], wherein the N297 glycan is a remodeled glycan.
[21] The antibody-drug conjugate according to [19] or [20], wherein the N297 glycan is N297-(Fuc)MSG1 or N297-(Fuc)SG having a structure represented by the following formula:

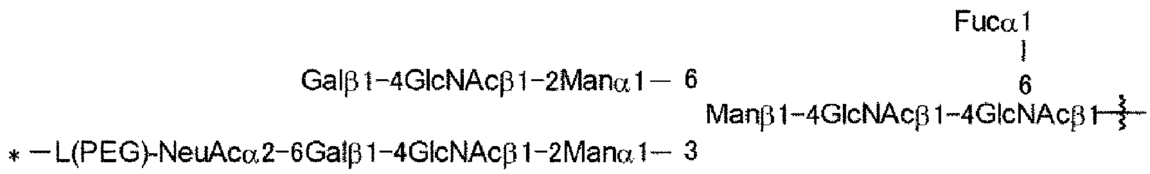

[N297-(Fuc)MSG1]

wherein the wavy line represents bonding to Asn297 of the antibody,
L(PEG) represents $-(CH_2-CH_2-O)n^5-CH_2-CH_2-NH-$,
wherein the amino group at the right end of the L(PEG) is bound via an amide bond to a carboxyl group at the 2-position of a sialic acid at the non-reducing terminal of the 1-3 branched chain of β-Man in the N297 glycan,
the asterisk represents bonding to a nitrogen atom at the 1- or 3-position of the 1,2,3-triazole ring of Lb in the linker L, and
$n^5$ is an integer of 2 to 5;

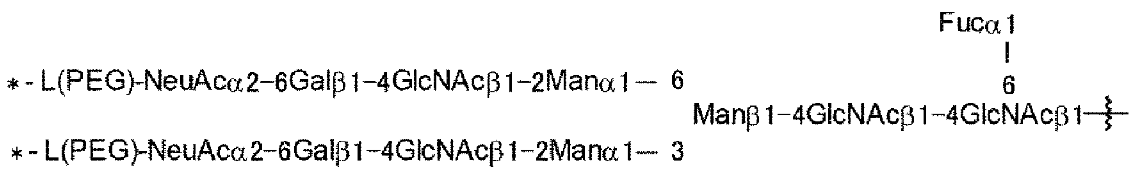

[N297-(Fuc)SG]

wherein the wavy line represents bonding to Asn297 of the antibody,
L(PEG) represents $-(CH_2-CH_2-O)n^5-CH_2-CH_2-NH-$,
wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L, and
$n^5$ is an integer of 2 to 5.

[22] The antibody-drug conjugate according to any one of [19] to [21], wherein the antibody-drug conjugate is represented by the following formula:

$$Ab\left[(N297\ glycan)\left[L - D\right]_{m^2}\right]_2$$

wherein $m^2$ represents an integer of 1 or 2,

L is a linker linking N297 glycan and D, as defined above,

Ab represents an antibody or a functional fragment of the antibody, wherein the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody,

N297 glycan is represented by N297-(Fuc)MSG1 or N297-(Fuc)SG having a structure represented by the following formula:

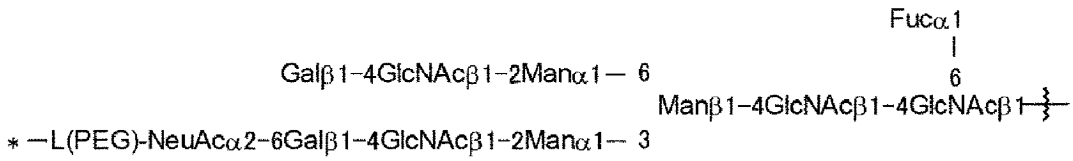

[N297-(Fuc)MSG1]

wherein the wavy line represents bonding to Asn297 of the antibody,

L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of the 1-3 branched chain of β-Man in the N297 glycan,

the asterisk represents bonding to a nitrogen atom at the 1- or 3-position of the 1,2,3-triazole ring of Lb in the linker L, and

$n^5$ represents an integer of 2 to 5; or

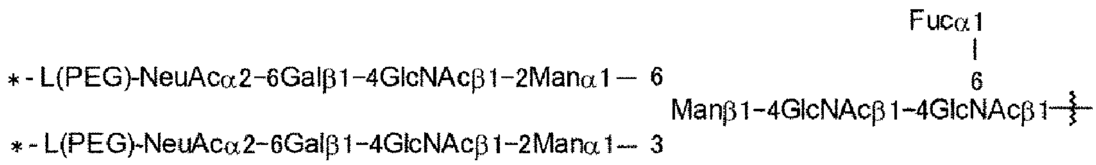

[N297-(Fuc)SG]

wherein the wavy line represents bonding to Asn297 of the antibody,

L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,

the asterisk represents bonding to a nitrogen atom at the 1- or 3-position of the 1,2,3-triazole ring of Lb in the linker L, and

$n^5$ represents an integer of 2 to 5,

D is represented by any one of the following four formulae:

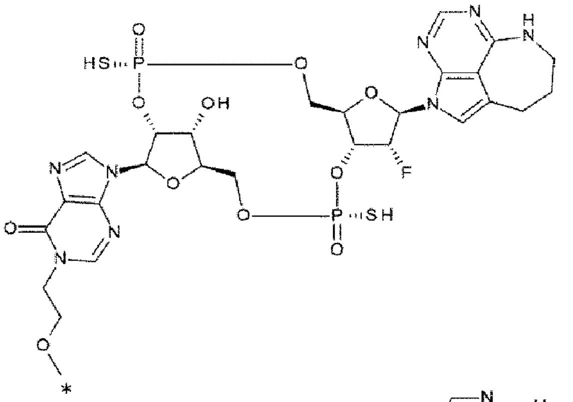

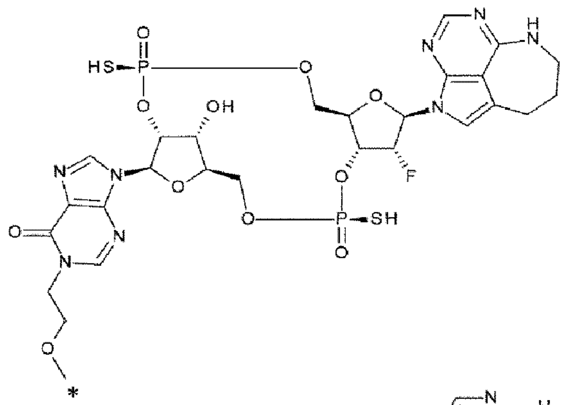

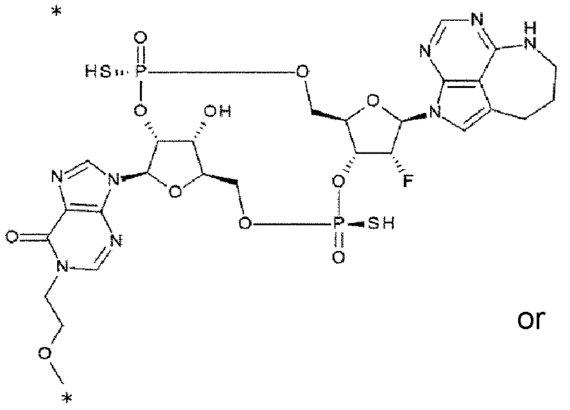

or

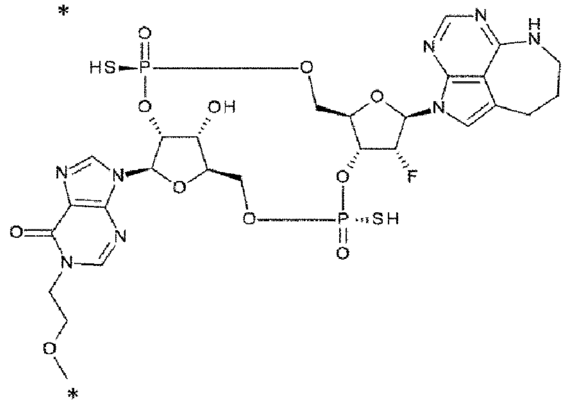

wherein the asterisk represents bonding to L.

[23] The antibody-drug conjugate according to [22], wherein the antibody-drug conjugate is selected from the following formulae:

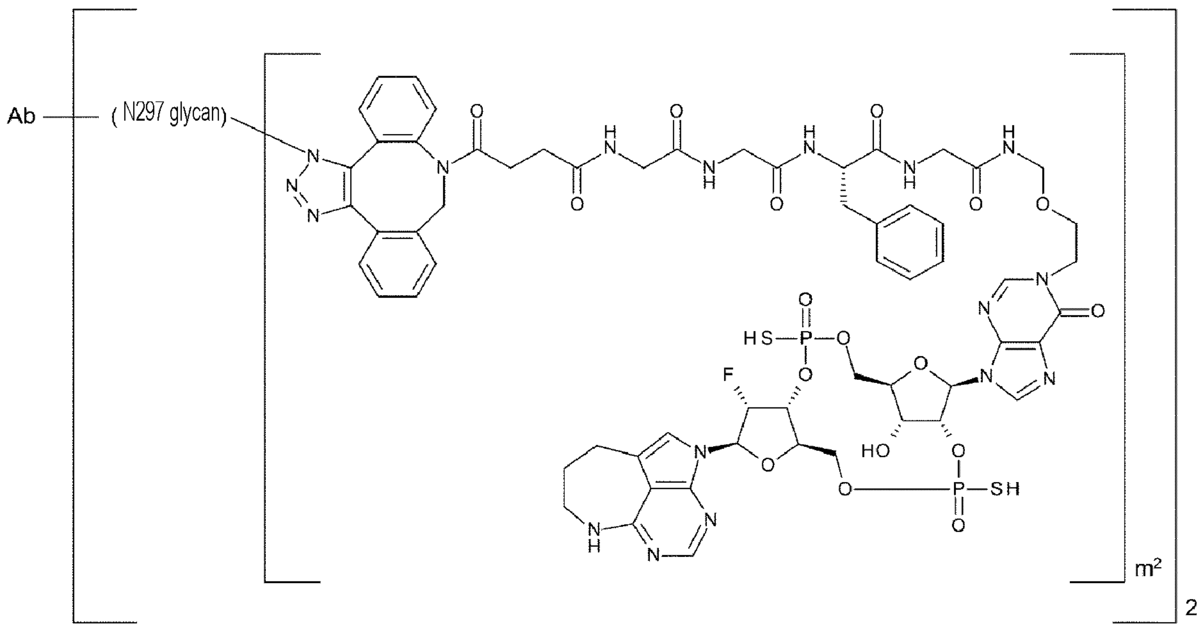

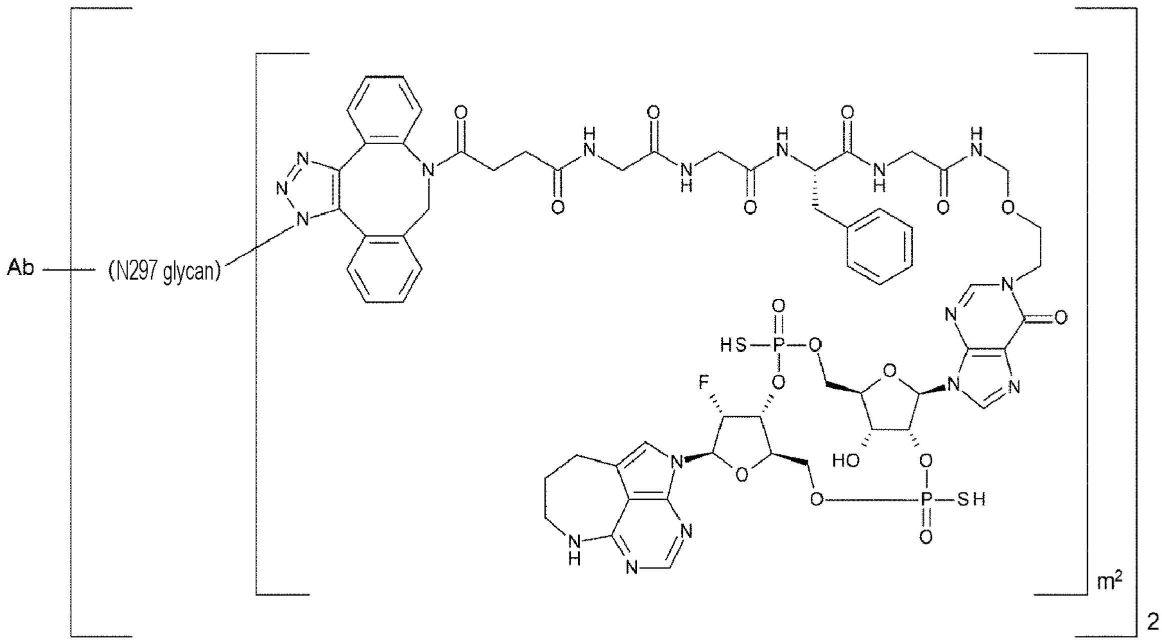

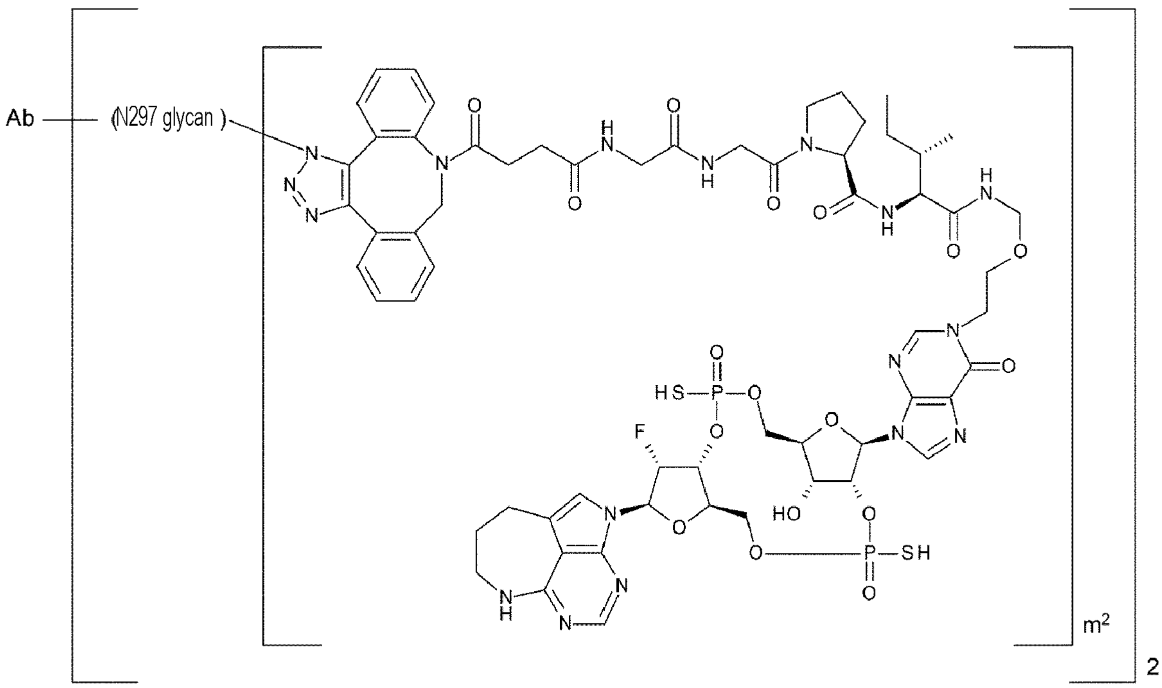

or

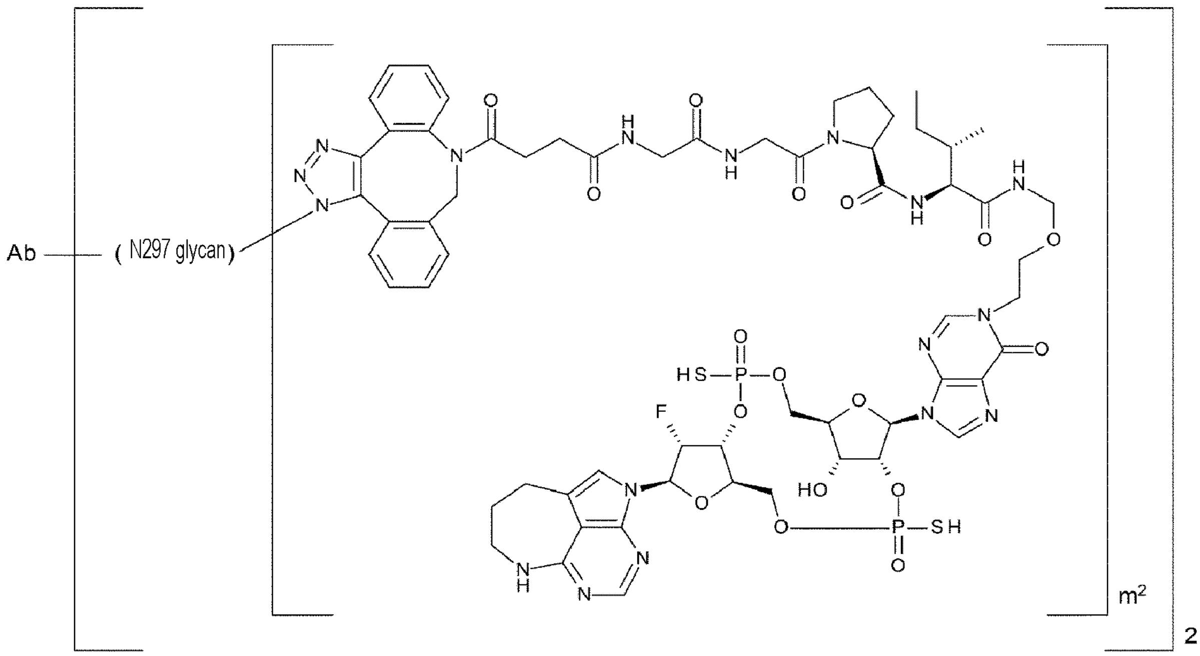

wherein, in each structural formula shown above, $m^2$ is an integer of 1 or 2,

Ab represents an antibody or a functional fragment of the antibody, wherein the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody,

N297 glycan is represented by any one of N297-(Fuc)MSG1 or N297-(Fuc)SG having a structure represented by the following formula:

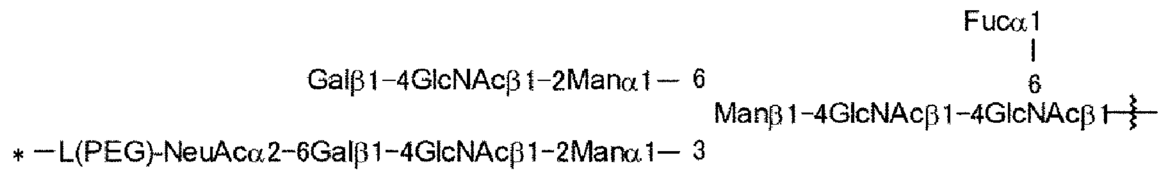

[N297-(Fuc)MSG1]

wherein the wavy line represents bonding to Asn297 of the antibody,

L(PEG) represents $-(CH_2-CH_2O)n^5-CH_2-CH_2-NH-$,

wherein the amino group at the right end of the L(PEG) is bound via an amide bond to a carboxyl group at the 2-position of a sialic acid at the non-reducing terminal of the 1-3 branched chain of β-Man in the N297 glycan, the asterisk represents bonding to a nitrogen atom at the 1- or 3-position of the 1,2,3-triazole ring of Lb in the linker L, and

$n^5$ represents an integer of 2 to 5; or

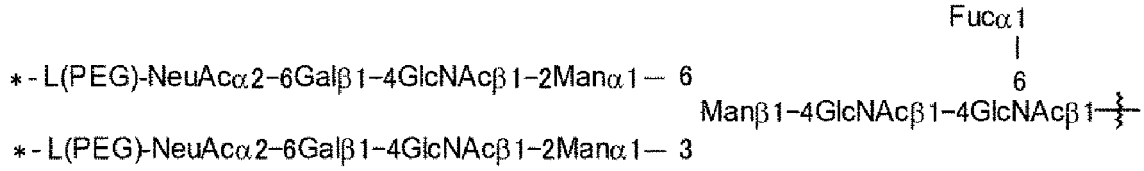

[N297-(Fuc)SG]

wherein the wavy line represents bonding to Asn297 of the antibody,

L(PEG) represents $-(CH_2-CH_2-O)n^5-CH_2-CH_2-NH-$,

wherein the amino group at the right end of the L(PEG) is bound via an amide bond to a carboxyl group at the 2-

position of a sialic acid at the non-reducing terminal of each of the 1-3 and 1-6 branched chains of β-Man in the N297 glycan,

the asterisk represents bonding to a nitrogen atom at the 1- or 3-position of the 1,2,3-triazole ring of Lb in the linker L, and

$n^5$ represents an integer of 2 to 5.

[24] The antibody-drug conjugate according to [23], wherein the antibody-drug conjugate is selected from the following formulae:

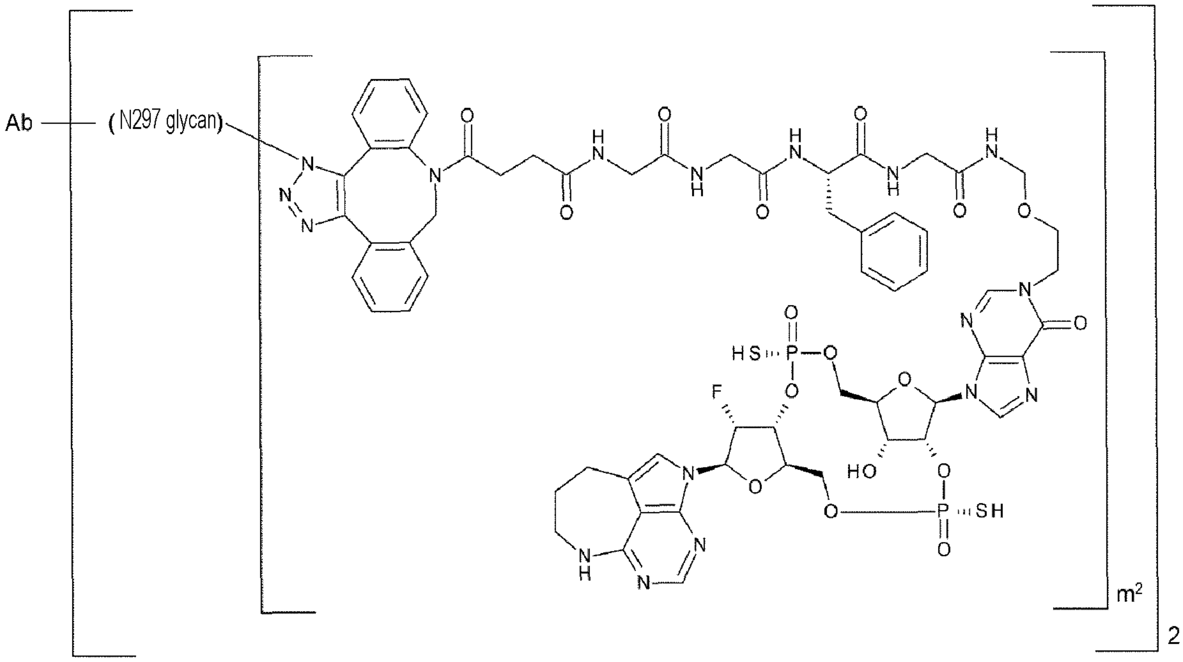

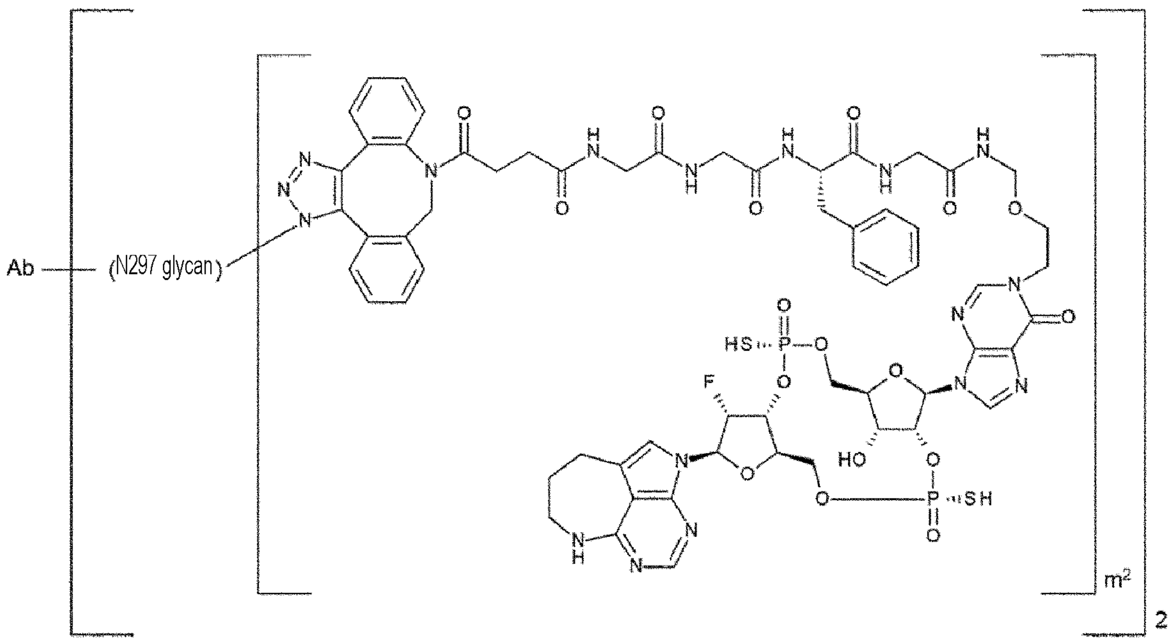

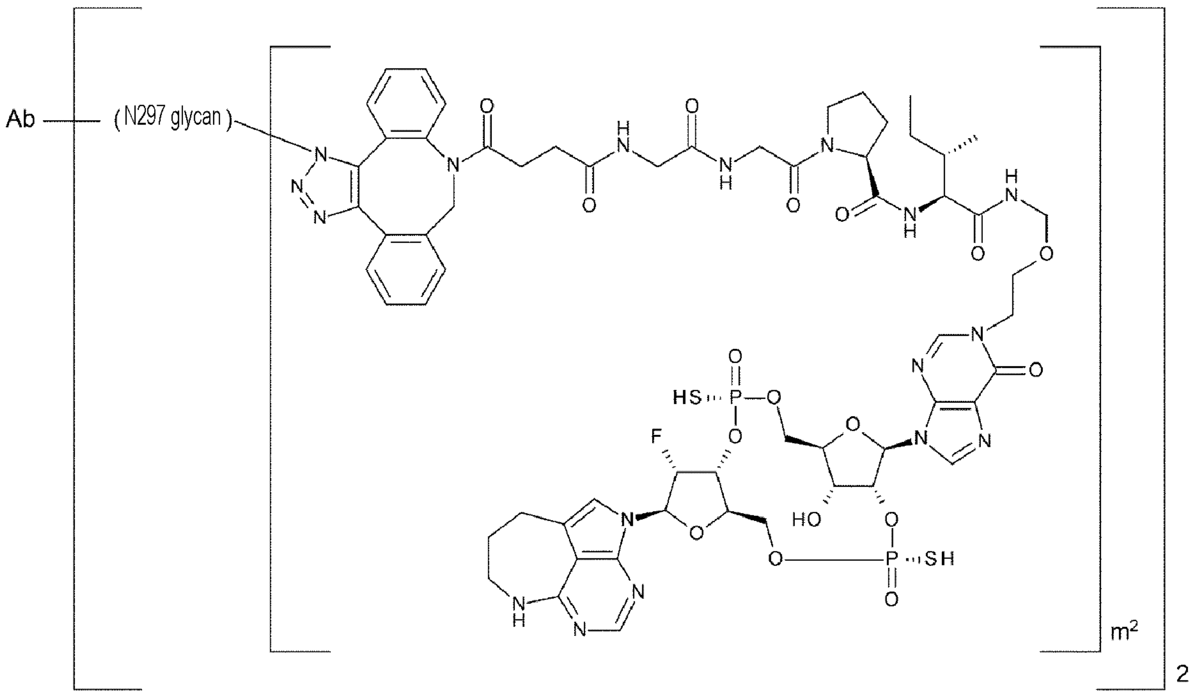

or

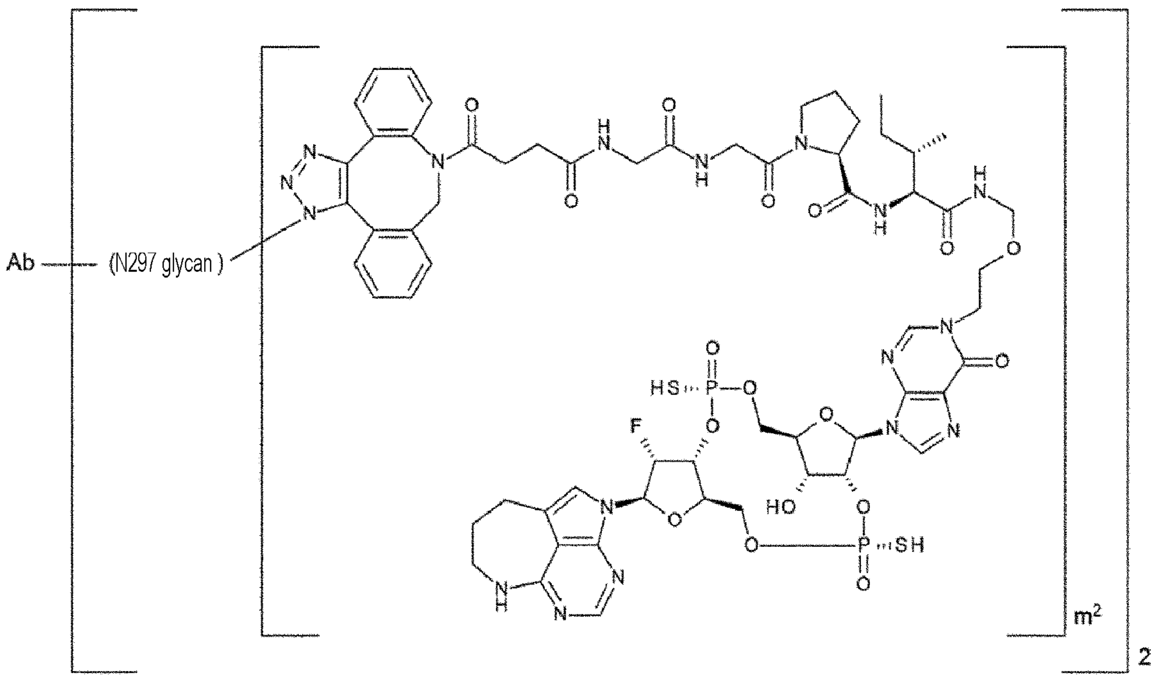

wherein, in each structural formula shown above, $m^2$ is an integer of 1 or 2,
Ab represents an antibody or a functional fragment of the antibody, wherein the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody,
N297 glycan is represented by any one of N297-(Fuc)MSG1 or N297-(Fuc)SG having a structure represented by the following formula:

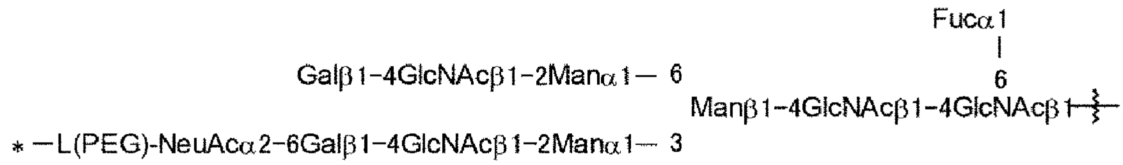

[N297-(Fuc)MSG1]

wherein the wavy line represents bonding to Asn297 of the antibody,
L (PEG) represents $-(CH_2-CH_2-O)n^5-CH_2-CH_2-NH-$,
wherein the amino group at the right end of the L(PEG) is bound via an amide bond to a carboxyl group at the 2-position of a sialic acid at the non-reducing terminal of a 1-3 branched chain of β-Man in the N297 glycan,
the asterisk represents bonding to a nitrogen atom at the 1- or 3-position of the 1,2,3-triazole ring of Lb in the linker L, and
$n^5$ represents an integer of 2 to 5;

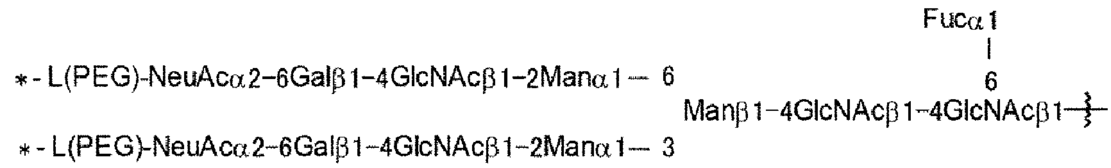

[N297-(Fuc)SG]

wherein the wavy line represents bonding to Asn297 of the antibody,
L(PEG) represents $-(CH_2-CH_2O)n^5-CH_2-CH_2-NH-$,
wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L, and
$n^5$ represents an integer of 2 to 5.

[25] The antibody-drug conjugate according to any one of [1] to [24], wherein the antibody is an anti-EGFR antibody.
[26] The antibody-drug conjugate according to any one of [1] to [24], wherein the antibody is an anti-CDH6 antibody.
[27] The antibody-drug conjugate according to [25], wherein the antibody is an antibody comprising a light chain consisting of an amino acid sequence set forth in SEQ ID NO: 30 and a heavy chain consisting of an amino acid sequence set forth in SEQ ID NO: 32, or an antibody comprising a light chain consisting of an amino acid sequence set forth in SEQ ID NO: 30 and a heavy chain consisting of an amino acid sequence set forth in SEQ ID NO: 33, or any one of these antibodies comprising a heavy chain in which one or several amino acid residues are deleted at the carboxyl terminus thereof.
[28] The antibody-drug conjugate according to [26], wherein the antibody is an antibody comprising a light chain consisting of an amino acid sequence set forth in SEQ ID NO: 44 and a heavy chain consisting of an amino acid sequence set forth in SEQ ID NO: 46, or any of these antibodies comprising a heavy chain in which one or several amino acid residues are deleted at the carboxyl terminus thereof.
[29] The antibody-drug conjugate according to [25], wherein the antibody is an antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, wherein the amino acid residues at positions 234, 235, and 265 (all according to EU numbering) of the Fc region of the antibody are Ala, Ala, and Gly, respectively.
[30] The antibody-drug conjugate according to [26], wherein the antibody is an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein the amino acid residues at positions 234, 235, and 265 (all according to EU numbering) of the Fc region of the antibody are Ala, Ala, and Gly, respectively.
[31] The antibody-drug conjugate according to [25], wherein the antibody is an antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO:

25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, wherein the amino acid residues at positions 234, 235, and 265 (all according to EU numbering) of the Fc region of the antibody are Ala, Ala, and Gly, respectively.

[32] The antibody-drug conjugate according to [26], wherein the antibody is an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein the amino acid residues at positions 234, 235, and 265 (all according to EU numbering) of the Fc region of the antibody are Ala, Ala, and Gly, respectively.

[33] An antibody-drug conjugate represented by the following formula:

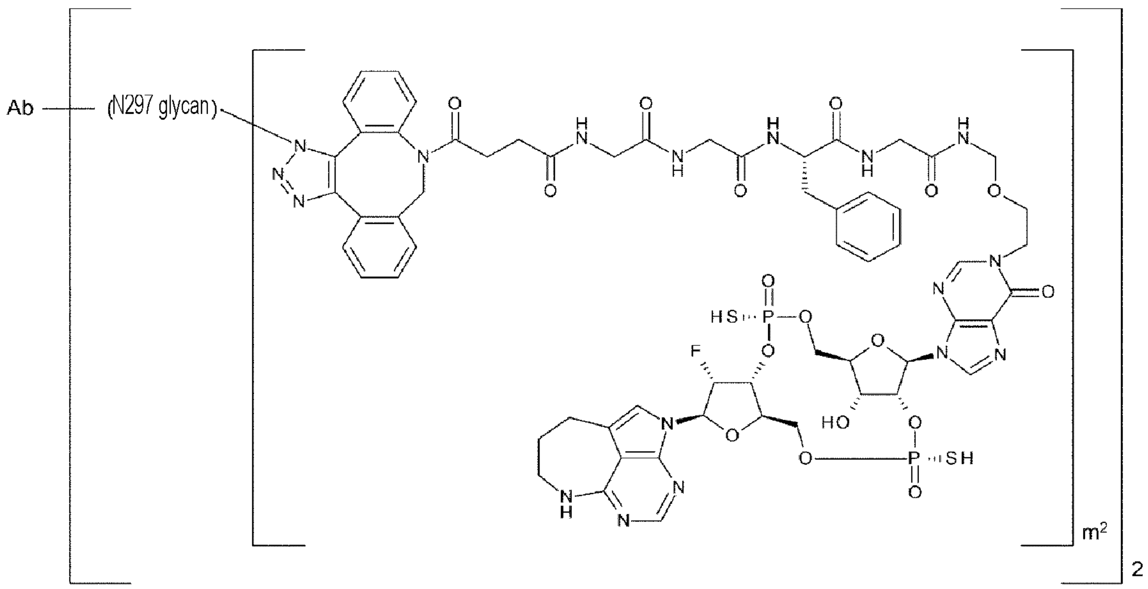

or

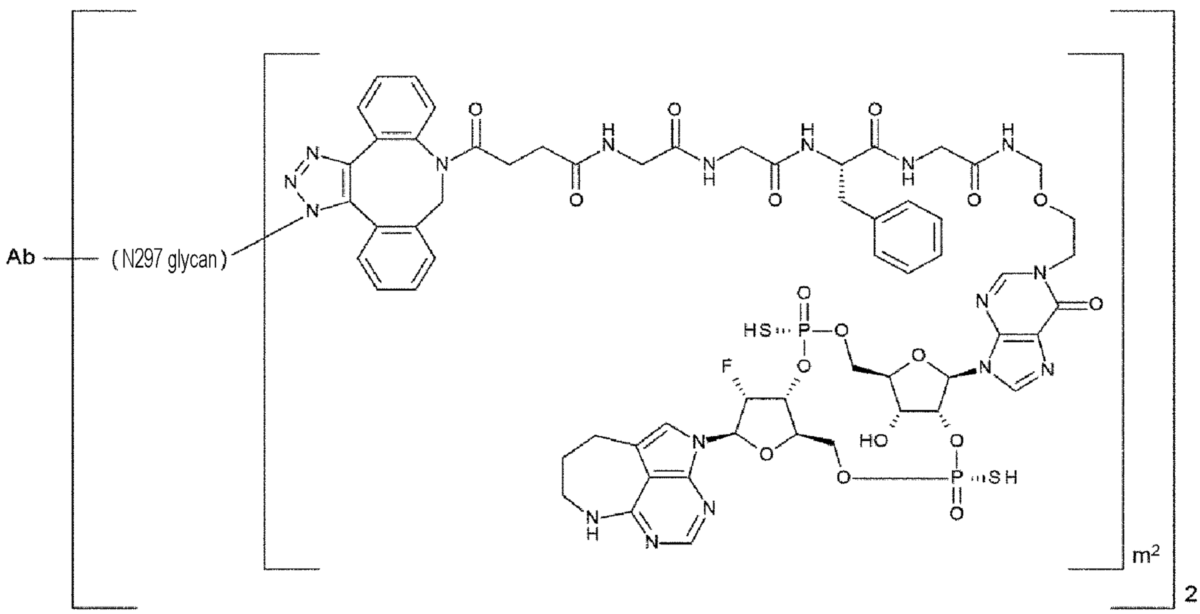

wherein Ab represents any one selected from the group consisting of the following:

an antibody comprising a light chain consisting of an amino acid sequence set forth in SEQ ID NO: 30 and a heavy

chain consisting of an amino acid sequence set forth in SEQ ID NO: 32,

an antibody comprising a light chain consisting of an amino acid sequence set forth in SEQ ID NO: 30 and a heavy chain consisting of an amino acid sequence set forth in SEQ ID NO: 33,

an antibody comprising a light chain consisting of an amino acid sequence set forth in SEQ ID NO: 44 and a heavy chain consisting of an amino acid sequence set forth in SEQ ID NO: 46, and

any one of these antibodies comprising a heavy chain in which one or several amino acid residues are deleted at the carboxyl terminus thereof,

wherein N297 glycan is represented by the following formula:

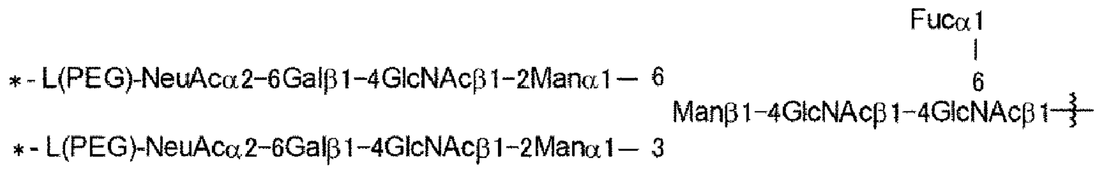

[N297-(Fuc)SG]

wherein the wavy line represents bonding to Asn297 of the antibody,

L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan

the asterisk represents bonding to a nitrogen atom at the 1- or 3-position of the 1,2,3-triazole ring of Lb in the linker L,

$n^5$ is 3, and

$m^2$ is 2.

[34] An antibody-drug conjugate represented by the following formula:

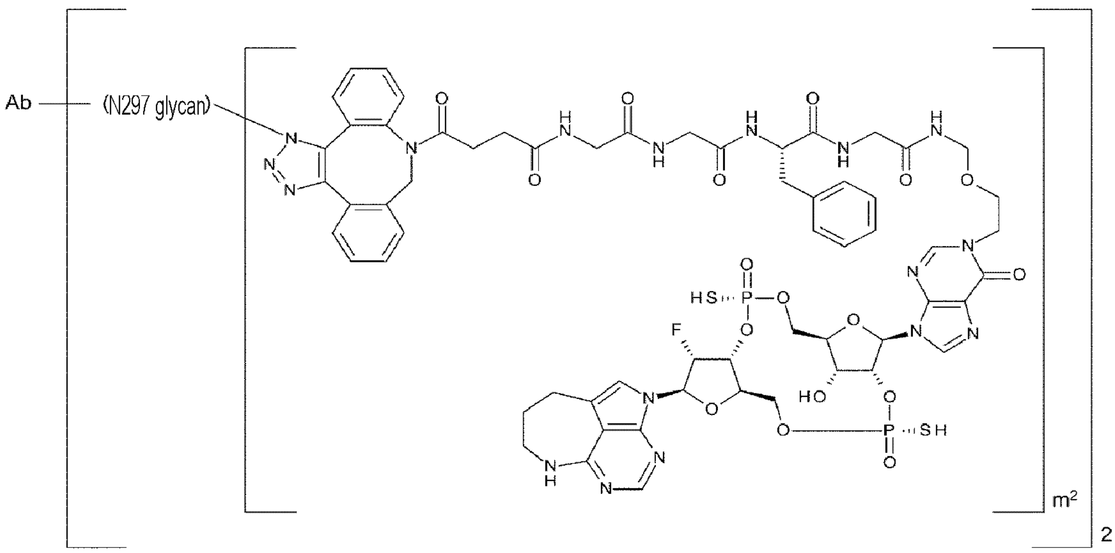

or

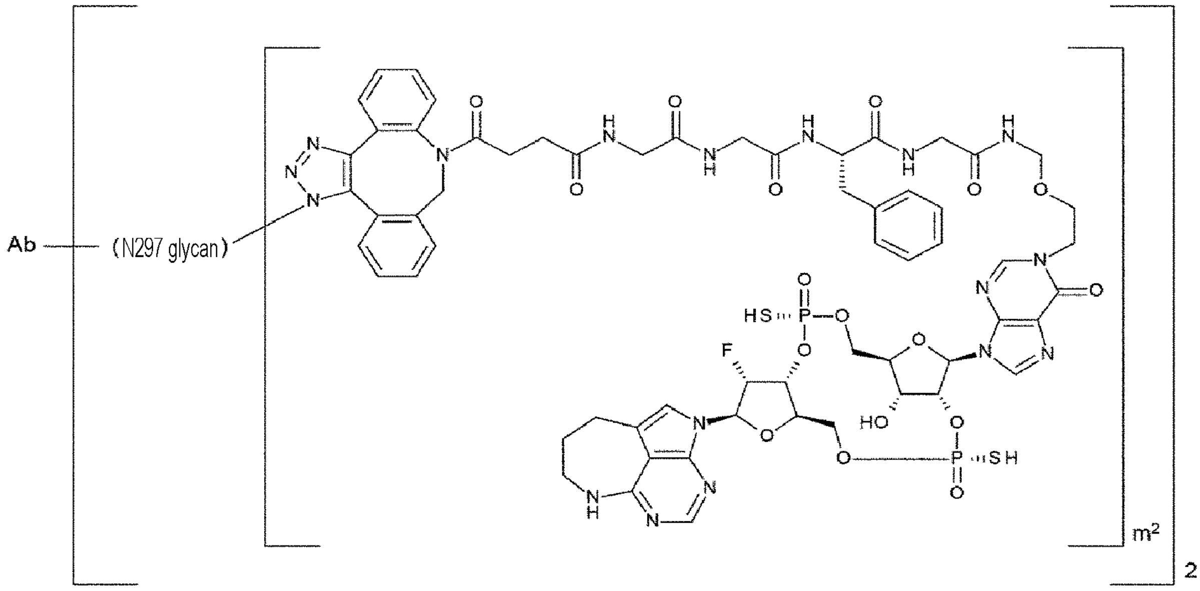

wherein Ab represents any one selected from the group consisting of the following:

an antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, and

an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein

the amino acid residues at positions 234, 235, and 265 (all according to EU numbering) of the Fc region of the antibody are Ala, Ala, and Gly, respectively,

wherein N297 glycan is represented by the following formula:

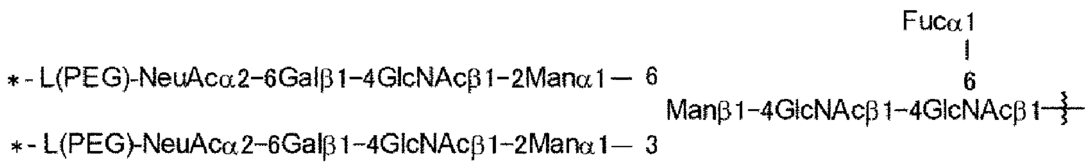

[N297-(Fuc)SG]

wherein the wavy line represents bonding to Asn297 of the antibody,

L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,

the asterisk represents bonding to a nitrogen atom at the 1- or 3-position of the 1,2,3-triazole ring of Lb in the linker L,

$n^5$ is 3, and

$m^2$ is 2.

[35] An antibody-drug conjugate represented by the following formula:

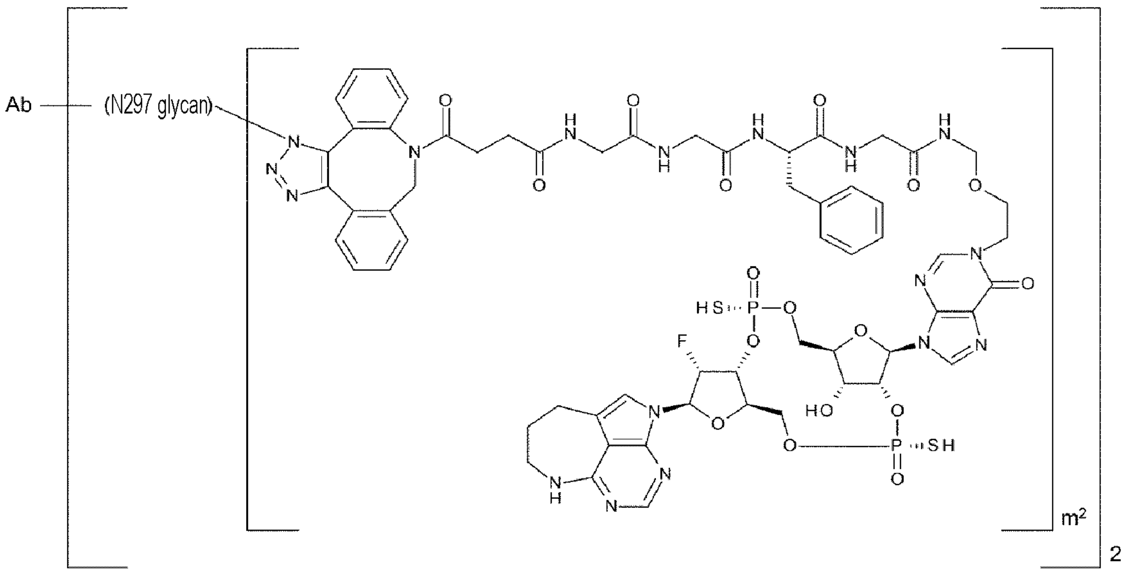

or

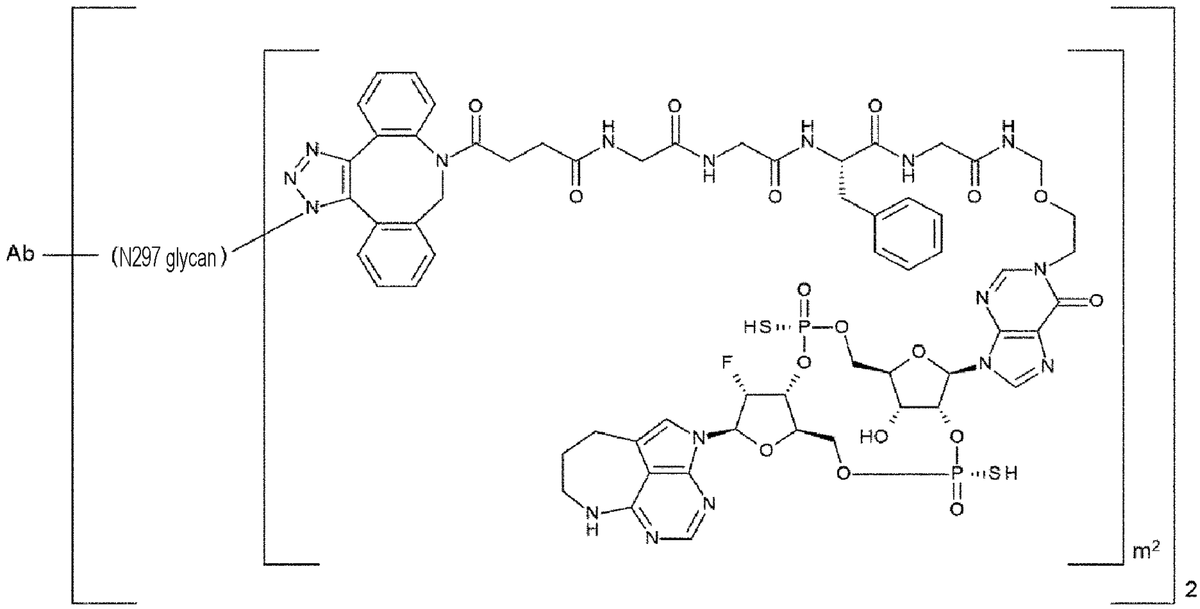

wherein Ab represents any one selected from the group consisting of the following:

an antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, and
an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein
the amino acid residues at positions 234, 235, and 265 (all according to EU numbering) of the Fc region of the antibody are Ala, Ala, and Gly, respectively,
wherein N297 glycan is represented by the following formula:

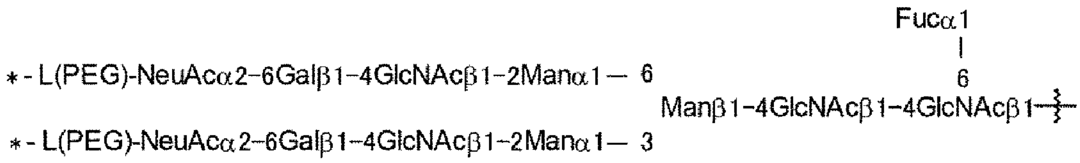

[N297-(Fuc)SG]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L,
$n^5$ is 3, and
$m^2$ is 2.

[36] An antibody-drug conjugate represented by the following formula:

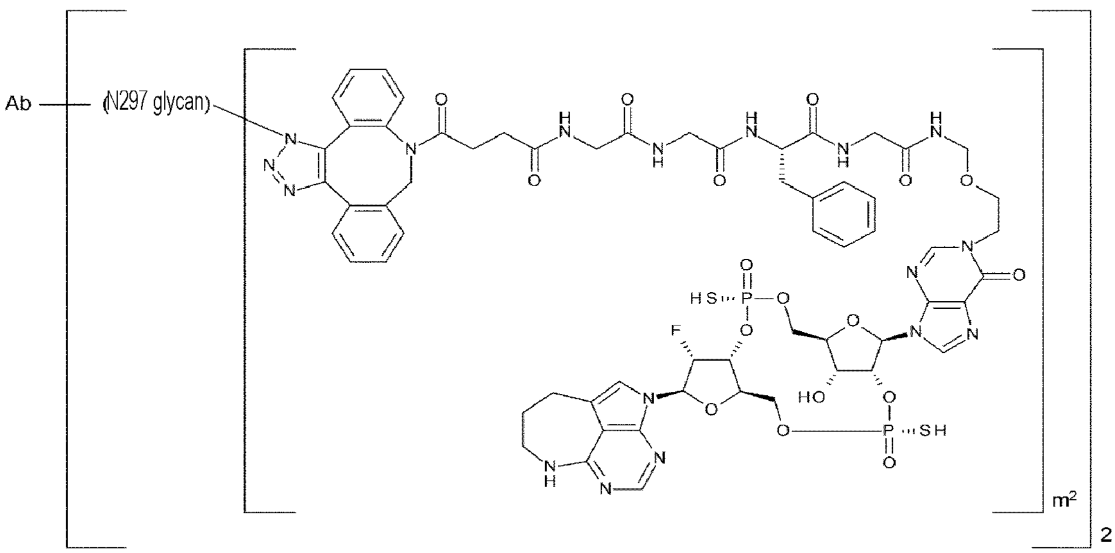

or

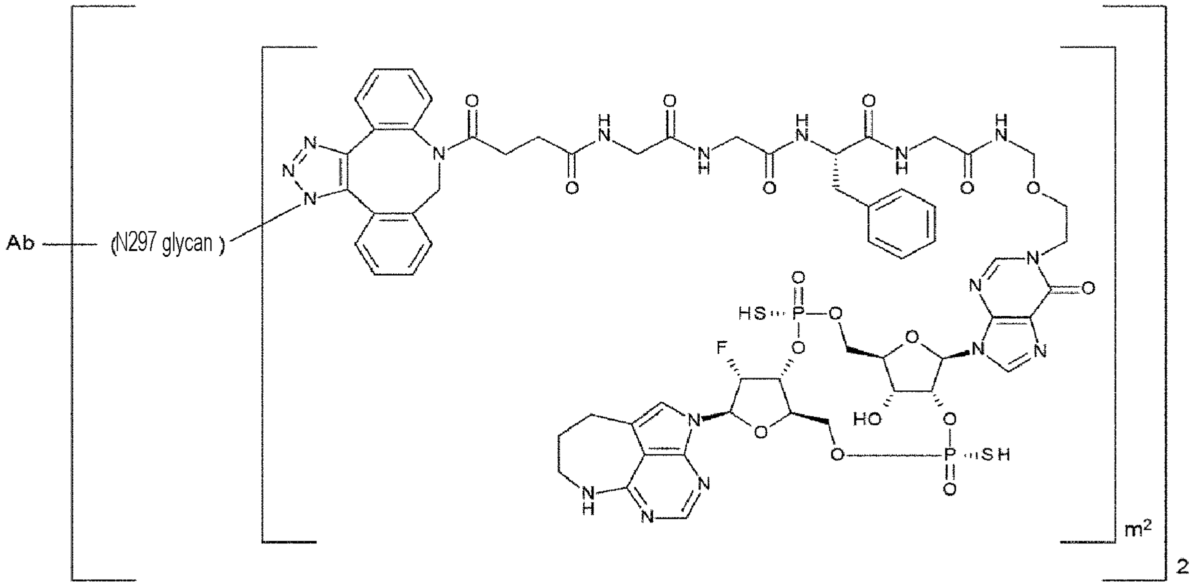

wherein Ab represents any one selected from the group consisting of the following:

an antibody comprising a light chain consisting of an amino acid sequence set forth in SEQ ID NO: 30 and a heavy chain consisting of an amino acid sequence set forth in SEQ ID NO: 32,
an antibody comprising a light chain consisting of an amino acid sequence set forth in SEQ ID NO: 30 and a heavy chain consisting of an amino acid sequence set forth in SEQ ID NO: 33,
an antibody comprising a light chain consisting of an amino acid sequence set forth in SEQ ID NO: 44 and a heavy chain consisting of an amino acid sequence set forth in SEQ ID NO: 46, and
any one of these antibodies comprising a heavy chain in which one or several amino acid residues are deleted at the carboxyl terminus thereof,
wherein N297 glycan is represented by the following formula:

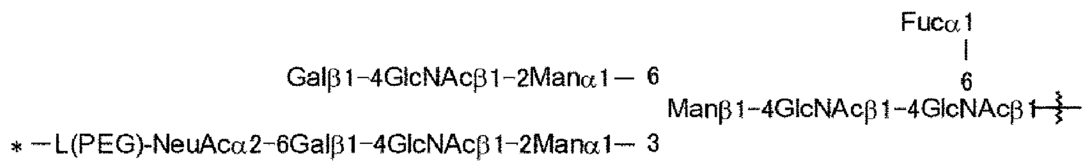

[N297-(Fuc)MSG1]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L,
$n^5$ is 3, and
$m^2$ is 1.

[37] An antibody-drug conjugate represented by the following formula:

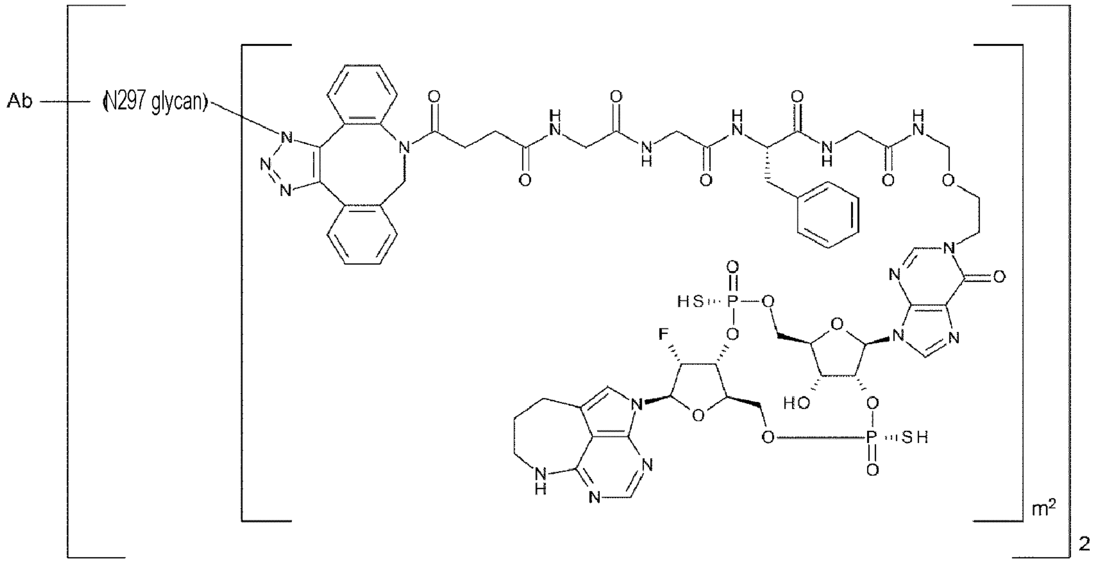

or

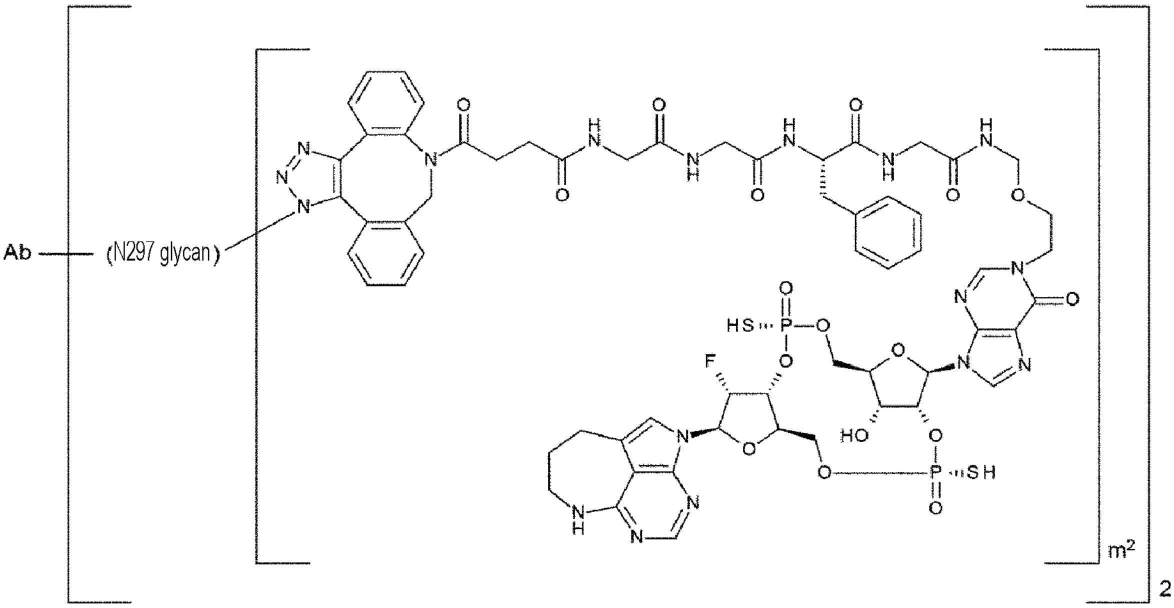

wherein Ab represents any one selected from the group consisting of the following:

an antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, and

an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein

the amino acid residues at positions 234, 235, and 265 (all according to EU numbering) of the Fc region of the antibody are Ala, Ala, and Gly, respectively, wherein

N297 glycan is represented by the following formula:

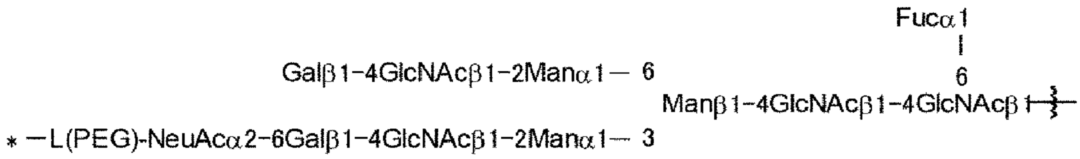

[N297-(Fuc)MSG1]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L,
$n^5$ is 3, and
$m^2$ is 1.

[38] An antibody-drug conjugate represented by the following formula:

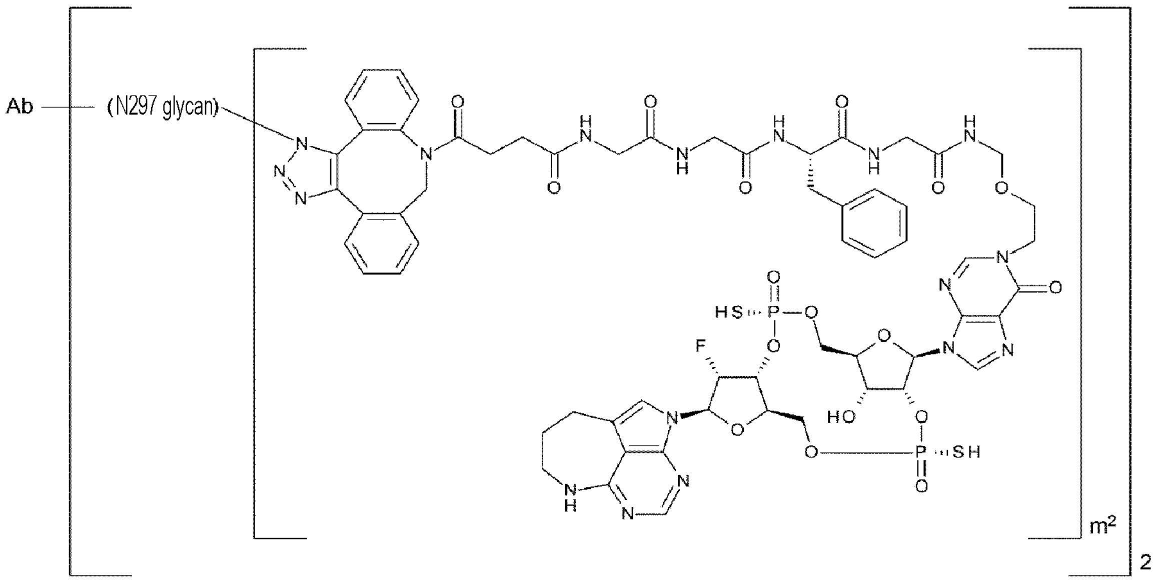

or

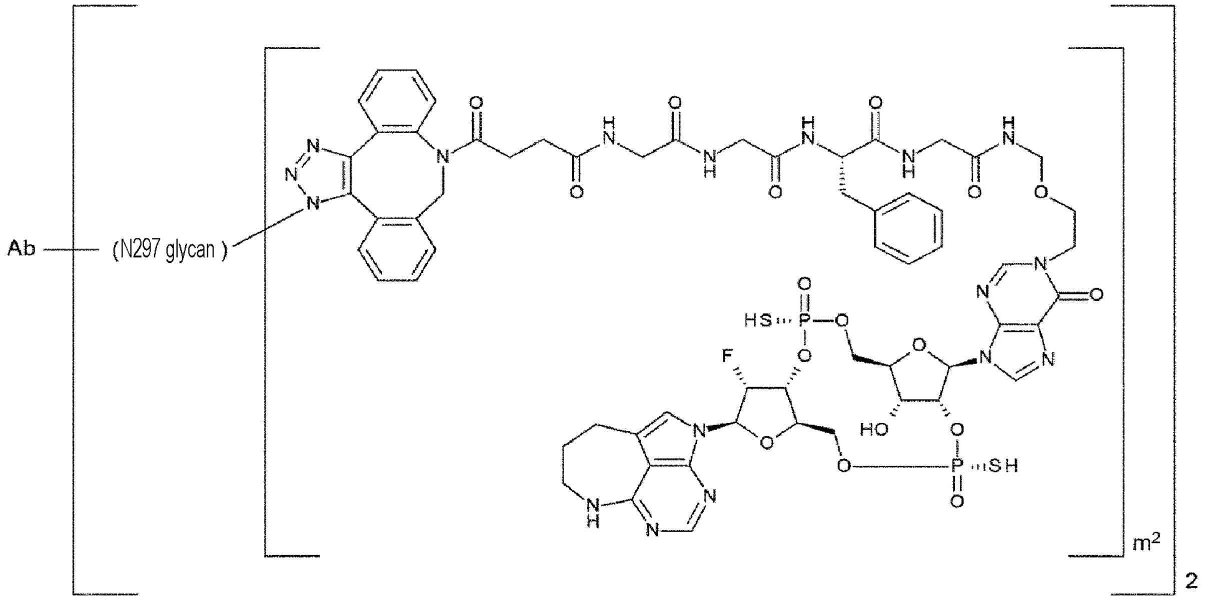

wherein Ab represents any one selected from the group consisting of the following:

an antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, and
an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein
the amino acid residues at positions 234, 235, and 265 (all according to EU numbering) of the Fc region of the antibody are Ala, Ala, and Gly, respectively,
wherein N297 glycan is represented by the following formula:

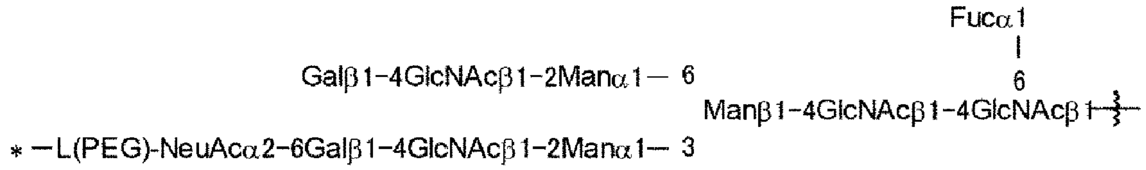

[N297-(Fuc)MSG1]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L,
$n^5$ is 3, and
$m^2$ is 1.

[39] A STING agonist comprising the antibody-drug conjugate according to any one of [1] to [38].
[40] A pharmaceutical composition comprising the antibody-drug conjugate according to any one of [1] to [38].
[41] An anti-tumor agent comprising the antibody-drug conjugate according to any one of [1] to [38].
[42] The anti-tumor agent according to [41], wherein the tumor is lung cancer, kidney cancer, urothelial cancer,

colorectal cancer, prostate cancer, glioblastoma multiforme, ovarian cancer, pancreatic cancer, breast cancer, melanoma, liver cancer, hepatocellular carcinoma, bladder cancer, gastric cancer, esophageal cancer, endometrial cancer, testicular cancer, cervical cancer, placental choriocarcinoma, brain tumor, head and neck cancer, thyroid cancer, mesothelioma, gastrointestinal stromal tumor (GIST), gallbladder cancer, bile duct cancer, adrenal cancer, squamous-cell carcinoma, pharyngeal cancer, tongue cancer, auditory organ cancer, thymus cancer, small intestine cancer, leukemia, malignant lymphoma, plasmacytoma, myeloma, sarcoma, EGFR mutation-positive cancer, or cancer having a mutation in an EGFR signaling molecule.

[42-2] The anti-tumor agent according to [41], wherein the tumor is a CDH6-positive cancer.
[42-3] The anti-tumor agent according to [41] or [42-2], wherein the tumor is ovarian cancer or kidney cancer.

[43] A method for treating cancer, comprising administering any one selected from the group consisting of the antibody-drug conjugate according to any one of [1] to [38], the STING agonist according to [39], the pharmaceutical composition according to [40], and the anti-tumor agent according to [41], [42], [42-2] or [42-3].
[44] The method according to [43], wherein the cancer is lung cancer, kidney cancer, urothelial cancer, colorectal cancer, prostate cancer, glioblastoma multiforme, ovarian cancer, pancreatic cancer, breast cancer, melanoma, liver cancer, hepatocellular carcinoma, bladder cancer, gastric cancer, esophageal cancer, endometrial cancer, testicular cancer, cervical cancer, placental choriocarcinoma, brain tumor, head and neck cancer, thyroid cancer, mesothelioma, gastrointestinal stromal tumor (GIST), gallbladder cancer, bile duct cancer, adrenal cancer, squamous-cell carcinoma, pharyngeal cancer, tongue cancer, auditory organ cancer, thymus cancer, small intestine cancer, leukemia, malignant lymphoma, plasmacytoma, myeloma, sarcoma, EGFR mutation-positive cancer, or cancer associated with a mutation in EGFR downstream signaling.

[44-2]
The method according to [43], wherein the cancer is a CDH6-positive cancer.
[44-3] The method according to [43] or [44-2], wherein the cancer is ovarian cancer or kidney cancer.

[45] (A) An antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, wherein the amino acid residues at positions 234, 235, and 265 (all according to EU numbering) of the Fc region of the antibody are Ala, Ala, and Gly, respectively; or
(B) an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein the amino acid residues at positions 234, 235, and 265 (all according to EU numbering) of the Fc region of the antibody are Ala, Ala, and Gly, respectively.
[45-2]

(A) An antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, wherein the amino acid residues at positions 234, 235, and 265 (all according to EU numbering) of the Fc region of the antibody are Ala, Ala, and Gly, respectively, and the antibody has binding activity against EGFR; or
(B) an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein the amino acid residues at positions 234, 235, and 265 (all according to EU numbering) of the Fc region of the antibody are Ala, Ala, and Gly, respectively, and the antibody has binding activity against CDH6.

[46] (A) An antibody comprising a light chain comprising a light chain variable region consisting of an amino acid sequence set forth in SEQ ID NO: 29 and a heavy chain comprising a heavy chain variable region consisting of an

amino acid sequence set forth in SEQ ID NO: 31, wherein the amino acid residues at positions 234, 235, and 265 (all according to EU numbering) of the Fc region of the antibody are Ala, Ala, and Gly, respectively; or
(B) an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein the amino acid residues at positions 234, 235, and 265 (all according to EU numbering) of the Fc region of the antibody are Ala, Ala, and Gly, respectively.
[46-2]

(A) An antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, wherein the amino acid residues at positions 234, 235, and 265 (all according to EU numbering) of the Fc region of the antibody are Ala, Ala, and Gly, respectively, and the antibody has binding activity against EGFR; or
(B) an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein the amino acid residues at positions 234, 235, and 265 (all according to EU numbering) of the Fc region of the antibody are Ala, Ala, and Gly, respectively, and the antibody has binding activity against CDH6.

[47] (A) An antibody comprising a light chain consisting of an amino acid sequence set forth in SEQ ID NO: 30 and a heavy chain consisting of an amino acid sequence set forth in SEQ ID NO: 32, or an antibody comprising a light chain consisting of an amino acid sequence set forth in SEQ ID NO: 30 and a heavy chain consisting of an amino acid sequence set forth in SEQ ID NO: 33;

(B) an antibody comprising a light chain consisting of an amino acid sequence set forth in SEQ ID NO: 44 and a heavy chain consisting of an amino acid sequence set forth in SEQ ID NO: 46; or
(C) an antibody comprising a heavy chain in which one or several amino acid residues are deleted at a carboxyl terminus in either one of the antibodies (A) and (B).

[47-2]

(A) An antibody comprising a light chain consisting of an amino acid sequence set forth in SEQ ID NO: 30 and a heavy chain consisting of an amino acid sequence set forth in SEQ ID NO: 32, or an antibody comprising a light chain consisting of an amino acid sequence set forth in SEQ ID NO: 30 and a heavy chain consisting of an amino acid sequence set forth in SEQ ID NO: 33, and having binding activity against EGFR;
(B) an antibody comprising a light chain consisting of an amino acid sequence set forth in SEQ ID NO: 44 and a heavy chain consisting of an amino acid sequence set forth in SEQ ID NO: 46, and having binding activity against CDH6; or
(C) an antibody comprising a heavy chain in which one or several amino acid residues are deleted at a carboxyl terminus in either one of the antibodies (A) and (B).

[48] The pharmaceutical composition according to [40] or the anti-tumor agent according to [41], [42], [42-2], or [42-3], wherein the pharmaceutical composition or the anti-tumor agent is administered in combination with an additional medicine.
[49] The pharmaceutical composition according to [40] or the anti-tumor agent according to [41], [42], [42-2], or [42-3], wherein the pharmaceutical composition or the anti-tumor agent comprises an additional medicine.
[50] The method according to [43], [44], [44-2], or [44-3], wherein any one selected from the group consisting of the antibody-drug conjugate according to any one of [1] to [38], the STING agonist according to [39], the pharmaceutical composition according to [40], and the anti-tumor agent according to [41], [42], [42-2], or [42-3] is administered in combination with an additional medicine.
[51] A polynucleotide encoding an amino acid sequence of the antibody according to any one of [45], [45-2], [46], [46-2], [47], and [47-2].
[52] An expression vector comprising the polynucleotide according to [51].
[53] A host cell transformed with the expression vector according to [52].
[54] A culture method comprising culturing the host cell according to [53].
[55] A method for producing an antibody, the method comprising the steps of culturing the host cell according to [53], and \collecting the antibody of interest from the resulting culture obtained in the culture step.
[56] A method for producing an Fc-containing molecule having an N297-linked glycan including a glycan donor

molecule-derived glycan, the method comprising the following step 1:
(Step 1) obtaining a reaction mixture by reacting an acceptor molecule which is an Fc-containing molecule having optionally fucosylated core GlcNAc as the N297-linked glycan with a glycan donor molecule comprising a glycan comprising GlcNAc with an unactivated reducing terminal in the presence of

- endo-β-N-acetylglucosaminidase whose substrate is the N297-linked glycan of the Fc-containing molecule (enzyme-A);
- endo-β-N-acetylglucosaminidase whose substrate is the glycan of the glycan donor molecule (enzyme-B); and
- an additive selected from a monovalent salt, an organic solvent, a surfactant, a saccharide, an amino acid, or a combination of any of them.

[57] The method according to [56], further comprising the following step **2:**
(Step 2) contacting the reaction mixture with a cation exchange chromatography medium or a multimode chromatography medium under acidic conditions, and collecting an Fc-containing molecule having a N297-linked glycan including a glycan donor molecule-derived glycan.
[58] The method according to [56] or [57], wherein the Fc-containing molecule is an antibody or a functional fragment of the antibody, wherein the amino acid residues at positions 234, 235, and 265 (all according to EU numbering) of the Fc region of the antibody or the functional fragment of the antibody are Ala, Ala, and Gly, respectively.
[59] The method according to any one of [56] to [58], wherein the additive is a monovalent salt.
[60] The method according to any one of [56] to [59], wherein the additive is a monovalent alkali metal salt.
[61] The method according to any one of [56] to [60], wherein the additive is sodium chloride.
[62] The method according to any one of [56] to [61], wherein the additive is 50 to 1000 mM of a monovalent salt.
[63] The method according to any one of [56] to [62], wherein the additive is 500 mM or less of sodium chloride.
[64] The method according to any one of [56] to [63], wherein a reaction time of step 1 is from 16 h to 24 h.
[65] The method according to any one of [56] to [64], wherein the Fc-containing molecule is an antibody, wherein the antibody comprises a light chain consisting of an amino acid sequence set forth in SEQ ID NO: 30 and a heavy chain consisting of an amino acid sequence set forth in SEQ ID NO: 33.
[66] The method according to any one of [56] to [64], wherein the Fc-containing molecule is an antibody, wherein the antibody comprises a light chain consisting of an amino acid sequence set forth in SEQ ID NO: 44 and a heavy chain consisting of an amino acid sequence set forth in SEQ ID NO: 46.

Advantageous Effects of Invention

The present invention provides a molecule having excellent safety while maintaining anti-tumor activity. The present invention also provides a novel antibody-drug conjugate that can be systemically administered and elicits anti-tumor effects in an antigen-expressing tumor. The present invention further provides a method for producing an improved Fc-containing molecule (particularly, an antibody) by use of a one-pot method.

BRIEF DESCRIPTION OF DRAWINGS

[Figure 1] Figure 1 is schematic diagrams of antibody-drug conjugates (the molecules of (II)) of the present invention, namely an antibody-drug conjugate (the molecule of (II) in Figure 1A) obtained from an SG-type glycan-remodeled antibody and an antibody-drug conjugate (a molecule (II) in Figure 1B) obtained from an MSG-type glycan-remodeled antibody. Here, (a) denotes a drug D, (b) denotes a linker L, (c) denotes a PEG linker (L(PEG)), and (d) denotes N297 glycan (where a white circle is NeuAc(Sia), a white hexagon is Man, a black hexagon is GlcNAc, a white diamond is Gal, and a white inverted triangle is Fuc), respectively. The white pentagon denotes a triazole ring formed by the reaction of the linker L-derived alkyne with the PEG linker-derived azide group. The Y-shaped diagram represents an antibody Ab. The PEG linker bonds via an amide bond to the carboxyl group at the 2-position of the sialic acid located at the non-reducing terminal. Unless otherwise stated, such a manner of illustration is applied throughout the specification.
[Figure 2] Figure 2 is schematic diagrams illustrating the structures of each production intermediate of an antibody-drug conjugate of the present invention, namely a (Fucα1,6)GlcNAc-antibody (the molecule of (III) in Figure 2A), a SG-type glycan-remodeled antibody (the molecule of (IV) in Figure 2B), and an MSG-type glycan-remodeled antibody (the molecule of (IV) in Figure 2C). In all of the diagrams, the Y-shaped diagram represents antibody Ab as in Figure 1. In Figure 2A, (e) denotes a N297 glycan consisting of a disaccharide in which GlcNAc at the 6-position is connected to the 1-position of Fuc via an α-glycoside bond. In Figures 2B and 2C, (d) denotes the same N297 glycan as in Figure 1, and

(f) denotes a PEG linker having an azide group at the end, wherein the azide group is for bonding to linker L. The mode of bonding of the PEG linker having an azide group is the same as the PEG linker in Figure 1.

[Figure 3] Figure 3 is schematic diagrams for the step of producing an SG-type glycan-remodeled antibody or an MSG-type glycan-remodeled antibody from an antibody produced in animal cells. Molecules (III) and (IV) in the Figure represent, as in Figure 2, a (Fucα1,6)GlcNAc-antibody and a SG-type glycan-remodeled antibody or an MSG-type glycan-remodeled antibody, respectively. Molecule (V) is an antibody produced in animal cells, and is a mixture of molecules with heterogeneous N297 glycans. Figure 3A illustrates the step of producing homogeneous (Fucα1,6) GlcNAc-antibody (III) by treating heterogeneous N297 glycans of (V) with hydrolase such as EndoS. Figure 3B illustrates the step of producing the SG-type glycan-remodeled antibody of (IV) by subjecting GlcNAc of the N297 glycan in antibody (III) to transglycosylation with an SG-type glycan donor molecule with use of glycosyltransferase such as an EndoS D233Q/Q303L mutant. Figure 3C illustrates the step, as in Figure 3B, of producing the MSG-type glycan-remodeled antibody of (IV) by subjecting antibody (III) to transglycosylation with an MSG-type glycan donor molecule. Each of the SG- and MSG-type glycan donor molecules used here has a sialic acid at each non-reducing terminal modified with a PEG linker having an azide group. Thus, the resulting SG- and MSG-type N-297 glycan-remodeled antibodies also have a sialic acid at the non-reducing terminal modified in the same manner as described for Figures 2B and 2C.

[Figure 4] Figure 4 shows the anti-tumor effects of intravenously administered anti-EGFR antibody 1-CDN conjugate 1 and anti-EGFR antibody 3-CDN conjugate **1.** The average number of conjugated drugs is about **4.** In the graph, the line (black squares) denotes the vehicle group; the line (black triangles) denotes the anti-EGFR antibody 3-CDN conjugate 1 administration group; and the line (black circles) denotes the anti-EGFR antibody 1-CDN conjugate 1 administration group. The vertical axis represents the tumor volume ($mm^3$) and the horizontal axis represents the number of days after tumor implantation.

[Figure 5] Figure 5 shows the anti-tumor effects of intravenously administered anti-EGFR antibody 2-CDN conjugate 1. The average number of conjugated drugs is about 4. In the graph, the line (black squares) denotes the vehicle group; and the line (black circles) denotes the anti-EGFR antibody 2-CDN conjugate 1 administration group. The vertical axis represents the tumor volume ($mm^3$) and the horizontal axis represents the number of days after tumor implantation.

[Figure 6] Figure 6 shows the anti-tumor effects of intravenously administered anti-EGFR antibody 1-CDN conjugate 2 and anti-EGFR antibody 3-CDN conjugate 2. The average number of conjugated drugs is about 2. In the graph, the line (black squares) denotes the vehicle group; the line (black triangles) denotes the anti-EGFR antibody 3-CDN conjugate 2 administration group; and the line (black circles) denotes the anti-EGFR antibody 1-CDN conjugate 2 administration group. The vertical axis represents the tumor volume ($mm^3$) and the horizontal axis represents the number of days after tumor implantation.

[Figure 7] Figure 7 shows the anti-tumor effects of intravenously administered anti-EGFR antibody 2-CDN conjugate 2. The average number of conjugated drugs is about 2. In the graph, the line (black squares) denotes the vehicle group; and the line (black circles) denotes the anti-EGFR antibody 2-CDN conjugate 2 administration group. The vertical axis represents the tumor volume ($mm^3$) and the horizontal axis represents the number of days after tumor implantation.

[Figure 8] Figure 8 shows the anti-tumor effects of intravenously administered anti-EGFR antibody 2-CDN conjugate 1. The average number of conjugated drugs is about 4. In the graph, the line (black squares) denotes the vehicle group; and the line (black circles) denotes the anti-EGFR antibody 2-CDN conjugate 1 administration group. The vertical axis represents the tumor volume ($mm^3$) and the horizontal axis represents the number of days after tumor implantation.

[Figure 9] Figure 9 shows the anti-tumor effects of intravenously administered anti-EGFR antibody 2-CDN conjugate 1. The average number of conjugated drugs is about 4. In the graph, the line (black squares) denotes the vehicle group; and the line (black circles) denotes the anti-EGFR antibody 2-CDN conjugate 1 administration group. The vertical axis represents the tumor volume ($mm^3$) and the horizontal axis represents the number of days after tumor implantation.

[Figure 10] Figure 10 shows the anti-tumor effects of intravenously administered anti-CDH6 antibody 1-CDN conjugate 1 and anti-CDH6 antibody 1-CDN conjugate 2. In the graph, the line (black squares) denotes the vehicle group; the line (white circles) denotes the anti-CDH6 antibody 1-CDN conjugate 1 administration group; and the line (inverted white triangles) denotes the anti-CDH6 antibody 1-CDN conjugate 2 administration group. The vertical axis represents the tumor volume ($mm^3$) and the horizontal axis represents the number of days after tumor implantation.

[Figure 11] Figure 11 shows the activity of anti-EGFR antibody 1-CDN conjugate 1, anti-EGFR antibody 3-CDN conjugate 1, anti-EGFR antibody 1-CDN conjugate 2, and anti-EGFR antibody 3-CDN conjugate 2 against reporter cells. In the graph, the line (black triangles) denotes the anti-EGFR antibody 3-CDN conjugate treated group; the line (black circles) denotes the anti-EGFR antibody 1-CDN conjugate treated group; the broken line denotes CDN conjugate 2 with an average drug conjugation number of about 2; and the solid line denotes CDN conjugate 1 with an

average drug conjugation number of about 4. The vertical axis represents the count value and the horizontal axis represents the concentration.

[Figure 12] Figure 12 shows the activity of anti-CDH6 antibody 1-CDN conjugate 1 and anti-CDH6 antibody 1-CDN conjugate 2 against reporter cells. In the graph, the line (black triangles) denotes the anti-CDH6 antibody 1-CDN conjugate treated group; the broken line denotes CDN conjugate 2 with an average drug conjugation number of about **2;** and the solid line denotes CDN conjugate 1 with an average drug conjugation number of about **4.** The vertical axis represents the count value and the horizontal axis represents the concentration.

[Figure 13] Figure 13 shows the anti-tumor effects of anti-EGFR antibody 1-CDN conjugate 1 in a tumor rechallenge test. The vertical axis represents the tumor volume ($mm^3$).

[Figure 14] Figure 14 shows the anti-tumor effects of anti-EGFR antibody 1-CDN conjugate 2 in a tumor rechallenge test. The vertical axis represents the tumor volume ($mm^3$).

[Figure 15A] Figure 15A shows the cytokine production of each antibody-CDN conjugate in a human whole blood test. The vertical axis represents the amount of the cytokine and the horizontal axis represents the Fc region of each antibody-CDN conjugate. The amount of the cytokine was calculated as a numeric value using the standard line of Log Scale (Logarithmic Scale).

[Figure 15B] Figure 15B shows the cytokine production of each antibody-CDN conjugate in a human whole blood test. The vertical axis represents the amount of the cytokine and the horizontal axis represents the Fc region of each antibody-CDN conjugate. The amount of the cytokine was calculated as a numeric value using the standard line of Linear Scale.

[Figure 16A] Figure 16A is a graph in which an anti-EGFR antibody (IgG2) (anti-EGFR antibody B), which is an IgG2-type antibody, an Fc substitution variant thereof (IgG1 WT) (anti-EGFR antibody A) obtained by replacing the Fc region with IgG1 type, a mutant (modified anti-EGFR antibody 3) obtained by introducing L234A/L235A (LALA) mutation into the Fc region of IgG1 WT, and a mutant (modified anti-EGFR antibody 1) obtained by introducing L234A/L235A/D265G (LALA-DG) mutation thereto were evaluated for their binding activity against human FcγRI by SPR. The horizontal axis represents the antibody concentration and the vertical axis represents the RU (Response Unit) value obtained at the antibody concentration.

[Figure 16B] Figure 16B is a graph in which the antibody groups used in Figure 16A were evaluated for their binding activity against human FcγRIIa by SPR. The horizontal axis represents the antibody concentration and the vertical axis represents the RU value obtained at the antibody concentration.

[Figure 16C] Figure 16C is a graph in which the antibody group used in Figure 16A were evaluated for their binding activity against human FcγRIIb/c by SPR. The horizontal axis represents the antibody concentration and the vertical axis represents the RU value obtained at the antibody concentration.

[Figure 16D] Figure 16D is a graph in which the antibody group used in Figure 16A were evaluated for their binding activity against human FcγRIIIa by SPR. The horizontal axis represents the antibody concentration and the vertical axis represents the RU value obtained at the antibody concentration.

[Figure 16E] Figure 16E is a graph in which the antibody group used in Figure 16A were evaluated for their binding activity against human FcγRIIIb by SPR. The horizontal axis represents the antibody concentration and the vertical axis represents the RU value obtained at the antibody concentration.

[Figure 17A] Figure 17A is a graph showing the RU value obtained at an antibody concentration of 4.7 μM when the antibody group used in Figure 16A and antibody-drug conjugates obtained by conjugating the antibody group with a STING agonist at DAR2 or DAR4 (DAR2: anti-EGFR antibody B-CDN conjugate 2, anti-EGFR antibody 3-CDN conjugate 2, modified anti-EGFR antibody 1-CDN
conjugate 2, or DAR4: anti-EGFR antibody A-CDN conjugate 1, anti-EGFR antibody 3-CDN conjugate 1, modified anti-EGFR antibody 1-CDN conjugate 1) were evaluated for their binding activity against human FcγRI by SPR.

[Figure 17B] Figure 17B is a graph showing the RU value obtained at an antibody concentration of 4.7 μM when the antibody group used in Figure 17A were evaluated for their binding activity against human FcγRIIa by SPR.

[Figure 17C] Figure 17C is a graph showing the RU value obtained at an antibody concentration of 4.7 μM when the antibody group used in Figure 17A were evaluated for their binding activity against human FcγRIIb/c by SPR.

[Figure 17D] Figure 17D is a graph showing the RU value obtained at an antibody concentration of 4.7 μM when the antibody group used in Figure 17A were evaluated for their binding activity against human FcγRIIIa by SPR.

[Figure 17E] Figure 17E is a graph showing the RU value obtained at an antibody concentration of 4.7 μM when the antibody group used in Figure 17A were evaluated for their binding activity against human FcγRIIIb by SPR.

[Figure 18] Figure 18 is a table showing the $T_m$ values of anti-EGFR antibody A-CDN conjugate 1 (IgG1 WT), anti-EGFR antibody 3-CDN conjugate 1 (LALA), and modified anti-EGFR antibody 1-CDN conjugate 1 (LALA-DG). Also, the difference in $T_m$ value ($\Delta T_m$) between anti-EGFR antibody A-CDN conjugate 1 and each anti-EGFR antibody-CDN conjugate 1 is shown.

[Figure 19] Figure 19 shows (a)the amino acid sequence of human wild-type STING (SEQ ID NO: 1), (b) the amino acid sequence of human STING REF mutant (R232H) (SEQ ID NO: 3), and (c) the amino

acid sequence of human STING HAQ mutant (R71H, G230A, R293Q) (SEQ ID NO: 5).

[Figure 20] Figure 20 shows (a) the CDRL1 amino acid sequence (SEQ ID NO: 23) of anti-EGFR antibodies (modified anti-EGFR antibodies 1 to 3), (b) the CDRL2 amino acid sequence (SEQ ID NO: 24) of anti-EGFR antibodies (modified anti-EGFR antibodies 1 to 3), (c) the CDRL3 amino acid sequence (SEQ ID NO: 25) of anti-EGFR antibodies (modified anti-EGFR antibodies 1 to 3), (d) the CDRH1 amino acid sequence (SEQ ID NO: 26) of anti-EGFR antibodies (modified anti-EGFR antibodies 1 to 3), (e) the CDRH2 amino acid sequence (SEQ ID NO: 27) of anti-EGFR antibodies (modified anti-EGFR antibodies 1 to 3), and (f) the CDRH3 amino acid sequence (SEQ ID NO: 28) of anti-EGFR antibodies (modified anti-EGFR antibodies 1 to 3).

[Figure 21-1] Figure 21-1 shows (a) the light chain variable region amino acid sequence (SEQ ID NO: 29) of anti-EGFR antibodies (modified anti-EGFR antibodies 1 to 3), (b) the light chain amino acid sequence (SEQ ID NO: 30) of anti-EGFR antibodies (modified anti-EGFR antibodies 1 to 3), and (c) the heavy chain variable region amino acid sequence (SEQ ID NO: 31) of anti-EGFR antibodies (modified anti-EGFR antibodies 1 to 3).

[Figure 21-2] Figure 21-2 shows (d) the heavy chain amino acid sequence (SEQ ID NO: 32) of the LALA-DG mutant anti-EGFR antibody (modified anti-EGFR antibody 1), (e) the heavy chain amino acid sequence (SEQ ID NO: 33) of the LALA-DG mutant anti-EGFR antibody (modified anti-EGFR antibody 2), and (f) the heavy chain amino acid sequence (SEQ ID NO: 34) of anti-EGFR antibody A.

[Figure 21-3] Figure 21-3 shows (g) the heavy chain amino acid sequence (SEQ ID NO: 35) of anti-EGFR antibody B (Vectibix), and (h) the heavy chain amino acid sequence (SEQ ID NO: 36) of the LALA mutant anti-EGFR antibody (modified anti-EGFR antibody 3).

[Figure 22] Figure 22 shows (a) the CDRL1 amino acid sequence (SEQ ID NO: 37) of an anti-CDH6 antibody (modified anti-CDH6 antibody 1), (b) the CDRL2 amino acid sequence (SEQ ID NO: 38) of an anti-CDH6 antibody (modified anti-CDH6 antibody 1), (c) the CDRL3 amino acid sequence (SEQ ID NO: 39) of an anti-CDH6 antibody (modified anti-CDH6 antibody 1), (d) the CDRH1 amino acid sequence (SEQ ID NO: 40) of an anti-CDH6 antibody (modified anti-CDH6 antibody 1), (e) the CDRH2 amino acid sequence (SEQ ID NO: 41) of an anti-CDH6 antibody (modified anti-CDH6 antibody 1), and (f) the CDRH3 amino acid sequence (SEQ ID NO: 42) of an anti-CDH6 antibody (modified anti-CDH6 antibody 1).

[Figure 23] Figure 23 shows (a) the light chain variable region amino acid sequence (SEQ ID NO: 43) of an anti-CDH6 antibody (modified anti-CDH6 antibody 1), (b) the light chain amino acid sequence (SEQ ID NO: 44) of an anti-CDH6 antibody (modified anti-CDH6 antibody 1), (c) the heavy chain variable region amino acid sequence (SEQ ID NO: 45) of an anti-CDH6 antibody (modified anti-CDH6 antibody 1), and (d) the heavy chain amino acid sequence (SEQ ID NO: 46) of a LALA-DG mutant anti-CDH6 antibody (modified anti-CDH6 antibody 1).

[Figure 24] Figure 24 shows the anti-tumor effects of anti-CDH6 antibody 1-CDN conjugate 1 and anti-CDH6 antibody 1-CDN conjugate 2 in the CT26.WT-hCDH6 rechallenge of the left axillary region of a mouse with complete tumor regression after intravenous administration. The vertical axis represents the tumor volume ($mm^3$) .

[Figure 25] Figure 25 shows the anti-tumor effects of intravenously administered anti-CDH6 antibody 1-CDN conjugate 1 and anti-CDH6 antibody 1-CDN conjugate 2. The vertical axis represents the tumor volume ($mm^3$) and the horizontal axis represents the number of days after tumor implantation.

[Figure 26] Figure 26 shows the anti-tumor effects of intravenously administered anti-CDH6 antibody 1-CDN conjugate 1. The vertical axis represents the tumor volume ($mm^3$) and the horizontal axis represents the number of days after tumor implantation.

[Figure 27] Figure 27 shows the anti-tumor effects of intravenously administered anti-CDH6 antibody 1-CDN conjugate 2. The vertical axis represents the tumor volume ($mm^3$) and the horizontal axis represents the number of days after tumor implantation.

[Figure 28] Figure 28 shows the anti-tumor effects of intravenously administered anti-CDH6 antibody 1-CDN conjugate 1. The vertical axis represents the tumor volume ($mm^3$) and the horizontal axis represents the number of days after tumor implantation.

[Figure 29] Figure 29 shows the anti-tumor effects of intravenously administered anti-CDH6 antibody 1-CDN conjugate 2. The vertical axis represents the tumor volume ($mm^3$) and the horizontal axis represents the number of days after tumor implantation.

[Figure 30] Figure 30 shows the amino acid sequence (SEQ ID NO: 47) of wild-type Endo-S. An amino acid residue corresponding to the central site of catalytic activity of the enzyme is underlined in bold. Amino acid residues that can be expected to be able to control enzymatic activity by mutation are indicated in bold.

[Figure 31] Figure 31 shows the amino acid sequence (SEQ ID NO: 48) of wild-type Endo-S2. An amino acid residue corresponding to the central site of catalytic activity of the enzyme is underlined in bold. Amino acid residues that can be expected to be able to control enzymatic activity by mutation are indicated in bold.

[Figure 32] Figure 32 shows the amino acid sequence (SEQ ID NO: 49) of wild-type Endo-Si. An amino acid residue corresponding to the central site of catalytic activity of the enzyme is underlined in bold. Amino acid residues that can be expected to be able to control enzymatic activity by mutation are indicated in bold.

[Figure 33] Figure 33 shows the amino acid sequence (SEQ ID NO: 50) of wild-type Endo-M. An amino acid residue corresponding to the central site of catalytic activity of the enzyme is underlined in bold. Amino acid residues that can be expected to be able to control enzymatic activity by mutation are indicated in bold.

[Figure 34] Figure 34 shows the amino acid sequence (SEQ ID NO: 51) of wild-type Endo-Rp. An amino acid residue corresponding to the central site of catalytic activity of the enzyme is underlined in bold. Amino acid residues that can be expected to be able to control enzymatic activity by mutation are indicated in bold.

[Figure 35] Figure 35 shows the amino acid sequence (SEQ ID NO: 52) of wild-type Endo-CC. An amino acid residue corresponding to the central site of catalytic activity of the enzyme is underlined in bold. Amino acid residues that can be expected to be able to control enzymatic activity by mutation are indicated in bold.

[Figure 36] Figure 36 shows the amino acid sequence (SEQ ID NO: 53) of wild-type Endo-Om. An amino acid residue corresponding to the central site of catalytic activity of the enzyme is underlined in boldface. Amino acid residues that can be expected to be able to control enzymatic activity by mutation are indicated in boldface.

[Figure 37] Figure 37 shows the amino acid sequence (SEQ ID NO: 54) of wild-type Endo-Sd. An amino acid residue corresponding to the central site of catalytic activity of the enzyme is underlined in boldface. Amino acid residues that can be expected to be able to control enzymatic activity by mutation are indicated in boldface.

[Figure 38] Figure 38 shows the amino acid sequence (SEQ ID NO: 55) of wild-type Endo-Sz. An amino acid residue corresponding to the central site of catalytic activity of the enzyme is underlined in boldface. Amino acid residues that can be expected to be able to control enzymatic activity by mutation are indicated in boldface.

[Figure 39] Figure 39 shows the amino acid sequence (SEQ ID NO: 56) of wild-type Endo-Se. An amino acid residue corresponding to the central site of catalytic activity of the enzyme is underlined in boldface. Amino acid residues that can be expected to be able to control enzymatic activity by mutation are indicated in boldface.

DESCRIPTION OF EMBODIMENTS

In one aspect, the present invention relates to a molecule comprising a target molecule-binding moiety, a mutated Fc region, and an immunostimulator, wherein the mutated Fc region comprises at least three amino acid substitutions compared to a wild-type Fc region, and the molecule has decreased binding activity against human Fcγ receptor, has a decreased ability to produce inflammatory cytokines, and has equivalent or enhanced *in vivo* anti-tumor activity compared to a corresponding molecule having the wild-type Fc region.

Herein, "target molecule-binding moiety" means a moiety that binds to a given target molecule and preferably a target molecule involved in a human disease. Examples of the moiety can include antibodies, functional fragments of the antibodies, antigen-binding fragments of the antibodies, non-immunoglobulin proteins and fragments thereof, receptor proteins, ligand proteins, proteins (polypeptides) such as protein scaffolds, aptamers, nucleic acid molecules such as antisense, organic synthetic small molecule compounds, and naturally-occurring small molecule compounds, and can preferably include antibodies, functional fragments of the antibodies, and antigen-binding fragments of the antibodies, further preferably antibodies and functional fragments of the antibodies, and particularly preferably antibodies, though the moiety is not limited thereto. In one aspect of the present invention, in the case where "target molecule-binding moiety" includes an Fc region (for example, in the case where "target molecule-binding moiety" is an antibody), the Fc region included in the target molecule-binding moiety can be a mutated Fc region. In other words, in one aspect of the present invention, the mutated Fc region may be included in the target molecule-binding moiety.

"Antibody", "functional fragment of the antibody", and "antigen-binding fragment of the antibody" will be described later in detail.

In the present invention, "immunostimulation" means the induction of activation in any way of immune cells that participate in anti-tumor immunity, such as monocytes, macrophages, dendritic cells, T cells, B cells, NK cells, and neutrophils. For example, the term refers to inducing the production of cytokines and chemokines, increased expression of immune-activation markers, decreased expression of immune-suppression markers, changes in phosphorylation and the like of intracellular signaling pathways, changes in gene expression, and any other changes in the structure and function of immune cells. In addition, the term includes causing changes in tumor cells that induce anti-tumor immunity. For example, the term refers to inducing the production of cytokines and chemokines that stimulate immune cells or induce migration, or increased sensitivity to immune cells.

In the present invention, "immunostimulator" means any material that brings about the immunostimulation defined above. In one aspect of the present invention, the immunostimulator is preferably a STING agonist, a TLR1 to 10 agonist, a CIITA agonist, a NAIPs agonist, a NOD1 agonist, a NOD2 agonist, a NLRC3 agonist, a NLRP1 to 14 agonist, a DEC205 agonist, a MMR agonist, a Dectin1 agonist, a Dectin2 agonist, a Mincle agonist, a DC-SIGN agonist, a DNGR-1 agonist, a MBL agonist, an AIM2 agonist, a MDA-5 agonist, a RIG-I agonist, or an AhR agonist, more preferably a TLR3 agonist, a TLR7 agonist, a TLR8 agonist, a TLR9 agonist, a RIG-I agonist, or a STING agonist, and particularly preferably a STING agonist.

Herein, "Fc region" is a C-terminal region of an immunoglobulin heavy chain and means a region containing a

CH2 domain and a CH3 domain. In one aspect of the present invention, "Fc region" comprises at least a portion of a hinge domain. In another aspect of the present invention, "Fc region" comprises at least a portion of a hinge domain, two CH2 domains, and two CH3 domains. "Fc region" usually means a homo- or heterodimer or a multimer containing C-terminal regions of two or more immunoglobulin heavy chains bound through an S-S bond and may be a single chain Fc region (scFc) in which these form one chain.

In one aspect, the "mutated Fc region" of the present invention has at least three amino acid substitutions compared to a wild-type Fc region. In one aspect of the present invention, the "wild-type Fc region" and "mutated Fc region" can be derived from any organism species and are preferably derived from a human, a monkey (examples thereof can include, but are not limited to, cynomolgus monkeys, rhesus macaques, common marmosets, and common squirrel monkeys), a mouse, a rat, or a rabbit, more preferably derived from a human or a monkey, and particularly preferably derived from a human. In one aspect of the present invention, the "wild-type Fc region" and "mutated Fc region" can be derived from any immunoglobulin molecule of IgG, IgE, IgM, IgD, IgA, and IgY and are preferably derived from IgG. Their subclass may be any of IgG1, IgG2, IgG3, IgG4, IgA1, and IgA2, and preferably is IgG1, IgG2, or IgG4, more preferably is IgG1 or IgG4, and particularly preferably is IgG1. Thus, in one aspect of the present invention, the Fc region in the "wild-type Fc region" and "mutated Fc region" is a human IgG1 Fc region or a human IgG4 Fc region, and is preferably a human IgG1 Fc region.

As described above, in one aspect of the present invention, the mutated Fc region has at least three amino acid substitutions compared to a wild-type Fc region. The at least three amino acid substitutions may be present in any one or two or more of a hinge domain, a CH2 domain, and a CH3 domain in the mutated Fc region and are preferably present in a CH2 domain of the mutated Fc region. In one aspect of the present invention, the at least three amino acid substitutions are present at all three positions 234, 235, and 265 (all according to the EU numbering) of the mutated Fc region. In a further aspect of the present invention, the at least three amino acid substitutions preferably include 234Ala, 235Ala, and 265Gly (all according to EU numbering). In the present invention, such a mutation consisting of 234Ala, 235Ala, and 265Gly (all according to EU numbering) in the Fc region may be also referred to as "LALA-**DG",** "L234A/L235A/D265G", "-L234A, L235A, D265G", or the like.

In one aspect of the present invention, the molecule comprising a target molecule-binding moiety, a mutated Fc region, and an immunostimulator may comprise an additional Fc region, and the additional Fc region can be a mutated Fc region. In another aspect of the present invention, the "mutated Fc region" comprises two CH2 domains, one or both of which comprise the at least three amino acid substitutions.

In one aspect, the molecule comprising a target molecule-binding moiety of the present invention, a mutated Fc region, and an immunostimulator has decreased binding activity against human Fcγ receptor compared to a corresponding molecule having the wild-type Fc region. Herein, "corresponding molecule having the wild-type Fc region" means a molecule identical or equivalent to the molecule comprising a target molecule-binding moiety, a mutated Fc region, and an immunostimulator according to the present invention except that the molecule comprises the wild-type Fc region, not the mutated Fc region. Herein, "human Fcγ receptor" (also referred to as "human FcγR") means one or more receptors selected from the group consisting of FcγRI (CD64) (including isoforms FcγRIa, FcγRIb, and FcγRIc), FcγRII (CD32) (including isoforms FcγRIIa (allotypes H131 (H type) and R131 (R type)), FcγRIIb (including FcγRIIb-1 and FcγRIIb-2), and FcγRIIc), and FcγRIII (CD16) (including isoforms FcγRIIIa (including allotypes V158 and F158), FcγRIIIb (including allotypes FcγRIIIb-NA1 and FcγRIIIb-NA2), and FcγRIIIc). In one aspect of the present invention, "human Fcγ receptor" is at least 1, at least 2, at least 3, at least 4, at least 5, at least 6, at least 7, at least 8, or all of the 9 isoforms listed above, and is preferably one or more receptors selected from the group consisting of human FcγRI, human FcγRIIa, human FcγRIIb/c, human FcγRIIIa, and human FcγRIIIb, and further preferably human FcγRI. The binding activity against the analyte human Fcγ receptor can be determined using any known method and can be determined by, for example, surface plasmon resonance (SPR). In one aspect of the present invention, the binding activity against the analyte human Fcγ receptor can be determined by allowing His-tagged human Fcγ receptor to be captured as a ligand onto an anti-His antibody-immobilized sensor chip, then adding the analyte to the sensor chip, and measuring the interaction between the ligand and the analyte by surface SPR and, specifically, can be determined using a method as disclosed in Test Examples 15 to 20 herein.

In one aspect, in the case where the target molecule-binding moiety of the present invention is an antibody and the mutated Fc region is included in the antibody, the molecule comprising a target molecule-binding moiety, a mutated Fc region, and an immunostimulator according to the present invention has decreased binding activity against human Fcγ receptor to the same level as that of a parent antibody. In one aspect, the decreased binding activity against human Fcγ receptor means decreased effector functions.

In one aspect, the molecule comprising a target molecule-binding moiety of the present invention, a mutated Fc region, and an immunostimulator has a decreased ability to produce inflammatory cytokines compared to a corresponding molecule having the wild-type Fc region. In the present invention, "inflammatory cytokine" means a cytokine that causes or promotes inflammatory response or inflammatory symptoms. In one aspect of the present invention, "inflammatory cytokine" is a cytokine that causes or promotes inflammatory response or inflammatory symptoms and means a cytokine

that is not expected or desired to increase its production by a test substance more than necessary. For example, a cytokine whose production is increased in a situation where cancer cells targeted by the target molecule-binding moiety are absent, as in Test Example 14 herein, generally corresponds to the cytokine that is not expected or desired to increase its production more than necessary and may therefore be included in "inflammatory cytokine" according to the present invention. In one aspect of the present invention, the "inflammatory cytokine" is interleukin, TNF-$\alpha$, TNF-$\beta$, interferon, or chemokine, and is preferably TNF-$\alpha$, IL-1, IL-6, IL-10, CCL2, CCL4, or IFN-y, and particularly preferably IL-6. The ability of a test substance (for example, the molecule comprising a mutated Fc region according to the present invention) to produce inflammatory cytokines can be determined using any known method and can be determined by, for example, immunoassay such as ELISA. In one aspect of the present invention, the ability of a test substance to produce inflammatory cytokines can be determined using a method as disclosed in Test Example 14 herein.

In one aspect, the molecule comprising a target molecule-binding moiety of the present invention, a mutated Fc region, and an immunostimulator has equivalent or enhanced *in vivo* tumor activity compared to a corresponding molecule having the wild-type Fc region.

Herein, "carcinoma" and "tumor" are used interchangeably.

In the present invention, "anti-tumor effects" refers to induction of a decrease or regression of the tumor while a drug exerts a direct or indirect effect on the tumor cells. For example, a drug can cause direct damage to tumor cells; the tumor cells stimulate anti-tumor immunity by drug stimulation; and the drug delivered to the tumor cells is released into an extracellular space, stimulating the anti-tumor immunity around the tumor cells. This, for instance, can cause a decrease in the number of tumor cells and damage the tumor or cause regression of the tumor. This is called an anti-tumor effect.

The *in vivo* anti-tumor activity of a test substance can be determined using any known method and can be determined using, for example, a method as disclosed in Test Examples 3 to 9 herein.

In one aspect, the molecule comprising a target molecule-binding moiety of the present invention, a mutated Fc region, and an immunostimulator has equivalent or improved thermal stability compared to a corresponding molecule having the wild-type Fc region and preferably has improved thermal stability. The thermal stability of the molecule as described above can be determined using any known method and can be determined, for example, by preparing a solution containing this molecule, and examining change in heat capacity $C_p$ (kcal/mol/°C) of the solution when the temperature is elevated. Specifically, the thermal stability can be determined using a method as disclosed in Test Example 21 herein. A peak in a curve that shows change in heat capacity indicates the thermal denaturation of each antibody domain, and thermal denaturation peaks are generally observed in the order of CH2, Fab, and CH3 (Biochem. Biophys. Res. Commun. 355, 751-757 (2007)). Thus, change in thermal stability can be determined by calculating a thermal denaturation midpoint ($T_m$) of a domain with an introduced mutation from a thermal denaturation peak, and comparing a molecule comprising a mutated Fc region to a corresponding molecule having the wild-type Fc region. Note that herein, a peak top temperature in a curve that shows change in heat capacity is defined as $T_m$. Herein, if the amount of change in the $T_m$ value ($\Delta T_m$)is less than 1°C, the thermal stability between the molecules is confirmed to be equivalent; and in the case where the amount of change is improved by 1°C or more, the molecule comprising a mutated Fc region is confirmed to have improved thermal stability compared to a corresponding molecule having the wild-type Fc region. For example, in the case where a mutation is present in a CH2 domain, if the amount of change in the $T_m$ value ($\Delta T_m$)of the CH2 domain is less than 1°C, the thermal stability between the molecules is confirmed to be equivalent; and in the case where the amount of change is improved by 1°C or more, the molecule comprising a mutated Fc region is confirmed to have improved thermal stability compared to a corresponding molecule having the wild-type Fc region.

In one aspect, in the case where the mutated Fc region has amino acid substitutions of 234Ala, 235Ala, and 265Gly (EU numbering), the molecule comprising a target molecule-binding moiety, a mutated Fc region, and an immunostimulator according to the present invention has excellent safety while maintaining a pharmaceutical effect, and also has decreased effector functions to the same level as that of a parent antibody. In one aspect, in the case where the mutated Fc region has amino acid substitutions of 234Ala, 235Ala, and 265Gly (EU numbering), the molecule comprising a target molecule-binding moiety, a mutated Fc region, and an immunostimulator according to the present invention is also excellent in thermal stability.

In one aspect, the present invention relates to a pharmaceutical composition comprising the above-described molecule comprising a target molecule-binding moiety, a mutated Fc region, and an immunostimulator. In one aspect of the present invention, the pharmaceutical composition is an anti-tumor agent. "Pharmaceutical composition" will be described later in detail.

In one aspect, the present invention relates to an antibody-drug conjugate comprising a CDN derivative having STING agonist activity and use thereof. The CDN derivative has STING agonist activity, stimulates immune cells and then induces the production of interferons and/or cytokines. In addition, the CDN derivative exerts the anti-tumor effects by stimulating the relevant immune cells. In one aspect, the antibody-drug conjugate of the present invention is produced by conjugating the CDN derivative to an antibody capable of recognizing and binding to a target cell (e.g., a tumor cell or immune cell) via a given linker, and can be administered systemically. Specific examples of the systemic administration include an intradermal, intramuscular, intraperitoneal, intravenous, and subcutaneous route. Note that the antibody-drug

conjugate can be regarded as one form of the above-described molecule comprising a target molecule-binding moiety, a mutated Fc region, and an immunostimulator.

STING (Stimulator of Interferon Genes) is a transmembrane adaptor protein localized in the endoplasmic reticulum. STING is known to have a high frequency of congenital polymorphisms (PLoS One, 2013 Oct, 21, 8(10), e77846). Examples of a STING mutant include a R232H mutant in which the amino acid at position 232 is mutated from arginine (R) to histidine (H), or a HAQ mutant in which the arginine (R) at position 71 is mutated to histidine (H), the glycine (G) at position 230 is mutated to alanine (A), and the arginine (R) at position 293 is mutated to glutamine (Q). Such STING polymorphisms are known to cause a difference in the strength of responses, such as the level of cytokine production induced by STING agonist stimulation (Genes and Immunity, 2011, 12, 263-269). Therefore, in order to make STING agonists stably effective in humans, the STING agonists should elicit activity against each type of STING.

In the present invention, "cellular cytotoxic activity" refers to causing pathological change to cells in any way, which includes not only direct injuries but also all types of damage in the structure and function of cells such as cleavage of DNA, formation of a base dimer, cleavage of a chromosome, damage of the mitotic apparatus, and lowered activity of various enzymes.

In the present invention, "cells" includes cells in individual animals and cultured cells.

<1. CDN Derivative>

A CDN derivative has a structure represented by the following formula (I):

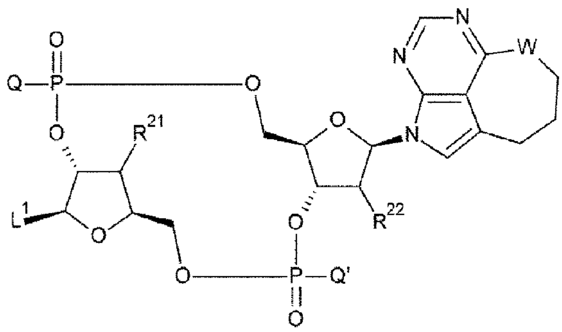

(I)

.

$L^1$ is a group represented by the following formula:

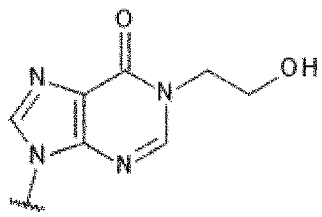

.

Q and Q', each independently, represent a hydroxy group or a thiol group. Preferably, Q and Q' are each a thiol group.

$R^{21}$ and $R^{22}$, each independently, represent a hydroxy group or a fluorine atom. $R^{21}$ is preferably a hydroxy group. $R^{22}$ is preferably a fluorine atom.

W is -NH- or a sulfur atom. W is preferably -NH-.

Methods for producing the CDN derivative will be described in the below-described section <3. Production Methods>.

<2. Antibody-Drug Conjugate>

In one aspect, it is possible to systemically administer an antibody-drug conjugate of the present invention as produced by conjugating the above-described CDN derivative to an antibody capable of recognizing and binding to a target cell (e.g., a tumor cell or immune cell) via a given linker. Note that hereinafter, "antibody-drug conjugate of the present invention" simply described herein means, in principle, an antibody-drug conjugate comprising the above-described CDN derivative as a drug moiety and also exceptionally means, in addition to this, the above-described "molecule comprising a target molecule-binding moiety, a mutated Fc region, and an immunostimulator".

An antibody-drug conjugate of the present invention is represented by the following formula (II):

$$Ab-[L-D]_{m^1} \quad (II)$$

.

Here, $m^1$ ranges from 1 to 10, and indicates the number of conjugated drug molecules per antibody molecule in the antibody-drug conjugate. Ab represents an antibody or a functional fragment thereof; L represents a linker that links Ab and D; and D represents the above-described CDN derivative (simply herein referred to as a "drug" when the CDN derivative is used as a part of the antibody-drug conjugate).

The drug D is a compound that has immune cell-stimulating activity, specifically, STING agonist activity. When part or all of the linker is cleaved in a target cell(s) (e.g., a tumor cell(s) or immune cell(s)), the drug D is released in its original structure and exerts its immunostimulatory effects. The desired functions can be elicited by increasing the sensitivity of target cells to immune cells or by stimulating immune cells through target cells. The functions of interest are not particularly limited if the functions involve STING agonist activity. However, they preferably involve anti-tumor activity. Specifically, the drug D conjugated, via a given linker, to a tumor-targeting antibody (e.g., anti-EGFR antibody, anti-CDH6 antibody) is delivered to a target cell(s) or tissue(s); the linker is partially or completely cleaved; and anti-tumor effects can be exerted through the enhancement of the sensitivity of target cells to immune cells or the target cell-mediated stimulation of immune cells (e.g., production of interferons and/or cytokines).

The drug D conjugated in an antibody-drug conjugate of the present invention is represented by the following formula (I):

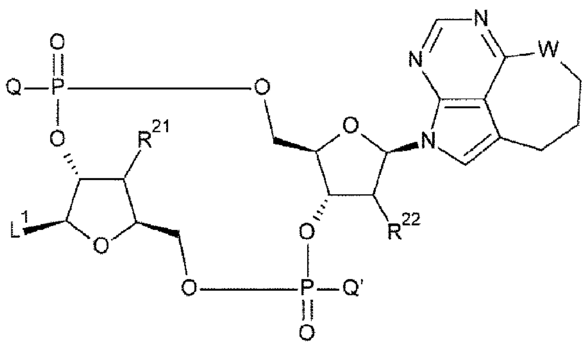

(I)

wherein $L^1$, Q, Q', $R^{21}$, $R^{22}$, and W are as specified above in the section <1. CDN Derivative>.

In addition, the drug D used in an antibody-drug conjugate of the present invention is preferably represented by any one of the following two formulae:

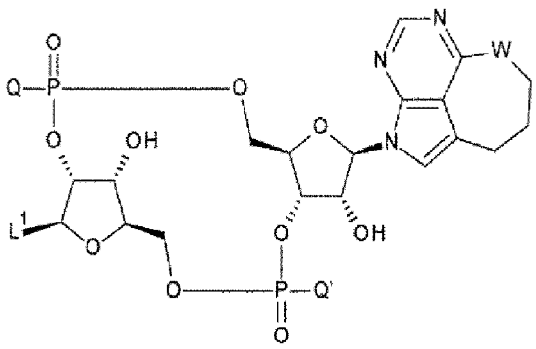

or

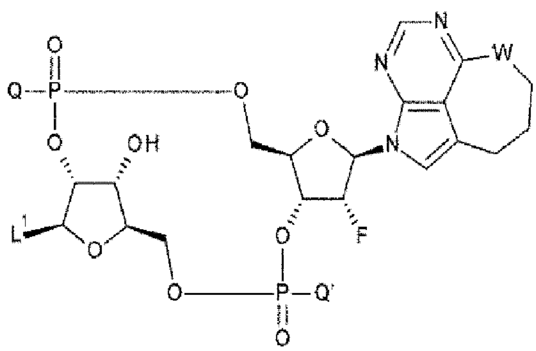

wherein $L^1$, Q, Q', and W are as specified in the above section <1. CDN Derivative>.

Further, the drug D used in an antibody-drug conjugate of the present invention is preferably represented by any one of the following two formulae:

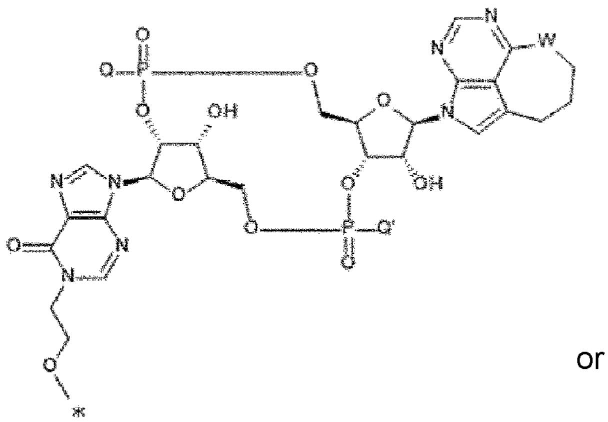

or

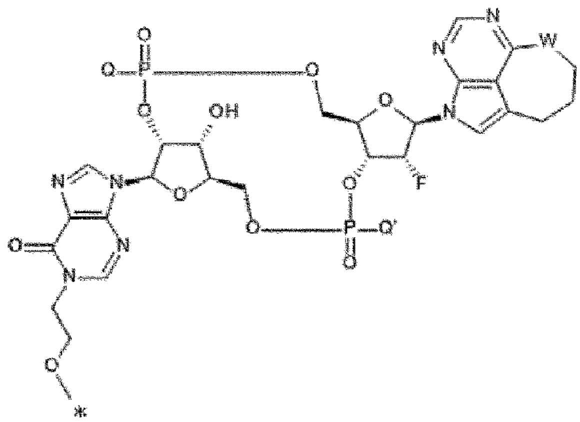

wherein the asterisk represents bonding to L, and Q, Q' and W are as specified in the above section <1. CDN Derivative>.

Furthermore, the drug D used in an antibody-drug conjugate of the present invention is preferably represented by any of the following three formulae:

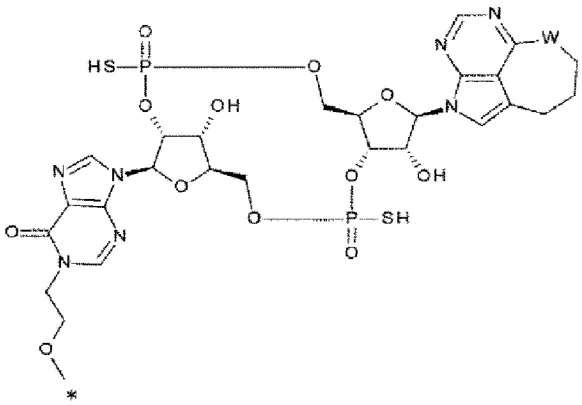

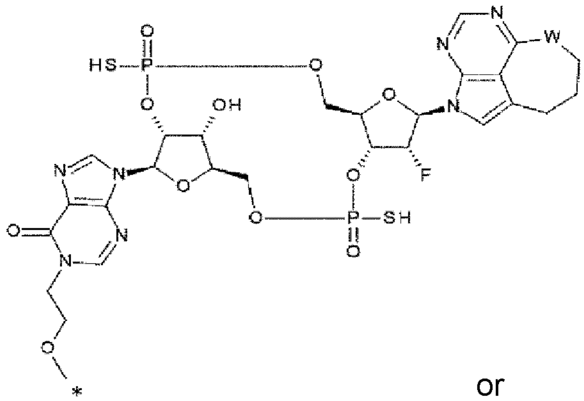

or

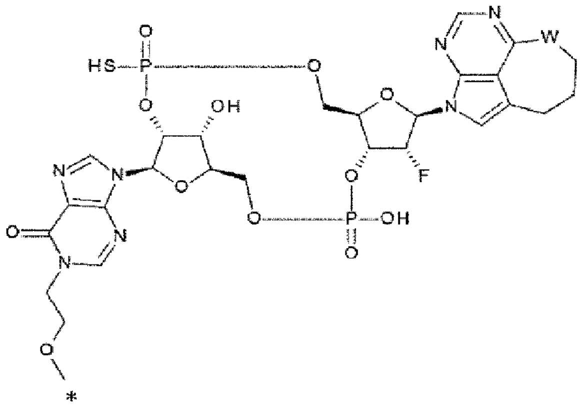

wherein the asterisk represents bonding to L, and W is as specified in the above section <1. CDN Derivative>.

Furthermore, the drug D used in an antibody-drug conjugate of the present invention is preferably represented by any of the following three formulae:

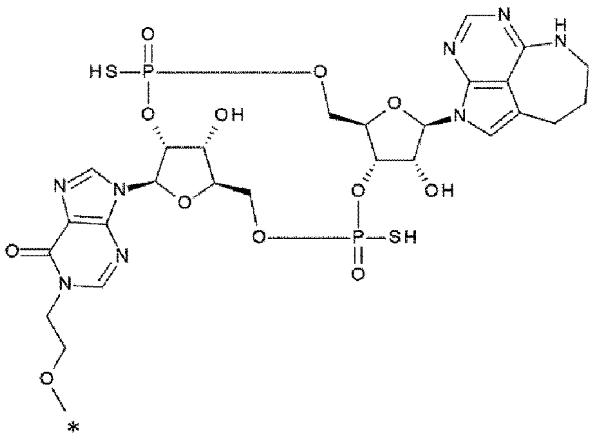

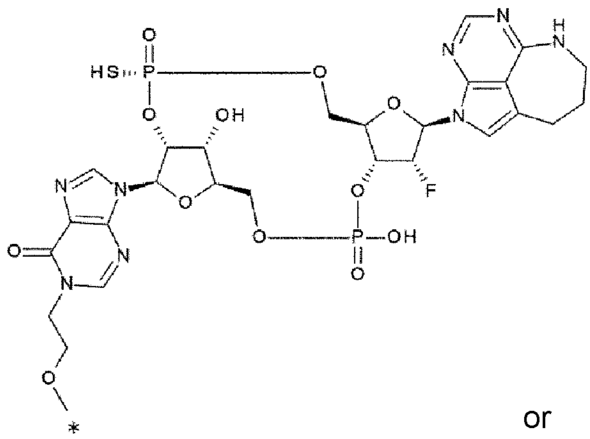

or

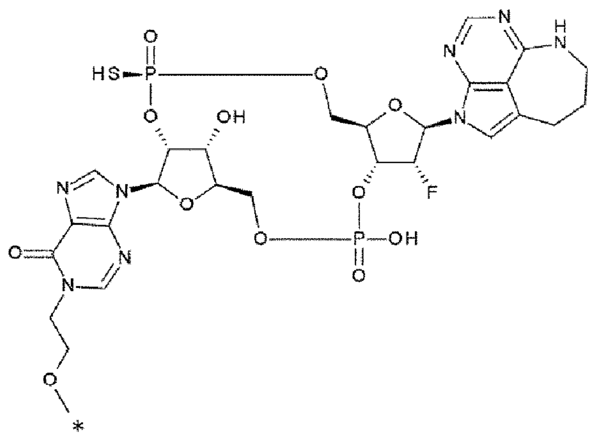

wherein the asterisk represents bonding to L.

Further, the drug D used in an antibody-drug conjugate of the present invention is preferably represented by any one of the following four formulae:

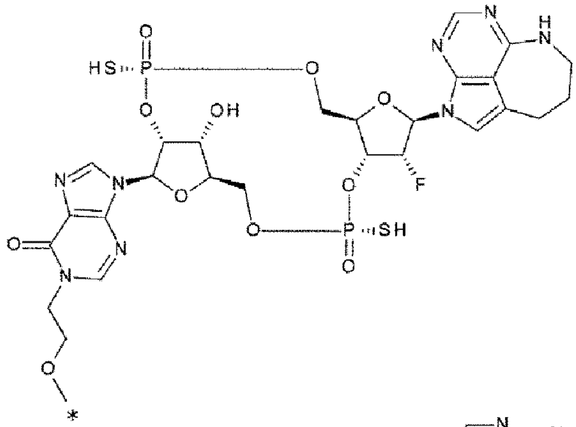

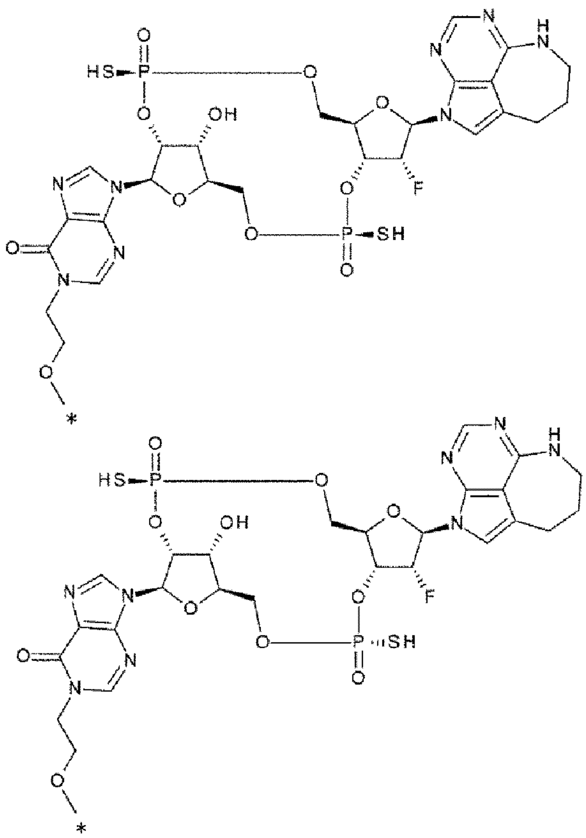

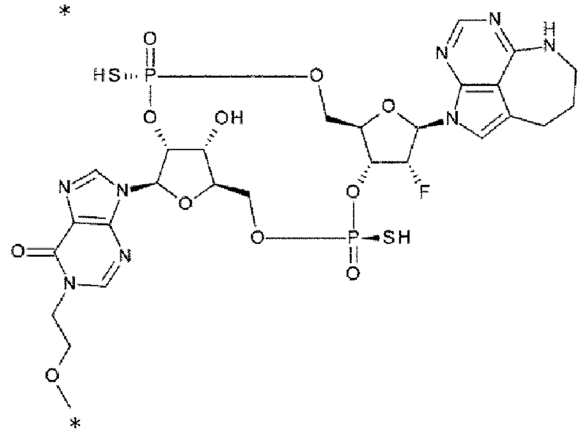

or

wherein the asterisk represents bonding to L.

Furthermore, the drug D used in an antibody-drug conjugate of the present invention is preferably represented by the following formula:

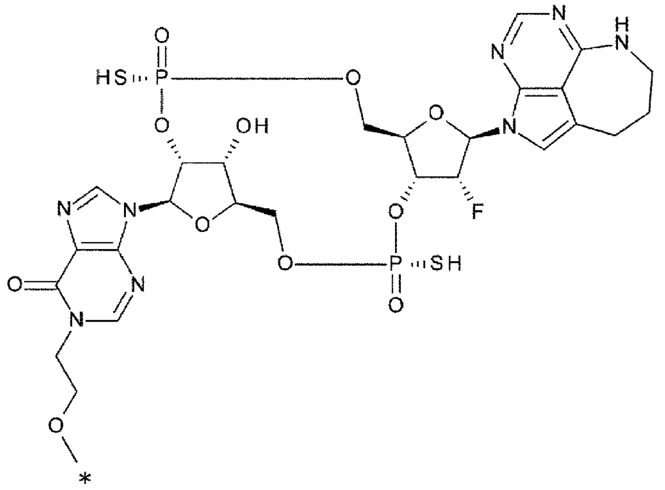

<2.1. Linker Structure>

The following describes the structure of a linker used to conjugate a drug to an antibody in an antibody-drug conjugate of the present invention. The linker used in the antibody-drug conjugate of the present invention is not particularly limited if the linker is understandable by those skilled in the art as a linker that conjugates the antibody to the drug. Examples of the linker used in the antibody-drug conjugate of the present invention include, but are not limited to, those described in Protein Cell, 2018, 9(1): 33-46, Pharm Res, 2015, 32: 3526-3540, or Int. J. Mol. Sci, 2016, 17, 561. The linker can be a linker that is cleaved *in vivo* or a linker that is not cleaved *in vivo,* but preferably a linker that is cleaved *in vivo.*

Examples of the linker used in the antibody-drug conjugate of the present invention include, but are not limited to,

a linker that conjugates the drug to a glycan or remodeled glycan of the Fc region of the antibody (sometimes herein referred to as "glycan conjugation") (e.g., as described in WO2018/003983), or a linker that conjugates the drug to a given amino acid residue of the antibody (e.g., a cysteine or lysine residue) (e.g., described in WO2014/057687). A linker that conjugates the drug to a given amino acid residue of the antibody preferably involves thioether bonding to the sulfhydryl group (SH group) of a cysteine of Ab (sometimes herein referred to as "cysteine conjugation") or amide bonding to the amino group ($NH_2$ group) of a lysine of Ab (sometimes herein referred to as "lysine conjugation"). Cysteine conjugation is preferred.

The linker L used in an antibody-drug conjugate of the present invention is preferably represented by the following formula:

-Lb-La-Lp-Lc-*

wherein the asterisk represents bonding to a drug D.

First, Lp will be described. Lp represents a linker consisting of an amino acid sequence that can be cleaved *in vivo* or in a target cell (sometimes herein referred to as a peptide linker), or is absent.

Lp can be cleaved, for example, by the action of enzyme such as a peptidase or esterase. Lp is a peptide consisting of 2 to 7 amino acids (preferably 2 to 4 amino acids). At its N-terminus, Lp forms an amide bond with the right end of the carbonyl group of La described below, and at its C-terminus, Lp forms an amide bond with the amino group (-NH-) of Lc. The amide bond on the C-terminal side of Lp is cleavable by an enzyme such as a peptidase.

The amino acids constituting Lp are not particularly limited, but can be, for example, L- or D-amino acids, preferably L-amino acids. The structure of each amino acid may involve the structure of α-amino acids as well as β-alanine, ε-aminocaproic acid, or γ-aminobutyric acid. Further, each amino acid may be a non-natural amino acid such as an N-methylated amino acid. The amino acid sequence of Lp consists of the amino acids which include but are not particularly limited to, for instance, glycine (Gly; G), valine (Val; V), alanine (Ala; A), phenylalanine (Phe; F), glutamic acid (Glu; E), isoleucine (Ile; I), proline (Pro; P), citrulline (Cit), leucine(Leu; L), methionine (Met; M), serine (Ser; S), lysine (Lys; K), and aspartic acid (Asp; D). Among them, preferred are glycine (Gly; G), valine (Val; V), alanine (Ala; A), phenylalanine (Phe; F), citrulline (Cit), isoleucine (Ile; I), and proline (Pro; P). Any of these amino acids may appear multiple times, and Lp has an amino acid sequence including freely selected amino acids. The drug release pattern may be controlled via amino acid type.

Specific examples of Lp include -GGFG-, -GGPI-, -GGVA-, - GGFM-, -GGVCit-, -GGFCit-, -GGICit-, -GGPL-, -GGAQ-, and -GGPP-. The linker Lp is preferably -GGFG- or -GGPI-, and more preferably -GGFG-.

Next, La will be described. La is represented by any one selected from the following group consisting of:

$-C(=O)-(CH_2CH_2)n^2-C(=O)-$,

$-C(=O)-(CH_2CH_2)n^2-CH_2-C(=O)-$,

$-C(=O)-(CH_2CH_2)n^2-C(=O)-NH-(CH_2CH_2)n^3-C(=O)-$,

$-C(=O)-(CH_2CH_2)n^2-C(=O)-NH-(CH_2CH_2)n^3-CH_2-C(=O)-$,

$-C(=O)-(CH_2CH_2)n^2-C(=O)-NH-(CH_2CH_2O)n^3-CH_2-C(=O)-$,

$-(CH_2)n^4-O-C(=O)-$,

and

$-(CH_2)n^9-C(=O)-$,

wherein $n^2$ represents an integer from 1 to 3 (preferably, 1 or 2), $n^3$ represents an integer from 1 to 5 (preferably, an integer from 2 to 5, more preferably, 3 or 4), $n^4$ represents an integer from 0 to 2 (preferably, 0 or 1), and $n^9$ represents an integer from 2 to 7 (preferably, an integer from 2 to 5, and more preferably 2, 3, or 5).

La is preferably represented by any one selected from the following group consisting of:

$-C(=O)-CH_2CH_2-C(=O)-$,

$-C(=O)-CH_2CH_2-C(=O)-NH-(CH_2CH_2O)_3-CH_2-C(=O)-$,

$-C(=O)-CH_2CH_2-C(=O)-NH-(CH_2CH_2O)_4-CH_2-C(=O)-$,

$-C(=O)-(CH_2CH_2)_2-C(=O)-$,

$-C(=O)-CH_2CH_2-C(=O)-NH-(CH_2CH_2)_2-C(=O)-$,

$-C(=O)-CH_2CH_2-C(=O)-NH-(CH_2CH_2)_2-CH_2-C(=O)-$,

$-CH_2-OC(=O)-$,

$-OC(=O)-$,

and

$-(CH_2)_5-C(=O)-$.

La is more preferably

$-C(=O)-CH_2CH_2-C(=O)-$,

$-C(=O)-CH_2CH_2-C(=O)-NH-(CH_2CH_2O)_3-CH_2-C(=O)-$,

$-C(=O)-CH_2CH_2-C(=O)-NH- (CH_2CH_2O)_4-CH_2-C(=O)-$,

or

$- (CH_2)_5-C(=O)-$.

La is more preferably $-C(=O)-CH_2CH_2-C(=O)-$.
Next, Lb will be described. Lb represents a spacer used in the linker for glycan conjugation (also herein referred to as a "spacer of linker for glycan conjugation"), or a spacer used in the linker for cysteine conjugation (also herein referred to as a "spacer of linker for cysteine conjugation").

<Case Where Lb Is A "Spacer of Linker for Glycan Conjugation">

In the case where Lb is a "spacer of linker for glycan conjugation", examples of Lb include, but are not particularly limited to, each spacer represented by the following formulae:

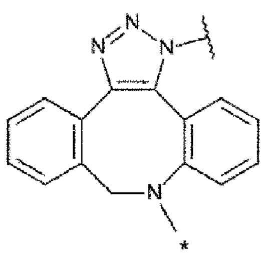

or

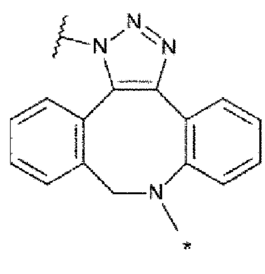

(Lb-1)

or

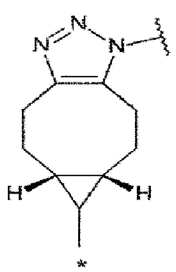

or

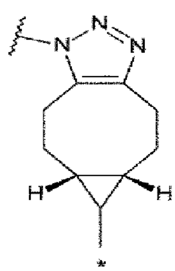

(Lb-3)

.

In each structural formula shown above, the asterisk represents bonding to -(C=O)-, $-CH_2-$, or -OC(=O)- at the left end of La. Each wavy line represents bonding to a glycan or remodeled glycan of Ab.

In the case where Lb-1 or Lb-3 is selected as Lb, the triazole ring provides structures of geometric isomers and Lb contains any one of the two structures or a mixture of both of them. The antibody-drug conjugate of the present invention allows multiple drugs to be conjugated to a single antibody molecule. When multiple drugs are conjugated to a single antibody molecule, a plurality of Lb portions are also present (e.g., see the scheme (1e) of the antibody-drug conjugate as shown in Method E of <3. Production Methods> described later). When Lb is selected from Lb-1 and Lb-3, and there are multiple Lb molecules for one antibody molecule (e.g., when $m^2$ is 1 or 2 as described below), the triazole ring provides structures of geometric isomers in each Lb, and Lb contains either one of the two structures or a mixture of them.

<Case Where Lb Is A "Spacer of Linker for Cysteine Conjugation">

In the case where Lb is a "spacer of linker for cysteine conjugation", examples of Lb include, but are not particularly limited to, -(succinimid-3-yl-N)-. In the present invention, "-(succinimid-3-yl-N)-" has the structure represented by the following formula:

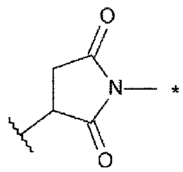

In the structural formula shown above, the asterisk represents bonding to La. The wavy line represents bonding, through thioether formation, to a side chain of a cysteine residue of an antibody.

Next, Lc will be described. Lc represents $-NH-CH_2-$, $-NH-phenyl-CH_2-O(C=O)-$, or $-NH-heteroaryl-CH_2-O(C=O)-$, or is absent. Here, the phenyl group is preferably a 1,4-phenyl group. The heteroaryl group is preferably a 2,5-pyridyl group, a 3,6-pyridyl group, a 2,5-pyrimidyl group, or a 2,5-thienyl group. Lc is preferably $-NH-CH_2-$, or is absent.

In the case where the form of antibody-drug bconjugation is "glycan conjugation", a more preferred linker L used in the antibody-drug conjugate of the present invention is

$-Z^{L1}-C(=O)-CH_2CH_2-C(=O)-GGFG-$,

$-Z^{L1}-C(=O)-CH_2CH_2-C(=O)-GGVA-$,

$-Z^{L1}-C(=O) -CH_2CH_2-C(=O)-GGVCit-$,

$-Z^{L1}-C(=O)-CH_2CH_2-C(=O)-GGFCit-$,

$-Z^{L1}-C(=O) -CH_2CH_2-C(=O)-GGICit-$,

$-Z^{L1}-C(=O)-CH_2CH_2-C(=O)-GGFM-$,

$-Z^{L1}-C(=O)-CH_2CH_2-C(=O)-GGPI-$,

$-Z^{L1}-C(=O)-CH_2CH_2-C(=O)-GGLM-$,

$-Z^{L1}-C(=O)-CH_2CH_2-C(=O)-FG-$,

$-Z^{L1}-C(=O)-CH_2CH_2-C(=O)-VA-$,

$-Z^{L1}-C(=O)-CH_2CH_2-C(=O)-GGFG-NH-CH_2-$,

$-Z^{L1}-C(=O)-CH_2CH_2-C(=O)-GGVA-NH-CH_2-$,

$-Z^{L1}-C(=O)-CH_2CH_2-C(=O)-GGVCit-NH-CH_2-$,

$-Z^{L1}-C(=O)-CH_2CH_2-C(=O)-GGFCit-NH-CH_2-$,

$-Z^{L1}-C(=O)-CH_2CH_2-C(=O)-NH-(CH_2CH_2O)_3-CH_2-C(=O)-$,

or

$-Z^{L1}-C(=O)-CH_2CH_2-C(=O)-NH-(CH_2CH_2O)_4-CH_2-C(=O)-$

where $Z^{L1}$ represents the following structural formula as described for Lb:

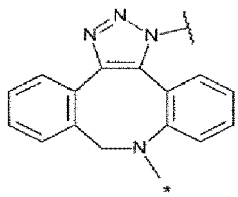

or

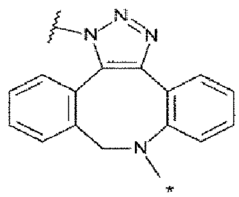

Alternatively, in the case where the form of the drug-antibody conjugation is "cysteine conjugation", the linker L is

$-Z^{L2}-(CH_2)_5-C(=O)-GGFG-$,

$-Z^{L2}-(CH_2)_5-C(=O)-GGVA-$,

$-Z^{L2}-(CH_2)_5-C(=O)-GGVCit-$,

$-Z^{L2}-(CH_2)_5-C(=O)-GGFCit-$,

$-Z^{L2}-(CH_2)_5-C(=O)-GGICit-$,

$-Z^{L2}-(CH_2)_5-C(=O)-GGFM-$,

$-Z^{L2}-(CH_2)_5-C(=O)-GGPI-$,

$-Z^{L2}-(CH_2)_5-C(=O)-GGLM-$,

$-Z^{L2}-(CH_2)_5-C(=O)-FG-$,

$-Z^{L2}-(CH_2)_5-C(=O)-VA-$,

$-Z^{L2}-(CH_2)_5-C(=O)-GGFG-NH-CH_2-$,

$-Z^{L2}-(CH_2)_5-C(=O)-GGVA-NH-CH_2-$,

$-Z^{L2}-(CH_2)_5-C(=O)-GGVCit-NH-CH_2-$,

$-Z^{L2}-(CH_2)_5-C(=O)-GGFCit-NH-CH_2-$,

$-Z^{L2}-(CH_2)_5-C(=O)-NH-(CH_2CH_2O)_3-CH_2-C(=O)-$,

or

$-Z^{L2}-(CH_2)_5-C(=O)-NH-(CH_2CH_2O)_4-CH_2-C(=O)-$,

where $Z^{L2}$ represents -(succinimid-3-yl-N)- represented by the following structural formula as described for Lb:

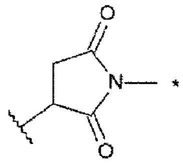

When the form of antibody-drug conjugation is "glycan conjugation", a more preferred linker L used in the antibody-drug conjugate of the present invention is

$$-Z^{L1}-C(=O)-CH_2CH_2-C(=O)-GGFG-NH-CH_2-,$$

or

$$-Z^{L1}-C(=O)-CH_2CH_2-C(=O)-GGPI-NH-CH_2-,$$

where $Z^{L1}$ represents the following structural formula as described for Lb:

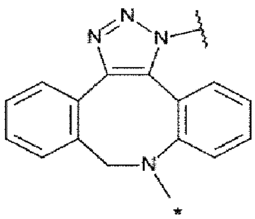

or

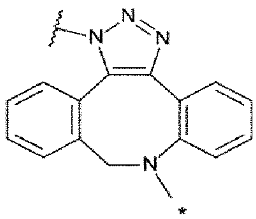

When the form of antibody-drug conjugation is "glycan conjugation", a yet more preferred linker L used in the antibody-drug conjugate of the present invention is

$$-Z^{L1}-C(=O)-CH_2CH_2-C(=O)-GGFG-NH-CH_2-,$$

where $Z^{L1}$ represents the following structural formula as described for Lb:

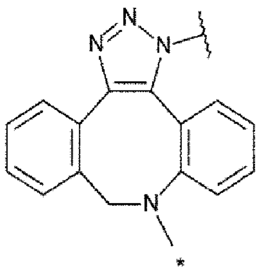

or

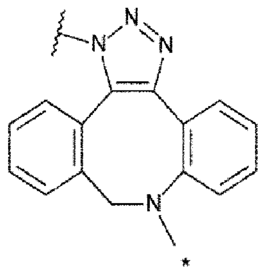

The right end of the above "preferred linker L", "more preferred linker L", "further preferred linker L", or "yet more preferred linker L" is linked to the drug D.

A "linker L-drug D" used in an antibody-drug conjugate of the present invention is preferably represented by the following four formulae:

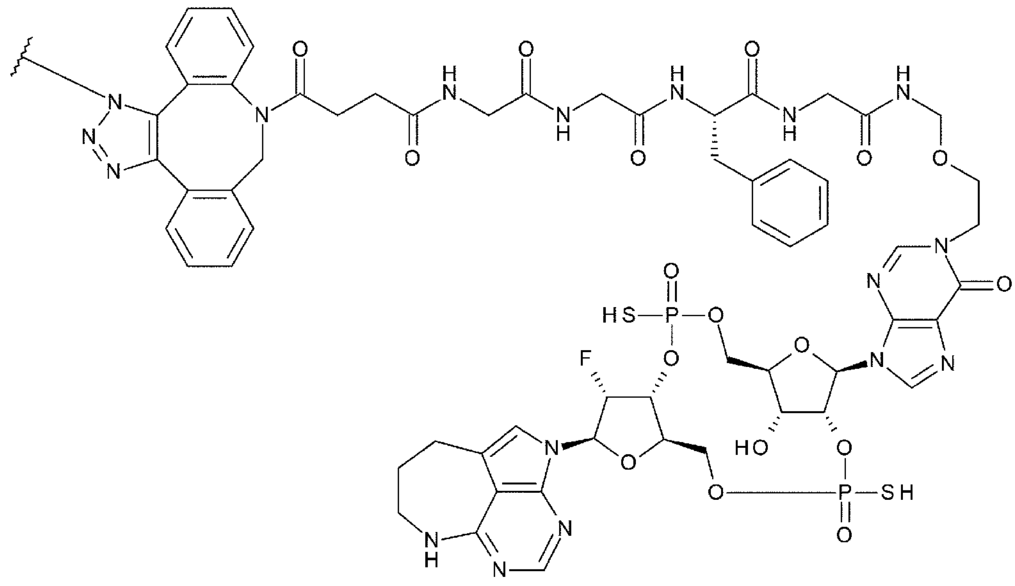

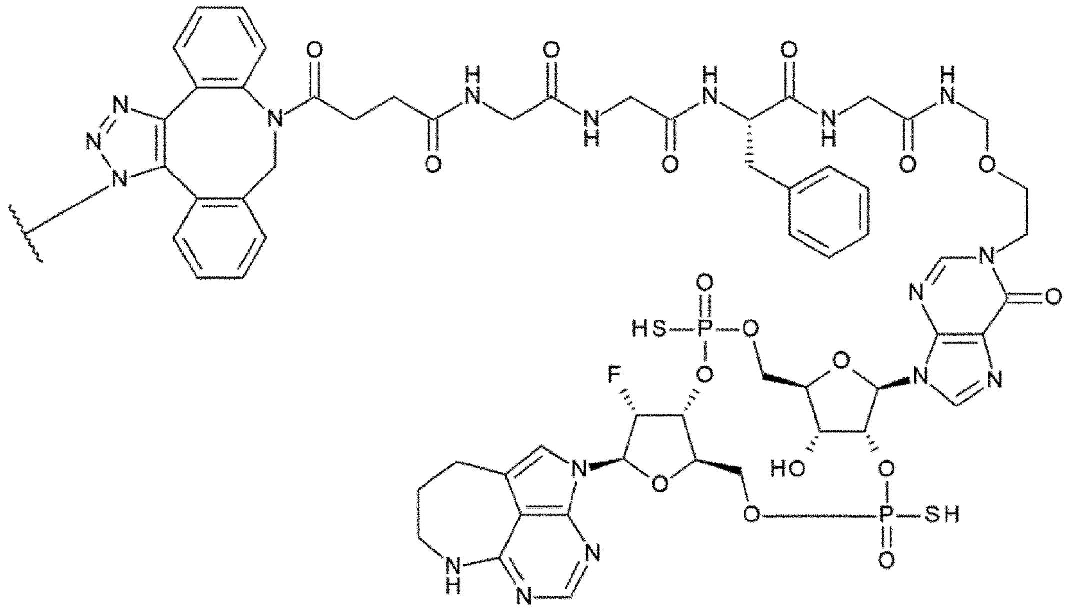

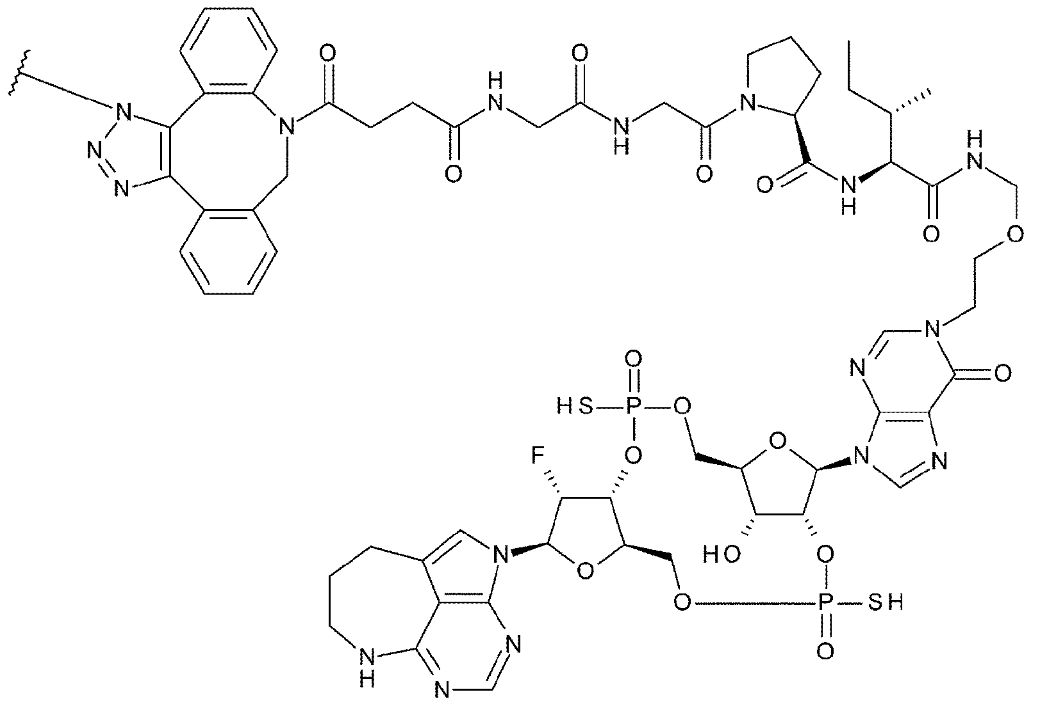

or

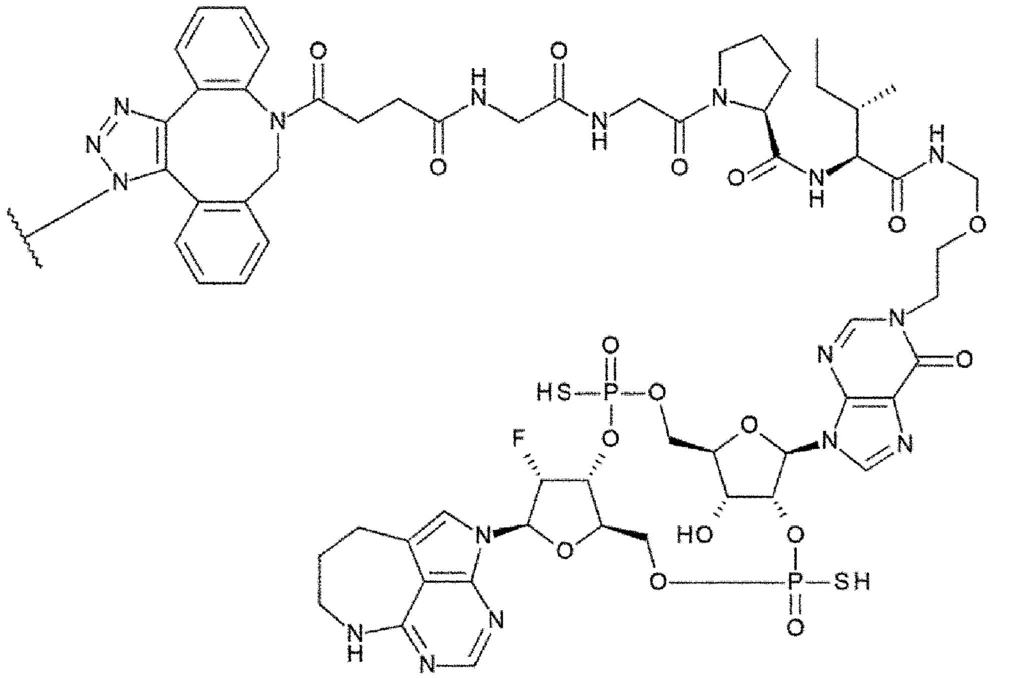

wherein the wavy line represents bonding to a glycan or remodeled glycan of Ab.

The "linker L-drug D" used in an antibody-drug conjugate of the present invention is more preferably represented by the following four formulae:

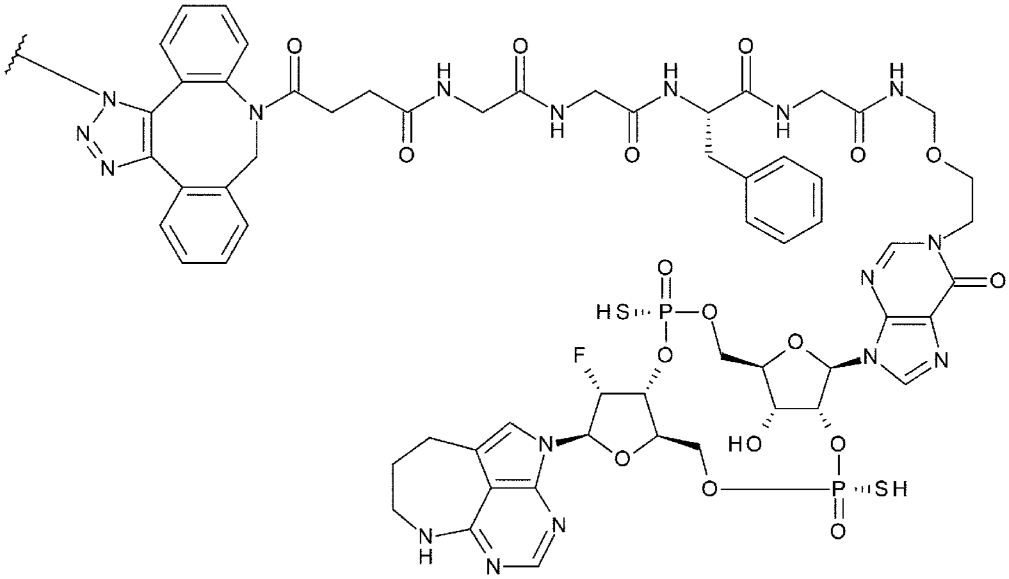

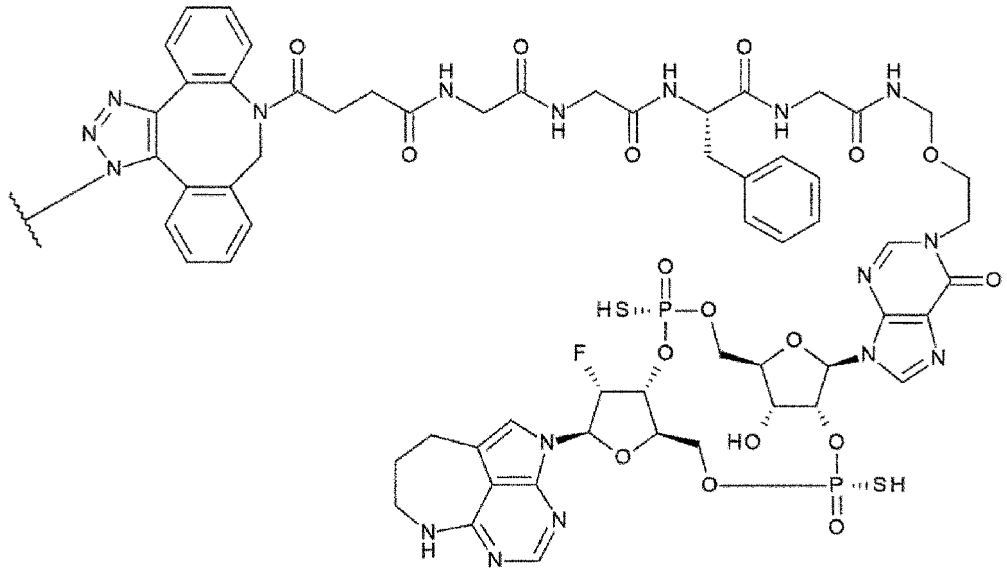

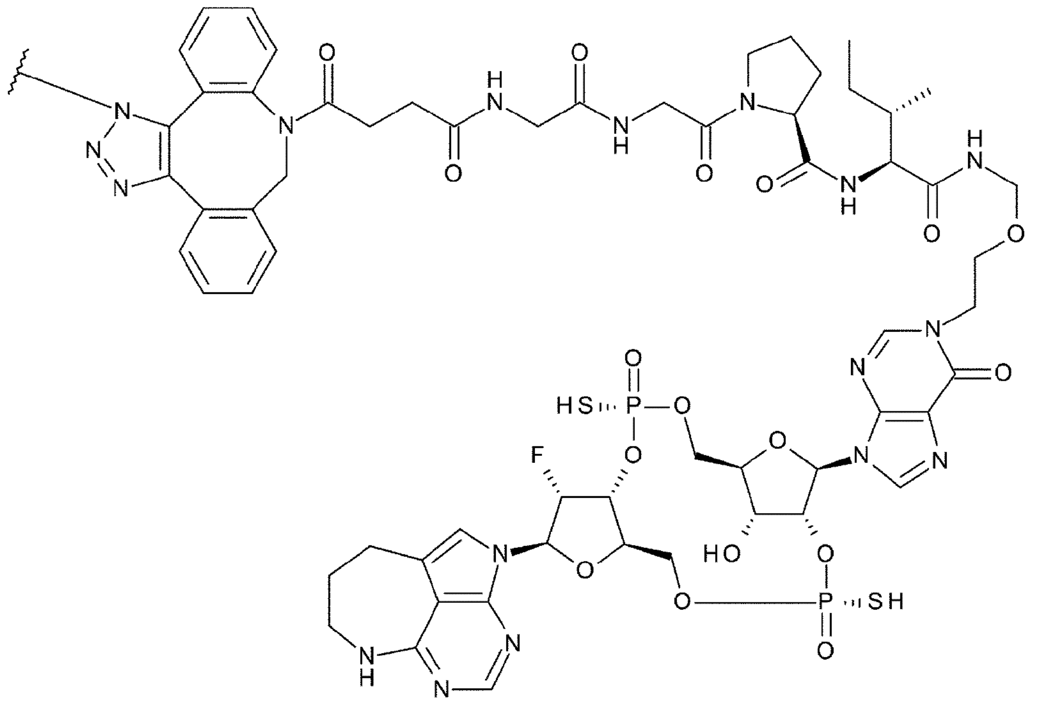

or

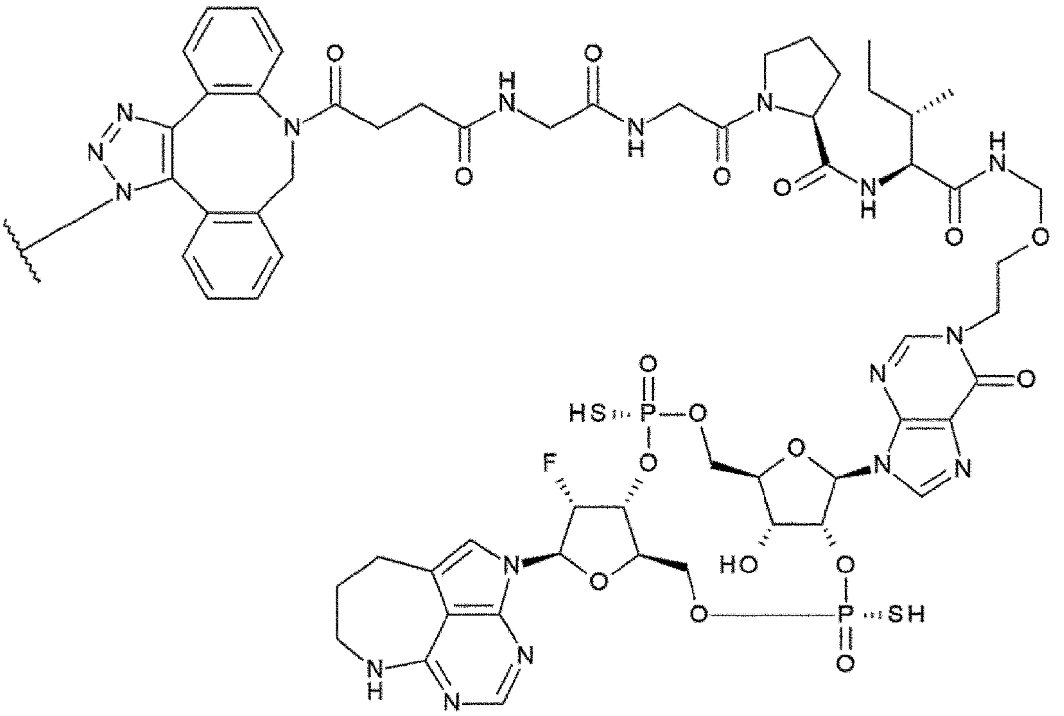

wherein the wavy line represents bonding to a glycan or remodeled glycan of Ab.

The "linker L-drug D" used in an antibody-drug conjugate of the present invention is further preferably represented by the following formula:

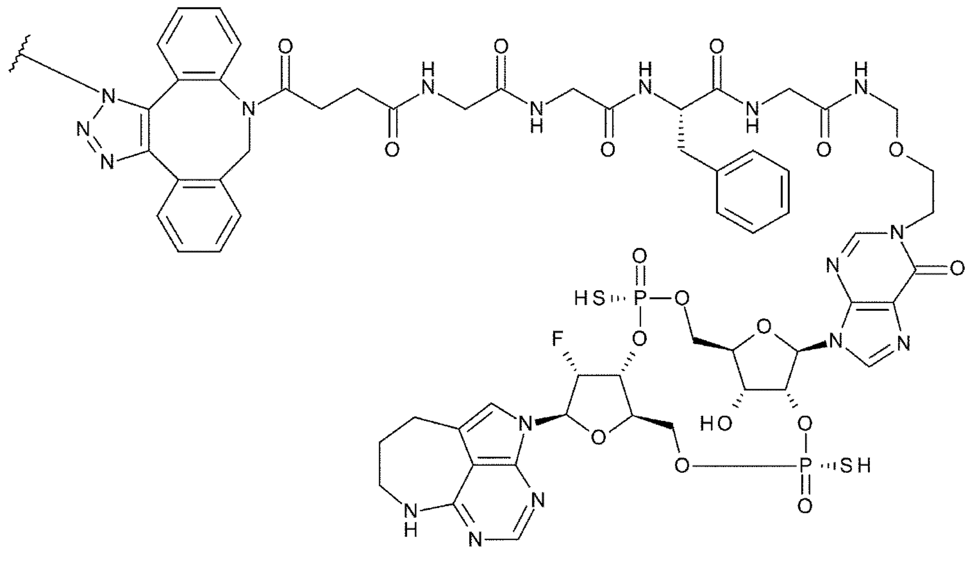

or

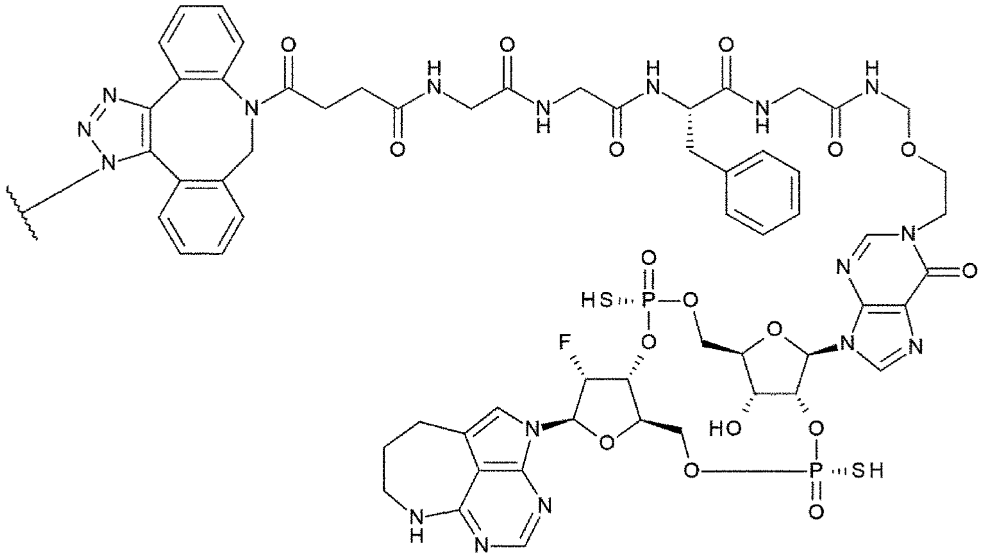

wherein the wavy line represents bonding to a glycan or remodeled glycan of Ab.

<2.2. Antibody and Its Glycosylation>

<2.2.1 Antibody>

Herein, a "gene" refers to nucleotides or a nucleotide sequence including a nucleotide sequence encoding amino acids of a protein or a complementary strand thereof. The meaning of a "gene" encompasses, for example, a polynucleotide, an oligonucleotide, DNA, mRNA, cDNA, and RNA as a nucleotide sequence including a nucleotide sequence encoding amino acids of a protein or a complementary strand thereof.

Herein, "nucleotides", "polynucleotide", and "nucleotide sequence" have the same meaning as that of "nucleic acids", and the meaning of "nucleotides" and "nucleotide sequence" encompasses, for example, DNA, RNA, a probe, an oligonucleotide, a polynucleotide, and a primer.

Herein, "polypeptide", "peptide", and "protein" are used interchangeably.

Herein, the "functional fragment of the antibody" is sometimes called an "antigen-binding fragment of the antibody", and means a partial fragment of the antibody having antigen-binding activity. Examples thereof include a linear antibody or multispecific antibody formed using an antibody fragment(s), etc, provided that the fragment is not limited to these molecules, as long as the fragment has the ability to bind to an antigen. In addition, these antigen-binding fragments include not only those obtained by treating a full-length molecule of an antibody protein with an appropriate enzyme, but

also proteins produced in appropriate host cells by using a gene which is modified by genetic engineering.

The functional fragment of the antibody used in an antibody-drug conjugate of the present invention includes a functional fragment that has an aspartic acid (Asn297) to be modified with a well-conserved N-linked glycan in the IgG heavy chain Fc region and amino acids around Asn297, wherein the functional fragment has the ability to bind to an antigen.

The antibody used in the antibody-drug conjugate of the present invention means an immunoglobulin, which is a molecule containing an antigen-binding site where an antigen can be immunospecifically bound. The antibody used in the antibody-drug conjugate of the present invention may be any of class IgG, IgE, IgM, IgD, IgA or IgY, and preferably is IgG. In addition, its subclass may be any of IgG1, IgG2, IgG3, IgG4, IgA1 or IgA2, and preferred is IgG1, IgG2 or IgG4, more preferred is IgG1 or IgG4, and particularly preferred is IgG1 (including an antibody with a mutation affecting the ADCC and/or ADCP activities in the Fc region of the IgG heavy chain). Thus, in one aspect of the present invention, the antibody used in the antibody-drug conjugate of the present invention is IgG1 or IgG4, and is preferably IgG1, and the functional fragment of the antibody used in the antibody-drug conjugate of the present invention is a functional fragment of an IgG1 or IgG4 antibody and preferably IgG1 antibody.

The antibody or the functional fragment of the antibody used in the antibody-drug conjugate of the present invention comprises a mutated Fc region in which the amino acid residues at positions 234, 235, and 265 (all according to EU numbering) are Ala, Ala, and Gly, respectively. Herein, the amino acid residues are numbered in accordance with the EU index or the EU numbering (Proceedings of the National Academy of Sciences of the United States of America, Vol. 63, No. 1 (May 15, 1969), pp.78-85) unless otherwise specified.

In one aspect of the present invention, in the case where the antibody used in an antibody-drug conjugate is IgG1 or the functional fragment of the antibody used in an antibody-drug conjugate is a functional fragment of an IgG1 antibody, the mutated Fc region has an IgG1 LALA-DG mutation (IgG1-L234A, L235A, D265G).

It has heretofore been known that effector functions can be adjusted by substituting part of the amino acid residues of the constant regions (see WO88/07089, WO94/28027, and WO94/29351). In particular, it has been also known that the possibility of activating immune cells at a site other than a tumor site can be circumvented by reducing effector functions. On the other hand, it has been reported that in a conjugate containing a drug having activity that stimulates immune cells, a pharmaceutical effect is also decreased when the effector functions of an antibody are decreased (Non-Patent Literatures 4 to 6).

In one aspect of the present invention, the antibody or the functional fragment of the antibody used in an antibody-drug conjugate can have decreased effector functions while maintaining a pharmaceutical effect, when allowed to have the above-described mutation in the Fc region. In one aspect of the present invention, in the case where the antibody or the functional fragment of the antibody used in an antibody-drug conjugate is allowed to have the above-described mutation in the Fc region, the antibody-drug conjugate has decreased binding activity to human Fcγ receptor, has a decreased ability to produce inflammatory cytokines, and/or has equivalent or enhanced *in vivo* anti-tumor activity compared to a corresponding antibody-drug conjugate having the wild-type Fc region. Here, "corresponding antibody-drug conjugate having the wild-type Fc region" means a molecule identical or equivalent to the antibody-drug conjugate of the present invention except that the molecule comprises the wild-type Fc region, not the mutated Fc region.

In one aspect of the present invention, in the case where the antibody or the functional fragment of the antibody used in an antibody-drug conjugate is allowed to have the above-described mutation in the Fc region, the antibody-drug conjugate has decreased effector functions to the same level as that of an antibody allowed to have the above-described mutation in the Fc region (parent antibody; an antibody used in the preparation of this antibody-drug conjugate). In addition, in one aspect of the present invention, in the case where the antibody or the functional fragment of the antibody used in an antibody-drug conjugate is allowed to have the above-described mutation in the Fc region, the antibody-drug conjugate has equivalent or improved thermal stability compared to a corresponding molecule having the wild-type Fc region. Particularly, in the case where the antibody or the functional fragment of the antibody used in an antibody-drug conjugate is allowed to have a LALA-DG mutation, the antibody-drug conjugate has improved thermal stability compared to a corresponding antibody-drug conjugate having the wild-type Fc region. Note that an IgG2 antibody is generally known to have lower effector functions than those of an IgG1 antibody (Front. Immunol. 2014, 5, 520).

In one aspect of the present invention, in the case where the mutated Fc region has a LALA-DG mutation, the antibody-drug conjugate of the present invention has excellent safety while maintaining a pharmaceutical effect. Furthermore, its effector functions are decreased to the same level as that of the parent antibody. In one aspect, in the case where the mutated Fc region has a LALA-DG mutation, the antibody-drug conjugate of the present invention also has excellent thermal stability.

The constant region of an antibody is known to have multiple allotypes. Examples in the IgG1 heavy chain include G1m17, G1m3, G1m1, and G1m2. Preferable examples of the constant region of the antibody used in the present invention include, but are not particularly limited to, G1m17 or G1m3. The amino acid sequence of a Fc region, without a Fab region, is commonly G1m17 or G1m3. A LALA-DG mutant of a Fc region, without a Fab region, has also exhibited an attenuating effect on FcγR-binding activity and improved thermal stability compared to a LALA mutant (data not shown).

Hence, the FcγR-binding activity-attenuating effect and thermal stability-improving effect of the molecule (particularly, an antibody or an antibody-drug conjugate) of the present invention are probably independent from the G1m17 and G1m3 allotypes.

It is known that each of heavy chains and light chains of an antibody molecule has three complementarity determining regions (CDRs). CDRs, which are also called the hypervariable regions, are located in variable regions of the heavy chains and light chains of an antibody and are sites with a particularly high variation of the primary structure. Three CDRs are separately located in the primary structure of the polypeptide chain of the heavy chains and light chains. Regarding CDRs of antibodies, herein, the CDRs of a heavy chain refer to CDRH1, CDRH2, and CDRH3 from the amino terminal side of the heavy chain amino acid sequence, and the CDRs of a light chain refer to CDRL1, CDRL2, and CDRL3 from the amino terminal side of the light chain amino acid sequence. These sites are located in the proximity of each other in the three-dimensional structure, determining specificity to an antibody to bind.

Note that herein, the amino acid sequence of a CDR contained in an antibody variable region is determined in accordance with the definition of KABAT (KABAT et al., Sequences of Proteins of Immunological Interest, 5th Ed. Public Health Service National Institutes of Health, Bethesda, MD. (1991)).

The antibody may be derived from any species. Preferable examples include a human, a rat, a mouse, or a rabbit. In the case where the antibody is derived from a species other than human, it is preferable to chimerize or humanize the antibody using well-known techniques. The antibody used in the present invention may be a polyclonal or monoclonal antibody, and is preferably a monoclonal antibody. Examples of the monoclonal antibody include each monoclonal antibody derived from a non-human animal (e.g., a rat, a mouse, and a rabbit antibodies), a chimeric antibody, a humanized antibody, a human antibody, or a functional fragment thereof, or a modified antibody thereof.

The antibody is preferably an antibody targeting a tumor cell or an immune cell, but is not limited thereto. The antibody is more preferably an antibody targeting a tumor cell.

The antibody may be used to target a tumor cell. In this case, it is preferable that the antibody should have one or more of the following characteristics: the ability to recognize tumor cells, the ability to bind to tumor cells, the ability to be taken-up and internalized by tumor cells, and the ability to damage tumor cells. The drug used in an antibody-drug conjugate of the present invention has STING agonist activity. The drug induces interferons by activating the signaling of interferon regulatory factor-3 (IRF3). Accordingly, the antibody against tumor cells as a target may be used in the antibody-drug conjugate of the present invention. In this case, the antibody-drug conjugate is administered into the body, delivered to the tumor site, and taken up by the tumor cells; and the linker portion is then cleaved by, for instance, a peptidase to release the drug moiety. The released drug moiety is thought to increase the sensitivity of the tumor cell to immune cells through STING agonist activity and to stimulate anti-tumor immunity, thereby exerting an anti-tumor effect. Alternatively, even if the antibody-drug conjugate accumulated on the tumor cells is not internalized, the tumor cells and/or the antibody-drug conjugate are/is taken up by the immune cells by phagocytosis or the like. Then, anti-tumor immunity is stimulated through STING agonist activity, and an anti-tumor effects may be exerted.

The binding activity of the antibody to tumor cells can be confirmed using flow cytometry. The incorporation of the antibody into tumor cells can be checked using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay for measuring a fluorescence intensity in cells of a secondary antibody (fluorescently labeled) which binds to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant protein complex of a diphtheria toxin catalytic domain and protein G may also be used.

In the case where an antibody against tumor cells is used in an antibody-drug conjugate of the present invention, it is desirable, but not essential, that the antibody itself has an anti-tumor effect.

The anti-tumor activity of the drug and the antibody-drug conjugate refers to cytotoxic activity on tumor cells, anti-cellular effects, and regression of the tumor volume. The anti-tumor activity can be checked by using a known *in vitro* or *in vivo* evaluation system.

The action and immunostimulatory activity of the drug and the antibody-drug conjugate refer to the increased sensitivity of tumor cells to immune cells or the activation of immune cells by tumor cells. Known *in vitro* or *in vivo* evaluation systems can be used to check the action and immunostimulatory activity of the drug and the antibody-drug conjugate.

Examples of the *in vitro* or *in vivo* evaluation system that can be used in the present invention can include, but are not limited to, a BALB/c mouse system, described in Test Examples 3, 4, 5, and 6, using subcutaneously implanted CT26.WT-hEGFR cells in which the human EGFR gene has been introduced into a mouse colon cancer cell line CT26.WT; a BALB/c-nu mouse system, described in Test Example 7, using subcutaneously transplanted cells of a human lung cancer cell line PC-9; a BALB/c-nu mouse system, described in Test Example 8, using a subcutaneously transplanted human lung cancer cell line NCI-H358; a BALB/c mouse system, described in Test Example 9, using subcutaneously transplanted CT26.WT-hCDH6 cells in which the human CDH6 gene has been introduced into CT26.WT; a co-culture assay system, described in Test Example 10, using THP1 reporter cells and a human lung cancer cell line HCC827; a co-culture assay system, described in Test Example 11, using THP1 reporter cells and a human ovarian cancer cell line OVCAR4; a BALB/c mouse system, described in Test Examples 12 and 13, using subcutaneously retransplanted CT26.WT cells; and an assay system described in Test Example 14, using whole human blood.

Examples of the antibody used in the present invention include an anti-CDH6 antibody and anti-EGFR antibody.

The antibody used in the present invention can be obtained by immunizing an animal with a polypeptide that serves as an antigen, and collecting and purifying the antibody produced *in vivo,* while using the methods routinely implemented in the art. The origin of the antigen is not limited to a human, and each animal can also be immunized with an antigen derived from a non-human animal (e.g., a mouse, a rat). In this case, any antibody applicable to human diseases can be selected by testing the cross-reactivity between the obtained antibody that can bind to a heterologous antigen and the corresponding human antigen.

Alternatively, antibody-producing cells which produce antibodies against the antigen are fused with myeloma cells according to the method known in the art (e.g., Kohler and Milstein, Nature (1975) 256, p.495-497; Kennett, R. ed., Monoclonal Antibodies, p.365-367, Plenum Press, N.Y. (1980)) to establish hybridomas, from which monoclonal antibodies can in turn be obtained.

Note that the antigen can be obtained by genetic engineering of a host cell to produce an antigen protein encoded by a gene of interest.

The antibody used in the antibody-drug conjugate of the present invention can be obtained according to known methods **(e.g.,** Proc. Natl. Acad. Sci. U.S.A., 81, 6851-6855, (1984); Nature (1986) 321, p.522-525, WO90/07861).

For example, an anti-EGFR antibody (e.g., WO1998/050433, WO2002/092771), and an anti-CDH6 antibody (e.g., WO2018/212136), can be obtained by known procedures.

The anti-EGFR antibody used in the present invention is not particularly limited, and should have, for example, the following characteristics.

(1) An anti-EGFR antibody capable of specifically binding to EGFR.
(2) The antibody described in (1), wherein the antibody can bind to an extracellular domain of human EGFR.
(3) The antibody described in (1) or (2), wherein the antibody is a monoclonal antibody.
(4) The antibody described in any of (1) to (3), wherein the antibody is a mouse monoclonal antibody, a chimeric monoclonal antibody, a human monoclonal antibody, or a humanized monoclonal antibody.
(5) The antibody described in any of (1) to (4), wherein a heavy chain constant region thereof is a heavy chain constant region of human IgG1 and includes a mutation which causes a decrease in ADCC and ADCP activities.
(6) The antibody described in (4), wherein a heavy chain constant region thereof is a heavy chain constant region of human IgG1, and the amino acid residues at positions 234, 235, and 265 (all according to EU numbering) of an Fc region are Ala, Ala, and Gly, respectively.
(7) The antibody described in (6), wherein the antibody is a humanized or human monoclonal antibody comprising a heavy chain consisting of an amino acid sequence set forth in SEQ ID NO: 32 and a light chain consisting of an amino acid sequence set forth in SEQ ID NO: 30.
(8) The antibody described in (6), wherein the antibody is a humanized or human monoclonal antibody comprising a heavy chain consisting of the amino acid sequence set forth in SEQ ID NO: 33 and a light chain consisting of the amino acid sequence set forth in SEQ ID NO: 30.
(9) The antibody described in (6), wherein the antibody is a humanized or human monoclonal antibody comprising the heavy chain variable region contained in the heavy chain an amino acid sequence set forth in SEQ ID NO: 32 or 33 and the light chain variable region contained in a light chain amino acid sequence set forth in SEQ ID NO: 30.
(10) The antibody described in (6), wherein the antibody is a humanized or human monoclonal antibody comprising: a light chain comprising CDRL1 consisting of the amino acid sequence set forth in SEQ ID NO: 23, CDRL2 consisting of the amino acid sequence set forth in SEQ ID NO: 24, and CDRL3 consisting of the amino acid sequence set forth in SEQ ID NO: 25; and a heavy chain comprising CDRH1 consisting of the amino acid sequence set forth in SEQ ID NO: 26, CDRH2 consisting of the amino acid sequence set forth in SEQ ID NO: 27, and CDRH3 consisting of the amino acid sequence set forth in SEQ ID NO: 28.
(11) The antibody described in any of (1) to (10), wherein one or two amino acids are deleted at a heavy chain carboxyl terminus.
(12) An antibody obtained by a method for producing the antibody, the method comprising the steps of culturing a host cell transfected with an expression vector containing a polynucleotide encoding the antibody described in any of (1) to (11), and collecting the antibody of interest from the resulting culture obtained in the former step.

Examples of the anti-EGFR antibody can include panitumumab, nimotuzumab, cetuximab, ametumumab (SY-101), SYN-004, SCT-200, tomuzotuximab, GC-1118, GR-1401, depatuxizumab (ABT-806), Serclutamab, AMG595, and matuzumab. Preferable examples include panitumumab or ABT-806.

The anti-CDH6 antibody used in the present invention is not particularly limited, and should have, for example, the

following characteristics.

(1) An anti- CDH6 antibody capable of specifically binding to CDH6.
(2) The antibody described in (1), wherein the antibody can bind to an extracellular domain of human CDH6.
(3) The antibody described in (1) or (2), wherein the antibody is a monoclonal antibody.
(4) The antibody described in any of (1) to (3), wherein the antibody is a mouse monoclonal antibody, a chimeric monoclonal antibody, a human monoclonal antibody, or a humanized monoclonal antibody.
(5) The antibody described in any of (1) to (4), wherein a heavy chain constant region thereof is a heavy chain constant region of human IgG1 and includes a mutation which causes a decrease in ADCC and ADCP activities.
(6) The antibody described in (5), wherein a heavy chain constant region thereof is a heavy chain constant region of human IgG1, and the amino acid residues at positions 234, 235, and 265 (all according to EU numbering) of the Fc region are Ala, Ala, and Gly, respectively.
(7) The antibody described in (6), wherein the antibody is a humanized or human monoclonal antibody comprising a heavy chain consisting of the amino acid sequence set forth in SEQ ID NO: 46 and a light chain consisting of the amino acid sequence set forth in SEQ ID NO: 44.
(8) The antibody described in (6), wherein the antibody is a humanized or human monoclonal antibody comprising the heavy chain variable region contained in the heavy chain amino acid sequence set forth in SEQ ID NO: 46 and the light chain variable region contained in the light chain amino acid sequence set forth in SEQ ID NO: 44.
(9) The antibody described in (6), wherein the antibody is a humanized or human monoclonal antibody comprising: a light chain comprising CDRL1 consisting of the amino acid sequence set forth in SEQ ID NO: 37, CDRL2 consisting of the amino acid sequence set forth in SEQ ID NO: 38, and CDRL3 consisting of the amino acid sequence set forth in SEQ ID NO: 39; and a heavy chain comprising CDRH1 consisting of the amino acid sequence set forth in SEQ ID NO: 40, CDRH2 consisting of the amino acid sequence set forth in SEQ ID NO: 41, and CDRH3 consisting of the amino acid sequence set forth in SEQ ID NO: 42.
(10) The antibody described in any of (1) to (9), wherein one or two amino acids are deleted at a heavy chain carboxyl terminus.
(11) An antibody obtained by a method for producing the antibody, the method comprising the steps of culturing a host cell transfected with an expression vector containing a polynucleotide encoding the antibody described in any of (1) to (10), and collecting the antibody of interest from the resulting culture obtained in the former step.

Examples of the anti-CDH6 antibody can include an antibody described in WO2018212136, comprising a light chain consisting of the amino acid sequence of positions 21 to 233 of SEQ ID NO: 61 and a heavy chain consisting of the amino acid sequence of positions 20 to 471 of SEQ ID NO: 69 (antibody X), NOV0712, and LTV977, and can preferably include antibody **X.**

The antibody used in the present invention may be an antibody with 80% to 99% amino acid identity to the heavy and/or light chains of each of the above antibodies. Here, the term "identity" has the general definition used in the art. The % identity refers to the percentage of identical amino acids per total number of amino acids (including gaps) when two amino acid sequences are aligned to maximize the amino acid identity. Such identity is generally greater than or equal to 80% identity, preferably greater than or equal to 90%, 91%, 92%, 93% or 94% identity, more preferably greater than or equal to 95%, 96%, 97% or 98% identity, and further preferably greater than or equal to 99% identity. It is also possible to select an antibody with various effects equivalent to those of the above antibodies by combining amino acid sequences in which one or several amino acid residues are substituted, deleted, and/or added to the amino acid sequence of the heavy chain and/or light chain. The number of amino acid residues to be substituted, deleted and/or added is generally 10 amino acid residues or less, preferably 5 to 6 amino acid residues or less, more preferably 2 to 3 amino acid residues or less, and further preferably 1 amino acid residue.

Note that the heavy chain of an antibody produced by cultured mammalian cells is known to lack a carboxyl-terminal lysine residue (Journal of Chromatography A, 705: 129-134 (1995)). Also, the heavy chain of such an antibody is known to lack two carboxyl-terminal amino acid residues (glycine and lysine) and instead have an amidated proline residue at the carboxyl terminus (Analytical Biochemistry, 360: 75-83 (2007)). However, such deletion or modification in the heavy chain sequence does not influence the ability of the antibody to bind to the antigen, nor its effector functions (complement activation, antibody-dependent cytotoxic effects, etc.). Thus, the antibody used in the present invention also includes an antibody and a functional fragment of the antibody that has undergone a deletion or a modification. Accordingly, in one aspect of the present invention, the antibody is an antibody comprising a heavy chain in which one or several amino acid residues are deleted at a carboxyl terminus. In this context, "one or several amino acid residues" preferably means 1 to 10 amino acid residues, 1 to 9 amino acid residues, 1 to 8 amino acid residues, 1 to 7 amino acid residues, 1 to 6 amino acid residues, 1 to 5 amino acid residues, 1 to 4 amino acid residues, 1 to 3 amino acid residues, 1 or 2 amino acid residues, or 1 amino acid residue. In one aspect of the present invention, the antibody used in the present invention also includes a deletion variant comprising a heavy chain in which one or two amino acids are deleted at the carboxyl terminus, an

amidated form of the deletion variant (for example, a heavy chain having an amidated proline residue at the carboxyl-terminal site), and the like. However, the deletion variant at the carboxyl terminus of the antibody heavy chain used in the present invention is not limited to the above types as long as the deletion or the modification (amidation), etc. at the carboxyl terminus does not significantly influence the ability to bind to the antigen and the effector functions. Two heavy chains constituting the antibody used in the present invention may be heavy chains of any one type selected from the group consisting of a full-length heavy chain and the above-described deletion variants, or may be a combination of heavy chains of any two types selected therefrom. The quantitative ratio of each deletion variant may be influenced by the type of cultured mammalian cells producing the antibody used in the present invention, and culture conditions. Examples of the antibody used in the present invention can preferably include an antibody in which one carboxyl-terminal amino acid residue is deleted in both of the two heavy chains.

<2.2.2 Polynucleotide, etc.>

In one aspect, the present invention provides a polynucleotide encoding the amino acid sequence of the above-described antibody. In one aspect, the present invention provides, for example, a polynucleotide encoding the amino acid sequence of the antibody described in any one of [45] to [47] (also including [45-2], [46-2], and [47-2]).

In one aspect, the present invention provides an expression vector containing the polynucleotide. The expression vector is not particularly limited, and any vector, such as a plasmid or a virus, which enables the polynucleotide to be expressed can be used. Any known method can be used as a method for constructing such an expression vector. Examples thereof can include a method of using an animal cell expression vector containing a gene of the wild-type Fc region as a template, and introducing a desired effector-less mutation into the Fc region by PCR using KOD-Plus-Mutagenesis Kit (TOYOBO), or synthesizing a gene for an Fc region having a desired effector-less mutation, and integrating the gene into an animal cell expression vector by ligation reaction.

In one aspect, the present invention provides a host cell transformed with the expression vector. The host cell is not particularly limited, and any type of eukaryotic or prokaryotic cell that can express the polynucleotide contained in the expression vector can be used. In one aspect of the present invention, the host cell of the present invention is a eukaryotic cell. Examples thereof can include animal cells, plant cells, and eukaryotic microbes. Examples of the animal cells can particularly include mammalian cells, for example, COS cells which are monkey cells (Cell (1981) 23, p. 175-182, ATCC CRL-1650), mouse fibroblasts NIH3T3 (ATCC No. CRL-1658), a dihydrofolate reductase-deficient line of Chinese hamster ovarian cells (CHO cells, ATCC CCL-61) (Proc. Natl. Acad. Sci. U.S.A. (1980) 77, p. 4126-4220), FreeStyle 293F cells (Invitrogen), lymphocytes, and myeloma cells. In another aspect of the present invention, the host cell of the present invention is a prokaryotic cell. Examples thereof can include *E. coli* and

*Bacillus subtilis.*

In one aspect, the present invention provides a culture method comprising culturing the host cell. The culture method is not particularly limited as long as the host cell can express the antibody of interest. Its culture conditions or the like can be appropriately determined according to the type or the like of the host cell or the expression vector used.

In one aspect, the present invention provides a method for producing an antibody, the method comprising the steps of culturing the host cell, and collecting the antibody of interest from the resulting culture obtained in the culture step. The above "step of culturing the host cell" is not particularly limited as long as the host cell can express the antibody of interest. Its culture conditions or the like can be appropriately determined according to the type of the host cell or the expression vector used or the like. The step of "collecting the antibody of interest from the resulting culture obtained in the culture step" is not particularly limited and can be appropriately determined according to the type of the host cell or the expression vector used or the like, the characteristic of the antibody of interest, etc. Examples of the method for producing an antibody can include, but are not limited to, a production method described in the Vectibix 100 mg Intravenous Infusion Review Results Report (March 5, 2010, Review and Administration Division, Pharmaceutical and Food Safety Bureau) and a production method described in WO2018/212136, as described in Examples 13 and 14 herein.

<2.2.3 Glycan Remodeling of Antibody>

It has recently been reported that the heterogeneous glycans of antibodies can be remodeled by enzymatic reactions so as to introduce functionalized glycans uniformly (ACS Chem. Biol. 2012, 7, 110-122, ACS Med. Chem. Lett. 2016, 7, 1005-1008). Using this glycan remodeling technology, attempts have been made to synthesize a homogeneous ADC by introducing a drug(s) in a site-specific manner (Bioconjugate Chem. 2015, 26, 2233-2242; Angew. Chem. Int. Ed. 2016, 55, 2361-2367, US2016361436).

In the glycan remodeling, heterogeneous glycans added to a protein (e.g., an antibody) are cleaved off, using hydrolase, to leave only GlcNAc at each terminus, thereby producing a homogenous protein moiety with GlcNAc

(hereinafter, referred to as an "acceptor"). Subsequently, a given glycan prepared separately (hereinafter, referred to as a "donor") is provided, and the acceptor and the donor are linked together using transglycosidase. A homogeneous glycoprotein with a given glycan structure can thereby be synthesized.

In the present invention, a "glycan" refers to a structural unit of two or more monosaccharides bonded together via glycosidic bonds. Specific monosaccharides and glycans are occasionally abbreviated, for example, as "GlcNAc-", **"SG-",** and so on. In the case where any of these abbreviations is used in a structural formula, the abbreviation is shown with an intention that an oxygen atom or nitrogen atom involved in a glycosidic bond at the reducing terminal to another structural unit is not included in the abbreviation indicating the glycan, unless specifically defined.

In the present invention, a monosaccharide as a basic unit of a glycan is indicated for convenience so that in the ring structure, the position of a carbon atom bonding to an oxygen atom constituting the ring and directly bonding to a hydroxy group (or an oxygen atom involved in a glycosidic bond) is defined as the 1-position (the 2-position only for sialic acids), unless otherwise specified. The names of compounds in the Examples are each provided in view of the chemical structure as a whole, and that rule is not necessarily applied.

In the case where a glycan is indicated as an abbreviation (e.g., SG, MSG, GlcNAc) in the present invention, the abbreviation is intended, unless otherwise defined, to include carbon atoms ranging to the reducing terminal and not to include N or O involved in an N- or O-glycosidic bond.

An antibody-drug conjugate of the present invention is represented by the following formula:

$$\mathrm{Ab} - \left[ \mathrm{L} - \mathrm{D} \right]_{m^1}$$

wherein an antibody Ab or a functional fragment thereof is bound to L directly through a side chain of an amino acid residue (e.g., cysteine, lysine) or is bound to L through a glycan or remodeled glycan of Ab.

Glycans in Ab of the present invention are N-linked glycans or O-linked glycans, and preferably N-linked glycans.

N-linked glycans and O-linked glycans each bond to an amino acid side chain of an antibody via an N-glycosidic bond and an O-glycosidic bond, respectively.

The Ab in the present invention is IgG, and is preferably IgG1, IgG2 or IgG4, more preferably IgG1 or IgG4, particularly preferably IgG1.

IgG has a well-conserved N-linked glycan (hereinafter, referred to as "Asn297 glycan or N297 glycan") on an asparagine residue (hereinafter, referred to as "Asn297 or N297") at position 297 of the Fc region of the heavy chain, and the N-linked glycan is known to contribute to the activity and kinetics of the antibody molecule (Eon-Duval, A. et al, Biotechnol. Prog. 2012, 28, 608-622; Sanglier-Cianferani, S., Anal. Chem. 2013, 85, 715-736).

The amino acid sequence in the constant region of IgG is well-conserved, and each amino acid is specified by the EU Index numbering in Edelman et al. (Proc. Natl. Acad. Sci. U.S.A., 63, 78-85, (1969)). For example, Asn297, to which an N-linked glycan is added in the Fc region, corresponds to position 297 in the EU numbering, and even if the actual amino acid position has varied through fragmentation or loss of the region of the molecule, the amino acid can be uniquely identified by using the EU numbering.

The diagram below shows the case where an antibody-drug conjugate of the present invention bonds via the antibody or a N297 glycan of its functional fragment to L.

$$\mathrm{Ab} - \left[ (\mathrm{N297\ glycan}) - \left[ \mathrm{L} - \mathrm{D} \right]_{m^2} \right]_2$$

Note that an antibody having a remodeled glycan is called a glycan-remodeled antibody.

SGP (α2,6-SGP), an abbreviation for sialyl glycopeptide, is a representative N-linked glycopeptide. SGP can be separated/purified from the yolk of a hen egg, for example, by using a method described in WO 2011/027868. Purified products of SGP are commercially available from Tokyo Chemical Industry Co., Ltd., or FUSHIMI Pharmaceutical Co., Ltd. Herein, the glycan moiety of SGP is represented as SG, and the glycan deleting one GlcNAc at the reducing terminal of SG is represented as SG(10). SG(10) can be prepared by enzymatic hydrolysis of SGP (see the report by Umekawa et al. (Biochim. Biophys. Acta 2010, 1800, 1203-1209). SG(10) can also be purchased from Tokyo Chemical Industry Co., Ltd., or FUSHIMI Pharmaceutical Co., Ltd.

Herein, a glycan structure formed by deleting a sialic acid at a non-reducing terminal only in either one of the branched chains of β-Man in SG(10) is referred to as MSG(9), and a structure having sialic acid only in 1-3 glycan of the branched chains is referred to as MSG1, and a structure having a sialic acid only in the 1-6 glycan of the branched chains is referred to as MSG2.

The remodeled glycan used in an antibody-drug conjugate of the present invention is N297-(Fuc)SG, N297-(Fuc)MSG1, N297-(Fuc)MSG2, or a mixture of N297-(Fuc)MSG1 and N297-(Fuc)MSG2, preferably N297-(Fuc)SG, N297-(Fuc)MSG1, or N297-(Fuc)MSG2, and more preferably N297-(Fuc)SG or N297-(Fuc)MSG1.

N297-(Fuc)SG is represented by the following structural formula or sequence formula.

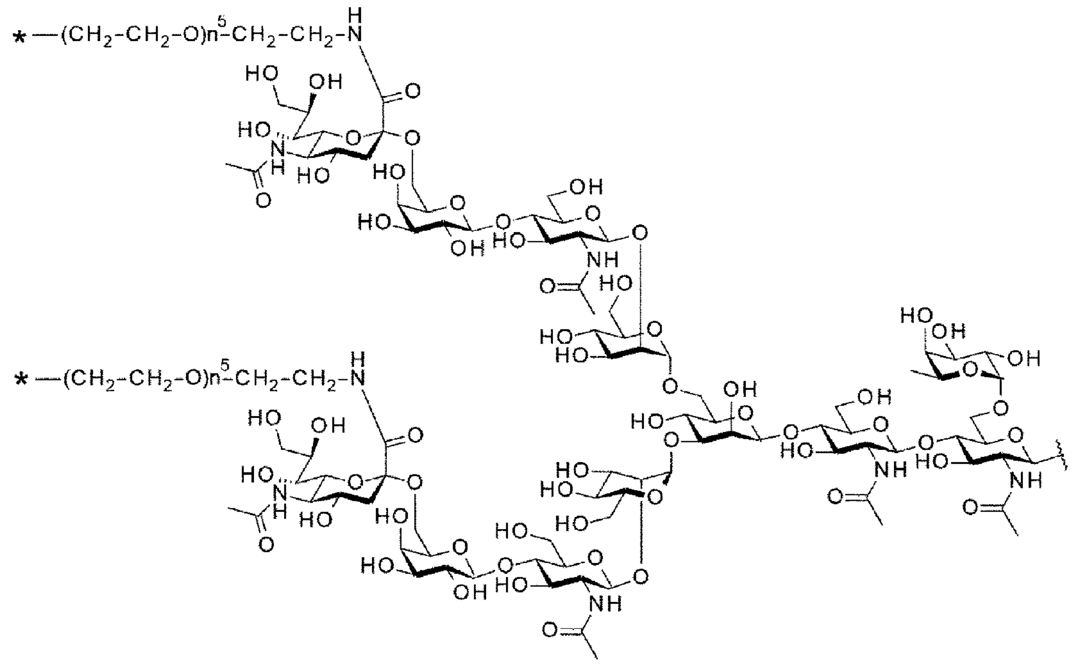

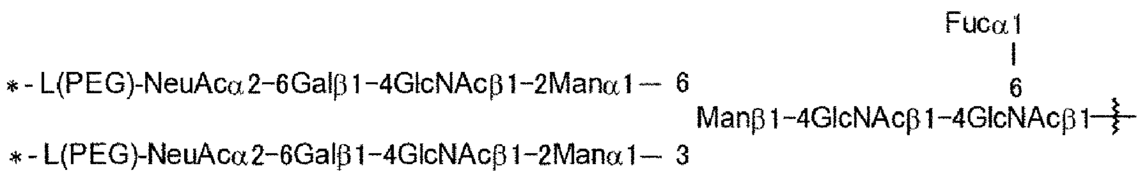

[N297-(Fuc)SG]

In the above formulae, the wavy line represents bonding to Asn297 of the antibody;

L (PEG) represents $-(CH_2-CH_2-O)n^5-CH_2-CH_2-NH-$ and the amino group at the right end of the L(PEG) represents bonding via an amide bond to a carboxyl group at the 2-position of a sialic acid at the non-reducing terminal of each of both the 1-3 and 1-6 glycan sides of the branched chains of β-Man in the N297 glycan; and
the asterisk represents bonding to the nitrogen atom at the 1- or 3-position of the 1,2,3-triazole ring of the linker L, in particular, Lb in the above linker L,
where $n^5$ is an integer from 2 to 10, preferably from 2 to 5.

N297-(Fuc)MSG1 is represented by the following structural formula or sequence formula.

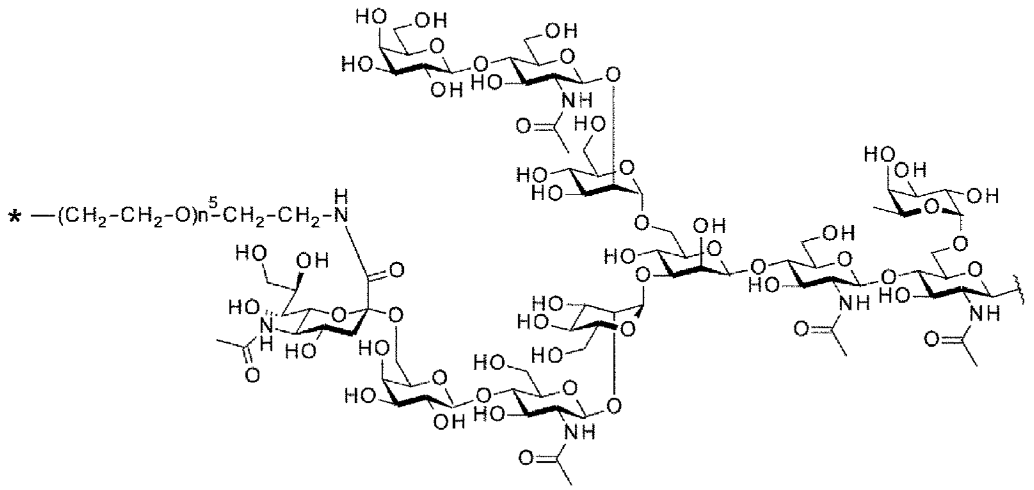

Fucα1

|

Galβ1-4GlcNAcβ1-2Manα1— 6 6

Manβ1-4GlcNAcβ1-4GlcNAcβ1—

* —L(PEG)-NeuAcα2-6Galβ1-4GlcNAcβ1-2Manα1— 3

[N297-(Fuc)MSG1]

In the above formulae, the wavy line represents bonding to Asn297 of the antibody;

L (PEG) represents - $(CH_2-CH_2-O)n^5-CH_2-CH_2-NH-$ and the amino group at the right end of the L(PEG) represents bonding via an amide bond to a carboxyl group at the 2-position of a sialic acid at the non-reducing terminal on the 1-3 glycan side of the branched chains of β-Man in the N297 glycan; and

the asterisk represents bonding to the nitrogen atom at the 1- or 3-position of the 1,2,3-triazole ring of the linker L, in particular, Lb in the above linker L,

where $n^5$ is an integer from 2 to 10, preferably from 2 to 5.

N297-(Fuc)MSG2 is represented by the following structural formula or sequence formula.

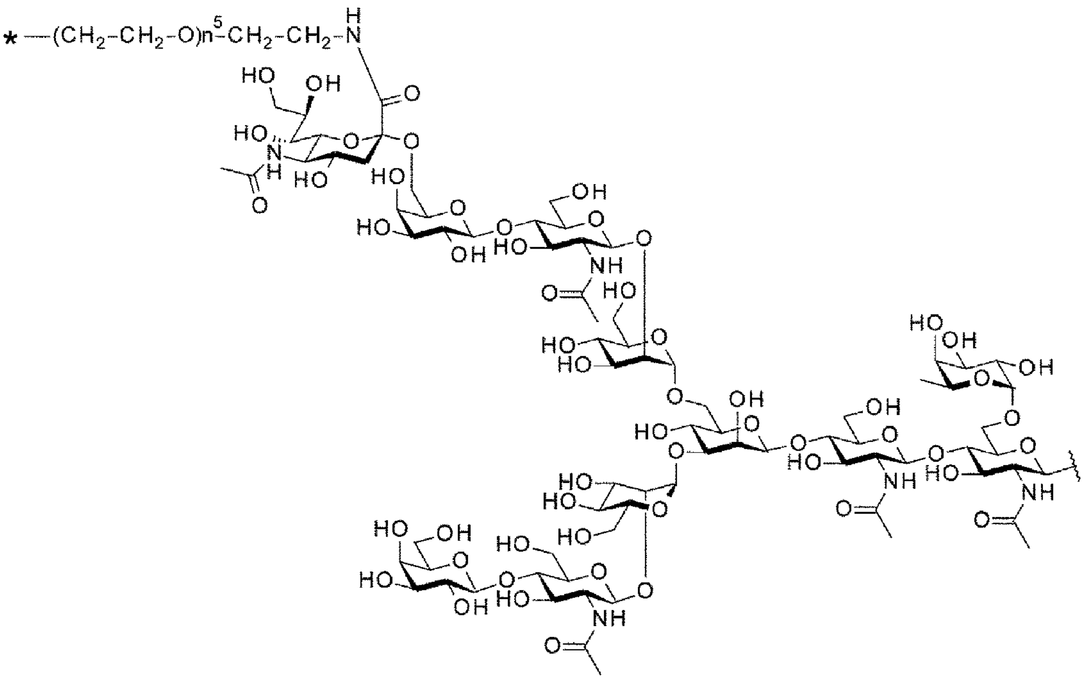

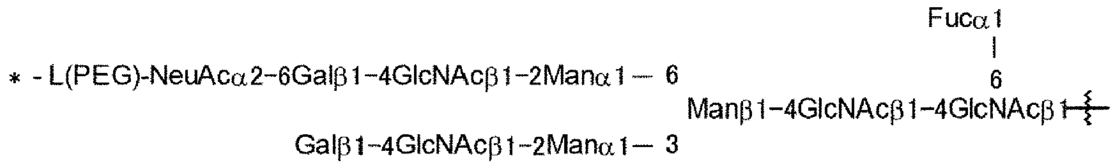

[N297-(Fuc)MSG2]

.

In the above formulae, the wavy line represents bonding to Asn297 of the antibody;

L (PEG) represents - $(CH_2-CH_2-O)n^5-CH_2-CH_2-NH-$ and the amino group at the right end of the L(PEG) represents bonding via an amide bond to a carboxyl group at the 2-position of a sialic acid at the non-reducing terminal on the 1-6 glycan side of the branched chains of β-Man in the N297 glycan; and
the asterisk represents bonding to the nitrogen atom at the 1- or 3-position of the 1,2,3-triazole ring of the linker L, in particular, Lb in the above linker L,
where $n^5$ is an integer from 2 to 10, preferably from 2 to 5.

The N297 glycan of the antibody in the antibody-drug conjugate of the present invention may be N297-(Fuc)SG. In this case, since one antibody molecule has two heavy chains, the antibody-drug conjugate is a molecule in which four linkers L and four drugs D are linked ($m^2 = 2$) (see Figure 1A). Specifically, the number of drugs D conjugated per heavy chain in the antibody-drug conjugate is 2.
The N297 glycan of the antibody in the antibody-drug conjugate of the present invention may be N297-(Fuc) MSG1 or N297-(Fuc)MSG2, or a mixture thereof. In this case, since one antibody molecule has two heavy chains, the antibody-drug conjugate is a molecule in which two linkers L and two drugs D are linked ($m^2 = 1$) (see Figure 1B). Specifically, the number of drugs D conjugated per heavy chain in the antibody-drug conjugate is 1.
The N297 glycan is preferably N297-(Fuc)SG or N297-(Fuc)MSG1 or N297-(Fuc)MSG2, more preferably N297-(Fuc)SG or N297-(Fuc)MSG1, and still more preferably N297-(Fuc)SG.
The N297 glycan of the antibody in the antibody-drug conjugate of the present invention may be N297-(Fuc)SG or N297-(Fuc)MSG1 or N297-(Fuc)MSG2. In this case, a highly homogeneous antibody-drug conjugate may be obtained.

<3. Production Methods>

The following describes representative methods for producing a CDN-derivative-containing antibody-drug conjugate according to the present invention or a production intermediate thereof. Note that hereinafter, the compound numbers designated in each reaction scheme are used to indicate each compound. Specifically, for instance, "compound of formula (1)" or "compound (1)" is used. Also, the same applies to compounds with numbers other than the above.
In the following Methods A to E, the substituent $L^1$ has the same meaning as above. The substituent $L^2$ represents a substituent selected from the following (i) or (ii):

(i) when bonded to L, $L^2$ represents -NHR', a hydroxy C1-C6 alkyl group, or an amino C1-C6 alkyl group, where R' is a hydrogen atom, a C1-C6 alkyl group, a C2-C6 alkenyl group, a C2-C6 alkynyl group, or a C3-C6 cycloalkyl group, wherein the C1-C6 alkyl group, C2-C6 alkenyl group, or C2-C6 alkynyl group is optionally substituted with 1 to 6 halogen atoms; or
(ii) when not bonded to L, $L^2$ represents a hydrogen atom or a halogen atom.

The substituent $W^1$ represents -NH- or a sulfur atom. The substituent $W^2$ represents -CH=. The substituents $Z^1$ to $Z^3$ together represent $-CH_2-CH_2-CH_2-$. The substituents $R^1$ to $R^3$, each independently, represent a hydrogen atom, a halogen atom, -OR', -OC(=O)R', $-N_3$, -NHR', -NR'R'', or -NHC(=O)R', where R' is as defined above and R'' represents a C1-C6 alkyl group, a C2-C6 alkenyl group, a C2-C6 alkynyl group, or a C3-C6 cycloalkyl group. The substituent $R^4$ represents a hydrogen atom. When $W^1$ is a nitrogen atom, the substituent $R^5$ represents a hydrogen atom; when $W^1$ is an oxygen atom, $R^5$ is absent. The substituents $R^a$, $R^c$, $R^e$, and $R^g$ each represent a side chain of natural α-amino acids. Examples of $R^a$, $R^c$, $R^e$, and $R^g$ include a methyl group, an isopropyl group, a sec-butyl group, an isobutyl group, or a benzyl group. $PRO^1$ represents a primary alcohol protecting group, and is preferably a 4,4'-dimethoxytrityl group, a 4-methoxytrityl group, and the like. $PRO^2$, $PRO^3$, $PRO^7$, and $PRO^8$ each denote a secondary alcohol protecting group. Preferable examples include a tert-butyldimethylsilyl group, triisopropylsilyloxymethyl group, benzoyl group, 2-nitrobenzyl group, and 4-methoxytetrahydropyran-4-yl group. $PRO^6$ represents a carboxylic acid protecting group, and is preferably a tert-butyl group, a benzyl group, and the like. $PRO^5$ and $PRO^9$ each denote an amine protecting group. $PRO^5$ is preferably a

tert-butyloxycarbonyl group, a 9-fluorenylmethyloxycarbonyl group, an allyloxycarbonyl group, a 2,2,2-trichloroethoxycarbonyl group, a benzyloxycarbonyl group, and the like. $PRO^9$ is preferably a 9-fluorenylmethyloxycarbonyl group or a 2-(trimethylsilyl)ethoxycarbonyl group. $PRO^4$ represents an alcohol or amine protecting group, and is preferably a tert-butyldimethylsilyl group, a benzoyl group, and the like in the case of alcohol and preferably a 2-(trimethylsilyl)ethoxycarbonyl group, an allyloxycarbonyl group, a tert-butyloxycarbonyl group, and the like in the case of amine. $Q^a$ represents an oxygen or sulfur atom, and $Q^b$ represents a hydroxy or thiol group. $Q^{a'}$ and $Q^{b'}$ each independently represent a negatively charged oxygen (O-) or sulfur (S-) atom. $R^x$ and $R^y$ each independently represent a halogen atom or -O-$PRO^2$. n represents an integer from 1 to 3.

Method A

A CDN derivative represented by (1) as used in an antibody-drug conjugate of the present invention can be produced according to Method A described below.

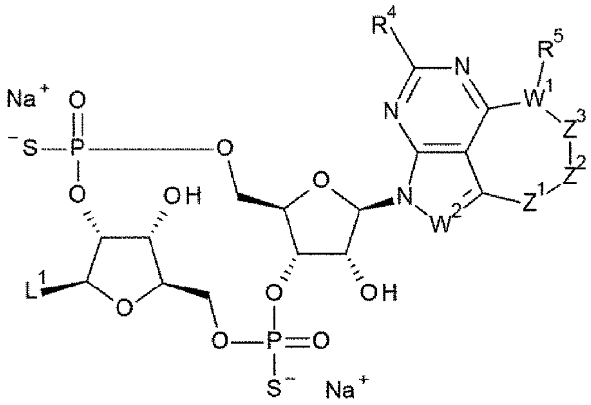

(1)

This synthetic scheme is a method for producing a compound represented by general formula (1). One-pot synthesis can be implemented from steps A-1 to A-5 in this production method according to the report of Gaffney and colleagues (Org. Lett. 2010, 12, 3269-3271).

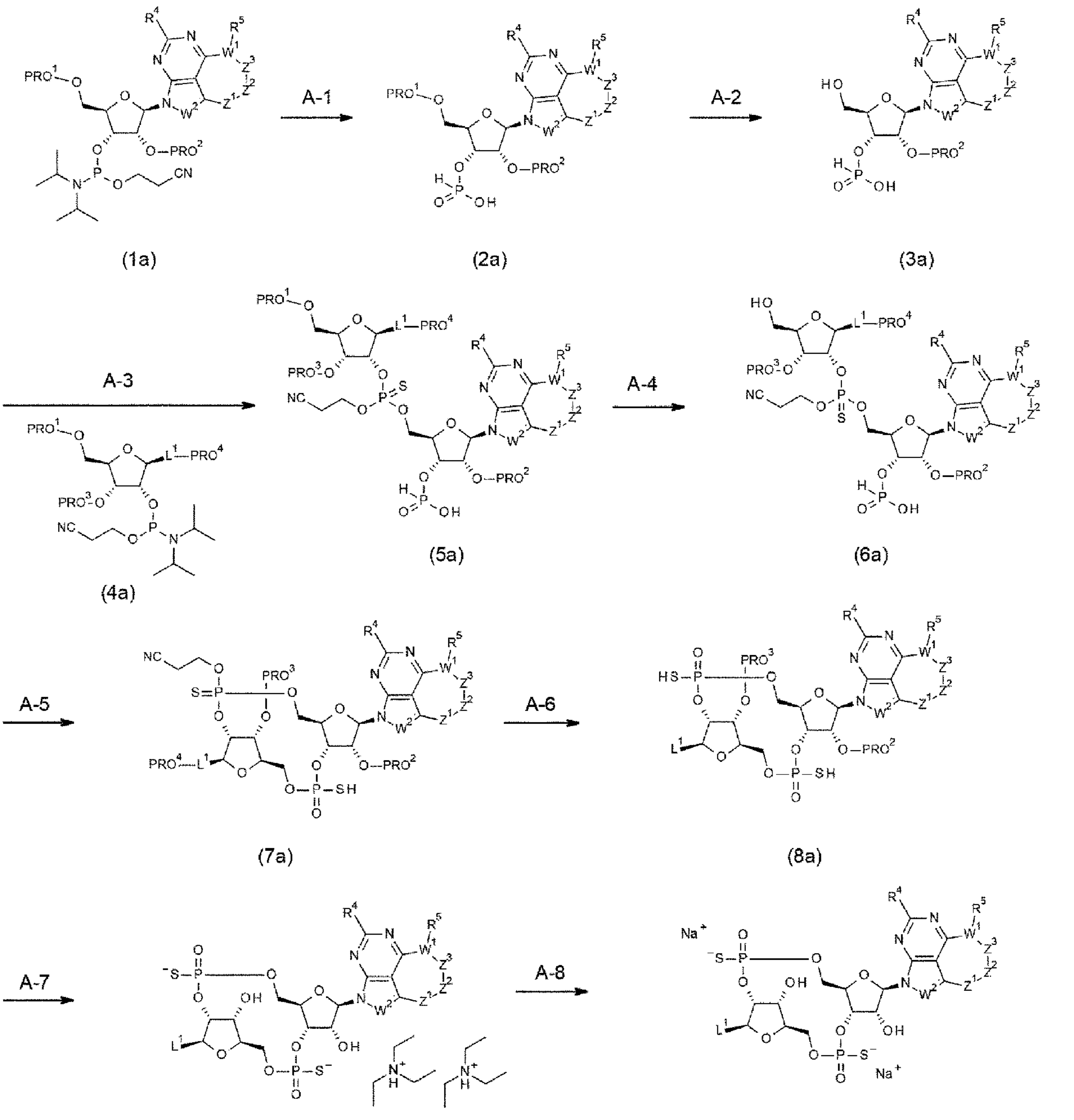


(1a) (2a) (3a) (4a) (5a) (6a) (7a) (8a) (9a) (1)

(Step A-1)

This step is a step of producing a compound of formula (2a) by using a known organic chemistry-based technique in order to subject a compound of formula (1a) to sequential hydrolysis and cyanoethyl group removal. The compound (1a) was hydrolyzed while treated with water and an acid (e.g., pyridine trifluoroacetate, 4,5-dicyanoimidazole, 1H-tetrazole) in a solvent (acetonitrile, tetrahydrofuran, N,N-dimethylformamide or a mixture of these solvents) at a temperature in the range of -10°C to the boiling point of the solvent used for the reaction, preferably from 15°C to 35°C. For 1 mole of compound (1a), 2 to excess moles and preferably 2 to 10 moles of water was used, and 1 mole to excess moles and preferably 1 mole to 5 moles of the acid was used. The reaction time is from 1 min to 3 h and preferably from 5 min to 30 min. Next, the cyanoethyl group was removed by adding a base (e.g., tert-butylamine) to the reaction mixture. For 1 mole of compound (1a), excess moles and preferably 30 to 50 moles of the base was used. The reaction time is from 5 min to 6 h and preferably from 15 min to 1 h. The reaction mixture was concentrated under reduced pressure to give a crude compound (2a). The crude compound (2a) can be sent to the next step without purification.

(Step A-2)

This step is a step of producing a compound of formula (3a) by using a known organic chemistry-based technique to remove a hydroxy group-protecting group from the compound of formula (2a). Before the start of the reaction in this step, the crude form of formula (2a) was dried while azeotroped once to three times with acetonitrile, if necessary. In the case where $PRO^1$ is a 4,4'- dimethoxytrityl group, the compound (2a) was placed in a solvent (e.g., dichloromethane, chloroform, dichloroethane) at a temperature in the range of -10°C to the boiling point of the solvent used for the reaction, preferably from 15°C to 35°C, and treated with water and an acid (e.g., dichloroacetic acid, trifluoroacetic acid) to remove the 4,4'-dimethoxytrityl group. For 1 mole of compound (2a), excess moles and preferably 10 to 20 moles of water was used; and the acid was diluted with the solvent used in the reaction to 1% to 50% (v/v) and preferably 5% to 10% (v/v), and excess moles and preferably 5 moles to 15 moles of the diluted solution was used. The reaction time is from 1 min to 3 h and preferably from 5 min to 30 min. Pyridine was added to the reaction mixture to stop the reaction. The amount of pyridine was an amount at which the acid can be sufficiently neutralized, and 2 moles to 10 moles of pyridine per mole of acid was preferably used. The reaction mixture was concentrated under reduced pressure to give a crude compound (3a). The crude compound (3a) was azeotroped three to five times using dehydrated acetonitrile. At the last azeotropic distillation, acetonitrile was left to prepare 0.01 M to 1 M compound (3a)-containing acetonitrile solution. The resulting acetonitrile solution was used directly in the next step.

(Step A-3)

This step is a step of producing a compound of formula (5a) by using a known organic chemistry-based technique to subject the compound of formula (3a) to a coupling reaction with a compound of formula (4a) and then subject the resulting coupling product to sulfidation in a sequential manner. Before the start of the reaction in this step, the compound (4a) was azeotroped three to five times using dehydrated acetonitrile. At the last azeotropic distillation, acetonitrile was left to prepare 0.01 M to 1 M compound (4a)-containing acetonitrile solution. A drying agent (Molecular Sieves 3A or Molecular Sieves 4A in a powder or pellet form) was added to the solution, and the resulting solution was stored under a nitrogen or argon atmosphere until use. The coupling reaction was carried out by adding the compound (4a)-containing acetonitrile solution, which had been dried by azeotropic distillation, to the compound (3a)-containing acetonitrile solution at a temperature of 5°C to 35°C. The reaction time is from 1 min to 24 h and preferably from 5 min to 6 h. Next, the reaction mixture was mixed with a sulfurizing agent (e.g., N,N-dimethyl-N'-(3-sulfaniliden-3H-1,2,4-dithiazol-5-yl)methane imidamide, 3H-1,2-benzodithiol-3-one). In this way, the sulfidation was carried out. For 1 mole of compound (3a), 1 to 5 moles and preferably 1 to 2 moles of the sulfurizing agent was used. The reaction time is from 5 min to 24 h and preferably from 30 min to 6 h. The reaction mixture was concentrated under reduced pressure to give a crude compound (5a). The resulting crude compound (5a) was used directly in the next step.

(Step A-4)

This step is a step of producing a compound of formula (6a) by using a known organic chemistry-based technique to remove a hydroxy group-protecting group from the compound of formula (5a). In the case where $PRO^1$ is a 4,4'-dimethoxytrityl group, the 4,4-dimethoxytrityl group was removed by treating the compound (5a) with water and an acid (e.g., dichloroacetic acid, trifluoroacetic acid) in a solvent (e.g., dichloromethane, chloroform, dichloroethane) at a temperature in the range of - 10°C to the boiling point of the solvent used for the reaction, preferably from 15°C to 35°C. For 1 mole of compound (5a), excess moles and preferably 10 to 20 moles of water was used; and the acid was diluted with the solvent used in the reaction to 1% to 50% (v/v) and preferably 5% to 10% (v/v), and excess moles and preferably 5 moles to 15 moles of the diluted solution was used. The reaction time is from 1 min to 3 h and preferably from 5 min to 30 min. Pyridine was added to the reaction mixture to stop the reaction. The amount of pyridine was an amount at which the acid can be sufficiently neutralized, and 10 moles to 200 moles of pyridine per mole of acid was preferably used. The reaction mixture was concentrated under reduced pressure to give a crude compound (6a). The resulting crude compound (6a) was used directly in the next step.

(Step A-5)

This step is a step of producing a compound of formula (7a) by using a known organic chemistry-based technique to subject the compound of formula (6a) to cyclization and sulfurization sequentially. The compound (6a) was dissolved in pyridine and then concentrated under reduced pressure to prepare a 0.01 M to 0.5 M pyridine solution. The cyclization was carried out by adding a dehydration condensation agent (e.g., 2-chloro-5,5-dimethyl-1,3,2$\lambda^5$-dioxaphosphinan-2-one) to the pyridine solution at a temperature of 5°C to 35°C. For 1 mole of compound (6a), 1 to excess moles and preferably 3 moles to 5 moles of the dehydration condensation agent was used. The reaction time is from 1 min to 6 h and preferably

from 5 min to 1 h. Next, water and a sulfurizing agent (e.g., 3H-1,2-benzodithiol-3-one, N,N-dimethyl-N'-(3-sulfanilidene-3H-1,2,4-dithiazol-5-yl)methane imidamide) were added to the reaction mixture to carry out the sulfidation. For 1 mole of compound (6a), excess moles and preferably 30 to 50 moles of water was used, and 1 mole to 5 moles and preferably 1 mole to 2 moles of the sulfurizing agent was used. The reaction time is from 5 min to 12 h and preferably from 30 min to 3 h. After the reaction mixture was added to aqueous sodium bicarbonate (0.1 M to 1 M), the mixture was stirred for 15 min to 24 h and the reaction was then stopped. The reaction mixture was extracted with an organic solvent (ethyl acetate, diethyl ether, toluene, or a mixture of these solvents) one to five times. After that, the extracts were combined, and dried over an anhydrous salt (anhydrous sodium sulfate or anhydrous magnesium sulfate). The drying agent was filtered off, and the filtrate was concentrated under reduced pressure. The resulting residue was purified by silica gel column chromatography [e.g., dichloromethane/methanol, ethyl acetate/methanol, hexane/ethyl acetate], C18 silica gel column chromatography [buffer solution/acetonitrile], or a combination of these procedures to yield a compound (7a) as a mixture of two or more diastereomers or as two or more separate diastereomers. In this step, two diastereomers are frequently obtained. However, depending on the starting material (1a) and (4a), one or two additional diastereomers may be obtained. Even if the resulting compound (7a) is a mixture of multiple diastereomers, the compound can be sent to the next step without further purification.

(Step A-6)

This step is a step of producing a compound of formula (8a) by using a known organic chemistry-based technique to simultaneously remove a cyanoethyl group and all the acyl-based protecting groups from the compound of formula (7a). This step was carried out in an autoclave or in a shielded tube, if necessary. In the case where $PRO^4$ was a benzoyl group, the cyanoethyl and benzoyl groups were removed by treating the compound (7a) with 28% (v/v) ammonia water in a solvent (methanol, ethanol, tetrahydrofuran, or a mixture of these solvents) at a temperature in the range of 5°C to the boiling point of the solvent used in the reaction. For 1 mole of compound (7a), excess moles and preferably 300 moles to 3000 moles of ammonia was used. The reaction time is from 30 min to 96 h and preferably from 2 h to 48 h. The reaction mixture was optionally concentrated. The residue was purified by preparative HPLC (e.g., buffer solution/acetonitrile, buffer solution/methanol), C18 silica gel column chromatography (e.g., buffer solution/acetonitrile, buffer solution/methanol), or a combination of these procedures to obtain the compound (8a). Even if the resulting compound (8a) is a mixture of diastereomers, the compound can be sent to the next step without further purification. Meanwhile, the compound may be sent directly to the next step without purification in this step.

(Step A-7)

This step is a step of producing a compound of formula (9a) by using a known organic chemistry-based technique to simultaneously remove all the silyl-based protecting groups from the compound of formula (8a). In the case where $PRO^2$ and $PRO^3$ are tert-butyldimethylsilyl groups, the tert-butyldimethylsilyl groups were removed by directly treating the compound (8a) with triethylamine trihydrofluoride at a temperature of 5°C to 100°C, preferably from 35°C to 60°C. For 1 mole of compound (8a), excess moles and preferably 100 to 200 moles of triethylamine trihydrofluoride was used. The reaction time is from 30 min to 24 h and preferably from 2 h to 12 h. The reaction mixture was cooled to room temperature. Then, an ice-cold mixture of 1 M aqueous triethylammonium bicarbonate and triethylamine at 3:1 to 10:1 (v/v) was gradually poured into the reaction mixture to stop the reaction. If necessary, the reaction mixture may be poured into an ice-cold mixture of 1 M aqueous triethylammonium bicarbonate and triethylamine. In this case, the reaction vessel was washed with acetonitrile and water. Triethylamine should be used in a quantity sufficient to change the reaction mixture property to be weakly basic. Preferably, for 1 mole of triethylamine trihydrofluoride, about 2 moles of triethylamine may be used. The organic solvent component of the reaction mixture was distilled off under reduced pressure. The residual aqueous solution was subjected to purification by preparative HPLC (e.g., buffer solution/acetonitrile, buffer solution/-methanol), C18 silica gel column chromatography (e.g., buffer solution/acetonitrile, buffer solution/methanol), or a combination of these procedures to obtain the compound (9a) as a single diastereomer.

(Step A-8)

This step is a step of producing a compound of formula (1) by using a known organic chemistry-based technique to subject the compound of formula (9a) to ion exchange. Cation exchange resin (BT AG (registered trademark); 50W-X2 resin; 100-200 mesh; hydrogen type) was suspended in pure water and packed into an empty column cartridge. The amount of the cation exchange resin used was 10 to 50 times the amount of compound (9a) by weight. After pure water in excess was allowed to flow down, three column volumes of 1M aqueous sodium hydroxide was allowed to flow down, followed by six column volumes of pure water. The compound (9a) was dissolved in about three column volumes of pure water and loaded into the column. If the compound is not readily soluble in pure water, a mixture with a small amount of

organic solvent (e.g., acetonitrile, methanol) may be used. The solution allowed to flow down was fractionated. Then, additional six column volumes of pure water or the like was used for elution, and the corresponding fractions were collected. Fractions containing the target product were combined and lyophilized to give the compound (1) as a single diastereomer.

Method A'

A CDN derivative represented by (1') as used in an antibody-drug conjugate of the present invention can be produced according to Method A' described below.

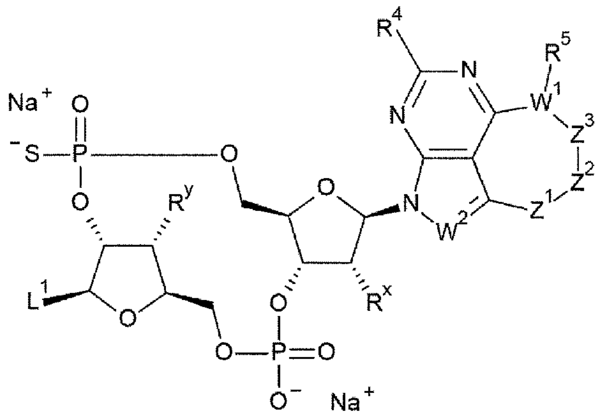

(1')

This synthetic scheme is a method for producing a compound represented by general formula (1') wherein part of Method A is modified. Specifically, the compound of general formula (1') can be produced by changing step A-5 of Method A to step A'-5 specified below. Meanwhile, if the substituents $R^x$ and $R^y$ are each a halogen atom, step A-7 may be omitted.

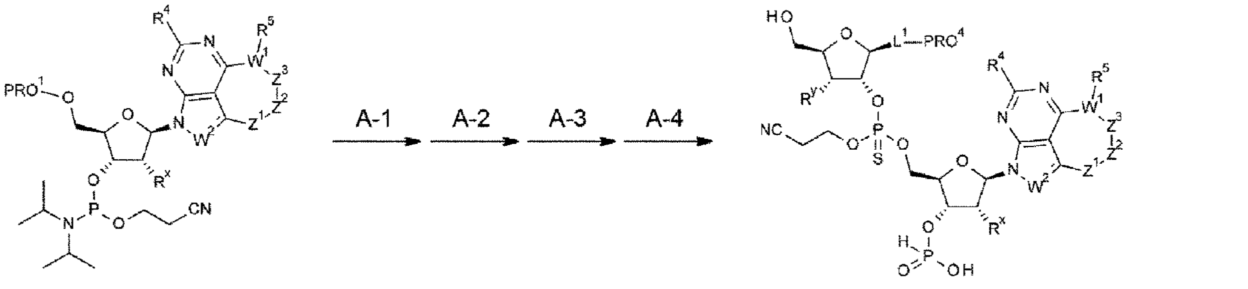


(1a') (6a')

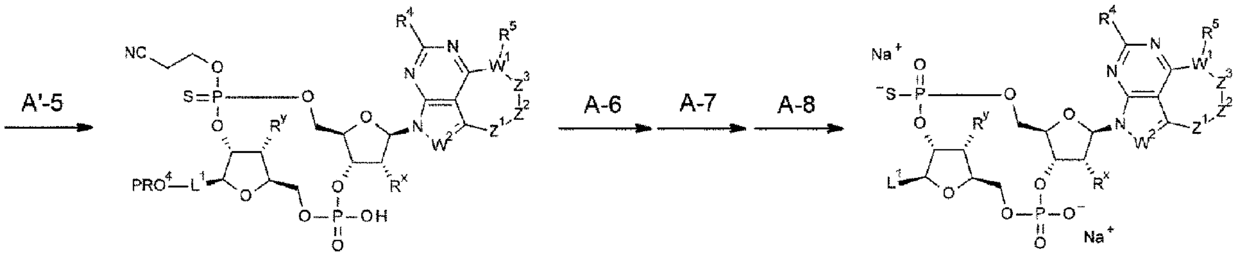


(7a') (1')

(Step A'-5)

This step is a step of producing a compound of formula (7a') by using a known organic chemistry-based technique to subject the compound of formula (6a') to cyclization and oxidation sequentially. The compound (6a') was dissolved in

pyridine and then concentrated under reduced pressure to prepare a 0.01 M to 0.5 M pyridine solution. The cyclization was carried out by adding a dehydration condensation agent (e.g., 2-chloro-5,5-dimethyl-1,3,2$\lambda^5$-dioxaphosphinan-2-one) to the pyridine solution at a temperature of 5°C to 35°C. For 1 mole of compound (6a'), 1 mole to excess moles and preferably 3 moles to 5 moles of the dehydration condensation agent was used. The reaction time is from 1 min to 6 h and preferably from 5 min to 1 h. Next, the oxidation was carried out by adding water and an oxidizing agent (e.g., iodine) to the reaction mixture. For 1 mole of compound (6a'), 0 mole to excess moles and preferably 30 moles to 50 moles of water was used, and 2 moles to 10 moles and preferably 3 moles to 5 moles of the oxidizing agent was used. The reaction time is from 5 min to 12 h and preferably from 30 min to 3 h. After the reaction mixture was added to aqueous sodium bicarbonate (0.1 M to 1 M), the mixture was stirred for 15 min to 24 h and the reaction was then stopped. The reaction mixture was extracted with an organic solvent (ethyl acetate, diethyl ether, toluene, or a mixture of these solvents) one to five times. After that, the extracts were combined, and dried over an anhydrous salt (anhydrous sodium sulfate or anhydrous magnesium sulfate). The drying agent was filtered off, and the filtrate was concentrated under reduced pressure. The resulting residue was purified by silica gel column chromatography (e.g., dichloromethane/methanol, ethyl acetate/methanol, hexane/ethyl acetate), C18 silica gel column chromatography (buffer solution/acetonitrile), or a combination of these procedures to give a compound (7a')

Method A''

A CDN derivative represented by (1'') as used in an antibody-drug conjugate of the present invention can be produced according to Method A'' described below.

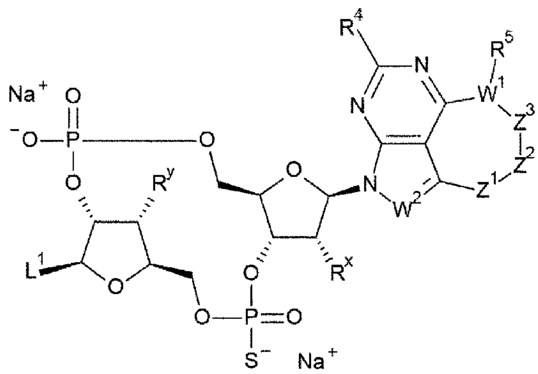

(1'')

This synthetic scheme is a method for producing a compound represented by general formula (1'') wherein part of Method A is modified. Specifically, the compound of general formula (1'') can be produced by changing step A-3 of Method A to step A''-3 specified below. Meanwhile, if the substituents $R^x$ and $R^y$ are each a halogen atom, step A-7 may be omitted.

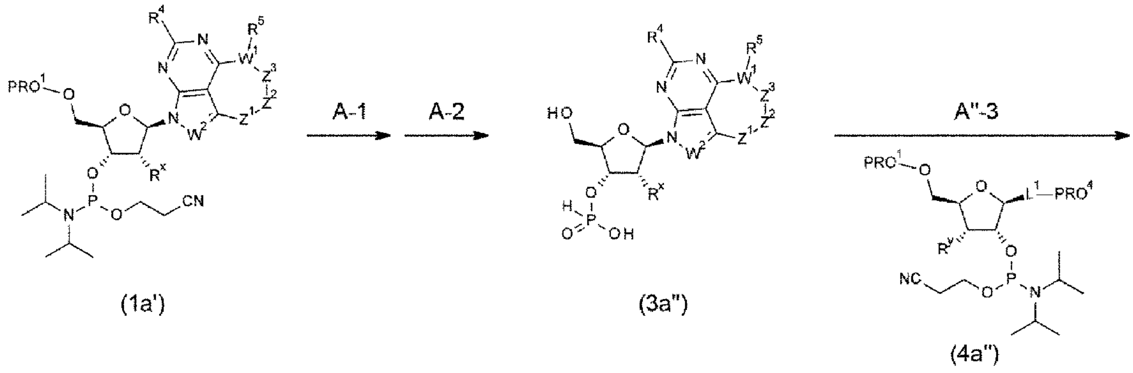


(1a') (3a") (4a")

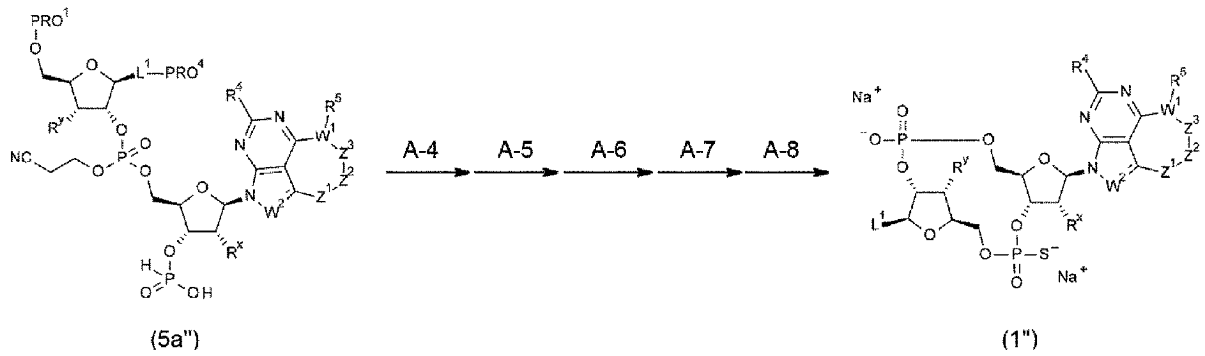


(5a") (1")

(Step A"-3)

This step is a step of producing a compound of formula (5a'') by using a known organic chemistry-based technique to subject the compound of formula (3a'') to a coupling reaction with a compound of formula (4a'') and then subject the resulting coupling product to oxidation in a sequential manner. Before the start of the reaction in this step, the compound (4a'') was azeotroped three to five times using dehydrated acetonitrile. At the last azeotropic distillation, acetonitrile was left to prepare 0.01 M to 1 M compound (4a'')-containing acetonitrile solution. A drying agent (Molecular Sieves 3A or Molecular Sieves 4A in a powder or pellet form) was added to the solution, and the resulting solution was stored under a nitrogen or argon atmosphere until use. The coupling reaction was carried out by adding the compound (4a'')-containing acetonitrile solution, which had been dried by azeotropic distillation, to the compound (3a'')-containing acetonitrile solution at a temperature of 5°C to 35°C. The reaction time is from 1 min to 24 h and preferably from 5 min to 6 h. Next, the oxidation was carried out by adding an oxidizing agent (e.g., tert-butyl hydroperoxide) to the reaction mixture. For 1 mole of compound (3a''), 1 mole to 5 moles and preferably 2 moles to 3 moles of the oxidizing agent was used. The reaction time is from 5 min to 24 h and preferably from 30 min to 6 h. Saturated aqueous sodium thiosulfate was added to the reaction mixture, the mixture was stirred for 10 min to 12 h, and the reaction was then stopped. The reaction mixture was extracted with an organic solvent (e.g., a mixed solvent of dichloromethane and methanol) one to five times. After that, the extracts were combined, and dried over an anhydrous salt (anhydrous sodium sulfate or anhydrous magnesium sulfate). The drying agent was filtered off, and the reaction mixture was concentrated under reduced pressure to give a crude compound (5a"). The resulting crude compound (5a") was used directly in the next step.

Method A'''

A CDN derivative represented by (1''') as used in an antibody-drug conjugate of the present invention can be produced according to Method A''' described below.

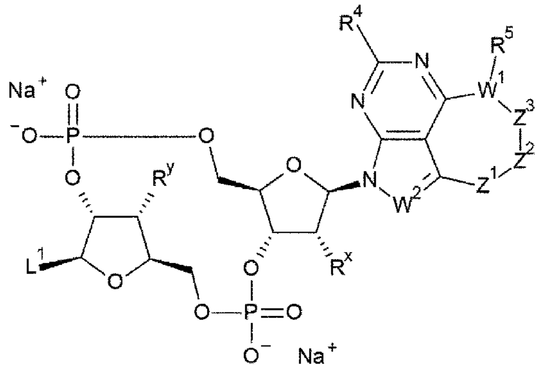

(1''')

This synthetic scheme is a method for producing a compound represented by general formula (1‴) wherein part of Method A is modified. Specifically, the compound of general formula (1‴) can be produced by changing step A-3 of Method A to step A"-3 and step A-5 of Method A to step A'-5. Meanwhile, if the substituents $R^x$ and $R^y$ are each a halogen atom, step A-7 may be omitted.

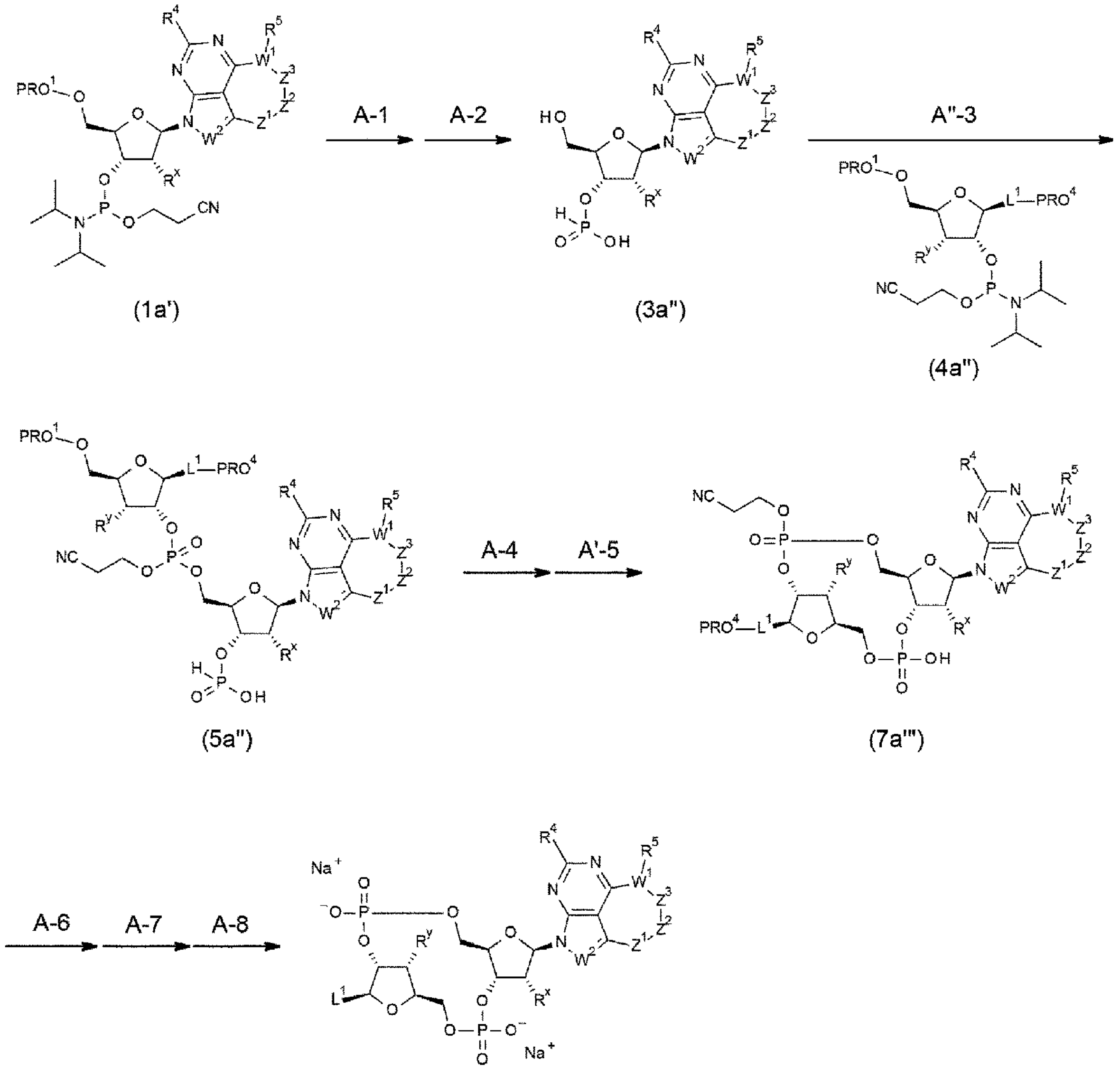


(1a') (3a") (4a") (5a") (7a''') (1''')

Method B: Conjugation Precursor (Glycan Conjugation)

A conjugation precursor represented by (2) as used in an antibody-drug conjugate of the present invention can be produced according to Method B described below.

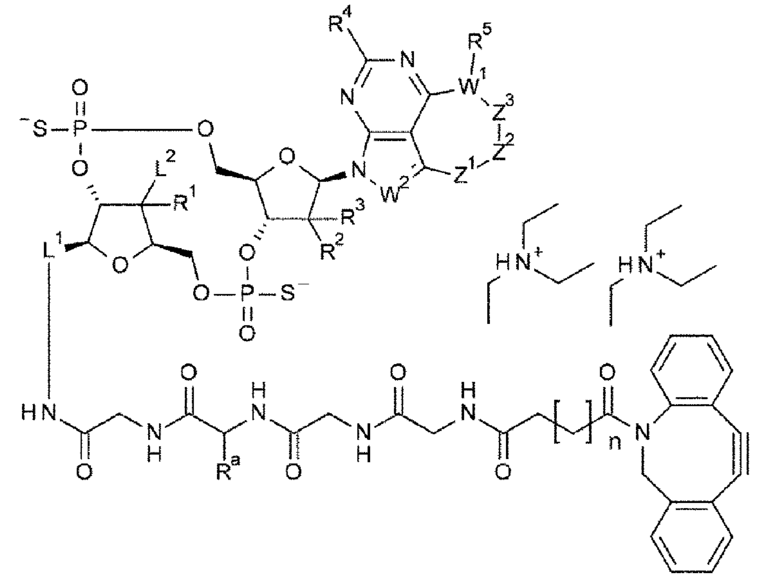

(2)

This synthetic scheme is a method for producing a conjugation precursor (2) in the case where $-NH_2$ is substituted at a given position of $L^1$.

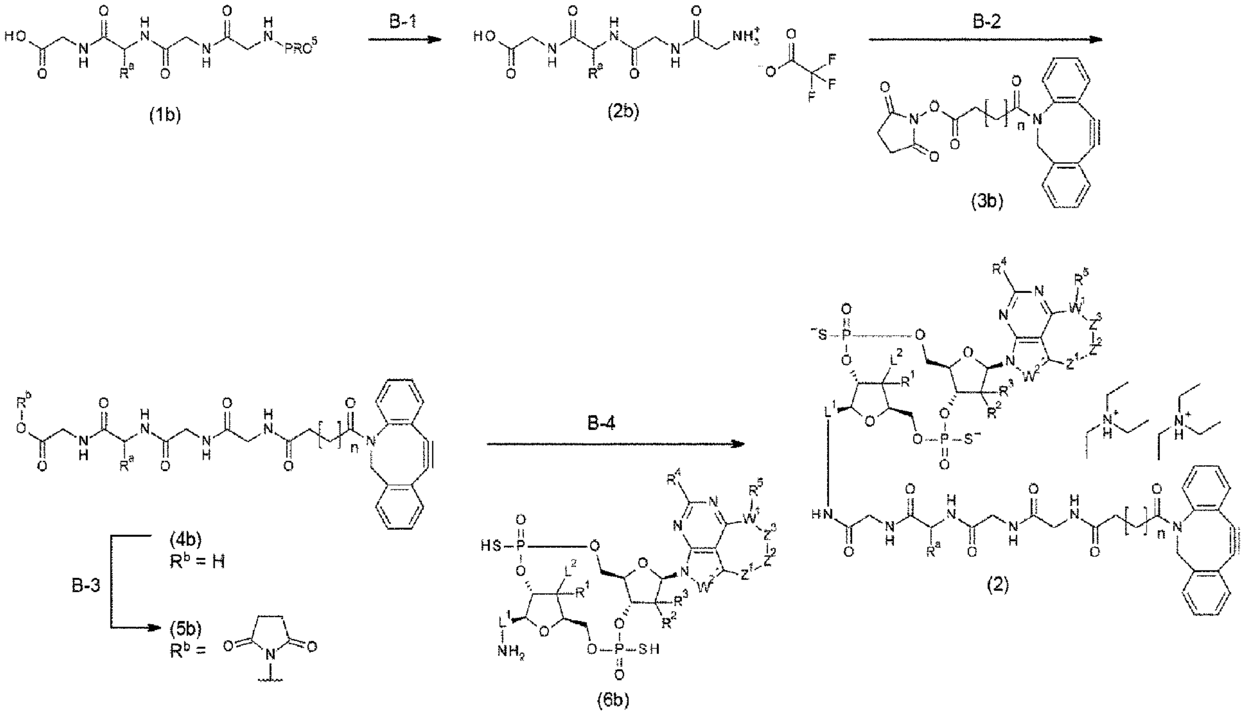


(Step B-1)

This step is a step of producing a compound of formula (2b) by using a known organic chemistry-based technique to remove a protecting group from a compound of formula (1b). In the case where $PRO^5$ was a tert-butyloxycarbonyl group, the protecting group was removed by treating the compound (1b) with trifluoroacetic acid in a solvent (dichloromethane,

dioxane, acetonitrile, ethyl acetate, tetrahydrofuran, or a mixture of these solvents) at a temperature in the range of -10°C to the boiling point of the solvent used in the reaction, preferably from 15°C to 35°C. For 1 mole of compound (1b), excess moles and preferably 20 moles to 50 moles of trifluoroacetic acid was used. The reaction time is from 5 min to 24 h and preferably from 30 min to 6 h. The reaction mixture was concentrated under reduced pressure, suspended in toluene, and then re-concentrated under reduced pressure. This procedure was repeated two to five times. A solvent (diethyl ether, diisopropyl ether, hexane, dichloromethane, ethyl acetate, or a mixture of these solvents) was added to form a slurry. The resulting solid was then filtered off to give a crude compound (2b). The crude compound (2b) was sent to the next step without further purification.

(Step B-2)

This step is a step of producing a compound of formula (4b) by using a known organic chemistry-based technique to subject the compound of formula (2b) to amidation with a compound of formula (3b). The amidation was carried out by reacting the compound (2b) with a base (e.g., triethylamine, N,N-diisopropylethylamine) and the compound (3b) in a solvent (e.g., N,N-dimethylformamide, N-methylpyrrolidone, N,N-dimethylacetamide, acetonitrile) at a temperature of 5°C to 35°C. For 1 mole of compound (2b), 1 mole to 5 moles of the base was used; and 0.5 mole to 1.5 moles of the compound (3b) was used. The reaction time is from 10 min to 72 h and preferably from 1 h to 24 h. The reaction mixture was poured into a two-layer mixture: an organic solvent (dichloromethane, chloroform, ethyl acetate, methanol, or a mixture of these solvents) and water or acidic aqueous solution (e.g., 0.1 to 1 M hydrochloric acid, aqueous citric acid solution), and extracted with the organic solvent one to five times. The resulting extracts were combined, washed with brine, and dried over an anhydrous salt (anhydrous sodium sulfate or anhydrous magnesium sulfate). The drying agent was filtered off, and the filtrate was concentrated under reduced pressure. Note that the above liquid separation procedure may be omitted, and the reaction mixture can be concentrated as it is under reduced pressure and sent to the next step of silica gel column purification. The resulting residue was purified by silica gel column chromatography (e.g., dichloromethane/methanol, ethyl acetate/methanol) to yield a compound (4b). If necessary, the resulting compound (4b) may be dissolved in a good solvent (ethyl acetate, acetonitrile, dichloromethane, methanol, or a mixture of these solvents); a poor solvent (e.g., diethyl ether, diisopropyl ether, hexane) may then be added to re-precipitate the compound; and the resulting solid may be filtered to increase the purity.

(Step B-3)

This step is a step of producing a compound of formula (5b) by using a known organic chemistry-based technique to subject the compound of formula (4b) to esterification. The esterification was carried out by reacting the compound (4b) with N-hydroxysuccinimide and a condensing agent (e.g., 1-ethyl-3-(3-dimethylaminopropyl)carbodiimide hydrochloride) in a solvent (e.g., N,N-dimethylformamide, N,N-dimethylacetamide, N-methylpyrrolidone, acetonitrile) at a temperature of 5°C to 35°C. For 1 mole of compound (4b), 1 mole to 3 moles of each of N-hydroxysuccinimide and the condensing agent was used. The reaction time is from 30 min to 72 h and preferably from 2 h to 24 h. The reaction mixture was diluted with an organic solvent (dichloromethane, chloroform, ethyl acetate, or a mixture of these solvents) and washed three to five times with ice water. The resulting organic layer was dried over an anhydrous salt (anhydrous sodium sulfate or anhydrous magnesium sulfate). The drying agent was filtered off, and the filtrate was concentrated under reduced pressure to give a crude compound (5b). If necessary, the resulting compound (5b) may be purified by C18 silica gel column chromatography (acetonitrile only). Also, the resulting compound (5b) may be dissolved in a good solvent (ethyl acetate, acetonitrile, dichloromethane, or a mixture of these solvents); a poor solvent (e.g., diethyl ether, diisopropyl ether, hexane) may then be added to re-precipitate the compound; and the resulting solid may be filtered to increase the purity.

(Step B-4)

This step is a step of producing a compound of formula (2) by using a known organic chemistry-based technique to subject the compound of formula (5b) to condensation with a compound of formula (6b). The condensation was carried out by reacting the compound (6b) with a base (e.g., triethylamine, N,N-diisopropylethylamine) and the compound (5b) in a solvent (e.g., N,N-dimethylformamide, N,N-dimethylacetamide, N-methylpyrrolidone, acetonitrile) at a temperature of -10°C to 100°C, preferably from 15°C to 35°C. For 1 mole of compound (6b), 2 moles to 5 moles of the base was used; and 1 mole to 2 moles of the compound (5b) was used. The reaction time is from 5 min to 24 h and preferably from 1 h to 6 h. Benzylamine was added to the reaction mixture to stop the reaction. For 1 mole of compound (6b), 4 moles to 10 moles of benzylamine was used. If necessary, the reaction mixture was partially concentrated under reduced pressure. The residual solution was subjected to purification by preparative HPLC (e.g., buffer solution/acetonitrile, buffer solution/-methanol), C18 silica gel column chromatography (e.g., buffer solution/acetonitrile, buffer solution/methanol), or a combination of these procedures to obtain the compound (2).

Method B': Conjugation Precursor (Cysteine Conjugation)

A conjugation precursor represented by (2') as used in an antibody-drug conjugate of the present invention can be produced according to Method B' described below.

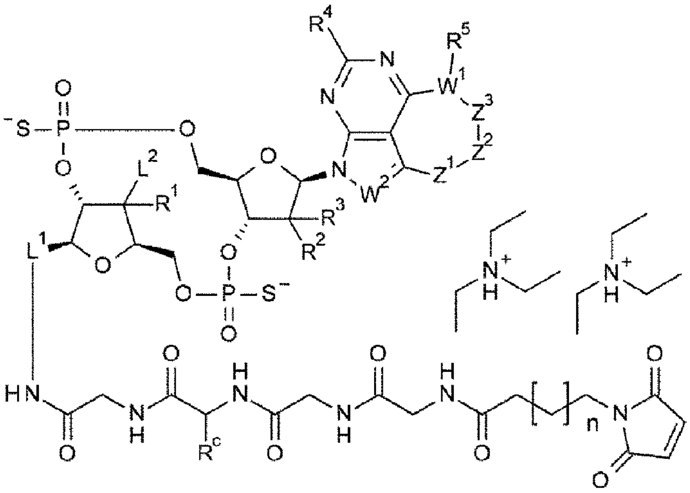

(2')

This synthetic scheme is a method for producing a conjugation precursor (2') in the case where $-NH_2$ is substituted at a given position of $L^1$.

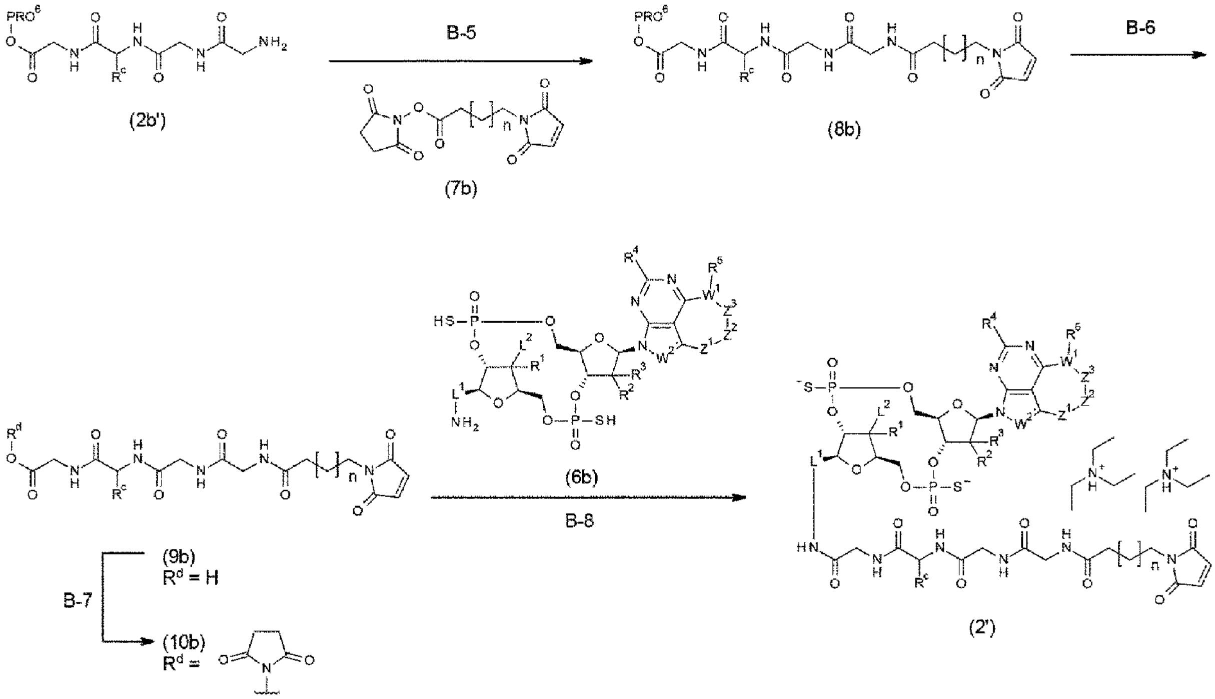


(2b') (7b) (8b) (6b) (9b) (10b) (2')

(Step B-5)

This step is a step of producing a compound of formula (8b) by using a known organic chemistry-based technique to subject the compound of formula (2b') to amidation with a compound of formula (7b). The compound (8b) was obtained according to the procedure described in step B-2 of Method **B,** except that no base was used.

(Step B-6)

This step is a step of producing a compound of formula (9b) by using a known organic chemistry-based technique to remove a protecting group from the compound of formula (8b). In the case where $PRO^6$ is a tert-butyl group, the compound (9b) was obtained according to the procedure described in step B-1 of Method B, except that silica gel column chromatography (dichloromethane/methanol) was used for purification.

(Step B-7)

This step is a step of producing a compound of formula (10b) by using a known organic chemistry-based technique to subject the compound of formula (9b) to esterification. The compound (10b) was obtained according to the procedure described in step B-3 of Method **B.**

(Step B-8)

This step is a step of producing a compound of formula (2') by using a known organic chemistry-based technique to subject the compound of formula (6b) to condensation with a compound of formula (10b). The compound (2') was obtained according to the procedure described in step B-4 of Method B.

Method C

A conjugation precursor represented by (3) as used in an antibody-drug conjugate of the present invention can be produced according to Method C described below.

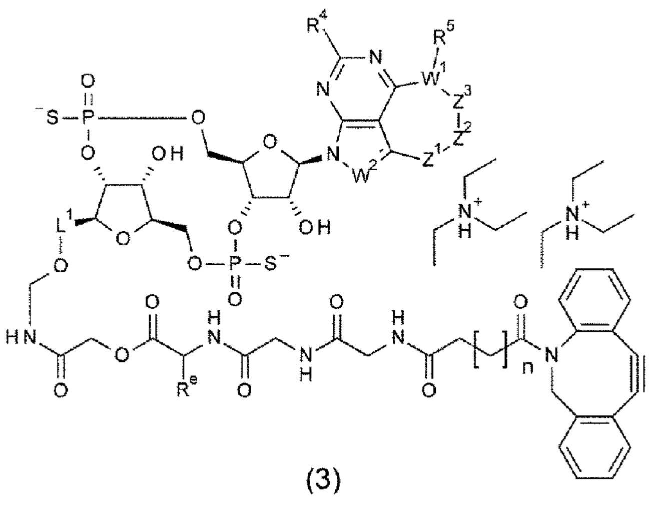

(3)

This synthetic scheme is a method for producing a conjugation precursor (3) in the case where a hydroxy group is substituted at a given position of $L^1$.

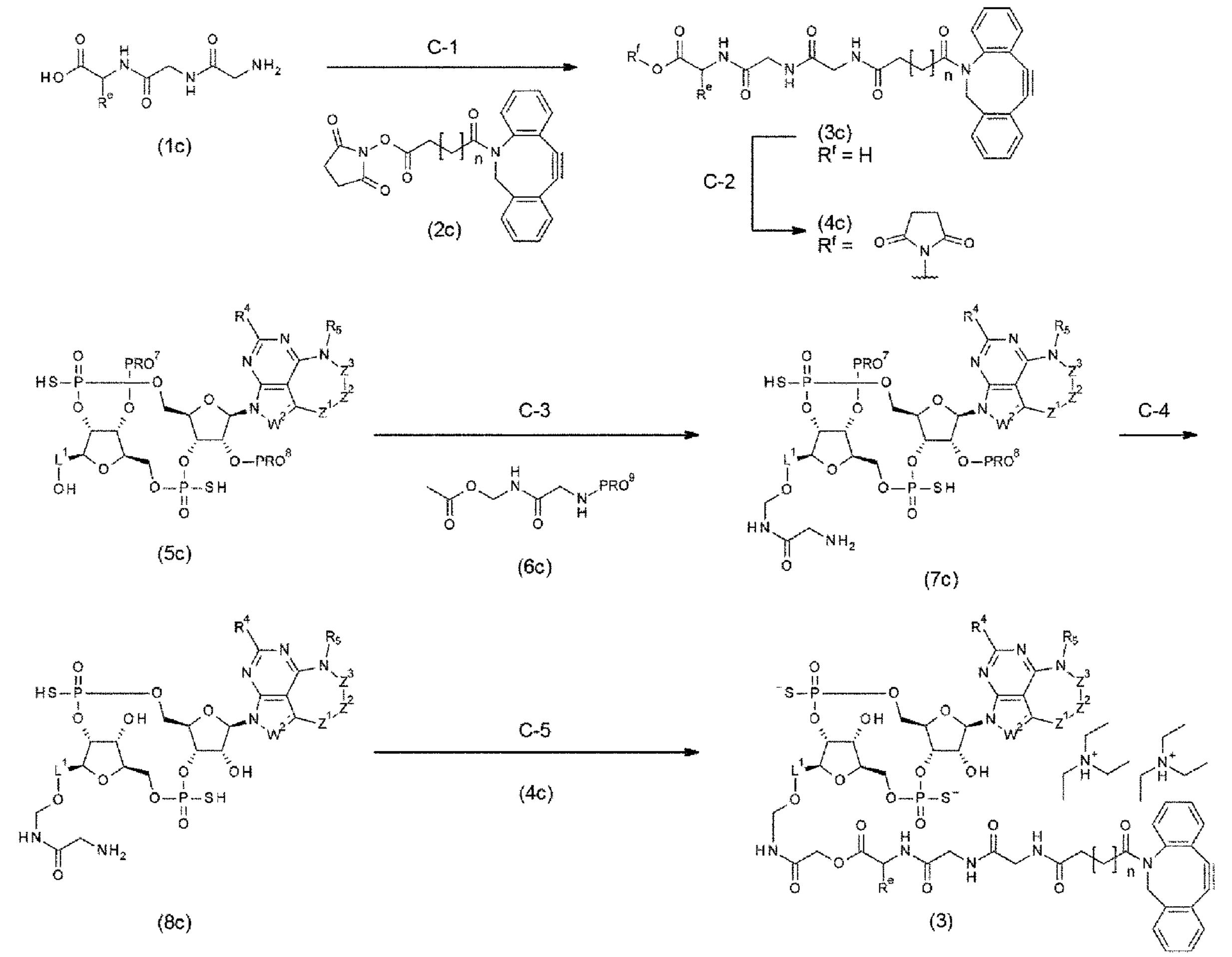


(1c) (2c) (3c) (4c) (5c) (6c) (7c) (8c) (3)

(Step C-1)

This step is a step of producing a compound of formula (3c) by using a known organic chemistry-based technique to subject a compound of formula (1c) to amidation with a compound of formula (2c). The compound (3c) was obtained according to the procedure described in step B-2 of Method B.

(Step C-2)

This step is a step of producing a compound of formula (4c) by using a known organic chemistry-based technique to subject the compound of formula (3c) to esterification. The compound (4c) was obtained according to the procedure described in step B-3 of Method B.

(Step C-3)

This step is a step of producing a compound of formula (7c) by using a known organic chemistry-based technique to subject a compound of formula (5c) to a coupling reaction (aminomethylenation) with a compound of formula (6c) and then subject the resulting coupling product to deprotection in a sequential manner. In the case where $PRO^9$ was a 9-fluorenylmethyloxycarbonyl group, the aminomethylenation was carried out by reacting the compound (5c) with the compound (6c) and an acid (e.g., p-toluenesulfonic acid) in tetrahydrofuran at a temperature of 5°C to 35°C. For 1 mole of compound (5c), 1 mole to 20 moles and preferably 2 moles to 10 moles of the compound (6c) was used, and 0.05 mole to excess moles and preferably 0.1 mole to 3 moles of the acid was used. The reaction time is from 30 min to 72 h and preferably from 2 h to 24 h. Next, a base (e.g., 1,8-diazabicyclo[5.4.0]-7-undecene) was added to the reaction mixture, and deprotection was thereby carried out. In the case where the reaction mixture is a suspension, a solvent (e.g., N,N-dimethylformamide) may be added and the reaction mixture is dissolved if necessary, and then the reaction can be carried out. For 1 mole of compound (5c), excess moles and preferably 5 moles to 20 moles of the base was used. The reaction time is from 10 min to 24 h and preferably from 2 h to 12 h. Water was added to the reaction mixture and the mixture was

subjected directly to purification by C18 silica gel column chromatography **(e.g.,** buffer solution/acetonitrile) to give a compound (7c).

(Step C-4)

This step is a step of producing a compound of formula (8c) by using a known organic chemistry-based technique to remove a protecting group from the compound of formula (7c). In the case where $PRO^7$ and $PRO^8$ are each a tert-butyldimethylsilyl group, the compound (8c) was obtained according to the procedure described in step A-7 of Method **A.**

(Step C-5)

This step is a step of producing a compound of formula (3) by using a known organic chemistry-based technique to subject the compound of formula (8c) to condensation with the compound of formula (4c). The compound (3) was obtained according to the procedure described in step B-4 of Method B.

Method C'

A conjugation precursor represented by (3') as used in an antibody-drug conjugate of the present invention can be produced according to Method C' described below.

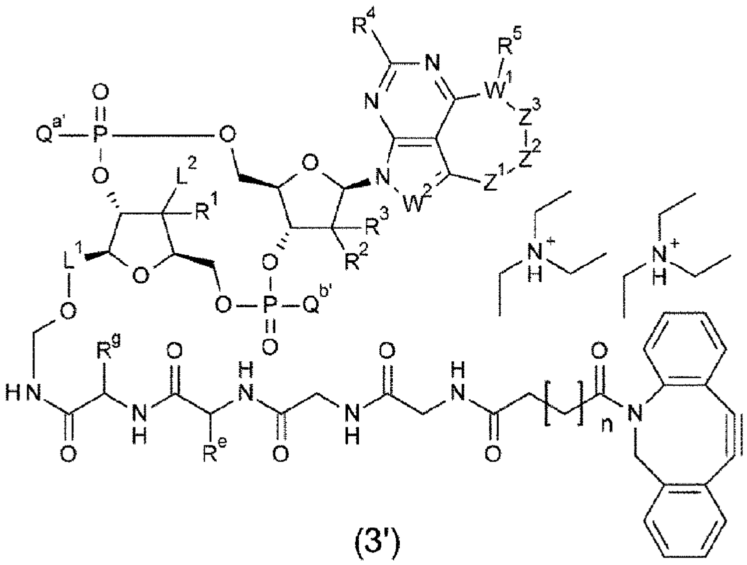

(3')

This synthetic scheme is a method for producing a conjugation precursor (3') in the case where a hydroxy group is substituted at a given position of $L^1$.

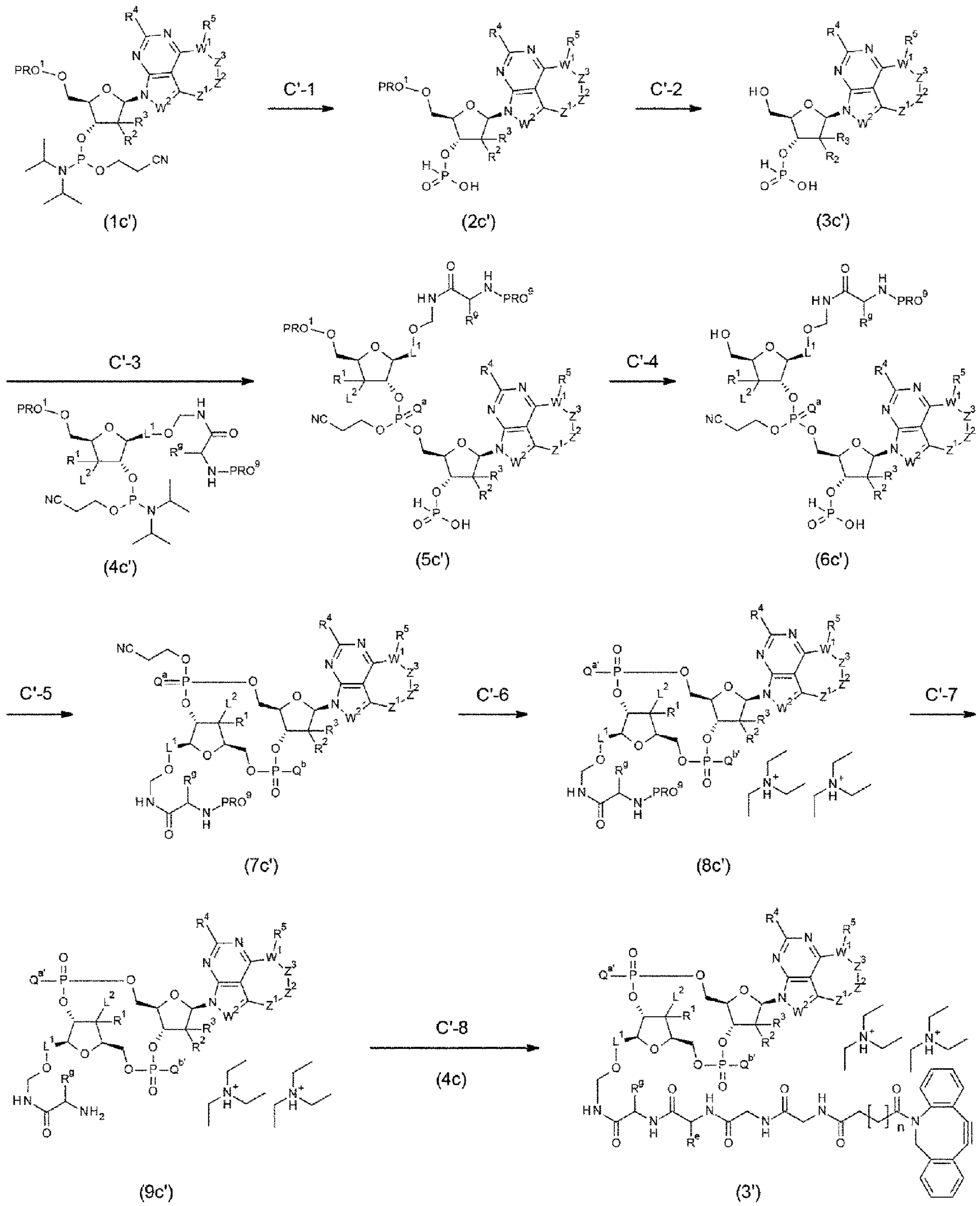


(Step C'-1)

This step is a step of producing a compound of formula (2c') by using a known organic chemistry-based technique to subject a compound of formula (1c') to sequential hydrolysis and cyanoethyl group removal. The compound (2c') was obtained according to the procedure described in step A-1 of Method A.

(Step C'-2)

This step is a step of producing a compound of formula (3c') by using a known organic chemistry-based technique to remove a hydroxy group-protecting group from the compound of formula (2c'). The compound (3c') was obtained according to the procedure described in step A-2 of Method A.

(Step C'-3)

This step is a step of producing a compound of formula (5c') by using a known organic chemistry-based technique to subject the compound of formula (3c') to a coupling reaction with a compound of formula (4c') and then subject the resulting coupling product to sulfidation or oxidation in a sequential manner. The compound (5c') was obtained according to the procedure described in step A-3 of Method A or step A''-3 of Method A''.

(Step C'-4)

This step is a step of producing a compound of formula (6c') by using a known organic chemistry-based technique to remove a hydroxy group-protecting group from the compound of formula (5c'). The compound (6c') was obtained according to the procedure described in step A-4 of Method A.

(Step C'-5)

This step is a step of producing a compound of formula (7c') by using a known organic chemistry-based technique to subject the compound of formula (6c') to sequential cyclization and sulfurization or oxidation. The compound (7c') was obtained according to the procedure described in step A-5 of Method A or step A'-5 of Method A'.

(Step C'-6)

This step is a step of producing a compound of formula (8c') by using a known organic chemistry-based technique to simultaneously remove a cyanoethyl group and all the acyl-based protecting groups from the compound of formula (7c'). The compound (8c') was obtained according to the procedure described in step A-6 of Method A.

(Step C'-7)

This step is a step of producing a compound of formula (9c') by using a known organic chemistry-based technique to simultaneously remove all the silyl-based protecting groups from the compound of formula (8c'). In the case where $PRO^9$ was a 2-(trimethylsilyl)ethoxycarbonyl group, the 2-(trimethylsilyl)ethoxycarbonyl group was removed by treating the compound (8c') with a tetrahydrofuran solution of tetrabutylammonium fluoride at a temperature of 5°C to 100°C, preferably from 35°C to 60°C. For 1 mole of compound (8c'), excess moles and preferably 10 to 30 moles of tetrabutylammonium fluoride was used. The reaction time is from 1 h to 48 h and preferably from 4 h to 24 h. The reaction mixture was diluted with a buffer solution, and the organic solvent component was then distilled off under reduced pressure as necessary. The residue was purified by preparative HPLC (e.g., buffer solution/acetonitrile, buffer solution/-methanol), C18 silica gel column chromatography (e.g., buffer solution/acetonitrile, buffer solution/methanol), or a combination of these procedures to obtain the compound (9c').

(Step C'-8)

This step is a step of producing a compound of formula (3') by using a known organic chemistry-based technique to subject the compound of formula (9c') to condensation with the compound of formula (4c). The compound (3') was obtained according to the procedure described in step B-4 of Method B.

Method D: Production of Glycan-Remodeled Antibody

A glycan-remodeled antibody can be produced by the method illustrated in the following scheme in accordance with the procedure described in, for instance, WO2018/003983, WO2020/050406, WO2021/177438, WO2022/050300, and PLos ONE 2018, 13, e0193534.

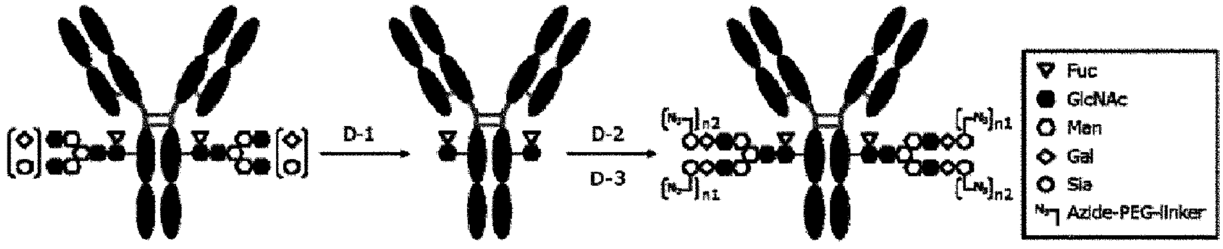


(1d) (2d) (3d)

(n1,n2)=(1,0)(0,1) or (1,1)

(Step D-1)

This step is a step of producing a glycan-cleaved antibody by using a known enzymatic reaction to hydrolyze and cleave a glycosidic linkage between GlcRAcβ1-4GlcNAc of the chitobiose structure at the reducing terminal of the N-linked glycan (N297-linked glycan) attached to asparagine at position 297 of the heavy chain amino acid sequence of an antibody. The hydrolysis reaction of the glycosidic linkage between GlcNAcβ1 and 4GlcNAc at the reducing terminal of the chitobiose structure of a desired antibody (1d) (10 mg/mL) was carried out using a hydrolase such as wild-type EndoS enzyme in a buffer solution (e.g., a phosphate buffer) at a temperature of 0°C to 40°C. The reaction time is from 10 min to 72 h and preferably from 1 h to 6 h. The amount of wild-type EndoS enzyme used was from 0.1 mg to 10 mg and preferably from 0.1 mg to 3 mg per 100 mg of the antibody (1d). After completion of the reaction, the antibody was purified by affinity chromatography (HiTrap rProtein A FF (5 ml) (produced by GE Healthcare)) and/or hydroxyapatite column (Bio-Scale Mini CHT Type I cartridge (5 ml) (produced by BIO-RAD)) to obtain a (Fucα1,6) GlcNAc antibody (2d).

(Step D-2)

This step is a step of producing a glycan-remodeled antibody (3d) by using a known enzymatic reaction to link the (Fucα1,6)GlcNAc antibody (2d) obtained in step D-1 with an SG-or MSG (MSG1, MSG2)-type glycan oxazoline moiety having an azide group-containing PEG linker (hereinafter referred to as an "azido-glycan oxazoline moiety").

The transglucosylation reaction was carried out by reacting the antibody (2d) with the azido-glycan oxazoline moiety in the presence of glycosyltransferase (e.g., EndoS (D233Q/Q303L)) in a buffer solution (e.g., a phosphate buffer) at a temperature of 0°C to 40°C. The reaction time is from 10 min to 72 h and preferably from 1 h to 6 h. The amount of the EndoS enzyme (D233Q/Q303L) used was from 1 mg to 10 mg and preferably from 1 mg to 3 mg per 100 mg of the antibody; and 2 equivalents to excess equivalents and preferably 4 equivalents to 20 equivalents of the azido-glycan oxazoline moiety was used. After completion of the reaction, the antibody was purified by affinity chromatography (HiTrap rProtein A FF (5 ml) (produced by GE Healthcare)) and a hydroxyapatite column (Bio-Scale Mini CHT Type I cartridge (5 ml) (produced by BIO-RAD)) to obtain the glycan-remodeled antibody (3d).

In the above preparation of the glycan-remodeled antibody, concentration of the antibody aqueous solution, concentration measurement, and buffer exchange may be carried out according to the following common operations A to C.

Note that the SG-type azido-glycan oxazoline moiety was synthesized in accordance with the procedure described in WO2018/003983. As an example, how to synthesize $[N_3\text{-PEG}(3)]_2$-SG(10)-Ox (compounds 1-10 described in WO2018/003983) is illustrated in the following scheme.

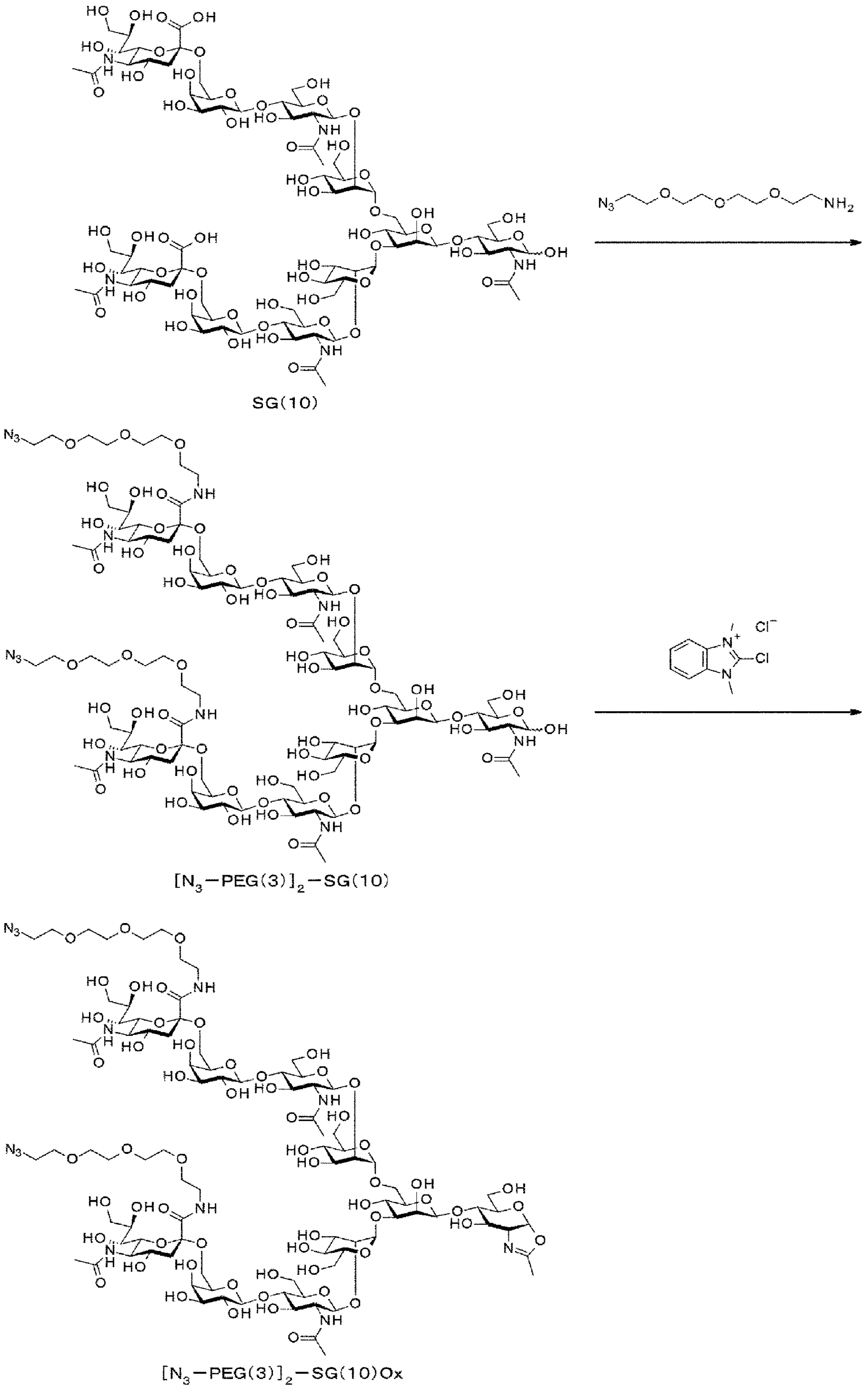

SG(10)

$[N_3-PEG(3)]_2-SG(10)$

$[N_3-PEG(3)]_2-SG(10)Ox$

The MSG-type azido-glycan oxazoline moiety was also synthesized in accordance with the procedure described in WO2018/003983. As an example, how to synthesize $[N_3\text{-PEG(3)}]_2$-MSG1(9)-Ox (compounds 1-11 described in WO2018/003983) is illustrated in the following scheme.

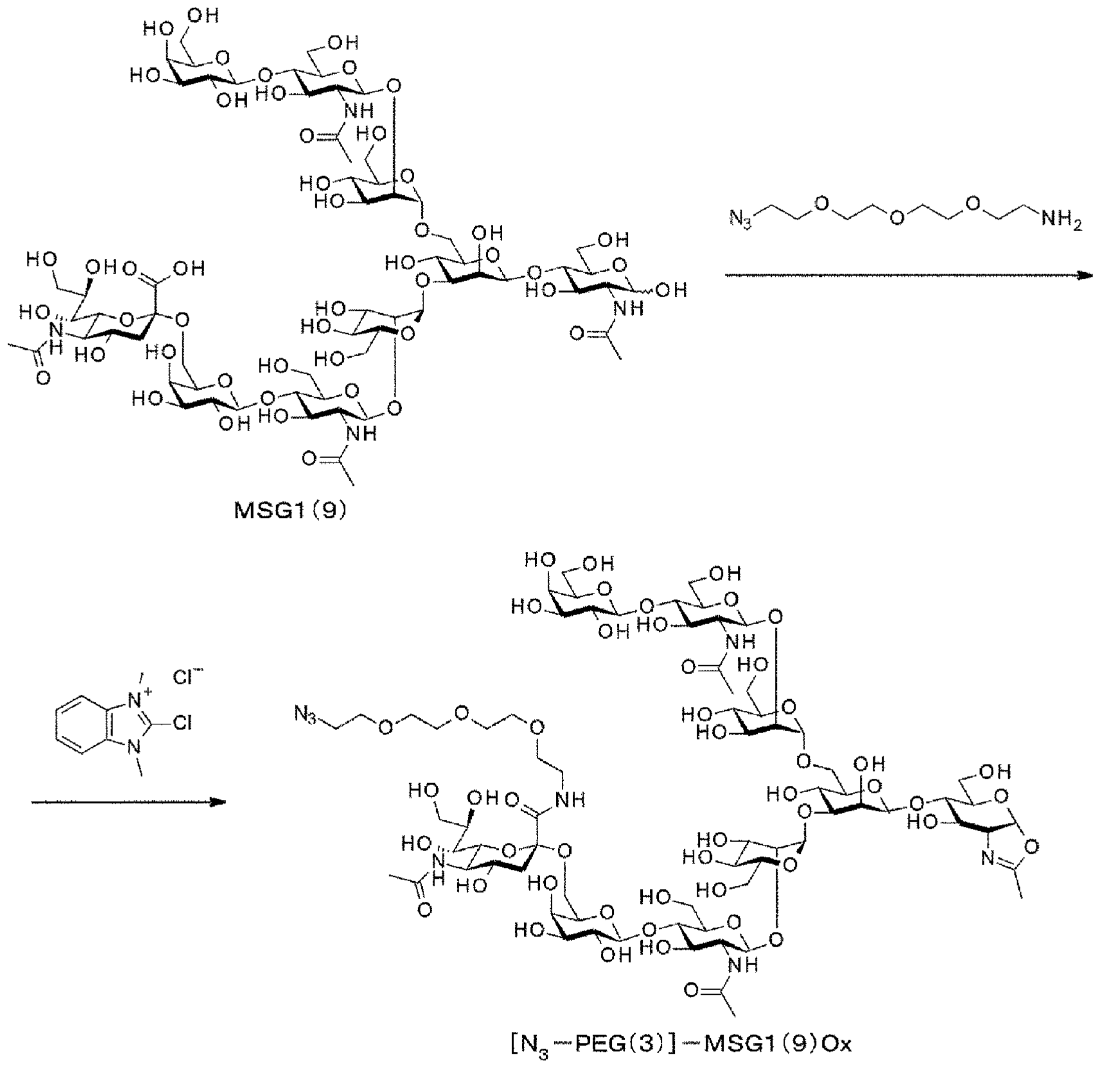

MSG1 (9)

$[N_3\text{-PEG(3)}]$-MSG1 (9) Ox

(Step D-3)

This step is a step of producing a glycan-remodeled antibody (3d) from the (Fucα1,6)GlcNAc antibody (2d) obtained in step D-1 by transglycosylation reaction using two types of Endo enzymes. Concurrent use of these two types of enzymes can directly subject the N297 glycan of the antibody to transglycosylation with a glycan donor with an unactivated reducing terminal, such as SGP or (SG)Asn.

Enzyme-B (EndoM-like enzyme) and enzyme-A (EndoS-like enzyme) can be suitably combined as the two types of Endo enzymes used.

Examples of enzyme-B can include EndoM, EndoOm, EndoCC, and their mutants with reduced hydrolytic activity, such as EndoM mutants, EndoOm mutants, and EndoCC mutants. Enzyme-B is preferably EndoM N175Q, EndoCC N180H, or EndoOm N194Q.

Examples of enzyme-A can include EndoS, EndoS2 (EndoS49), and their mutants with reduced hydrolytic activity, such as EndoS mutants and EndoS2 (EndoS49) mutants. Enzyme-A is preferably EndoS D233Q, EndoS D233Q/Q303L, EndoS D233Q/E350A, EndoS D233Q/E350Q, EndoS D233Q/E350D, EndoS D233Q/E350N, EndoS D233Q/D405A, EndoS2 D184M, EndoS2 T138Q, or the like.

Examples of the glycan donor that can be used include ($[N_3\text{-PEG(3)}]_2$-SG)-Asn-PEG(3)-$N_3$, $[N_3\text{-PEG(3)}]$-MSG1-Asn-PEG(3)-$N_3$, and $[N_3\text{-PEG(3)}]$-MSG2-Asn-PEG(3)-$N_3$.

The transglycosylation reaction was carried out by reacting the antibody (2d) with ($[N_3\text{-PEG(3)}]_2$-SG)-Asn-PEG(3)-$N_3$ in the presence of glycosyltransferases of enzyme-B (EndoM-like enzyme) and enzyme-A (EndoS-like

enzyme) in a buffer solution (e.g., a Tris buffer). The reaction temperature can be appropriately selected according to the optimum temperatures for the enzymes used and is usually 15 to 50°C and preferably 25 to 40°C. The reaction time can be appropriately selected from 2 h to 48 h. After completion of the reaction, a purification method (affinity chromatography, a hydroxyapatite column, etc.) or an ultrafiltration method (ultrafiltration membrane) suitable for a reaction scale can be selected to obtain the glycan-remodeled antibody (3d).

In the above preparation of the glycan-remodeled antibody, concentration of the antibody aqueous solution, concentration measurement, and buffer exchange may be carried out according to the following common operations A to C.

Note that ([$N_3$-PEG(3)]$_2$-SG)-Asn-PEG(3)-$N_3$ is synthesized in accordance with a method described in WO2018/003983. ([$N_3$-PEG(3)]$_2$-SG)-Asn-PEG(3)-$N_3$ (compound 1-13 described in WO2018/003983) can be obtained by reacting a free form of Fmoc-(SG-)Asn prepared from Fmoc-(SG-)Asn (1S2S-11NC-Asn-Fmoc, produced by GlyTech, Inc.) with 11-azido-3,6,9-trioxaundecan-1-amine in step 1-2A described in WO2018/003983.

The MSG-type glycan donor [$N_3$-PEG(3)]-MSG1-Asn-PEG(3)-$N_3$ or [$N_3$-PEG(3)]-MSG2-Asn-PEG(3)-$N_3$ can also be synthesized in accordance with a method described in steps 1 to 3 of Example 154 of WO2019065964.

Note that step D-3 can also be carried out in accordance with the description of <5. Method for producing Fc-containing molecule> below.

Method E: To Conjugate Antibody and Drug (Glycan Conjugation 1)

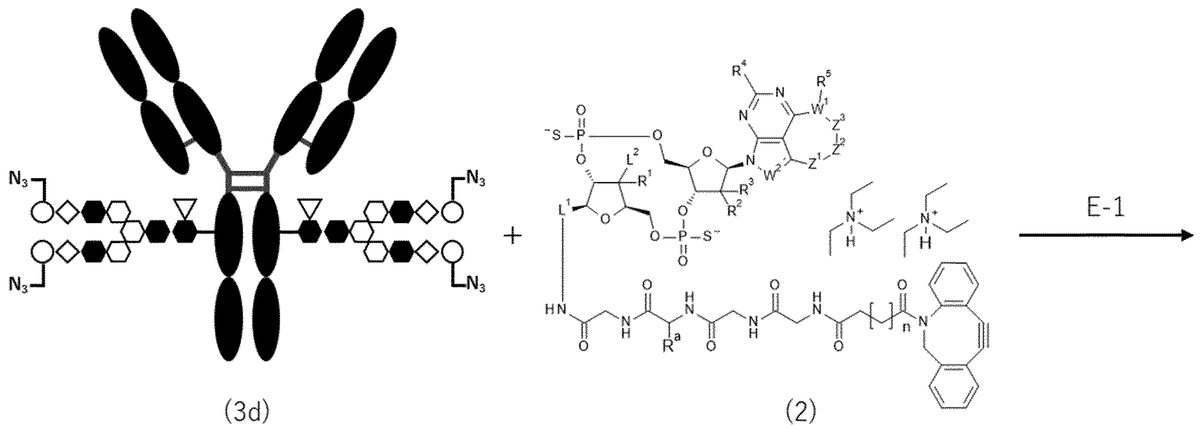


(3d) (2)

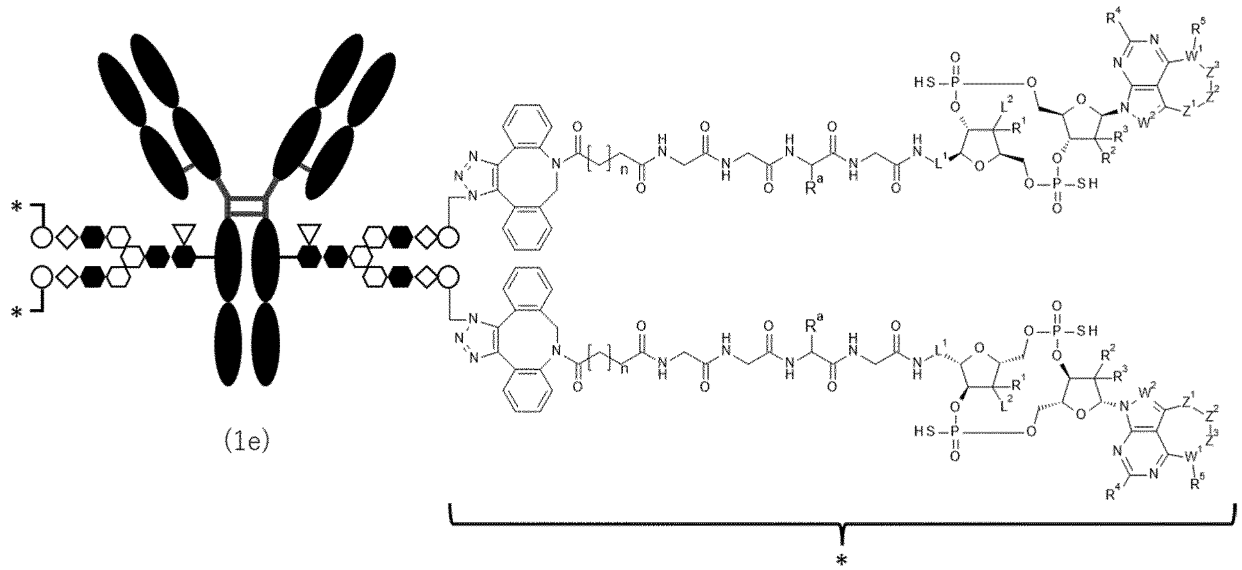

(1e)

Wherein, the two asterisks (*) on the left side of the antibody-drug conjugate (1e) each indicate a drug-linker portion which is also represented by the asterisk on the right side.

This synthetic scheme is a method for producing an antibody-drug conjugate (1e) by using a SPAAC (strain-promoted azide-alkyne cycloaddition: J. Am. Chem. Soc. 2004, 126, 15046-15047) reaction to conjugate the glycan-remodeled antibody (3d) obtained in step D-2 of Method D with the conjugation precursor (2) obtained in step B-4 of Method B.

(Step E-1)

The SPAAC reaction was carried out by mixing the glycan-remodeled antibody (3d)-containing buffer solution (e.g., phosphate buffer, acetate buffer, borate buffer) with a solution in which the conjugation precursor (2) had been dissolved in an appropriate solvent (dimethyl sulfoxide, N,N-dimethylformamide, N,N-dimethylacetamide, N-methylpyrrolidone, propylene glycol, or a mixture of these solvents). For 1 mole of the glycan-remodeled antibody (3d), 2 moles to excess moles and preferably 4 moles to 30 moles of the conjugation precursor (2) is used; and the percentage of the organic solvent is preferably from 1% to 200% (v/v) based on the antibody buffer solution. The reaction temperature is from 0°C to 37°C and preferably from 15°C to 25°C. The reaction time is from 1 h to 150 h and preferably from 6 h to 72 h. The pH of the reaction mixture is preferably from 5 to 9. The reaction mixture was subjected to purification by the method described in common operation D to give the antibody-drug conjugate (1e).

Method E': To Conjugate Antibody and Drug (Cysteine Conjugation)

An antibody-drug conjugate with cysteine conjugation according to the present invention can be produced by the procedure described in, for instance, WO2014/057687 while using the desired antibody prepared according to, for instance, Reference Example 3 and the conjugation precursor (2') having a maleimide group as obtained in step B-8 of Method B'.

Method E'': To Conjugate Antibody and Drug (Glycan Conjugation 2)

The conjugation precursor (2) was switched, in Method E, to the conjugation precursor (3') obtained in step C'-8 of Method C' to obtain an antibody-drug conjugate (1e'') represented by the following formula:

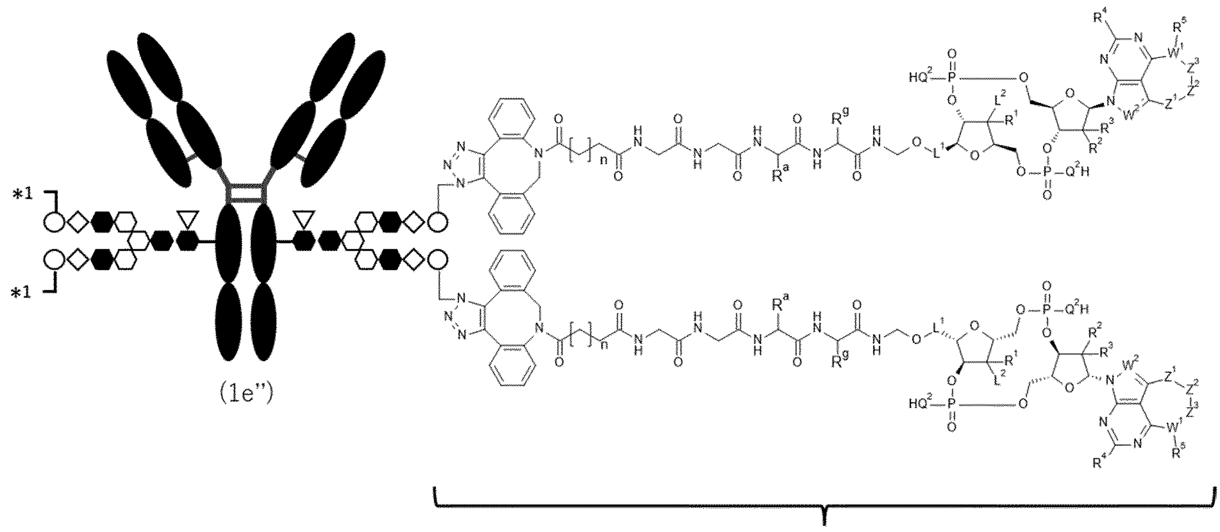

(1e'')

wherein the two asterisks (*1) on the left side of the antibody-drug conjugate (1e'') indicate a drug-linker portion which is also represented by the asterisk on the right side.

According to the below-described common operations D to G, antibody-drug conjugates can be identified from each other through buffer exchange, purification, measurement of antibody concentration, and measurement of the average number of conjugated drugs per antibody molecule.

Method F: To Produce Antibody Comprising Mutated Fc Region

An antibody comprising a mutated Fc region can be prepared by a known method using an Fc effector-less mutant animal cell expression vector. Examples of the method for constructing the Fc effector-less mutant animal cell expression vector include the following method.

Construction of Fc Effector-Less Mutant Animal Cell Expression Vector

An animal cell expression vector containing a gene of the wild-type Fc region is used as a template, and a desired effector-less mutation is introduced into the Fc region by PCR using KOD-Plus-Mutagenesis Kit (TOYOBO). Alternatively, a gene of an Fc region having a desired effector-less mutation may be synthesized and integrated into an animal cell expression vector by ligation reaction.

Note that the antibody-drug conjugate comprising a CDN derivative according to the present invention or its production intermediate may be produced with reference to WO2022163846. The molecule comprising a target molecule-binding moiety, a mutated Fc region, and an immunostimulator according to the present invention, wherein the immunostimulator is other than the CDN derivative, can be produced by appropriately modifying the above-described production method with reference to a known method, or using a known method.

Common operation A: Concentration of Antibody Aqueous Solution

An antibody or antibody-drug conjugate solution was placed in an Amicon (registered trademark) Ultra centrifugal filter device (50,000 NMWL, Merck Millipore, Ltd.) and centrifuged (at 2000 G to 4000 G for 5 to 20 min) using a centrifuge (Allegra **X-**15R, Beckman Coulter, Inc.). In this way, the antibody or antibody-drug conjugate solution was concentrated.

Common operation B: Measurement of Antibody Concentration

The antibody concentration was measured using a UV meter (Nanodrop 1000, Thermo Fisher Scientific, Inc.) in accordance with the method specified by the manufacturer. At that time, the absorbance coefficients at 280 nm (1.3 $mLmg^{-1}$ $cm^{-1}$ to 1.8 $mLmg^{-1}$ $cm^{-1}$) were different for each antibody.

Common operation C: Exchange Buffer for Antibody

A buffer solution (e.g., phosphate buffered saline (pH 6.0), phosphate buffer (pH 6.0)) was added to the antibody aqueous solution, and the solution was concentrated according to the method described in common operation A. This operation was repeated several times, and the antibody concentration was then measured according to the method described in common operation B. A buffer solution (e.g., phosphate buffered saline (pH 6.0), phosphate buffer (pH 6.0)) was added, if appropriate, to the antibody buffer solution to prepare the antibody buffer solution at a desired concentration (e.g., about 10 mg/mL).

Common operation D: Purification of Antibody-Drug Conjugate (Gel Filtration Chromatography)

Each NAP column (NAP-5, NAP-10, or NAP-25 (produced by GE Healthcare)) was equilibrated with acetate buffer (10 mM acetate buffer, 5% sorbitol, pH 5.5; herein referred to as ABS) or another suitable buffer solution. The antibody-drug conjugate reaction mixture was loaded into this NAP column. A buffer solution at a volume specified by the manufacturer was allowed to flow down. Then, the antibody fractions were collected. The fractions were reloaded into the NAP column. Subsequently, a buffer solution at a volume specified by the manufacturer was allowed to flow down, and the antibody fractions were then collected. This operation was repeated a total of 2 to 3 times to yield an antibody-drug conjugate from which an unbound drug-linker, dimethylsulfoxide, and propyleneglycol had been removed. As necessary, the concentration of the antibody-drug conjugate solution was adjusted using common operations A and C.

Common operation E: Measurement of Antibody Concentration and Average Number of Conjugated Drugs Per Antibody Molecule in Antibody-Drug Conjugate (UV Method).

The concentration of drug conjugated in the antibody-drug conjugate can be calculated by using a spectrophotometer (UV/VIS Spectrometer Lambda 25, PerkinElmer, Inc.) to measure absorbance of the aqueous solution of the antibody-drug conjugate at two wavelengths, 280 nm and 250 nm, and then performing the following calculation. The total absorbance at a certain wavelength is equal to the sum of each absorbance of all the absorption chemical species (each absorbance can be added). Therefore, it is assumed that there is no change in the molar extinction coefficients of the antibody and the drug between before and after the antibody and the drug are conjugated. In this case, the antibody concentration or the drug concentration in the antibody-drug conjugate can be expressed using the following expressions:

$$A_{280} = A_{D,280} + A_{A,280} = \varepsilon_{D,280}C_D + \varepsilon_{A,280}C_A \quad \text{Expression (I);}$$

and

$$A_{250} = A_{D,250} + A_{A,250} = \varepsilon_{D,250}C_D + \varepsilon_{A,250}C_A \quad \text{Expression (II)},$$

wherein, $A_{280}$ represents absorbance at 280 nm by antibody-drug conjugate aqueous solution; $A_{250}$ represents absorbance at 250 nm by the antibody-drug conjugate aqueous solution; $A_{A,280}$ represents absorbance at 280 nm by the antibody; $A_{A,250}$ represents absorbance at 250 nm by the antibody; $A_{D,280}$ represents absorbance at 280 nm by the conjugate precursor; $A_{D,250}$ represents absorbance at 250 nm by the conjugate precursor; $\varepsilon_{A,280}$ represents the molar extinction coefficient at 280 nm by the antibody; $\varepsilon_{A,250}$ represents the molar extinction coefficient at 250 nm of the antibody; $\varepsilon_{D,280}$ represents the molar extinction coefficient at 280 nm of the conjugate precursor; $\varepsilon_{D,250}$ represents the molar extinction coefficient at 250 nm of the conjugate precursor; $C_A$ represents the concentration of the antibody in the antibody-drug conjugate; and $C_D$ represents the concentration of the drug in the antibody-drug conjugate; wherein, for each of $\varepsilon_{A,280}$, $\varepsilon_{A,250}$, $\varepsilon_{D,280}$, and $\varepsilon_{D,250}$, a calculated estimate or a measured value is used. For instance, $\varepsilon_{A,280}$ may be estimated from the amino acid sequence of the antibody by using a known calculation process (Protein Science, 1995, vol.4, 2411-2423). The value $\varepsilon_{A,250}$ used was calculated from the values measured obtained from UV measurement of the antibody and the value estimated for $\varepsilon_{A,280}$. In the Examples, the molar extinction coefficients of anti-EGFR antibody 1 were $\varepsilon_{A,280}$ = 203460 and $\varepsilon_{A,250}$ = 70151 or 63837. The molar extinction coefficients of anti-EGFR antibody 2 were $\varepsilon_{A,280}$ = 203460 and $\varepsilon_{A,250}$ = 63051 or 63370. The molar extinction coefficients of anti-EGFR antibody 3 were $\varepsilon_{A,280}$ = 203460 and $\varepsilon_{A,250}$ = 62692 or 63853. The molar extinction coefficients of anti-EGFR antibody A were $\varepsilon_{A,280}$ = 203460 and $\varepsilon_{A,250}$ = 69640. The molar extinction coefficients of anti-EGFR antibody B were $\varepsilon_{A,280}$ = 197750 and $\varepsilon_{A,250}$ = 66256. The molar extinction coefficients of anti-CDH6 antibody 1 were $\varepsilon_{A,280}$ = 223400 and $\varepsilon_{A,250}$ = 74671 or 71530. Meanwhile, $\varepsilon_{D,280}$ and $\varepsilon_{D,250}$ are obtained by measuring the absorbance of a solution in which the conjugate precursor has been dissolved to a certain molar concentration, and applying Lambert-Beer's law (absorbance = molar concentration × molar extinction coefficient × cell's optical path length). The molar extinction coefficients of the conjugate precursor in the Examples were obtained by UV measurement on a case-by-case basis. $A_{280}$ and $A_{250}$ of the antibody-drug conjugate aqueous solution may be measured; these values may be assigned to Expressions (I) and (II) to solve the simultaneous expressions; and $C_A$ and $C_D$ can thus be calculated. Furthermore, by dividing $C_D$ by $C_A$, the average number of conjugated drugs per antibody molecule may be calculated.

Common operation F: Measurement of Antibody Concentration and Average Number of Conjugated Drugs Per Antibody Molecule in Antibody-Drug Conjugate (Reverse-Phase High Performance Liquid Chromatography Method: RP-HPLC)

The antibody concentration and the average number of conjugated drugs per antibody molecule in the antibody-drug conjugate can be calculated by the above-described common operation E as well as high performance liquid chromatography analysis using the following methods.

[F-1. Preparation of Samples for HPLC Analysis (To Reduce Antibody-Drug Conjugate)]

A solution of an antibody-drug conjugate solution (about 1 mg/mL; 60 μL) was mixed with an aqueous solution of dithiothreitol (DTT) (100 mM; 15 μL). The mixture was incubated at 37°C for 30 min to cleave each disulfide bond between the L and H chains of the antibody-drug conjugate. The reaction mixture was used directly for HPLC analysis.

[F-2. HPLC Analysis]

Representative analysis conditions are as follows.

HPLC system: Agilent 1290 HPLC system (Agilent Technologies)
Detector: UV spectrophotometer (measurement wavelength: 280 nm)
Column: Acquity BEH Phenyl (2.1 × 50 mm, 1.7 μm; produced by Waters)
Column temperature: 75°C
Flow rate: 0.8 mL/min
Sample injection volume: 10 μL
Mobile phase A: 0.1% trifluoroacetic acid (TFA), 15% isopropyl alcohol solution
Mobile phase B: 0.075% TFA, 15% isopropyl alcohol acetonitrile solution
Gradient program (mobile phase B): 14%-36% (0 min-15 min), 36%-80% (15-17 min), 80%-14% (17 min-17.1 min), 14 %-14% (17.1 min-23 min)

[F-3. Data Analysis]

[F-3-1] In the case of glycan conjugation in the SPAAC reaction, the drug-conjugated H-chain (H-chain having one drug conjugated: H1, H-chain having two drugs conjugated: H2) is more hydrophobic than the drug-free L-chain (L0)

and H-chain (H0) of the antibody. The hydrophobicity and the retention time increase in proportion to the number of conjugated drugs. Accordingly, in principle, L0, H0, H1, and H2 are eluted in this order. By comparing the retention time between L0 and H0, the detection peak can be assigned to any of L0, H0, H1, and H2. In the case of cysteine conjugation, the drug-conjugated L-chain (L chain having one drug conjugated: L1) and the drug-conjugated H-chain (H-chain having one conjugated drug molecule: H1, H-chain having two conjugated drug : H2, H-chain having three conjugated drug : H3) are more hydrophobic in proportion to the number of conjugated drugs. Also, the retention time increases. Accordingly, in principle, L0, L1, H0, H1, H2, and H3 are eluted in this order. By comparing the retention time between L0 and H0, the detection peak can be assigned to any of L0, L1, H0, H1, H2, and H3.

[F-3-2] In the case of glycan conjugation in the SPAAC reaction, the peak area was corrected according to the following expression while using the molar extinction coefficients of the H chain and the drug-linker in response to the number of drug-linkers conjugated, because each drug-linker absorbs UV light. In the case of cysteine conjugation, where the drug is also conjugated to the L-chain, the peak area was corrected likewise for the L-chain.

[Expression 1]

$$\text{Corrected H-chain peak area } (HPA_i) = \text{Peak area} \times \frac{\text{Molar extinction coefficient of H-chain}}{\text{Molar extinction coefficient of H-chain} + \text{Number of drugs conjugated} \times \text{Molar extinction coefficient of drug-linker}}$$

For the molar extinction coefficients (280 nm) of the L chain and H chain in each antibody, values estimated from the known calculation method described in common operation E were used. In the case of anti-EGFR antibody 1, 2, 3 and A, 23232 was used as the molar extinction coefficient of the L chain and 78498 was used as the molar extinction coefficient of the H chain. In the case of anti-EGFR antibody B, 23232 was used as the molar extinction coefficient of the L chain and 75642 was used as the molar extinction coefficient of the H chain. In the case of anti-CDH6 antibody 1, 31712 was used as the molar extinction coefficient of the L chain and 79988 was used as the molar extinction coefficient of the H chain. The molar extinction coefficient (at 280 nm) of the drug-linker was a value measured for the conjugation precursor in the case of glycan conjugation in the SPAAC reaction. In the case of cysteine conjugation, the conjugation precursor was reacted with mercaptoethanol or N-acetylcysteine, and the value measured for the compound, in which the maleimide group had been converted to a succinimide thioether, was used.

[F-3-3] The peak area ratio (%) of each chain based on the total of corrected peak areas was calculated according to the following expression.

[Expression 2]

$$\text{H-chain peak area ratio } (\%HPA_i) = \frac{HPA_i}{HPA_0 + HPA_1 + HPA_2} \times 100$$

[F-3-4] The average number of conjugated drugs per antibody molecule in an antibody-drug conjugate (DAR) was calculated according to the following expression.

[Expression 3]

$$\text{Average number of drugs conjugated } (DAR) = \frac{0 \times \%HPA_0 + 1 \times \%HPA_1 + 2 \times \%HPA_2}{100} \times 2$$

[F-3-5] The antibody concentration in the antibody-drug conjugate was calculated according to the following expression.

[Expression 4]

$$\text{Antibody concentration}(C_A)\ [mg/mL] = \frac{\text{Absorbance of antibody-drug conjugate} \times \text{Dilution factor} \times \text{Molecular weight of antibody}}{\text{Molar extinction coefficient of antibody} + \text{Average number of drugs conjugated} \times \text{Molar extinction coefficient of drug-linker}}$$

For the absorbance (280 nm) of the antibody-drug conjugate, values measured from the antibody-drug conjugate aqueous solution were used. The dilution factor indicates how many times the antibody-drug conjugate aqueous solution has been diluted for absorbance measurement, and is usually 4. For the molar extinction coefficient (280 nm) of the antibody, values estimated from the known calculation method described in common operation E were used. For the average number of conjugated drugs, values obtained in [F-3-4] were used. For the molar extinction coefficient (280 nm) of the drug-linker, values measured for the conjugation precursor in the case of glycan conjugation in the SPAAC reaction were used. In the case of cysteine conjugation, the conjugation precursor was reacted with mercaptoethanol or N-acetylcysteine, and the value measured for the compound, in which the maleimide group had been converted to a succinimide thioether, was used.

Common operation G: Measurement of Antibody Concentration and Average Number of Conjugated Drugs Per Antibody Molecule in Antibody-Drug Conjugate (Hydrophobic Interaction-High Performance Liquid Chromatography Method: HI-HPLC).

The antibody concentration and the average number of conjugated drugs per antibody molecule in the antibody-drug conjugate can be calculated by the above-described common operations E and F as well as high performance liquid chromatography analysis using the following methods.

[G-1. Preparation of Samples for HPLC Analysis]

The antibody-drug conjugate solution (approximately 1 mg/mL, 60 μL) was used directly for HPLC analysis.

[G-2. HPLC Analysis]

Representative analysis conditions are the following two different conditions.

HPLC system: SHIMADZU CBM-20A (Shimadzu Corporation)
Detector: UV spectrophotometer (measurement wavelength: 280 nm)
Column: TSK-gel Butyl-NPR (4.6 × 100 mm, 2.5 μm; produced by TOSOH)
Column temperature: Constant temperature at or near 25°C
Mobile phase A: 25 mM phosphate buffer solution containing 1.5 M ammonium sulfate (pH = 7.0)
Mobile phase B: 25 mM phosphate buffer solution (pH = 7.0)/isopropyl alcohol mixture (3:1)
Flow rate: 0.8 mL/min
Sample injection volume: 15 μL
Gradient program (mobile phase B): 10%-15% (0 min-5 min), 15%-65% (5 min-20 min)
or
HPLC system: SHIMADZU CBM-20A (Shimadzu Corporation)
Detector: UV spectrophotometer (measurement wavelength: 280 nm)
Column: PolyPROPYL A (4.6 × 100 mm, 3 μm, 1500 Å; produced by PolyLC)
Column temperature: Constant temperature at or near 40°C
Mobile phase A: 20 mM phosphate buffer solution containing 1.5 M ammonium sulfate (pH = 7.4)
Mobile phase B: 20 mM phosphate buffer solution (pH = 7.4)
Flow rate: 0.8 mL/min
Sample injection volume: 15 μL
Gradient program (mobile phase B): 40%-80% (0 min-20 min)

[G-3. Data Analysis]

[G-3-1] The hydrophobicity increases in proportion to the number of conjugated drugs per antibody, and the retention time increases accordingly. Thus, in the case of glycan conjugation in the SPAAC reaction, in principle, DAR = 0, DAR = 2, and DAR = 4 are eluted in this order. By comparing the retention time of DAR = 0, the detection peak can be assigned to either DAR = 2 or DAR = 4. The peak representing DAR = 1 or DAR = 3 can also be detected depending on the

kind of antibody and drug-linker. The DAR at the detection peak may be estimated by measuring the mass spectrum after the peak is fractionated by HI-HPLC.

[G-3-2] The peak area was corrected according to the following expression by using the molar extinction coefficients of the antibody and the drug-linker in response to the number of conjugated drug-linkers, because each drug-linker absorbs UV light.

[Expression 5]

$$\text{Corrected antibody peak area } (WPA_i) = \text{Peak area} \times \frac{\text{Molar extinction coefficient of antibody}}{\text{Molar extinction coefficient of antibody} + \text{Number of drugs conjugated} \times \text{Molar extinction coefficient of drug-linker}}$$

For the molar extinction coefficient (280 nm) of the antibody, values estimated from the known calculation method described in common operation E were used. The molar extinction coefficient (at 280 nm) of the drug-linker was a value measured for the conjugation precursor.

[G-3-3] The peak area ratio (%) of the antibody based on the total of corrected peak areas was calculated according to the following expression.

[Expression 6]

$$\text{Antibody peak area ratio } (\%WPA_i) = \frac{WPA_i}{WPA_0 + WPA_1 + WPA_2 + WPA_3 + WPA_4} \times 100$$

[G-3-4] The average number of conjugated drugs per antibody molecule in an antibody-drug conjugate was calculated according to the following expression.

[Expression 7]

$$\text{Average number of conjugated drugs } (DAR) = \frac{0 \times \%WPA_0 + 1 \times \%WPA_1 + 2 \times \%WPA_2 + 3 \times \%WPA_3 + 4 \times \%WPA_4}{100}$$

[G-3-5] The antibody concentration in the antibody-drug conjugate was calculated according to the expression described in [F-3-5]. At that time, for the average number of conjugated drugs, values obtained in [G-3-4] were used.

The antibody-drug conjugate or its production intermediate may contain stereoisomers, optical isomers caused by asymmetric carbon atoms, geometric isomers, tautomers, or optical isomers (e.g., d-, l-, or atrop-isomers). However, all of these isomers, optical isomers, and mixtures of them are included in the invention.

The number of conjugated drugs per antibody molecule is an important factor having an influence on the efficacy and safety of the antibody-drug conjugate of the present invention. The antibody-drug conjugate is produced under specified reaction conditions (e.g., the amounts of starting materials or reagents to be reacted) so that the number of conjugated drugs can be constant. However, unlike the chemical reaction of small molecule compounds, a mixture with different numbers of conjugated drugs is usually obtained. It is possible to determine the average number of conjugated drugs (DAR), namely the averaged value for the number of conjugated drugs per antibody molecule. The number of cyclic dinucleotide derivatives conjugated to the antibody molecule is controllable. The average number of conjugated cyclic dinucleotide derivatives per antibody may be1 to 10. The number is preferably from 1 to 8, more preferably from 1 to 5, and still more preferably from 2 to 4. In the present invention, the average number of conjugated drugs and the average number of the conjugated drug molecules have the same meaning and can be used interchangeably.

In an antibody-drug conjugate of the present invention, if the antibody Ab bonds to L through a remodeled glycan of the antibody Ab, $m^2$, which indicates the number of conjugated drugs per heavy chain of the antibody in the antibody-drug conjugate, is an integer of 1 or 2. In the case where the glycan is N297 glycan and the glycan is N297-(Fuc)SG, $m^2$ is 2, and DAR is in the range of 3 to 5 (preferably in the range of 3.2 to 4.8, and more preferably in the range of 3.5 to 4.2). In the case where the N297 glycan is N297-(Fuc)MSG1, N297-(Fuc)MSG2, or a mixture of N297-(Fuc)MSG1 and N297-(Fuc)MSG2, $m^2$ is 1, and DAR is in the range of 1 to 3 (preferably in the range of 1.0 to 2.5, and more preferably in the range of 1.2 to 2.2).

Note that a person skilled in the art can design, from the description of the Examples in the present application, a reaction in which the necessary number of drugs are conjugated to the antibody. Thus, it is possible to obtain an antibody in which the number of cyclic dinucleotide derivatives conjugated is controlled.

Note that an antibody-drug conjugate or production intermediate of the present invention may be left in the atmosphere or recrystallized. This may cause moisture absorption, adhesion of absorbed water, or a hydrate. Such compounds and salts containing water are also included in the present invention.

The antibody-drug conjugate or production intermediate in the present invention may be converted into a pharmaceutically acceptable salt, as desired, if it has a basic group such as an amino group. Examples of such salts may include hydrogen halide salts such as hydrochlorides and hydroiodides; inorganic acid salts such as nitrates, perchlorates, sulfates, and phosphates; lower alkanesulfonates such as methanesulfonates, trifluoromethanesulfonates, and ethanesulfonates; arylsufonates such as benzenesulfonates and p-toluenesulfonates; organic acid salts such as formates, acetates, malates, fumarates, succinates, citrates, tartrates, oxalates, and maleates; and amino acid salts such as ornithinates, glutamates, and aspartates.

The antibody-drug conjugate of the present invention contains a phosphate group and/or a thiophosphate group in its structure, so that a base addition salt can be generally formed. In addition, if the production intermediate has an acidic group such as a carboxyl group, it is generally possible to form a base addition salt. Examples of pharmaceutical acceptable salts may include alkali metal salts such as sodium salts, potassium salts, and lithium salts; alkali earth metal salts such as calcium salts and magnesium salts; inorganic salts such as ammonium salts; and organic amine salts such as dibenzylamine salts, morpholine salts, phenylglycine alkyl ester salts, ethylenediamine salts, N-methylglucamates, diethylamine salts, triethylamine salts, cyclohexylamine salts, dicyclohexylamine salts, N,N'-dibenzylethylenediamine salts, diethanolamine salts, N-benzyl-N-(2-phenylethoxy)amine salts, piperazine salts, tetramethylammonium salts, and tris(hydroxymethyl)aminomethane salts.

The antibody-drug conjugate and production intermediate of the present invention may exist as a hydrate, for example, by absorbing moisture from the air. The solvate in the present invention is not limited to a particular solvate and may be any pharmaceutically acceptable solvate, and specifically hydrates, ethanol solvates, 2-propanol solvates, and so on are preferred. Additionally, the antibody-drug conjugate and production intermediate of the present invention may be in an N-oxide form if a nitrogen atom is present therein, and these solvates and N-oxide forms are included in the scope of the present invention. In addition, the antibody-drug conjugate and production intermediate of the present invention may be in an sulfoxide form if a sulfur atom is present therein, and these solvates and sulfoxide forms are included in the scope of the present invention.

The present invention also includes compounds labeled with various radioactive or nonradioactive isotopes. The antibody-drug conjugate and production intermediate of the present invention may contain one or more constituent atoms in which atomic isotopes are present at a non-natural ratio. Examples of atomic isotopes may include deuterium (2H), tritium (3H), iodine-125 (1251), and carbon-14 (14C). The compound of the present invention may be radiolabeled with a radioactive isotope such as tritium (3H), iodine-125 (125I), or carbon-14 (14C). The radiolabeled compound is useful as a therapeutic or prophylactic agent, a reagent for research such as an assay reagent, and a diagnostic agent such as a diagnostic agent for *in vivo* imaging. Isotopic variants of the antibody-drug conjugate of the present invention are all included in the scope of the present invention, regardless of whether they are radioactive or not.

<4. Medicine>

The antibody-drug conjugate of the present invention exhibits anti-tumor immunity or cytotoxicity against cancer cells and also has excellent safety, and thus can be used as a medicine, especially as a therapeutic and/or prophylactic agent against cancer, or as an anti-tumor agent. The antibody medicine conjugate of the present invention can be used as a medicine, further as a therapeutic and/or prophylactic agent against infection. In one aspect of the present invention, the antibody-drug conjugate of the present invention can be used as a medicine highly stable against heat.

Examples of cancers to which the antibody-drug conjugate of the present invention is applicable may include lung cancer (e.g., non-small cell lung cancer, small cell lung cancer), kidney cancer, urothelial cancer, colorectal cancer, prostate cancer, glioblastoma multiforme, ovarian cancer (e.g., superficial epithelial tumor, stromal tumor, germ cell tumor), pancreatic cancer, breast cancer, melanoma, liver cancer, hepatocellular carcinoma, bladder cancer, gastric cancer, esophageal cancer, endometrial cancer, testicular cancer (seminoma, non-seminoma), cervical cancer, placental choriocarcinoma, brain tumor, head and neck cancer, thyroid cancer, mesothelioma, Gastrointestinal Stromal Tumor (GIST), gallbladder cancer, bile duct cancer, adrenal cancer, pharyngeal cancer, tongue cancer, auditory organ cancer, thymus cancer, small intestine cancer, squamous-cell carcinoma, leukemia, malignant lymphoma, plasmacytoma, myeloma, or sarcoma. The antibody-drug conjugate of the present invention can be also applied to EGFR-positive cancer or CDH6-positive cancer. The EGFR-positive cancer is cancer expressing EGFR. Examples thereof include lung cancer (e.g., non-small cell lung cancer, small cell lung cancer), kidney cancer, urothelial cancer, colorectal cancer, prostate cancer, glioblastoma multiforme, ovarian cancer (e.g., superficial epithelial tumor, stromal tumor, germ cell tumor), pancreatic cancer, breast cancer, melanoma, liver cancer, hepatocellular carcinoma, bladder cancer, gastric cancer, esophageal cancer, endometrial cancer, cervical cancer, brain tumor, head and neck cancer, thyroid cancer, mesothelioma, Gastrointestinal Stromal Tumor (GIST), gallbladder cancer, bile duct cancer, squamous-cell carcinoma,

pharyngeal cancer, tongue cancer, thymus cancer, and small intestine cancer. A CDH6-positive cancer is cancer expressing CDH6. Examples thereof include lung cancer (e.g., non-small cell lung cancer, small cell lung cancer), kidney cancer, urothelial cancer, ovarian cancer (e.g., superficial epithelial tumor, stromal tumor, germ cell tumor), pancreatic cancer, endometrial cancer, cervical cancer, thyroid cancer, mesothelioma, gallbladder cancer, bile duct cancer, and sarcoma. The antibody-drug conjugate of the present invention can be further applied to an EGFR signal mutation-positive cancer. An EGFR signal mutation-positive cancer includes a cancer having a mutation in EGFR (EGFR mutation-positive cancer) and a cancer having a mutation in an EGFR signaling molecule (cancer having a mutation in EGFR downstream signal). Examples of a cancer having a mutation in EGFR can include lung cancer and head and neck cancer having at least one mutation in exons 18 to 21, and preferred is lung cancer having an exon 19 mutation. Examples of a cancer having a mutation in an EGFR signaling molecule can include a cancer having at least one mutation in RAS, RAF, PI3K, or the like. Preferable examples thereof can include a RAS mutation cancer such as lung cancer, colorectal cancer, and pancreatic cancer having at least one mutation in RAS (KRAS, NRAS, HRAS), and more preferred is lung cancer or colorectal cancer having a KRAS mutation, and further preferred is lung cancer having a KRAS mutation. The cancer to which the antibody-drug conjugate of the present invention is applicable is not limited thereto so long as the cancer to be treated expresses a protein that can be recognized by the antibody in the antibody-drug conjugate.

The antibody-drug conjugate of the present invention can be preferably administered to mammals, and is more preferably administered to humans.

Substances used in a pharmaceutical composition containing the antibody-drug conjugate of the present invention may be suitably selected from formulation additives or the like that are generally used in the field in view of the dose or concentration for administration.

The antibody-drug conjugate of the present invention may be administered as a pharmaceutical composition containing one or more pharmaceutically applicable components. For example, the pharmaceutical composition typically contains one or more pharmaceutical carriers (e.g., sterilized liquid (including water and oil (petroleum oil and oil of animal origin, plant origin, or synthetic origin (such as peanut oil, soybean oil, mineral oil, and sesame oil)))). Water is a more typical carrier when the pharmaceutical composition above is intravenously administered. Saline solution, an aqueous dextrose solution, or an aqueous glycerol solution can be also used as a liquid carrier, in particular, for an injectable solution. Suitable pharmaceutical vehicles are known in the art. If desired, the composition above may also contain a trace amount of a moisturizing agent, an emulsifying agent, or a pH buffering agent. Examples of suitable pharmaceutical carriers are disclosed in "Remington's Pharmaceutical Sciences" by E. W. Martin. The formulations correspond to the mode of administration.

Various delivery systems are known and they may be used for administering the antibody-drug conjugate of the present invention. Examples of the administration route include, but not limited to, intradermal, intramuscular, intraperitoneal, intravenous, and subcutaneous routes. The administration may be by injection or bolus injection, for example. According to a specific preferred embodiment, the administration of the above antibody-drug conjugate is done by injection. Parenteral administration is a preferred administration route.

According to a representative embodiment, a pharmaceutical composition containing the antibody-drug conjugate is prescribed, as a pharmaceutical composition suitable for intravenous administration to humans, according to conventional procedures. A composition for intravenous administration is typically a solution in a sterile and isotonic aqueous buffer. If necessary, the pharmaceutical composition may contain a solubilizing agent and a local anesthetic to alleviate pain at an injection site (e.g., lignocaine). Generally, the ingredients above are provided either separately as a dried lyophilized powder or an anhydrous concentrate in sealed containers, such as an ampoule or a sachet, with an indication of the amount of the active agent, or as a mixture in a unit dosage form. When the pharmaceutical composition is to be administered by injection, it may be administered from an injection bottle containing water or saline of sterile pharmaceutical grade. When the pharmaceutical composition is administered by injection, an ampoule of sterile water or saline for injection may be provided so that the aforementioned ingredients are admixed with each other before administration. The pharmaceutical composition may be provided as a solution.

The pharmaceutical composition of the present invention may be a pharmaceutical composition containing only the antibody-drug conjugate of the present invention, or a pharmaceutical composition containing the antibody-drug conjugate of the present invention and other medicines (including cancer treating agents). The antibody-drug conjugate of the present invention may be administered with (simultaneously with, separately from, or in a staggered manner) or in combination with one or two or more other medicines (including, but not limited to, cancer treating agents), and thereby the anti-tumor effect may be enhanced or the safety may be improved. Other medicines used for such a purpose may be administered to an individual simultaneously with, separately from, or subsequently to the antibody-drug conjugate, and may be administered while varying the administration interval for each. Examples of such other cancer treating agents may include hormone regulators (e.g., LH-RH analogs (e.g., leuprorelin, goserelin, estramustine), estrogen antagonists (e.g., tamoxifen, raloxifene)), aromatase inhibitors (e.g., anastrozole, letrozole, exemestane), kinase inhibitors, PARP inhibitors, bone destruction inhibitors, osteogenesis promoters, metastasis inhibitors, anti-regulatory T-cell drugs (e.g., an anti-GITR antibody, an anti-GARP antibody, an anti-TIGIT antibody, an anti-CCR8 antibody), immune activators (e.g., an

anti-4-1BB antibody, an anti-OX40 antibody, an anti-CD40 antibody, an anti-CD3 antibody, an anti-CD28 antibody, an IL-2 analog, cytokines, an TLR agonist), immunomodulators (e.g., an anti-CD47 antibody, an anti-SIRPα antibody, inhibitory myeloid modulators), antibody medicines with ADCC (Antibody Dependent Cellular Cytotoxicity) activity, ADCP (Antibody Dependent Cellular Phagocytosis) activity, or complement activity, BiTE (Bi-specific T-cell engagers), ADCs in combination with photodynamic therapy, as well as anti-tumor vaccines, anti-tumor cell therapy (e.g., CART, TCR-T, dendritic cells, NK cells), anti-tumor bacterial treatments, or anti-tumor viral treatments. However, the cancer treating agents are not limited so long as the agents have an anti-tumor activity. In addition, the antibody-drug conjugate of the present invention can be administered together with another antibody-drug conjugate having an anti-tumor activity. This can enhance the anti-tumor effects. Besides, the antibody-drug conjugate of the present invention can enhance the anti-tumor effects not only by the drug alone, but also by use in combination with an anti-tumor effect-exerting treatment (e.g., radiation, heavy particle radiation, surgery, bone marrow transplantation). The treatment is not limited as long as it has the anti-tumor effects.

The pharmaceutical composition can be formulated as a lyophilization formulation or a liquid formulation as a formulation having the selected composition and required purity. When formulated as a lyophilization formulation, it may be a formulation containing suitable formulation additives that are used in the art. Also, for a liquid formulation, it may be formulated as a liquid formulation containing various formulation additives that are used in the art.

The composition and concentration of the pharmaceutical composition may vary depending on the administration method. However, the antibody-drug conjugate contained in the pharmaceutical composition of the present invention can exhibit a pharmaceutical effect even at a small dosage when the antibody-drug conjugate has a higher affinity for an antigen, that is, a higher affinity (lower Kd value) in terms of the dissociation constant (Kd value) for the antigen. Thus, for determining the dosage of the antibody-drug conjugate, the dosage may be set in view of the situation relating to the affinity of the antibody-drug conjugate with the antigen. When the antibody-drug conjugate of the present invention is administered to a human, for example, about 0.001 to 100 mg/kg can be administered once or administered in several portions with intervals of 1 to 180 days.

<5. Method for Producing Fc-Containing Molecule>

In one aspect, the present invention provides a method for producing an Fc-containing molecule having an N297-linked glycan including a glycan donor molecule-derived glycan.

In one aspect, the present invention provides a method for producing an Fc-containing molecule having an N297-linked glycan including a glycan donor molecule-derived glycan, the method comprising the following step 1:
(Step 1) producing a reaction mixture by reacting an acceptor molecule which is an Fc-containing molecule having optionally fucosylated core GlcNAc as the N297-linked glycan with a glycan donor molecule comprising a glycan comprising GlcNAc with an unactivated reducing terminal in the presence of

- endo-β-N-acetylglucosaminidase whose substrate is the N297-linked glycan of the Fc-containing molecule (enzyme-A);
- endo-β-N-acetylglucosaminidase whose substrate is the glycan of the glycan donor molecule (enzyme-B); and
- an additive selected from a monovalent salt, an organic solvent, a surfactant, a saccharide, an amino acid, or a combination of any of them.

<N297-Linked Glycan>

In one aspect of the present invention, "N297-linked glycan" means an N-linked glycan in a state linked to a side chain of Asn at position 297 of an IgG heavy chain. Even if the position of addition of the N-linked glycan to the IgG heavy chain is changed by a given technique, the resulting N-linked glycan is included in the N297-linked glycan so long as the N-linked glycan receives the action of enzyme-A. In one aspect of the present invention, for example, in the case of fragmenting an IgG heavy chain, a corresponding glycan linked to the Asn in a peptide fragment containing the Asn is also included in the N297-linked glycan. Usually, the N297-linked glycan in IgG produced in an animal or the like has a basic structure consisting of a structure of the following formula (I) or (II), and the non-reducing terminal may be further chemically modified and, for example, Gal or a sialic acid may be added thereto.

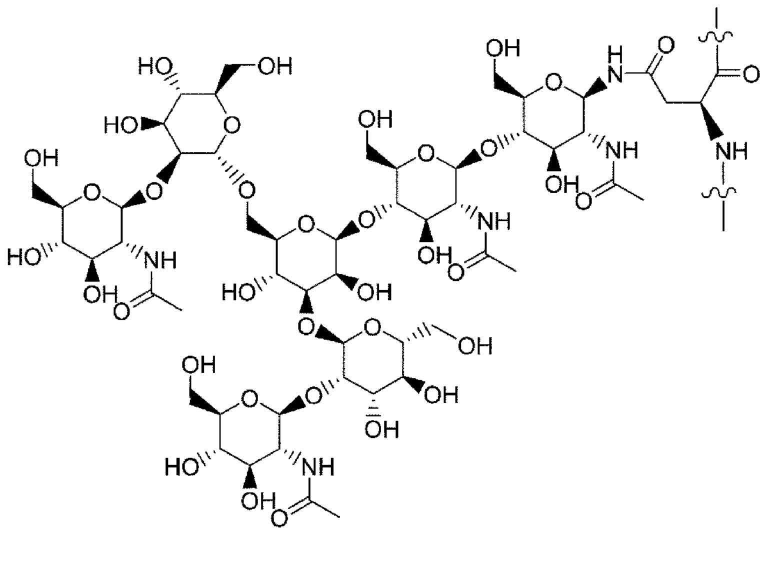

(I)

GlcNAcβ1-2Manα1 - 6
Manβ1-4GlcNAcβ1-4GlcNAcβ1-(Asn297)
GlcNAcβ1-2Manα1 - 3

(II)

Most of the N297-linked glycans of IgG produced by cells have diverse glycan structures including a glycan further linked to GlcNAc at the reducing terminal (core GlcNAc), the non-reducing terminal, branched sugar, or the like in the basic structure. The N297-linked glycan may have a structure having a core fucose in which fucose (Fuc) is α1,6 linked to the 6-position of core GlcNAc ((Fucα1,6)GlcNAc). The branched sugar Man may form a three-branched glycan in which a glycan containing GlcNAc is further linked to the 5-position thereof. A glycan containing galactose (Gal) or a sialic acid (Sia) may be further linked to GlcNAc at the non-reducing terminal.

<Glycan Donor Molecule>

In one aspect of the present invention, "glycan donor molecule" (also referred to as "glycan donor") means a molecule that donates a glycan to an acceptor molecule.

For use in glycan remodeling aimed at drug development, it is preferable to adopt a glycan donor molecule having a human glycan or a human-compatible glycan, which presents few problems when applied to humans. Such a glycan is known to exhibit no antigenicity in the human body. For example, high mannose, hybrid, and complex types are known as N-linked glycans. These three types of glycans have a common basic structure. The high mannose type is a glycan having a mannose-rich structure where a plurality of mannose residues are connected to two branched chains (1-3 and 1-6 chains) from mannose (β-mannose) positioned near the reducing terminal. The hybrid type assumes a structure having GlcNAc in one of the two branched chains (1-3 and 1-6 chains) from mannose (β-mannose) positioned near the reducing terminal. The complex type assumes a structure having GlcNAc in both the two branched chains (1-3 and 1-6 chains) from mannose (β-mannose) positioned near the reducing terminal and has various structures including the presence or absence of galactose, the presence or absence of a sialic acid, and binding isomerism and positional isomerism thereof. Biantennary, triantennary, and tetraantennary complex-type glycans are known.

Structural examples of the high mannose, hybrid, and complex types are shown below. Note that their common basic structures are boxed in the dotted line (the structures boxed in the dotted line include both the presence and absence of GlcNAc (bisecting GlcNAc) introduced to the 4-position of β-mannose, and both the structures correspond to the common basic structures herein).

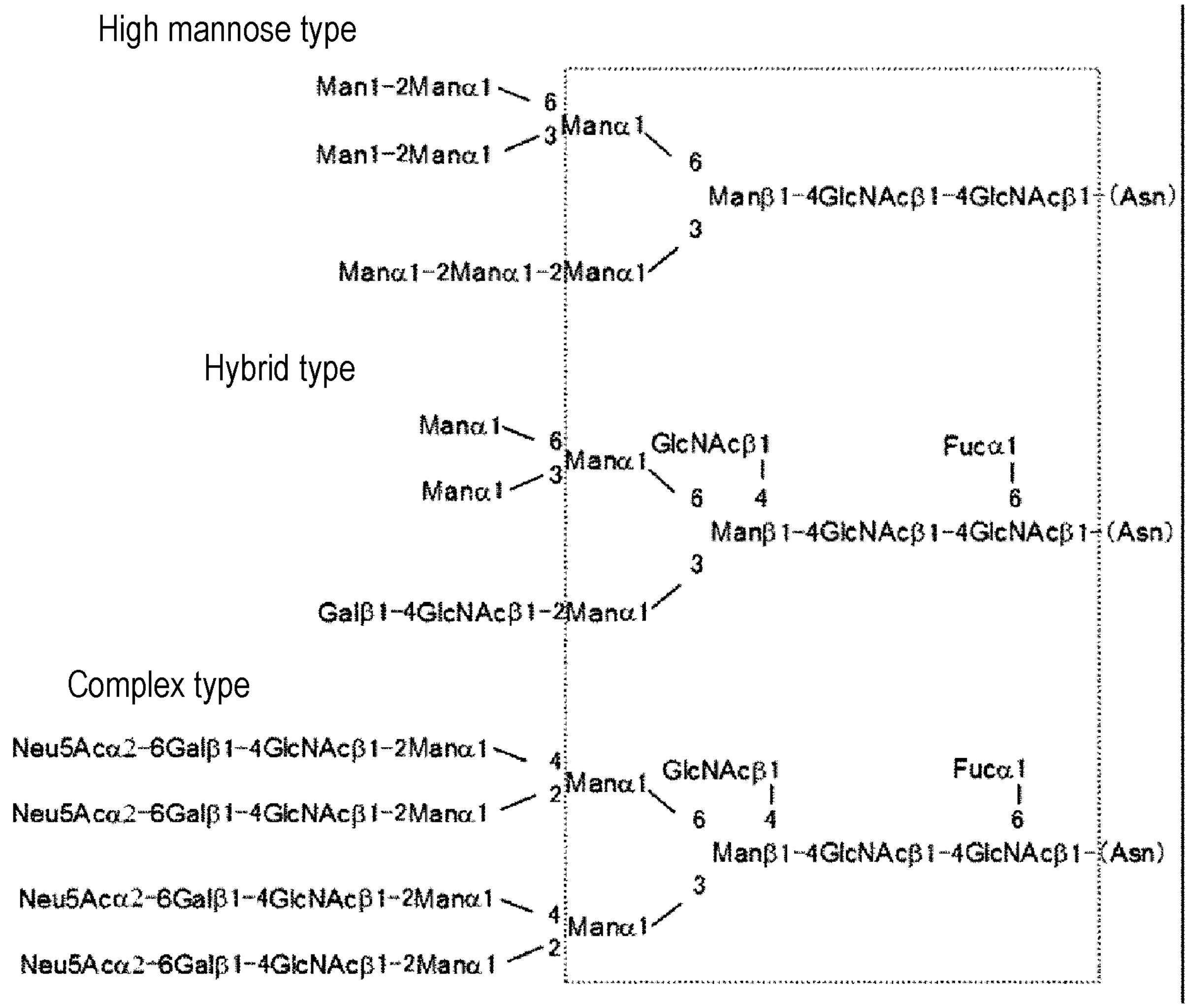

High mannose type

Hybrid type

Complex type

In one aspect of the present invention, the glycan moiety in the glycan donor molecule may be any of high mannose-type, hybrid-type, and complex-type glycans, and is preferably a high mannose-type or complex-type glycan, and particularly preferably a complex-type glycan. Thus, in one aspect of the present invention, the glycan donor molecule comprises a complex-type glycan. In the present invention, the complex-type glycan may be biantennary, triantennary, or tetraantennary. Preferably, the glycan donor molecule of the present invention comprises a biantennary complex-type glycan.

In one aspect of the present invention, the glycan donor molecule is a glycan-containing molecule having GlcNAc with an unactivated reducing terminal of the glycan. Preferable examples thereof can include SGP, (SG-)Asn, (MSG1-)Asn, (MSG2-)Asn, and a mixture of (MSG1-)Asn and (MSG2-)Asn. These glycan moieties are representative N-linked glycans and include Sialyl Glycan (hereinafter, referred to as "SG") composed of a structure represented by the structural formula or the sequence formula given below. The linking mode of a reducing terminal-side glycan may be changed from the N-linked form to an O-linked form. Note that herein, a substructure linked to a glycan, in a side chain of an amino acid, is represented by, for example, "(SG-)Asn" as described above wherein the side chain moiety is included in the parentheses, unless otherwise specified.

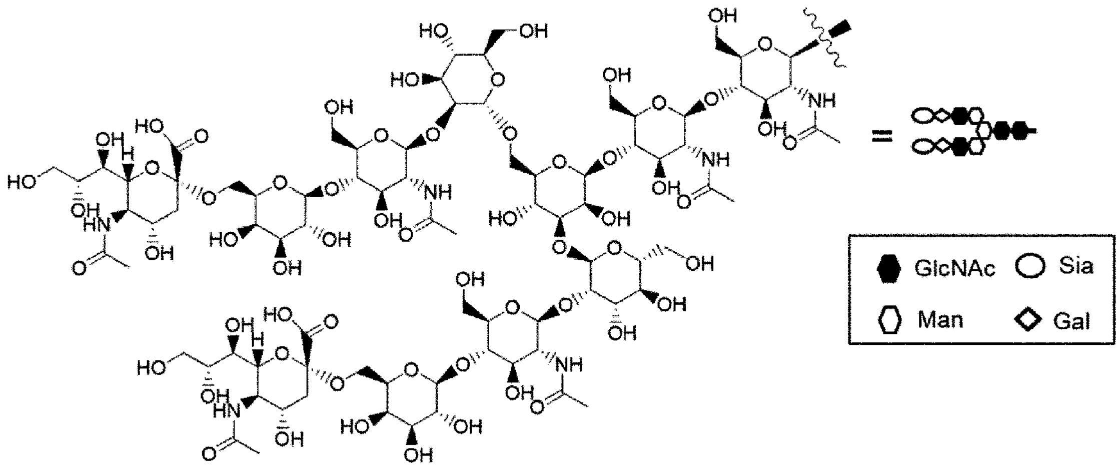


NeuAcα2-6Galβ1-4GlcNAcβ1-2Manα1 - 6
Manβ1-4GlcNAcβ1-4GlcNAcβ1-(N/O)
NeuAcα2-6Galβ1-4GlcNAcβ1-2Manα1 - 3

[SG]

wherein "-(N/O)" represents binding to a N atom or an O atom via a glycosidic bond.

Specific examples of the glycan moiety in the glycan donor molecule that can be used in the present invention can include AG(9) which has two non-reducing terminal NeuAc residues removed by the neuraminidase treatment of SG (AG(9) of the following structural formula and sequence formula), and AG(7) which has two non-reducing terminal Gal residues removed by the galactosidase treatment of AG(9) (AG(7) of the following structural formula and sequence formula).

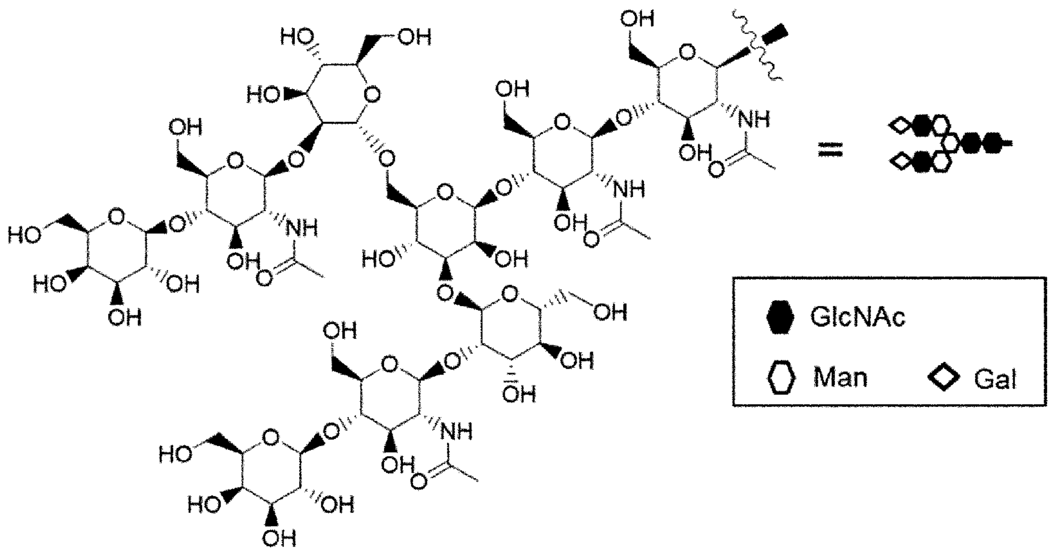


Galβ1-4GlcNAcβ1-2Manα1 -6
Manβ1-4GlcNAcβ1-4GlcNAcβ1-(N/O)
Galβ1-4GlcNAcβ1-2Manα1 -3

[AG(9)]

wherein "-(N/O)" represents binding to a N atom or an O atom via a glycosidic bond.

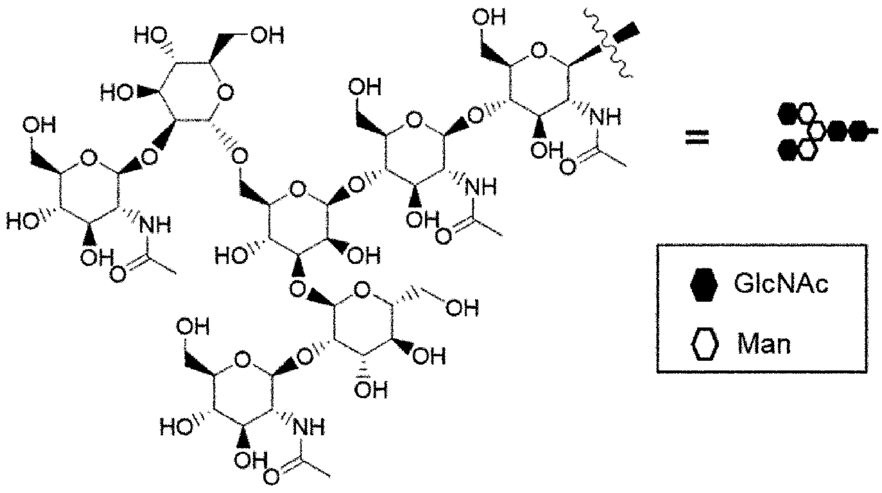


GlcNAcβ1-2Manα1 -6
Manβ1-4GlcNAcβ1-4GlcNAcβ1-(N/O)
GlcNAcβ1-2Manα1 -3

[AG(7)]

wherein "-(N/O)" represents binding to a N atom or an O atom via a glycosidic bond.

In the present invention, the glycan donor molecule may be chemically modified and, for example, its non-reducing terminal may be chemically modified.

Examples of the glycan moiety in the glycan donor molecule with an optionally chemically modified non-reducing terminal that can be used in the present invention include an SG-type derivative in which a site on the wild-type sialic acid is chemically modified with a given substituent.

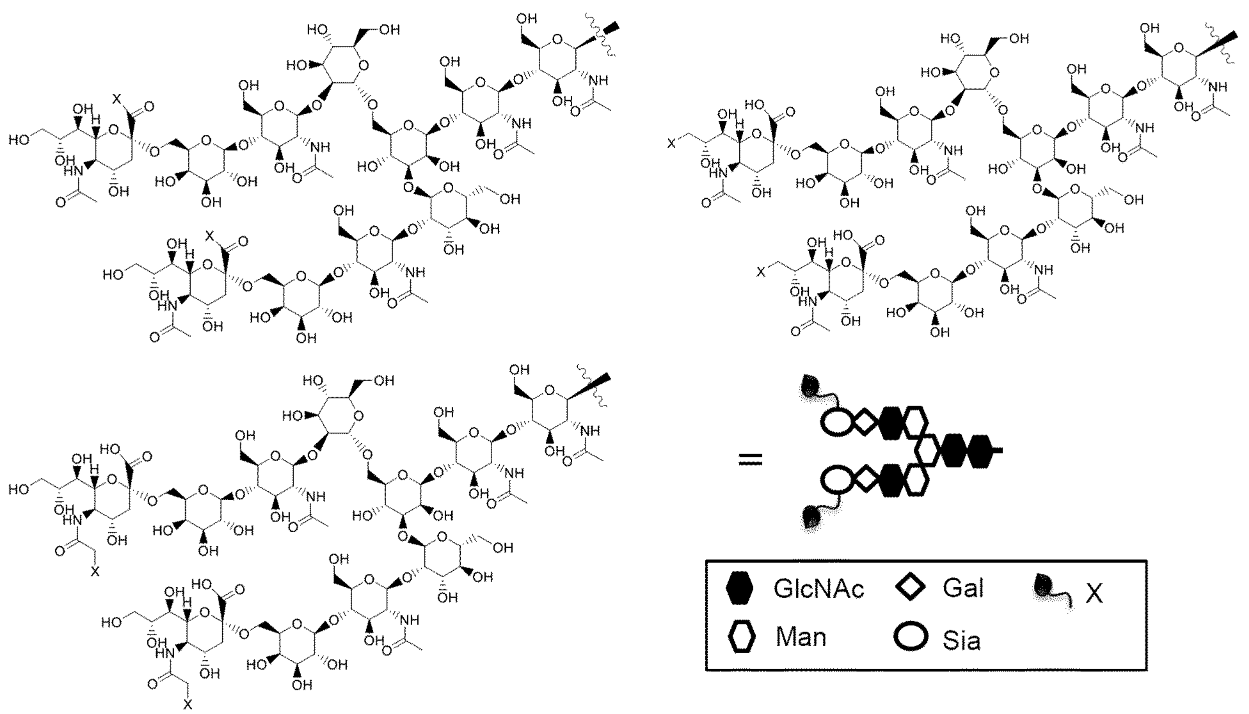

(X)-NeuAcα2-6Galβ1-4GlcNAcβ1-2Manα1 - 6
Manβ1-4GlcNAcβ1-4GlcNAcβ1-(N/O)
(X)-NeuAcα2-6Galβ1-4GlcNAcβ1-2Manα1 - 3

[X-SG]

wherein X represents a substituent introduced to sialic acids at the non-reducing terminals of both the 1-3 and 1-6 branched chains of β-Man, the substituent can be synthesized by use of various methods known in the field of organic synthetic chemistry, and with reference to substituents used in binding methods applied to antibody-drug conjugate synthesis (Bioconjugate Chem. 2015, 26, 2198-2215), examples thereof can include an acetylene group, an azide group ($N_3$-), a dibenzylcyclooctene (DBCO) group, a bicyclo[6.1.0]non-4-ene (BCN) group, a structure having a 1,2,4,5-tetrazine ring, an aldehyde group, an SH group, and a methylsulfonyltriazole group, and can preferably include an acetylene group and an azide group ($N_3$-), and further preferably a substituent having an azide group ($N_3$-), though the substituent is not limited thereto; "substituent having an azide group ($N_3$-)" is, for example, a polyethylene glycol linker having $N_3$, namely the upper limit of the number of ethylene glycol units ($-CH_2-CH_2-O-$) contained in the linker structure is about 20 or less, preferably about 11 or less, and more preferably about 7 or less, and examples of "substituent having an azide group ($N_3$-)" can include, but are not limited to, polyethylene glycol linkers having $N_3$ (for example, linear substituents such as a 35-azido-3,6,9,12,15,18,21,24,27,30,33-undecaoxapentatriacontan-1-amino group, a 32-azido-3,6,9,12,15,18,21,24,27,30-decaoxadotriacontan-1-amino group, a 29-azido-3,6,9,12,15,18,21,24,27-nonaoxanonacosan-1-amino group, a 26-azido-3,6,9,12,15,18,21,24-octaoxahexacosan-1-amino group, a 23-azido-3,6,9,12,15,18,21-heptaoxatricosan-1-amino group, a 20-azido-3,6,9,12,15,18-hexaoxaeicosan-1-amino group, a 17-azido-3,6,9,12,15-pentaoxaheptadecan-1-amino group, a 14-azido-3,6,9,12-tetraoxatetradecan-1-amino group, a 11-azido-3,6,9-trioxaundecan-1-amino group, a 2-[2-(2-azidoethoxy)ethoxy]ethylamino group, a 2-(azidoethoxy)ethylamino group, a 2-azidoethylamino group, and a 2-[2-[2-(2-azidoethoxy)ethoxy]-N-[2-[2-[2-(2-azidoethoxy)ethoxy]ethoxy]ethyl]ethanamine group, or branched substituents such as a 1,3-bis[2-[2-(2-azidoethoxy)ethoxy]ethoxy]propan-2-amino group and a 3-[2-[2-(2-azidoethoxy)ethoxy]ethoxy]-2-[2-[2-(2-azidoethoxy)ethoxy]ethoxymethyl]propan-1-amino group); and "-(N/O)" represents binding to a N atom or an O atom via a glycosidic bond.

Among the X-SG-type structures shown here, X-MSG1 type which is a structure lacking a chemically modified sialic acid in a 1-6 branched chain of β-Man, and X-MSG2 type which is a structure lacking a chemically modified sialic acid in a 1-3 branched chain thereof are also included in the examples of the glycan moiety in the glycan donor molecule with an optionally chemically modified non-reducing terminal.

The optionally chemically modified site may be a site other than sialic acid. Examples thereof include the following AG(9)-type and AG(7)-type derivatives.

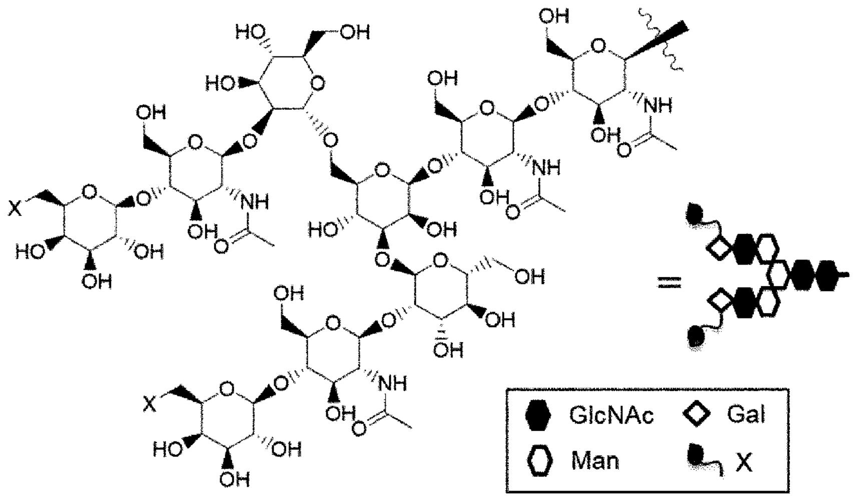


(X)-Galβ1-4GlcNAcβ1-2Manα1 - 6
Manβ1-4GlcNAcβ1-4GlcNAcβ1-(N/O)
(X)-Galβ1-4GlcNAcβ1-2Manα1 -3

[X-AG (9)]

wherein X represents a substituent introduced to galactose residues at the non-reducing terminals of both the 1-3 and 1-6 branched chains of β-Man and specifically, is as defined above; and "-(N/O)" represents binding to a N atom or an O atom via a glycosidic bond.

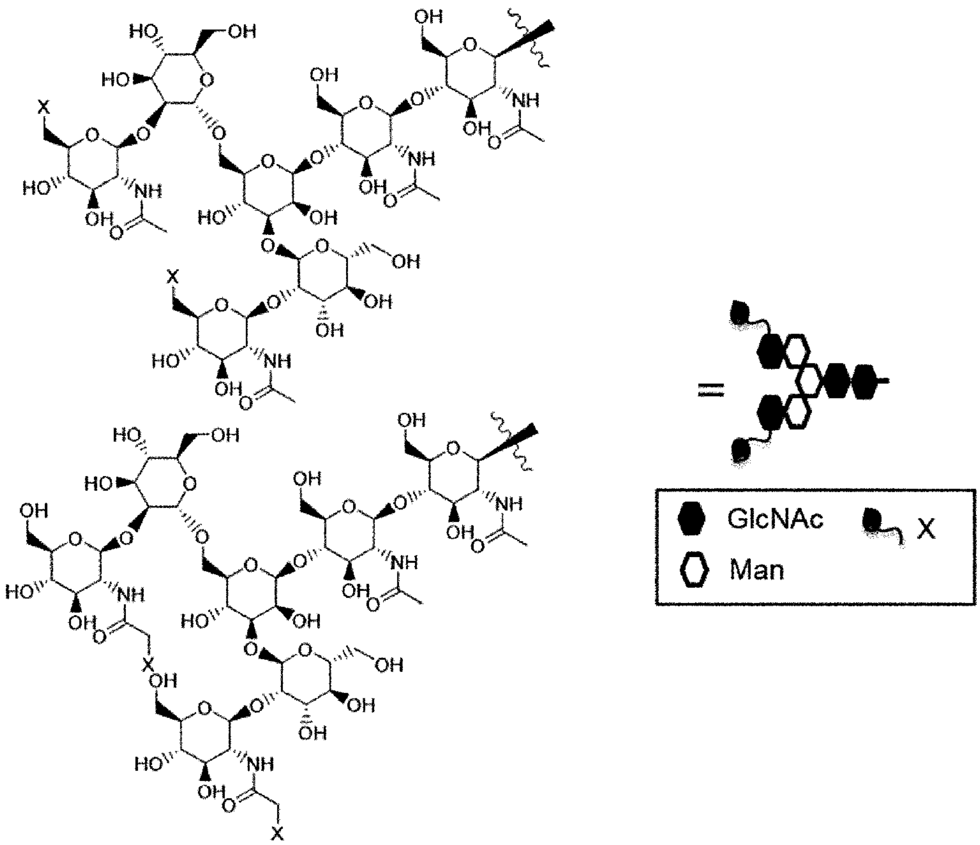


(X)-GlcNAcβ1-2Manα1 -6
Manβ1-4GlcNAcβ1-4GlcNAcβ1-(N/O)
(X)-GlcNAcβ1-2Manα1 -3

[X-AG (7)]

wherein X represents a substituent introduced to GlcNAc residues at the non-reducing terminals of both the 1-3 and 1-6 branched chains of β-Man and specifically, is as defined above; and "-(N/O)" represents binding to a N atom or an O atom via a glycosidic bond.

Among the X-AG(9)-type structures shown here, a structure lacking chemically modified galactose in a 1-6 branched chain of β-Man and a structure lacking chemically modified galactose in a 1-3 branched chain thereof, or among the X-AG(7)-type structures, a structure lacking chemically modified GlcNAc in a 1-6 branched chain of β-Man and a structure lacking chemically modified GlcNAc in a 1-3 branched chain thereof are also included in the examples of the glycan moiety in the donor molecule.

Examples of the glycan donor molecule of the present invention can preferably include SGP, AG(9)-P, AG(7)-P, SG-Asn, AG(9)-Asn, AG(7)-Asn, SG-OR, AG(9)-OR, AG(7)-OR, (MSG1)-Asn, (MSG2)-Asn, a mixture of (MSG1)-Asn and (MSG2)-Asn, MSG1-OR, MSG2-OR, and a mixture of MSG1-OR and MSG2-OR, with an optionally chemically modified non-reducing terminal, or can include glycan donor molecules having the following structures.

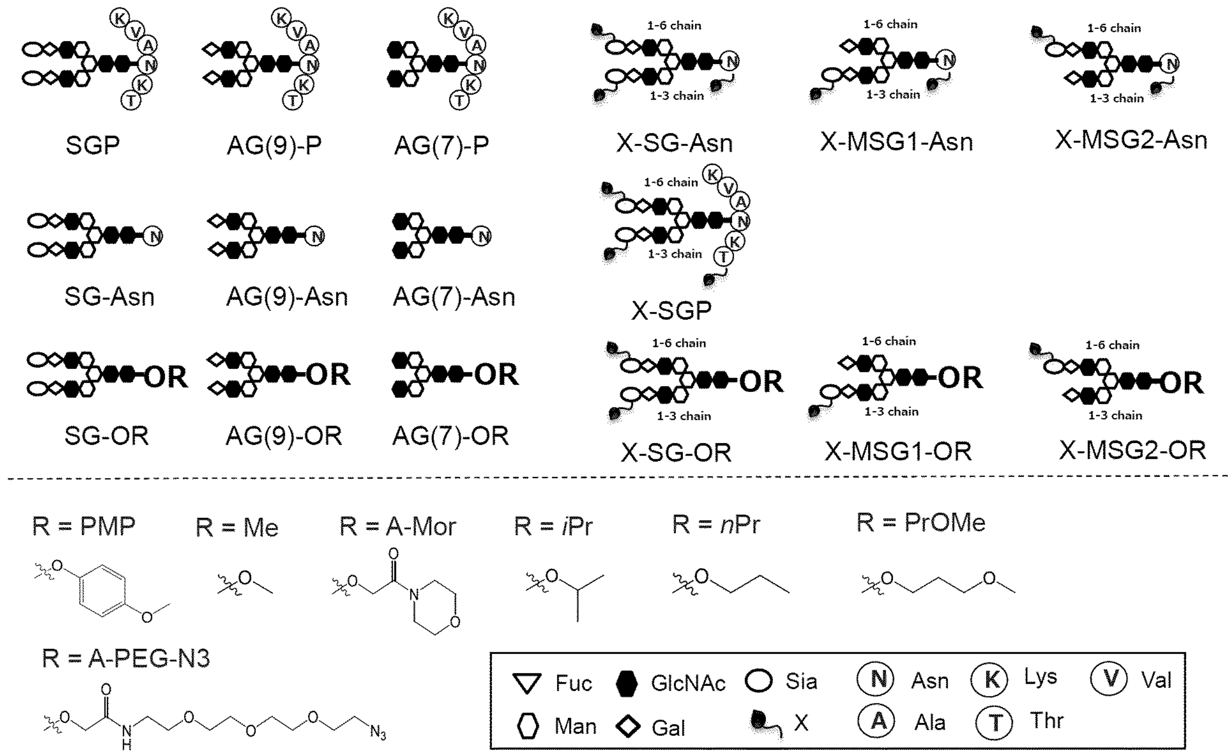

SGP AG(9)-P AG(7)-P X-SG-Asn X-MSG1-Asn X-MSG2-Asn

SG-Asn AG(9)-Asn AG(7)-Asn X-SGP

SG-OR AG(9)-OR AG(7)-OR X-SG-OR X-MSG1-OR X-MSG2-OR

R = PMP R = Me R = A-Mor R = *i*Pr R = *n*Pr R = PrOMe

R = A-PEG-N3

Fuc GlcNAc Sia Asn Lys Val

Man Gal X Ala Thr

Note that in the above structural formulas, "P" represents a peptide moiety derived from SGP, and "X" is as defined above. Examples of the glycan donor molecule of the present invention can further preferably include ([$N_3$-PEG(3)]$_2$-SG)-P-PEG(3)-$N_3$ ([$N_3$-PEG(3)]$_2$-SG)-Asn-PEG(3)-$N_3$, ([$N_3$-PEG(3)]-MSG1)-Asn-PEG(3)-$N_3$, ([$N_3$-PEG(3)]-MSG2)-Asn-PEG(3)-$N_3$, and a mixture of ([$N_3$-PEG(3)]-MSG1)-Asn-PEG(3)-$N_3$ and ([$N_3$-PEG(3)]-MSG2)-Asn-PEG(3)-$N_3$, including an N-linked glycan; and ([$N_3$-PEG(3)]$_2$-SG)-OR, ([$N_3$-PEG(3)]-MSG1)-OR, ([$N_3$-PEG(3)]-MSG2)-OR, and a mixture of ([$N_3$-PEG(3)]-MSG1)-OR and ([$N_3$-PEG(3)]-MSG2)-OR, including an O-linked glycan.

In the above chemical formulas, "R" represents a given substituent linked to an oxygen atom via at least one carbon atom, and preferably represents a C1 to C6 alkyl group or an optionally substituted aryl group. "C1 to C6 alkyl group" means a linear or branched alkyl group having 1 to 6 carbon atoms. Examples thereof can include, but are not limited to, a methyl group, an ethyl group, a n-propyl group, an i-propyl group, a n-butyl group, an i-butyl group, a s-butyl group, a t-butyl, a n-pentyl group, and a n-hexyl group. Examples of "aryl group" can include, but are not limited to, a phenyl group, a benzyl group, an indenyl group, a naphthyl group, a fluorenyl group, an anthranyl group, and a phenanthrenyl group. Examples of the substituent for the aryl group can include, but are not limited to, C1 to C6 alkoxy groups (for example, a methoxy group, an ethoxy group, an isopropoxy group), C1 to C6 alkyl groups (for example, a methyl group, an ethyl group), and halogen groups. Examples of the substituted aryl group can include, but are not limited to, a p-methoxyphenyl group and a p-methylphenyl group. In one aspect of the present invention, "R" is selected from the group consisting of PMP, Me, A-Mor, iPr, nPr, PrOMe, and A-PEG-$N_3$ (wherein A represents an acetyl group in a glycolic acid unit, namely a moiety sandwiched between an anomer hydroxy group and an amino group in Mor or PEG).

In one aspect of the present invention, preferable examples of the glycan donor molecule can include the following glycan donor molecules.

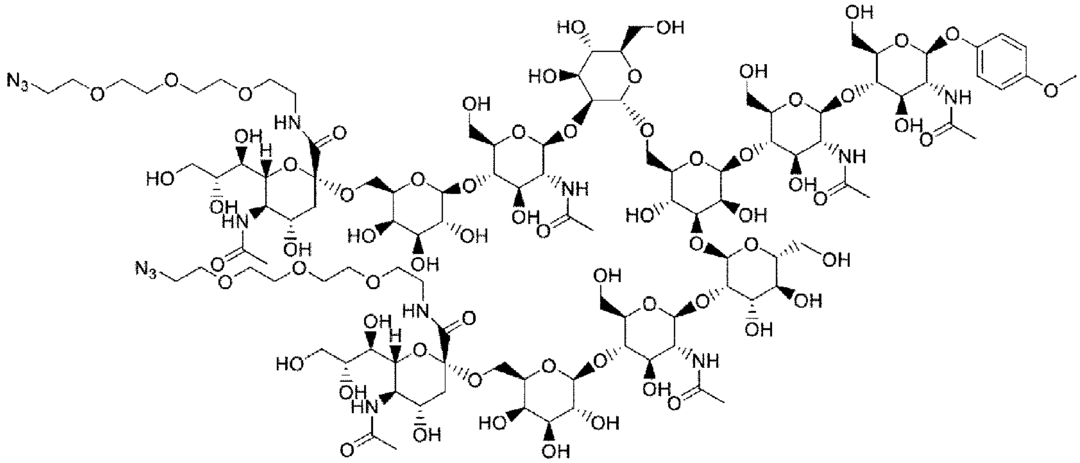

G-6

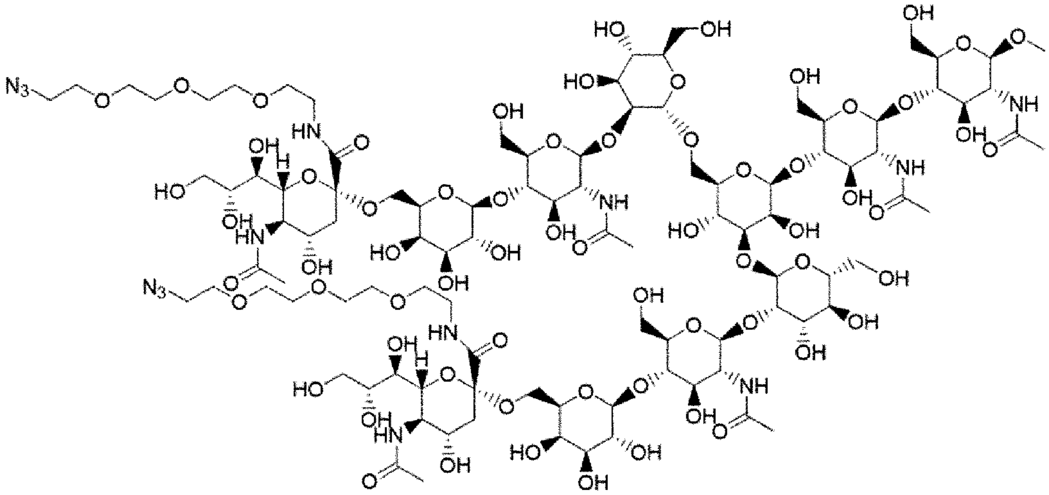

G-7

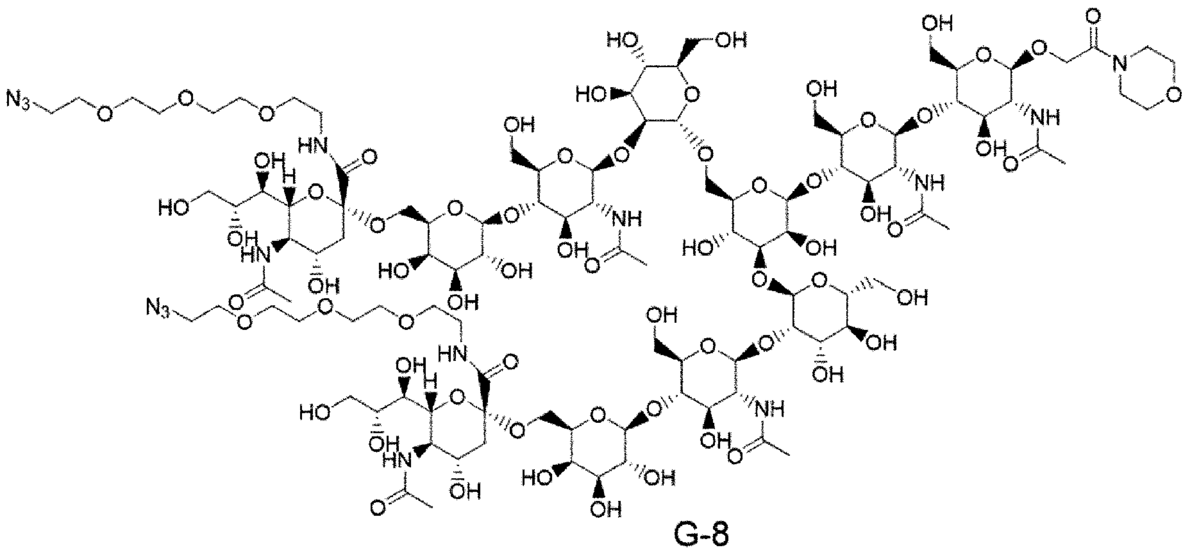

G-8

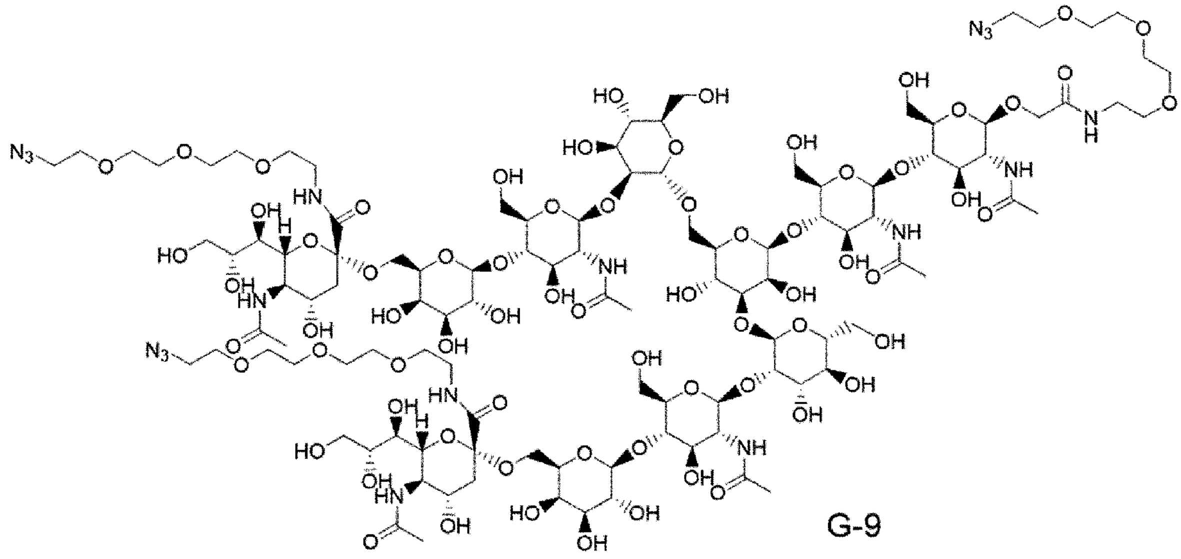

G-9

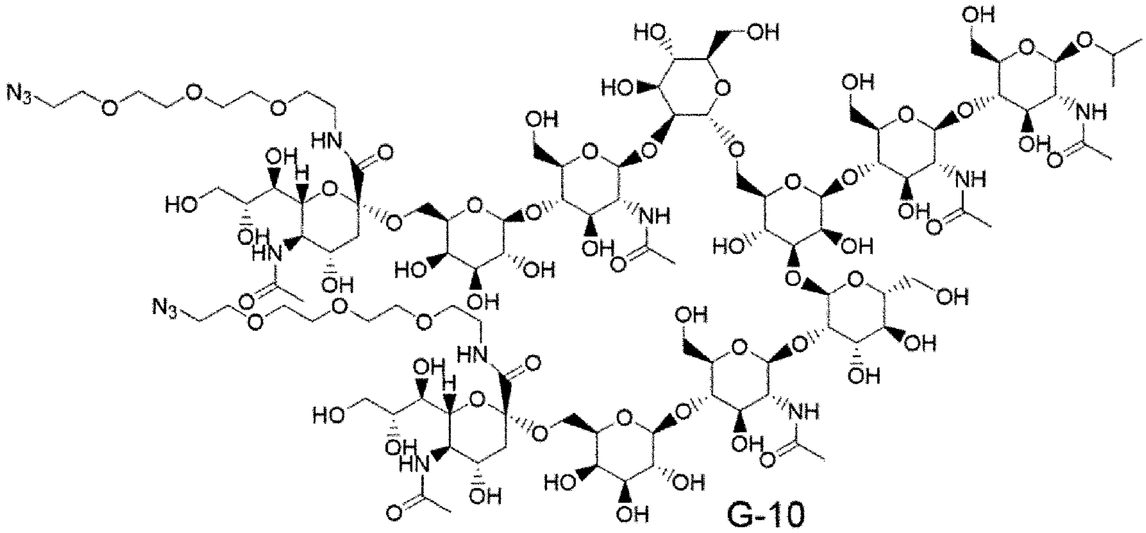

G-10

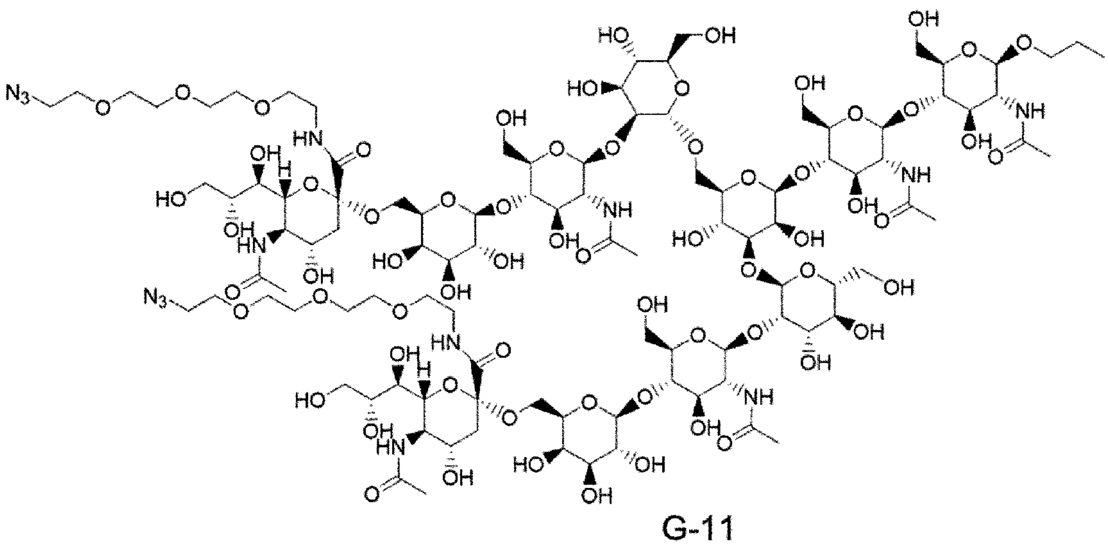

G-11

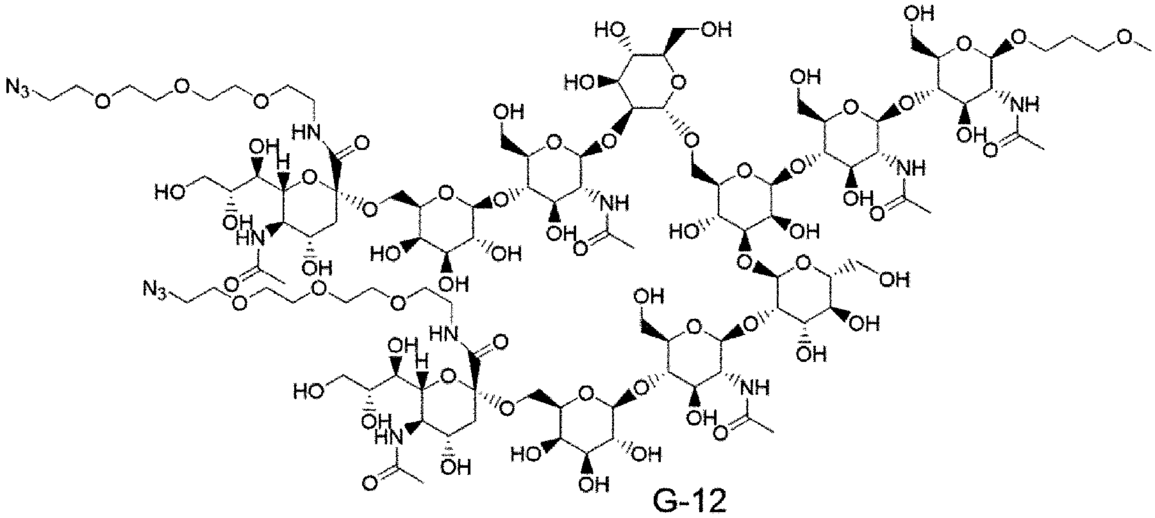

G-12

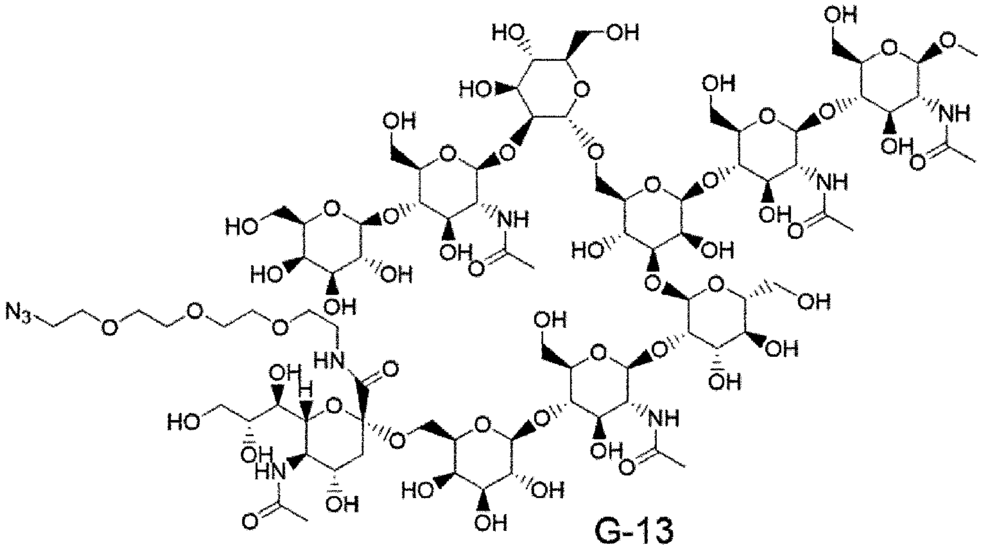

G-13

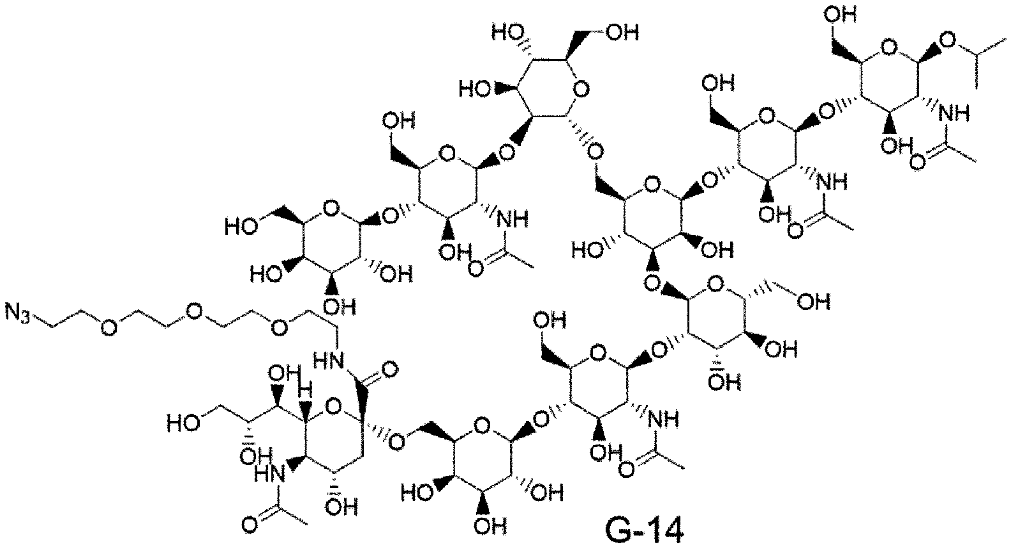

G-14

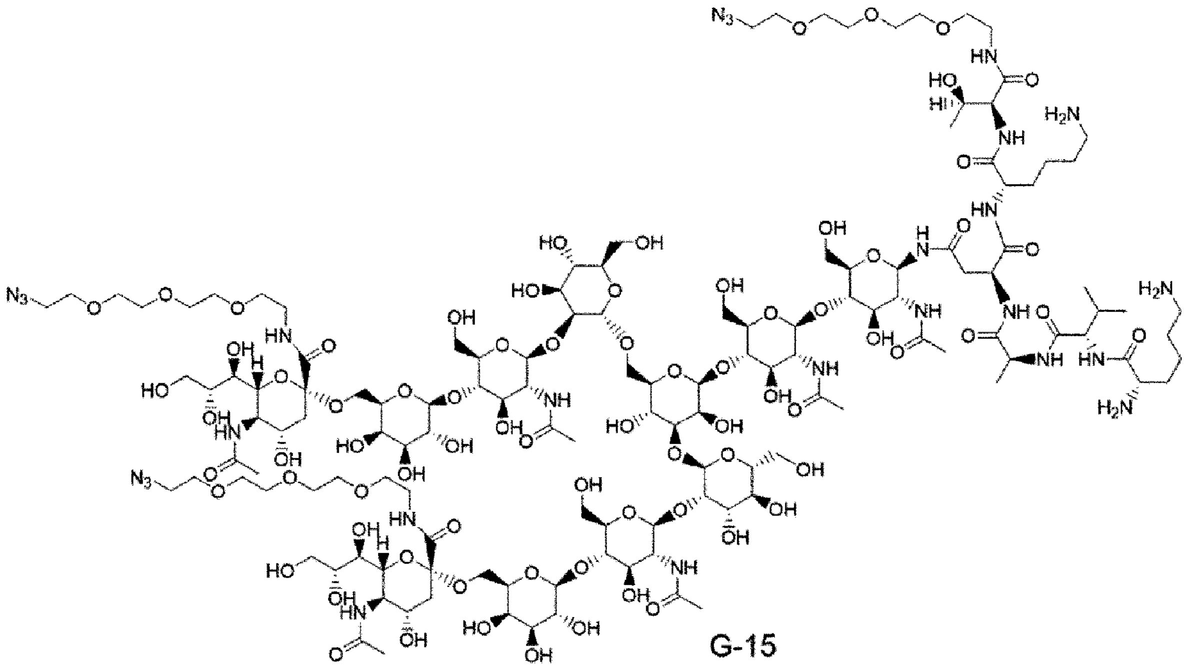

G-15

and

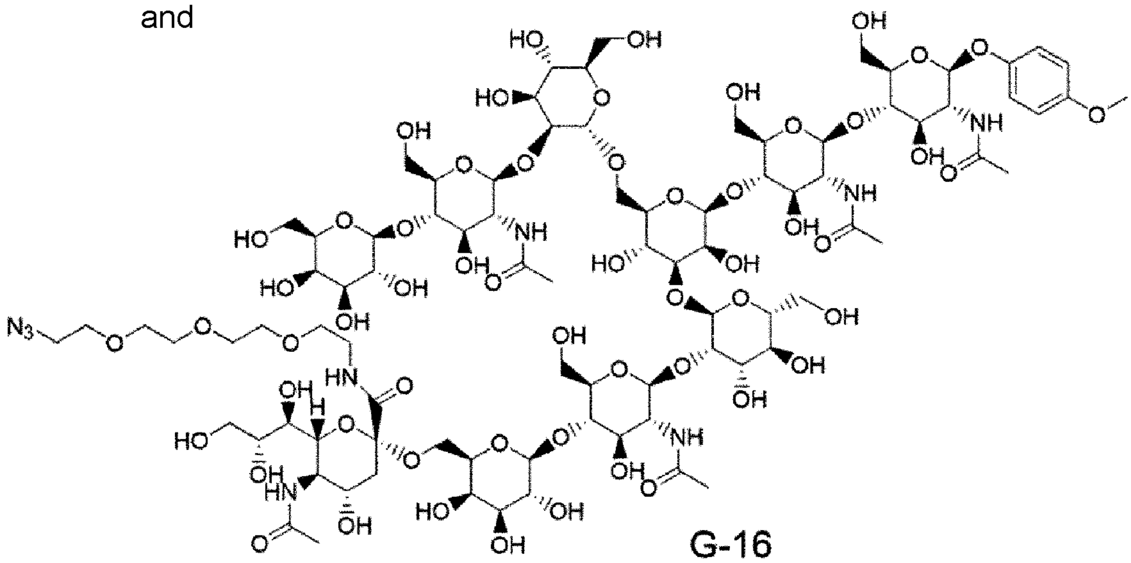

G-16

It is preferable to appropriately select the glycan donor molecule of the present invention according to conditions such as the combination of enzyme-A and enzyme-B used.

In one aspect of the present invention, the glycan donor molecule comprises a high mannose-type glycan. Examples of such a glycan donor molecule can include, but are not limited to, Man9-GlcNAc$_2$-Asn ("M9-Asn") having the following structure, and Man8-GlcNAc$_2$-Asn ("M8-Asn"), Man7-GlcNAc$_2$-Asn ("M7-Asn"), and Man6-GlcNAc$_2$-Asn ("M6-Asn") having a structure obtained by deleting one, two, or three mannose residues from M9-Asn.

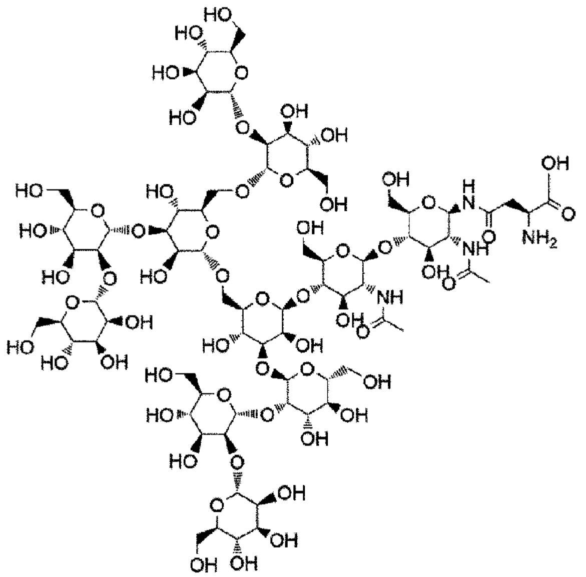

Manα1-2Manα1-6
Manα1-6
Manα1-2Manα1-3
Manβ1-4GlcNAcβ1-4GlcNAcβ1-Asn
Manα1–2Manα1-2Manα1-3

[ Man9-GlcNAc$_2$-Asn ] = [ M9-Asn ]

The glycan donor molecule can be appropriately purchased as a commercially available product or prepared by use or application of a known method.

<Fc-Containing Molecule>

In one aspect of the present invention, "Fc-containing molecule" (also referred to as "Fc region-containing molecule") refers to a molecule that contains an Fc region. Examples thereof can include antibodies (immunoglobulins), Fc-fusion proteins, and Fc fragments (or Fc-containing fragments). Examples of the Fc-containing fragments other than antibodies can include, but are not limited to, CLCH, for example, CLCH consisting of IgG (particularly, IgG-type monoclonal antibody) Fc fragments or constant regions (Patent Literature 1), and a bispecific antibody comprising Fc fragments (Expert Opin Ther Pat. 2018 28, 251-276). In one aspect of the present invention, the Fc-containing molecule is an antibody or a functional fragment of the antibody, and the antibody or the functional fragment can include all of those described above. Thus, in one aspect of the present invention, the Fc-containing molecule is, for example, an antibody or a functional fragment of the antibody, wherein the amino acid residues at positions 234, 235, and 265 (all according to EU numbering) of an Fc region are Ala, Ala, and Gly, respectively. In another aspect of the present invention, the Fc-containing molecule is an antibody comprising a light chain consisting of an amino acid sequence set forth in SEQ ID NO: 30 and a heavy chain consisting of an amino acid sequence set forth in SEQ ID NO: 33. In a further alternative aspect of the present invention, the Fc-containing molecule is an antibody comprising a light chain consisting of an amino acid sequence set forth in SEQ ID NO: 44 and a heavy chain consisting of an amino acid sequence set forth in SEQ ID NO: 46.

<Acceptor Molecule>

In one aspect of the present invention, "acceptor molecule" means a molecule that receives a glycan from the glycan donor molecule. The acceptor molecule is typically a monoclonal antibody-derived IgG having an N297-linked glycan consisting of only core GlcNAc to which core Fuc may be linked, or an Fc fragment thereof. Note that herein, "core Fuc" (or "core fucose") means fucose linked to the core GlnNAc. The core Fuc may or may not be linked to the core GlcNAc according to the antibody of its origin or a production method therefor. Thus, in one aspect of the present invention, the acceptor molecule is an Fc-containing molecule (for example, an antibody or its Fc-containing fragment) having optionally fucosylated core GlcNAc as an N297-linked glycan. Preferable examples thereof can include an Fc-containing molecule (for example, an antibody or its Fc-containing fragment) having an N297-linked glycan consisting of GlcNAc or (Fucα1,6) GlcNAc. Various monoclonal antibodies or glycan-containing molecules or Fc region-containing molecules (for example,

Fc, CLCH composed of a combination of CH consisting of only a heavy chain constant region without a variable region and CL consisting of only a light chain constant region) can be used as the origin of the acceptor molecule. Preferable examples of the acceptor molecule of the present invention include (Fucα1,6)-GlcNAc-IgG (for example, (Fucα1,6)GlcNAc-mAb1), (Fucα1,6)-GlcNAc-Fc, (Fucα1,6)-GlcNAc-CLCH, and GlcNAc-mAb4.

In one aspect of the invention, the Fc-containing molecule having an N297-linked glycan can be derived from an antibody.

The acceptor molecule can be appropriately purchased as a commercially available product or prepared by use or application of a known method. Typically, the acceptor molecule can be prepared by treating the N297-linked glycan of an Fc-containing molecule (typically, an Fc-containing molecule such as an IgG-type monoclonal antibody or an Fc fragment or CLCH (constant regions) of the antibody) targeted for glycan modification (also referred to as glycan remodeling) with ENGase which maintains activity that specifically hydrolyzes a 1,4-glycosidic bond (GlcRAcβ1-4GlcNAc) between GlcNAc residues in the core chitobiose structure of the N297-linked glycan. In this case, various enzymes including Endo-Si WT, Endo-A, Endo-D, Endo-E, Endo-F3, Endo-H, Endo-S, Endo-S2, and Endo-Si can be used as ENGase. Thus, in one aspect of the present invention, the acceptor molecule can be provided by a step independent of the transglycosylation reaction of step 1 by a technique of, for example, reacting an enzyme the same as or different from enzyme-A (enzyme having activity that transfers a glycan in the glycan donor molecule to the acceptor molecule) used in step 1 with a starting material Fc-containing molecule (an Fc-containing molecule targeted for glycan modification and typically, an Fc-containing molecule having a host cell-derived N297-linked glycan, heterogeneous N297-linked glycans, or host cell-derived heterogeneous N297-linked glycans). A feature of enzyme-A that can be used in step 1 is that "hydrolytic activity may remain". Hence, the acceptor molecule may be prepared *in situ* by the action of enzyme-A on an Fc-containing molecule having host cell-derived heterogeneous glycans, instead of such treatment with ENGase beforehand. Thus, in one aspect of the present invention, the acceptor molecule is prepared *in situ* and/or in one pot by the action of enzyme-A on a starting material Fc-containing molecule (an Fc-containing molecule targeted for glycan modification and typically, an Fc-containing molecule having a host cell-derived N297-linked glycan, heterogeneous N297-linked glycans, or host cell-derived heterogeneous N297-linked glycans). IgG or the Fc-containing molecule used in glycan modification (glycan remodeling) is preferably derived from IgG heavy chains consisting of an identical amino acid sequence and can be produced in a form having an N297-linked glycan. Its production method is not limited and, for example, IgG produced by a known method for producing a monoclonal antibody, CLCH of the IgG, or an Fc fragment obtained therefrom by enzymatic treatment can be used. Such an IgG or Fc fragment may adopt a mixture of IgG or Fc fragment samples obtained by different production methods or in different lots.

<Endo-β-N-acetylglucosaminidase>

"Endo-β-N-acetylglucosaminidase" is glycoside hydrolase also known as ENGase: Endo-β-N-acetylglucosaminidase and is responsible for the cleavage of a chitobiose structure in a glycan structure, and bond formation. This enzyme has different names depending on its origin. Herein, endo-β-N-acetylglucosaminidase whose substrate is the N297-linked glycan of the Fc-containing molecule is referred to as "enzyme-A", and endo-β-N-acetylglucosaminidase whose substrate is the glycan of the glycan donor molecule, for example, the glycan of a fucose-free glycan starting material (for example, SGP) is referred to as "enzyme-B". Hereinafter, enzyme-A and enzyme-B in the present invention will be described.

<Enzyme-A>

"Enzyme-A" means endo-β-N-acetylglucosaminidase whose substrate is the N297-linked glycan of the Fc-containing molecule (for example, an antibody). Enzyme-A generally comprises a domain having a high affinity for the Fc-containing molecule, a domain having a high affinity for the glycan, and a transglycosylation reaction catalyst domain and typically has both hydrolytic activity and transglycosylation activity against the N297-linked glycan of the Fc-containing molecule (for example, an antibody) as a substrate. For the present invention, activity that transfers a glycan (particularly, a glycan activated by a chemical technique or an enzymatic technique) in the glycan donor molecule to the acceptor molecule is important. Therefore, the hydrolytic activity may be attenuated or canceled. Rather, an enzyme that maintains strong hydrolytic activity might also hydrolyze a glycan transferred to core GlcNAc of the acceptor molecule as a substrate through transglycosylation activity. Therefore, enzyme-A according to the present invention preferably has relatively decreased hydrolytic activity. Note that as described above, in the case where enzyme -A retains hydrolytic activity, the advantage may be enjoyed that the acceptor molecule in step 1 can be prepared *in situ* in the same reaction vessel in step 1, without being separately prepared. Thus, in one aspect of the present invention, enzyme-A has activity that can act on the glycan of the Fc-containing molecule (particularly, transglycosylation activity against the N297-linked glycan of the Fc-containing molecule as a substrate). In another aspect of the present invention, enzyme-A has activity that can act on the glycan of the Fc-containing molecule while its hydrolytic activity is retained or is canceled.

The hydrolytic activity of enzyme-A means activity that specifically hydrolyzes a β1,4-glycosidic bond contained in core chitobiose in the common basic structures of glycans and particularly means activity that specifically hydrolyzes a β1,4-glycosidic bond contained in core chitobiose in the common basic structures of N297-linked glycans of Fc-containing molecules.

The transglycosylation activity of enzyme-A means activity that links the reducing terminal of the glycan donor molecule via a glycosidic bond to the acceptor molecule comprising an Fc site having only core GlcNAc (to which core fucose may or may not be added) in N297.

Examples of enzyme-A according to the present invention can include Endo-S (*Streptococcus* pyogenes-derived enzyme) (see Collin M and Olsen A., EMBO J. 2001, 20, 3046-3055, and Goodfellow JJ, et al., J Am Chem Soc. 2012, 134, 8030-8033), EndoS2 or EndoS49 (see Sjogren J, et al., Biochem J. 2013, 455, 107-118, and Shivatare SS, et al., Chem Commun (Camb). 2018, 54, 6161-6164), Endo-F3 (*Flavobacterium meningosepticum-derived* enzyme) (see Huang W, et al., Chembiochem. 2011, 12, 932-941, and Giddens JP, et al., J Biol Chem. 2016, 291, 9356-9370), and their mutated enzymes. Examples of enzyme-A according to the present invention can also include *Streptococcus iniae* (see Pier GB and Madin SH., Int J Syst Bacteriol. 1976 26, 545-553)-derived endo-β-N-acetylglucosaminidase (hereinafter, referred to as "Endo-Si"; its amino acid sequence (SEQ ID NO: 49) is shown in Figure 32) and its mutated enzymes. Examples of enzyme-A according to the present invention can further include an enzyme whose sequence is identified from genome information of the genus *Streptococcus* (see Vincent P. Richard, et al., Genome Biol. Evol. 2014, 6, 741-753) and its mutated enzymes.

Enzyme-A of the present invention is not limited to the specific enzymes used in the Examples and may be an enzyme isolated from nature or an enzyme artificially prepared or modified on the basis of sequence information of the enzyme of the present invention so long as the enzyme has the above-described characteristics. In the case where enzyme A is isolated from nature, the source organism species source thereof is not particularly limited and is preferably a bacterium.

The active domain and Carbohydrate-Binding Module (CBM) of Endo-Si are presumed to be regions from amino acid residues 106 to 447 and from amino acid residues 762 to 897, respectively, of SEQ ID NO: 49 by sequence comparison with EndoS which has been subjected to crystal structure analysis (B. Trastoy et al., PNAS (2014) vol. 111, No. 18, pp. 6714-6719). These two sites may be important for the interaction of the hydrolytic activity and/or transfer activity with an antibody. Thus, examples of enzyme-A of the present invention include a polypeptide that comprises amino acid residues 106 to 447 and/or amino acid residues 762 to 897 of SEQ ID NO: 49, preferably comprises amino acid residues 106 to 897 of SEQ ID NO: 49, more preferably comprises amino acid residues 106 to 928 of SEQ ID NO: 49, and further preferably comprises amino acid residues 34 to 928 of SEQ ID NO: 49 and exhibits transglycosylation activity.

As described above, endo-β-N-acetylglucosaminidase usually has both hydrolytic activity and transglycosylation activity, and an enzyme that maintains strong hydrolytic activity may also hydrolyze a glycan transferred to core GlcNAc of the acceptor molecule (an antibody having core GlcNAc as the N297-linked glycan or an Fc region-containing molecule thereof) as a substrate through transglycosylation activity so that a desired transglycosylation product cannot be suitably obtained. Hence, a mutated enzyme having relatively decreased hydrolytic activity compared to transglycosylation activity is useful in the synthesis of a glycan-remodeled antibody or a glycan-modified compound. Thus, in one aspect of the present invention, enzyme-A is a mutated enzyme of Endo-S, EndoS-2, Endo-Si, Endo-Sd, Endo-Se, or Endo-Sz, the mutated enzyme having lower hydrolytic activity than that of its corresponding wild-type enzyme. Preferable examples thereof can include, but are not limited to, mutated enzymes obtained by appropriate substitution of amino acid residues indicated in boldface in Figs. 30 to 32 and Figs. 37 to 39, and particularly preferably Endo-S D233Q, Endo-S D233Q/Q303L, Endo-S D233Q/E350A, Endo-S D233Q/E350Q, Endo-S D233Q/E350D, Endo-S D233Q/E350N, Endo-S D233Q/D405A, Endo-Si D241Q, Endo-Si D241Q/Q311L, Endo-Si D241Q/E360Q, Endo-Si D241M, Endo-Si D241M/Q311L, Endo-Si D241M/E360Q, Endo-Si T190Q, Endo-Si T190/D241Q, Endo-Si T190Q/D241M, Endo-Si D241$X_1$ (wherein $X_1$ represents any amino acid residue other than K, R, and D and specifically represents A, N, C, Q, E, G, H, I, L, M, F, P, S, T, W, Y, or V, and preferably A, Q, E, H, I, L, M, F, P, S, T, W, Y, or V), Endo-Si T190$X_2$ (wherein $X_2$ represents an amino acid residue F, H, K, M, Q, R, W, or Y), Endo-Si Q311$X_3$ (wherein $X_3$ represents an amino acid residue F, N, or Y), Endo-Si E360K, Endo-S2 D184M, Endo-S2 T138Q, Endo-S2 D184Q, Endo-S2 D184Q/Q250L, Endo-S2 D184Q/E289Q, Endo-S2 D184M/Q250L, Endo-S2 D184M/E289Q, Endo-S2 D182Q, Endo-S2 D226Q, Endo-S2 T227Q, Endo-S2 T228Q, Endo-Sd D232Q, Endo-Sd D232M, Endo-Sd D232Q/Q302L, Endo-Sd D232M/Q302L, Endo-Se D233Q, Endo-Se D233M, Endo-Se D233Q/Q303L, Endo-Se D233M/Q303L, Endo-Sz D234Q, Endo-Sz D234M, Endo-Sz D234Q/Q304L, and Endo-Sz D234M/Q304L. Enzyme-A may be further mutated, in addition to the above-described specific mutations. For example, 1 to 30, 1 to 20, 1 to 10, 1 to 5, 1 to 4, 1 to 3, 1 to 2, or 1 amino acid residues may be substituted, deleted, inserted, and/or added with reference to the patent literature (International Publication No. WO 2022/050300), without influencing the present enzymatic activity. Examples of the amino acid sequence of enzyme-A include an amino acid sequence having homology or identity equal to or greater than at least 80%, preferably 85%, more preferably 90%, and further preferably 95%, 96%, 97%, 98%, or 99% to amino acid residues other than the specific mutated amino acid residues with reference to the patent literature (International Publication No. WO 2022/050300).

<Enzyme-B>

"Enzyme-B" means endo-β-N-acetylglucosaminidase whose substrate is the glycan of the glycan donor molecule, particularly, endo-β-N-acetylglucosaminidase whose substrate is the glycan of the glycan donor molecule but is not the N297-linked glycan (in other words, which is low reactive, preferably very low reactive, with the N297-linked glycan). The glycan moiety, serving as the substrate of enzyme-B, on the glycan donor molecule may be any of high mannose-type, hybrid-type, and complex-type glycans, and is preferably a high mannose-type or complex-type glycan, and particularly preferably a complex-type glycan. Thus, in one aspect of the present invention, the glycan moiety, serving as the substrate of enzyme-B, on the glycan donor molecule is a high mannose-type or complex-type glycan, and is preferably a complex-type glycan. In the present invention, the complex-type glycan may be biantennary, triantennary, or tetraantennary. Preferably, the glycan moiety, serving as the substrate of enzyme-B, on the glycan donor molecule is a biantennary complex-type glycan. In one aspect of the present invention, enzyme-B is endo-β-N-acetylglucosaminidase whose substrate is a fucose-free glycan starting material (glycopeptide such as SGP or glycoprotein), and is preferably endo-β-N-acetylglucosaminidase whose substrate is a fucose-free glycan starting material comprising a biantennary complex-type glycan.

Enzyme-B of the present invention is preferably an enzyme having a characteristic of acting on the glycan donor molecule comprising a glycan comprising GlcNAc with an unactivated reducing terminal in the presence of enzyme-A to produce an activated intermediate with the reducing terminal rendered usable in transglycosylation reaction, consequently promoting the enzyme-A-catalyzed transglycosylation reaction of the glycan donor molecule-derived glycan to the acceptor molecule. Here, the transglycosylation activity of enzyme-B itself is considered to be correlated with the ability to activate the glycan donor molecule. Therefore, in one aspect of the present invention, enzyme-B can be selected from an endo-β-N-acetylglucosaminidase group whose substrate is the glycan of the glycan donor molecule (for example, SGP) comprising GlcNAc with an unactivated reducing terminal but is not the N297-linked glycan, by using the transglycosylation activity against an acceptor having GlcNAc (for example, a GlnNAc derivative such as 1-methylethyl-2-(acetylamino)-2-deoxy-β-D-glucopyranoside or 2-(acetylamino)-2-deoxy-β-D-glucopyranoside) as an indicator. Thus, in one aspect of the present invention, enzyme-B is an enzyme having a transglycosylation activity from SGP to a GlcNAc derivative.

The activating effect of enzyme-B on the glycan donor molecule acts on the glycan donor molecule comprising GlcNAc with an unactivated reducing terminal to produce an activated intermediate with the reducing terminal rendered usable in transglycosylation reaction. If enzyme-B has a high hydrolytic activity, the activated intermediate might react with a $H_2O$ molecule present in the reaction system, without reacting with the acceptor molecule via enzyme-A, to produce many free glycans. Thus, enzyme-B according to the present invention preferably has a relatively decreased hydrolytic activity compared to the glycan-activating effect.

Examples of enzyme-B of the present invention include Endo-M (*Mucor* hiemalis-derived enzyme) (see Yamamoto K, et al., Biochem Biophys Res Commun. 1994, 203, 244-252, and Umekawa M, et al., J. Biol Chem. 2021, 285, 511-521), Endo-CC (*Coprinopsis cinerea-derived* enzyme) (see Eshima Y, et al., PLoS One. 2015, 10, e0132859), Endo-Om (*Ogataea* minuta-derived enzyme) (see Murakami S, et al., Glycobiology. 2013, 23, 736-744), Endo-Rp (*Rhizomucor* pusillus-derived enzyme) (WO2018101454), and their mutated enzymes (preferably mutated enzymes having a decreased hydrolytic activity compared to the wild type). In one aspect of the present invention, enzyme-B is a mutated enzyme of Endo-M, Endo-Om, Endo-CC, or Endo-Rp, the mutated enzyme having a lower hydrolytic activity than that of its corresponding wild-type enzyme. Preferable examples thereof can include, but are not limited to, mutated enzymes obtained by appropriate substitution of amino acid residues indicated in boldface in Figs. 33 to 36, and particularly preferably, an enzyme selected from the group consisting of Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, Endo-Rp W214F/F307Y/L306I, Endo-M N175Q, Endo-M N175Q/Y217F, Endo-CC N180H, and Endo-Om N194Q. Enzyme-B may be further mutated, in addition to the above-described specific mutations. For example, 1 to 30, 1 to 20, 1 to 10, 1 to 5, 1 to 4, 1 to 3, 1 to 2, or 1 amino acid residues may be substituted, deleted, inserted, and/or added with reference to the patent literature (International Publication No. WO 2022/050300), without influencing the present enzymatic activity. Examples of the amino acid sequence of enzyme-B include an amino acid sequence having homology or identity equal to or greater than at least 80%, preferably 85%, more preferably 90%, and further preferably 95%, 96%, 97%, 98%, or 99% to amino acid residues other than the specific mutated amino acid residues with reference to the patent literature (International Publication No. WO 2022/050300).

In a typical embodiment of the production method according to the present invention, enzyme-A and enzyme-B are added as separate molecules in step 1, or enzyme-A and enzyme-B may be added in a state directly or indirectly bound with each other, to the reaction system so long as these enzymes play their respective desired roles. Thus, in one aspect of the present invention, enzyme-A and enzyme-B may be added to the reaction system in a state directly or indirectly chemically bound with each other . For example, both of the molecules may be added to the reaction system in the form of

enzymes immobilized on a given solid phase support or a fusion protein of the enzymes linked via a given amino acid, polymer (e.g., PEG), oligomer linker, or the like. In other words, in one aspect of the present invention, enzyme-A and enzyme-B may be directly or indirectly chemically bound with each other (=enzyme-A and enzyme-B may be fused with each other) and added, for example, as enzymes immobilized on a given solid phase support or as a fusion protein of the enzymes linked via a given amino acid, polymer (e.g., PEG), oligomer linker, or the like, and preferably an amino acid linker, to the reaction system. Note that in the present invention, each of enzyme-A or enzyme-B may be a functional fragment, not the full-length molecule without deletion. In such a case, the fragment is also regarded as one form of enzyme-A or enzyme-B so long as the functional fragment has a desired function. Accordingly, enzyme-A and enzyme-B according to the present invention also include, for example, "enzyme-A" and "enzyme-B", "enzyme fragment having the function of the enzyme-A" and "enzyme fragment having the function of the enzyme-B", "enzyme-A" and "enzyme fragment having the function of the enzyme-B", or "enzyme fragment having the function of the enzyme-A" and "enzyme-B" in a state directly or indirectly chemically bound with each other. Examples of "enzyme fragment having the function of enzyme-A" can include a fragment having an amino acid sequence obtained by fragmenting the full-length sequence of the above-described enzyme-A or Endo-BI1 (GenBank Accession number: ACJ53522.1), Endo-BI2 (GenBankAccession number: BAJ71450.1), Endo-BT-3987 (GenBank Accession number: AAO79092.1), Endo-E (GenBankAccession number: AAR20477.1), Endo-F (GenBankAccession number: AAA24922.1), Endo-F2 (GenBankAccession number: AAA24923.1), Endo-F3 (GenBank Accession number: AAA24924.1), Endo-CoM (GenBank Accession number: XP006673222.1), or Endo-SB (GenBank Accession number: BBB35949.1) into a specific functional sequence, the amino acid sequence optionally having a specific mutation added thereto. Examples of "enzyme fragment having the function of enzyme-B" can include a fragment having an amino acid sequence obtained by fragmenting a catalytic activity domain of the above-described enzyme-B or the full-length sequence of Endo-A (GenBank Accession number: AAD10851.1), Endo-CE (GenBank Accession number: BAB84821.1), Endo-Tsp1006 (GenBank Accession number: CCY37287.1), Endo-Tsp1263 (GenBank Accession number: CDD88945.1), Endo-Tsp1457 (GenBank Accession number: CDD89351.1), Endo-BB (GenBank Accession number: AAN25135.1), Endo-BH (GenBank Accession number: BAB04504.1), Endo-BN (GenBank Accession number: WP_007484749.1), Endo-CC1 (GenBank Accession number: XP_001839402.1), Endo-CC2 (GenBank Accession number: XP_002911817.1), Endo-D (GenBank Accession number: AAK74656.1), or Endo-Pm (GenBank Accession number: ADK97032.1) into a specific functional sequence, the amino acid sequence optionally having a specific mutation added thereto. As for the respective functional sequences of enzyme-A and enzyme-B, for example, 1 to 30, 1 to 20, 1 to 10, 1 to 5, 1 to 4, 1 to 3, 1 to 2, or 1 amino acid residues may be substituted, deleted, inserted, and/or added with reference to the patent literature (International Publication No. WO 2022/050300), without influencing the above-described functions. Examples of their respective amino acid sequences include amino acid sequences having homology or identity equal to or greater than at least 80%, preferably 85%, more preferably 90%, and further preferably 95%, 96%, 97%, 98%, or 99% to amino acid residues other than the specific amino acid residues that influence activity with reference to the patent literature (International Publication No. WO 2022/050300).

A given combination can be used as the combination of enzyme-A and enzyme-B so long as enzyme-A and enzyme-B play their respective roles. For example, given combinations of enzyme-A and enzyme-B described in the following table can be used.

[Table 1]

| | Enzyme — A | | Enzyme — B |
|---|---|---|---|
| A-1 | Endo-Si D241M Q311L | B-1 | Endo-Rp N172H |
| A-2 | Endo-Si D241Q | B-2 | Endo-Rp N172Q |
| A-3 | Endo-Si D241Q E360Q | B-3 | Endo-Rp N172V |
| A-4 | Endo-Si D241M | B-4 | Endo-M N175Q |
| A-5 | Endo-Si D241Q Q311L | B-5 | Endo-M N175Q Y217F |
| A-6 | Endo-Si D241M E360Q | | |
| A-7 | Endo-Si WT | | |
| A-8 | Endo-S D233Q E350Q | | |
| A-9 | Endo-S WT | | |
| A-10 | Endo-S2 D184M | | |
| A-11 | Endo-S2 T138Q | | |
| A-12 | Endo-S2 WT | | |

| | Enzyme — A | | Enzyme — A |
|---|---|---|---|
| A-13 | Endo-Si T190F | A-26 | Endo-Si D241L |
| A-14 | Endo-Si T190H | A-27 | Endo-Si D241P |
| A-15 | Endo-Si T190K | A-28 | Endo-Si D241R |
| A-16 | Endo-Si T190M | A-29 | Endo-Si D241S |
| A-17 | Endo-Si T190Q | A-30 | Endo-Si D241T |
| A-18 | Endo-Si T190R | A-31 | Endo-Si D241V |
| A-19 | Endo-Si T190W | A-32 | Endo-Si D241W |
| A-20 | Endo-Si T190Y | A-33 | Endo-Si D241Y |
| A-21 | Endo-Si D241A | A-34 | Endo-Si Q311F |
| A-22 | Endo-Si D241E | A-35 | Endo-Si Q311K |
| A-23 | Endo-Si D241F | A-36 | Endo-Si Q311N |
| A-24 | Endo-Si D241H | A-37 | Endo-Si Q311Y |
| A-25 | Endo-Si D241I | A-38 | Endo-Si E360K |

| | Enzyme — A |
|---|---|
| A-39 | Endo-S2 D184M/Q250L |
| A-40 | Endo-S2 D184Q/Q250L |
| A-41 | Endo-Sd WT |
| A-42 | Endo-Se WT |
| A-43 | Endo-Se D233M |
| A-44 | Endo-Sz WT |
| A-45 | Endo-Sz D234M |
| A-46 | Endo-Sz D234M/Q304L |

As described above, in the case where enzyme-A and enzyme-B are fused with each other, enzyme-A and enzyme-B can be used in a given combination so long as these enzymes play their respective roles. More specifically, enzyme-A is an Endo-Si mutant and enzyme-B is an Endo-Rp mutant, for example, enzyme-A is Endo-Si D241M/Q311L or Endo-Si D241Q/Q311L and enzyme-B is Endo-Rp N172H or Endo-Rp N172V. In a further alternative specific aspect, the fusion enzyme is a fusion protein of Endo-Rp N172H and Endo-Si D241M/Q311L, a fusion protein of Endo-Rp N172V and Endo-Si D241Q/Q311L, or Endo-Si D241Q/Q311L and Endo-Rp N172V. Examples thereof can include [enzyme-A]-[enzyme-B] fusion protein enzyme or [enzyme-B]-[enzyme-A] fusion protein enzyme described in the following table.

[Table 2]

| | [Enzyme-A]-[enzyme-B] fusion protein enzyme or [enzyme-B]-[enzyme-A] fusion protein enzyme |
|---|---|
| BA-1 | [Endo-Rp N172H]-[Endo-Si D241M/Q311L] |

(continued)

| | [Enzyme-A]-[enzyme-B] fusion protein enzyme or [enzyme-B]-[enzyme-A] fusion protein enzyme |
|---|---|
| BA-2 | [Endo-Rp N172V]-[Endo-Si D241Q/Q311L] |

The fusion protein of enzyme-A and enzyme-B can be produced in accordance with a routine method with reference to the non-patent literature (Joaquim, et al., Processes. 2022, 10, 494.) and can be produced, for example, by bonding enzyme-A and enzyme-B to a given solid phase support via physical or chemical interaction. Alternatively, the fusion protein can be obtained by genetically engineering a host cell to produce the fusion protein with reference to the non-patent literatures (F Grueninger-Leitch, et al., Protein Sci. 1996, 12, 2617-22, and, Emily MK, et al., Protein Eng Des Sel. 2005, 10, 497-501) and the patent literature (WO2017137459). Namely, genes encoding enzyme-A and enzyme-B are bound to each other, and a host cell can be genetically engineered to produce a single enzyme having functions derived from enzyme-A and enzyme-B. Such genetic engineering of a host cell to produce the fusion enzyme eliminates the need to separately provide enzyme-A and enzyme-B and can decrease the number of enzymes to be provided and therefore, can reduce the cost and work for enzyme production. Enzyme-A and enzyme-B may be linked via an amino acid linker. In one aspect, the linker that can be used is constituted by one or more amino acids and has a typical characteristic known in the art with reference to the non-patent literature (Chen, et al., Adv Drug Deliv Rev. 2013 Oct 15; 65 (10): 1357-1369.). The linker that can be used may be a flexible or rigid linker or a linker having a cleavable or uncleavable characteristic and is a long or short linker. In one aspect, the linker can comprise (SGGS)n, (GGGS)n, (GGGGS)n, (G)n, (EAAAK)n, (EF)n, or (GGS)n, or a combination thereof as an amino acid sequence fragment (wherein n is an integer of 1 to 30, preferably an integer of 1 to 20, more preferably an integer of 1 to 10, and further preferably an integer of 1 to 6). The linker can comprise an amino acid sequence that is commonly used as a purification tag such as polyhistidine tag, HA tag, FLAG tag, CBD tag, Fc tag, or GST tag. In one aspect, the purification tag is polyhistidine tag, HA tag, FLAG tag, Fc tag, or GST tag, and is preferably polyhistidine tag, HA tag, FLAG tag, or CBD tag, more preferably polyhistidine tag or FLAG tag, and further preferably polyhistidine tag.

<Additive>

In one aspect of the present invention, the above step 1 is a step of obtaining a reaction mixture by reacting an acceptor molecule with a glycan donor molecule in the presence of enzyme-A, enzyme-B, and a specific additive. The additive is capable of positively influencing the rate of transglycosylation in a one-pot reaction via electrostatic interaction, hydrophobic interaction, a solvent effect, or the like. In particular, a mutation, if present, in the Fc region of the Fc-containing molecule (typically, an antibody) may alter the three-dimensional structure of the Fc region so that the rate of transglycosylation changes. For example, some mutation present in the Fc region of an antibody used as the acceptor molecule is capable of decreasing the rate of transglycosylation compared to the absence of the mutation. The additive is useful in adjusting the rate of transglycosylation of the Fc-containing molecule (typically an antibody) used as the acceptor molecule and is especially useful when the Fc region of the Fc-containing molecule (typically an antibody) used as the acceptor molecule has a mutation which is capable of causing a decrease in the rate of transglycosylation.

In one aspect of the present invention, the additive used in step 1 is a monovalent salt. Herein, "monovalent salt" means a salt containing a monovalent anion or a monovalent cation. Examples of the monovalent salt can include, but are not limited to, inorganic salts (e.g., alkali metal salts such as sodium chloride, lithium chloride, potassium chloride, potassium iodide, sodium iodide, lithium iodide, potassium bromide, sodium fluoride, and potassium fluoride, ammonium salts such as ammonium fluoride, ammonium chloride, and ammonium sulfate) and organic salts (e.g., ammonium acetate, sodium acetate, potassium acetate). In one aspect of the present invention, the additive used in step 1 is sodium chloride, potassium acetate, ammonium acetate, lithium chloride, or sodium acetate, and is preferably sodium chloride, ammonium acetate, or sodium acetate. In another aspect of the present invention, the additive used in step 1 is a monovalent alkali metal salt, and is preferably sodium chloride, potassium acetate, lithium chloride, or sodium acetate, and further preferably sodium chloride.

In one aspect of the present invention, the additive used in step 1 is an organic solvent. Examples of an organic solvent that can be used in step 1 can include, but are not limited to, polar protic solvents such as tetrahydrofuran, acetone, acetonitrile, N,N-dimethylformamide, and dimethyl sulfoxide, and polar protic solvents such as 1-butanol, 2-propanol, 1-propanol, ethanol, and methanol. In one aspect of the present invention, the organic solvent used in step 1 is ethanol or dimethyl sulfoxide.

In one aspect of the present invention, the additive used in step 1 is a surfactant. Examples of the surfactant that can be used in step 1 can include, but are not limited to, anionic surfactants, cationic surfactants, nonionic surfactants, and amphoteric surfactants. Examples of anionic surfactants can include surfactants of carboxylate type such as sodium octanoate and sodium decanoate, sulfonate type such as sodium 1-hexanesulfonate and sodium 1-octanesulfonate,

sulfuric acid ester type such as sodium lauryl sulfate and sodium myristyl sulfate, and phosphoric acid ester type such as lauryl phosphate and sodium lauryl phosphate. Examples of cationic surfactants can include surfactants of quaternary ammonium salt type such as tetramethylammonium chloride and hexadecyl trimethylammonium bromide, alkylamine salt type such as monomethylamine hydrochloride and dimethylamine hydrochloride, and pyridine ring-containing type such as butylpyridinium chloride and cetylpyridinium chloride. Examples of nonionic surfactants can include surfactants of ester type such as glycerin laurate and sorbitan fatty acid ester, polyethylene glycol (polyethylene glycol 2000, polyethylene glycol 4000, polyethylene glycol 6000, polyethylene glycol 11000, polyethylene glycol 20000, etc.), ether type such as polyoxyethylene alkyl phenyl ether, ester ether type such as polyoxyethylene glycerin fatty acid ester and polyoxyethylene sorbitan fatty acid ester (polysorbate 20, polysorbate 60, polysorbate 65, polysorbate 80, etc.), alkanolamide type such as lauric acid diethanolamide and oleic acid diethanolamide, alkyl glycoside type such as octyl glucoside and decyl glucoside, and higher alcohol type such as cetanol and stearyl alcohol. Examples of amphoteric surfactants can include surfactants of alkyl betaine type such as lauryl dimethylaminoacetic acid betaine, fatty acid amidopropyl betaine such as cocamidopropyl betaine and cocamidopropyl hydroxysultaine, amino acid type such as sodium lauroyl glutamate and potassium lauroyl glutamate, and amine oxide type such as lauryl dimethylamine N-oxide and oleyl dimethylamine N-oxide. In one aspect of the present invention, the surfactant used in step 1 is a nonionic surfactant. In another aspect of the present invention, the surfactant used in step 1 is an ether-type or ester ether-type nonionic surfactant. In a further alternative aspect of the present invention, the surfactant used in step 1 is polyethylene glycol or polyoxyethylene sorbitan fatty acid ester, and is preferably polyethylene glycol 6000 or polysorbate 20.

In one aspect of the present invention, the additive used in step 1 is a saccharide. Examples of a saccharide that can be used in step 1 can include, but are not limited to, monosaccharides, disaccharides, oligosaccharides (trisaccharides or higher saccharides are referred to as oligosaccharides herein), deoxy sugar, uronic acid, amino sugar, sugar alcohols, lactone, and polysaccharides. Examples of monosaccharides that can be used as the additive in step 1 can include aldotriose such as glyceraldehyde, aldotetrose such as erythrose and threose, aldopentose such as ribose and xylose, aldohexose such as glucose, mannose, and galactose, ketotriose such as dihydroxyacetone, ketotetrose such as erythrulose, ketopentose such as xylulose and ribulose, and ketohexose such as fructose and sorbose. Note that in the case of using Endo-Rp enzyme (including a mutant) as the enzyme-B of step 1, glucose might serve as an acceptor of a glycan activated by the Endo-Rp enzyme and may therefore decrease the rate of transglycosylation. Thus, in one aspect of the present invention, the saccharide used as the additive in step 1 includes no glucose. Examples of disaccharides that can be used as the additive in step 1 can include trehalose, isotrehalose, kojibiose, sophorose, nigerose, laminaribiose, maltose, cellobiose, isomaltose, gentiobiose, lactose, and sucrose. Examples of oligosaccharides can include fructooligosaccharide and galactooligosaccharide. Examples of a deoxy sugar can include deoxyribose and fucose. Examples of a uronic acid can include glucuronic acid and galacturonic acid. Examples of an amino sugar can include glucosamine and galactosamine sugar. Examples of sugar alcohols can include glycerin, xylitol, and sorbitol. Examples of a lactone can include ascorbic acid and glucuronolactone. Examples of polysaccharides can include starch, amylose, amylopectin, glycogen, cellulose, pectin, and glucomannan. In one aspect of the present invention, the saccharide used in step 1 is a monosaccharide, a disaccharide, or amino sugar. In another aspect of the present invention, the saccharide used in step 1 is a disaccharide or amino sugar. In a further alternative aspect of the present invention, the saccharide used in step 1 is trehalose or sorbitol.

In one aspect of the present invention, the additive used in step 1 is an amino acid. The amino acid that can be used as the additive in step 1 is not particularly limited so long as it is an organic compound having both functional groups (an amino group and a carboxyl group). Preferable examples thereof can include basic amino acids such as arginine, histidine, and lysine, acidic amino acids such as glutamic acid and aspartic acid, neutral polar amino acids such as asparagine, glutamine, serine, threonine, tyrosine, and cysteine, and neutral non-polar amino acids such as isoleucine, leucine, valine, methionine, phenylalanine, tryptophan, alanine, glycine, and proline. In one aspect of the present invention, the amino acid used as the additive in step 1 is a basic amino acid, and is preferably arginine, histidine, or lysine, and further preferably arginine or histidine. The amino acid that can be used as the additive in step 1 may be added in the form of a salt. For example, the basic amino acid arginine, histidine, or lysine can be added as a hydrochloride. Accordingly, in one aspect of the present invention, the amino acid used as the additive in step 1 is arginine hydrochloride, histidine hydrochloride, or lysine hydrochloride, and is further preferably arginine hydrochloride or histidine hydrochloride.

The concentration of the additive used in step 1 can be arbitrarily set without precipitating the Fc-containing molecule or the enzymes by the addition of the additive, namely as long as the reactivity of one-pot reaction remains. The concentration can be set, for example, by use or application of a method described in Example 15-3 herein. In one aspect of the present invention, in the case where the additive used in step 1 is a monovalent salt (for example, sodium chloride), its concentration is 1500 mM or lower, 1000 mM or lower, 900 mM or lower, 800 mM or lower, 700 mM or lower, 600 mM or lower, 500 mM or lower, 450 mM or lower, 400 mM or lower, 350 mM or lower, 300 mM or lower, 250 mM or lower, 200 mM or lower, 150 mM or lower, 100 mM or lower, or 50 mM or lower. In another aspect of the present invention, in the case where the additive used in step 1 is a monovalent salt (for example, sodium chloride), its concentration is 1 mM or higher, 5 mM or higher, 10 mM or higher, 20 mM or higher, 30 mM or higher, 40 mM or higher, 50 mM or higher, 100 mM or higher, 150 mM or

higher, 200 mM or higher, 250 mM or higher, 300 mM or higher, 350 mM or higher, 400 mM or higher, 450 mM or higher, 500 mM or higher, 600 mM or higher, 700 mM or higher, 800 mM or higher, 900 mM or higher or 1000 mM or higher. In a further alternative aspect of the present invention, in the case where the additive used in step 1 is a monovalent salt (for example, sodium chloride), its concentration can be set to within a given concentration range specified by any of the upper and lower limits listed above and can be set within the range of, for example, 1 to 700 mM, 10 to 600 mM, 100 to 550 mM, or 50 to 1000 mM.

In one aspect of the present invention, the additive may be included beforehand or may be added in step 1.

In one aspect of the present invention, in the case where the additive used in step 1 is an organic solvent (for example, ethanol or DMSO), its concentration is 0.1 to 20% v/v, and is preferably 0.2 to 10% v/v, more preferably 0.3 to 5% v/v, further preferably 0.4 to 3% v/v, particularly preferably 0.5 to 2% v/v, and most preferably about 1% v/v.

In one aspect of the present invention, in the case where the additive used in step 1 is a surfactant (for example, polyethylene glycol 6000 or polysorbate 20), its concentration is 0.1 to 20% v/v, and is preferably 0.2 to 10% v/v, more preferably 0.3 to 5% v/v, further preferably 0.4 to 3% v/v, particularly preferably 0.5 to 2% v/v, and most preferably about 1% v/v.

In one aspect of the present invention, in the case where the additive used in step 1 is a saccharide (for example, trehalose or sorbitol), its concentration is 1 mM to 10 M, and is preferably 5 to 1000 mM, more preferably 10 to 500 mM, further preferably 15 to 200 mM, particularly preferably 20 to 100 mM, and most preferably about 40 mM.

In one aspect of the present invention, in the case where the additive used in step 1 is an amino acid (for example, arginine hydrochloride or histidine hydrochloride), its concentration is 1 mM to 10 M, and is preferably 5 to 1000 mM, more preferably 10 to 500 mM, further preferably 15 to 200 mM, particularly preferably 20 to 100 mM, and most preferably about 40 mM.

<Step 1>

In one aspect of the present invention, step 1 is a step of obtaining a reaction mixture by reacting an acceptor molecule with a glycan donor molecule in the presence of enzyme-A, enzyme-B, and a specific additive. The reaction conditions of this step 1 can be appropriately selected in accordance with reaction conditions of known transglycosylation reaction using GlcNAc with an unactivated reducing terminal as a glycan donor molecule or by use or application of reaction conditions of known transglycosylation reaction.

The reaction of step 1 is preferably performed in a buffer solution and is desirably performed under conditions that do not accelerate the degradation of the glycan donor molecule comprising GlcNAc with an unactivated reducing terminal. From this viewpoint, in one aspect of the present invention, the pH of the reaction system of step 1 can be appropriately selected from 5.8 to 9.5, and is preferably in the range of 6.2 to 8.5, more preferably 6.5 to 8.0, and further preferably 6.5 to 7.5. The buffer solution can be appropriately selected from a phosphate buffer solution (pH 6.0 to 7.5), a MOPS-NaOH buffer solution (pH 6.5 to 8.0), a Tris-HCl buffer solution (pH 7.0 to 9.0), and the like, and is preferably a Tris-HCl buffer solution (pH 7.0 to 9.0). For the purpose of stabilizing the enzymes, the reaction mixture may be supplemented with a further additive that does not inhibit enzymatic reaction.

The reaction temperature can be appropriately selected from 4°C to 50°C, and is preferably 15°C to 45°C, more preferably 20°C to 40°C, and further preferably 25°C to 40°C.

The reaction time can be appropriately selected from 10 min to 96 h, and is preferably 0.5 h to 80 h, more preferably 2 h to 70 h, further preferably 4 h to 60 h, particularly preferably 6 to 48 h or 8 to 30 h, and most preferably 16 to 24 h. The completion of the reaction may be determined while the degree of progression of the transglycosylation reaction is checked by collecting a small amount of the reaction mixture over time. In general, the degree of progression of the transglycosylation reaction can be monitored by, for example, sodium dodecyl sulfate-polyacrylamide gel electrophoresis (SDS-PAGE), capillary electrophoresis, microchip electrophoresis, a fully automated electrophoresis system, or liquid chromatography-mass spectrometry (LC-MS) and can be monitored, for example, by fragmenting a glycan-remodeled antibody into a heavy chain and a light chain, and then confirming the change in the retention time of only the heavy chain with the N297-linked glycan using a fully automated electrophoresis system.

The step 1 can be performed by a one-pot method of directly transferring the glycan of the glycan donor molecule to the acceptor molecule antibody or Fc region-containing molecule thereof having optionally fucosylated core GlcNAc as the N297-linked glycan.

In glycan remodeling using a glycan donor molecule comprising a glycan with an oxazolinated reducing terminal, an antibody concentration in a reaction mixture is routinely set to be low because glycation, which is a non-Endo enzyme-mediated chemical reaction, easily progresses for Lys in an acceptor molecule. On the other hand, in the case of using a glycan donor molecule comprising GlnNAc with an unactivated reducing terminal, it is unnecessary to be concerned about glycation because a solution of a chemically synthesized oxazoline moiety responsible for glycation is not added. Accordingly, a relatively high concentration of an acceptor molecule can be used. Rather, the addition of a high concentration of the acceptor molecule is efficient from the viewpoint of enhancing the frequency of contact between

the molecules. Thus, the acceptor molecule concentration in step 1 can be appropriately set in accordance with a known method. For example, the upper limit value of the final concentration of the acceptor molecule in the reaction system of step 1 can be 200 mg/mL, preferably 160 mg/mL, more preferably 120 mg/mL, further preferably 100 mg/mL, and particularly preferably 80 mg/mL, and the lower limit value thereof can be 10 mg/mL, preferably 14 mg/mL, more preferably 18 mg/mL, and further preferably 20 mg/mL. A concentration range can be a given combination of the above-described upper limit and lower limit values or 10 mg/mL to 200 mg/mL, preferably 14 mg/mL to 160 mg/mL, more preferably 18 mg/mL to 120 mg/mL, further preferably 20 mg/mL to 100 mg/mL, and particularly preferably 20 mg/mL to 80 mg/mL, though the concentration is not limited thereto.

The amount of the glycan donor molecule added in step 1 can be appropriately set by use or application of a known method. In one aspect of the present invention, for example, the lower limit value of the amount of the glycan donor molecule added (used) in step 1 can be 2 equivalents, 3 equivalents, 4 equivalents, 5 equivalents, 6 equivalents, 7 equivalents, 8 equivalents, 9 equivalents, 10 equivalents, 11 equivalents, 12 equivalents, 13 equivalents, 14 equivalents, 15 equivalents, 16 equivalents, 17 equivalents, 18 equivalents, 19 equivalents, or 20 equivalents, per equivalent of the acceptor molecule, and the upper limit value of the amount of the glycan donor molecule can be 300 equivalents, 200 equivalents, 150 equivalents, 100 equivalents, 90 equivalents, 80 equivalents, 75 equivalents, 70 equivalents, 65 equivalents, 60 equivalents, 55 equivalents, 45 equivalents, 40 equivalents, 35 equivalents, 30 equivalents, 25 equivalents, or 20 equivalents, though the amount is not limited thereto. A range of the amount of the glycan donor molecule added (used) in step 1 can be a given combination of the above-described upper limit and lower limit values or 5 to 80 equivalents, preferably 7 to 60 equivalents, more preferably 8 to 50 equivalents, and particularly preferably 10 to 40 equivalents per equivalent of the acceptor molecule, though the amount is not limited thereto.

In one aspect of the present invention, the rate of transglycosylation in step 1 can be set to a given numeric value and can be set to, for example, 50% or more, 55% or more, 60% or more, 65% or more, 70% or more, 75% or more, 80% or more, 85% or more, 90% or more, or 95% or more and on the other hand, can be also set to 95% or less, 90% or less, 85% or less, 80% or less, 75% or less, 70% or less, 65% or less, or 60% or less. In one aspect of the present invention, the rate of transglycosylation in step 1 exceeds 80%, preferably exceeds 85%, and particularly preferably exceeds 90%. Note that the above-described "rate of transglycosylation" can be determined in accordance with a routine method and can be determined, for example, using a fully automated electrophoresis system disclosed in Example 15-2B. Note that the timing of measurement of the rate of transglycosylation differs depending on the enzymes used and is 1 min or more and 100 h or less, and is preferably 1 h or more and 80 h or less, and more preferably 2 h or more and 72 h or less.

Note that the acceptor molecule used in step 1 can be prepared by a separate preparation method independent from step 1. Thus, in one aspect of the present invention, the method of the present invention further comprises a step of separately preparing the acceptor molecule prior to step 1. In this aspect, the acceptor molecule can be provided by a step independent from the transglycosylation reaction of step 1 by a technique of, for example, reacting an enzyme the same as or different from enzyme-A (enzyme having activity that transfers a glycan in the glycan donor molecule to the acceptor molecule) used in step 1 with a starting material Fc-containing molecule (an Fc-containing molecule targeted for glycan modification and typically, an Fc-containing molecule having a host cell-derived N297-linked glycan, heterogeneous N297-linked glycans, or host cell-derived heterogeneous N297-linked glycans). In one aspect of the present invention, the acceptor molecule can be prepared, for example, by treating the N297-linked glycan of an Fc-containing molecule targeted for glycan modification (also referred to as glycan remodeling) with ENGase which maintains activity that specifically hydrolyzes a 1,4-glycosidic bond (GlcNAcβ1-4GlcNAc) between GlcNAc residues in the core chitobiose structure of the N297-linked glycan.

Alternatively, in the case where hydrolytic activity remains in enzyme-A used in step 1, the acceptor molecule in step 1 can be prepared *in situ* in the same vessel as that of step 1. Thus, step 1 of the present invention includes an aspect in which the acceptor molecule is prepared *in situ.* In this aspect, the production process is simplified because the step of separately preparing the acceptor molecule prior to step 1 can be omitted. The simplified production process also eliminates the need of a step of removing ENGase used in the separate step and further eliminates the need of treatment to separate or purify a relatively unstable acceptor molecule resulting from the separate step and therefore, is very beneficial. Furthermore, the same enzyme-A is used as an enzyme hydrolyzing the N297-linked glycan of a starting material Fc-containing molecule and an enzyme for transferring a glycan in the glycan donor molecule. This could offer an advantage in a production process: the number and types of enzymes used can be decreased (this point is more marked when a fusion protein of enzyme-A and enzyme-B is used). In this aspect, an Fc-containing molecule (typically, an Fc-containing molecule such as an IgG-type monoclonal antibody or an Fc fragment or CLCH (constant regions) of the antibody) targeted for glycan modification is added to the reaction system in step 1, and not the acceptor molecule itself, which is a molecule that directly receives a glycan from the glycan donor molecule. The N297-linked glycan of such an Fc-containing molecule to be added as a starting material is not particularly limited so long as it is different from the glycan donor molecule-derived glycan which is the target product. Typical examples thereof can include an N297-linked glycan derived from the host cell used in producing the Fc-containing molecule, heterogeneous N297-linked glycans, or heterogeneous N297-linked glycans derived from the host cell. When such an Fc-containing molecule is added to the reaction system of step 1, the 1,4-glycosidic bond (GlcNAcβ1-4GlcNAc) between GlcNAc residues in the core chitobiose structure of the N297-linked glycan of the Fc-containing molecule is specifically hydrolyzed by the hydrolytic activity of enzyme-A to produce the acceptor molecule *in situ.* Subsequently, the glycan of the glycan donor molecule is transferred to the acceptor molecule produced *in situ* by the transglycosylation activity of enzyme-A that produces an Fc-containing molecule having an N297-linked glycan including the glycan donor molecule-derived glycan as the target product in one pot. In this aspect, the acceptor molecule, albeit produced *in situ,* is not isolated or purified and therefore looks as if the N297-linked glycan of the Fc-containing molecule added as a starting material is directly exchanged with the glycan donor molecule-derived glycan in one step. Hence, herein, such an aspect is appropriately referred to as a "direct exchange reaction of the N297-linked glycan".

In the direct exchange reaction of the N297-linked glycan, any type of glycan donor molecule as described above can be used without particular limitations. Note that the conditions, such as the reaction temperature, for the direct exchange reaction of the N297-linked glycan are as described above, whereas it is preferable to set the reaction time to a longer time compared to the aspect comprising the step of separately preparing the acceptor molecule.

In one aspect of the present invention, the method for producing an Fc-containing molecule further comprises a step of collecting an Fc-containing molecule having a N297-linked glycan including a glycan donor molecule-derived glycan from the reaction mixture obtained in step 1. This collection can be appropriately carried out by (application of) a method well known in the art, and is particularly preferably carried out by step 2 described below. Accordingly, in one aspect of the present invention, the method for producing an Fc-containing molecule further comprises step 2 described below.

<Step 2>

In one aspect of the present invention, step 2 is a step of contacting the reaction mixture obtained in step 1 with a cation exchange chromatography medium or a multimode chromatography medium under acidic conditions, and collecting an Fc-containing molecule having a N297-linked glycan including a glycan donor molecule-derived glycan, and is preferably a step of contacting the reaction mixture obtained in step 1 with a cation exchange chromatography medium or a multimode chromatography medium under acidic conditions, and isolating and collecting an Fc-containing molecule having an N297-linked glycan including a glycan donor molecule-derived glycan from a partially or wholly unreacted Fc-containing molecule (acceptor molecule) contained in the reaction mixture. In one aspect of the present invention, step 2 is a step of directly using the reaction mixture obtained in step 1 as a load solution or preparing a load solution by appropriately adding a buffer solution to the reaction mixture, and contacting the load solution with a cation exchange chromatography medium or a multimode chromatography medium under acidic conditions so that an unreacted acceptor molecule is selectively adsorbed to the medium, while collecting a flow-through solution containing an Fc-containing molecule having an N297-linked glycan including a glycan donor molecule-derived glycan, and thereby selectively collecting the Fc-containing molecule.

In step 2, the target Fc-containing molecule with the glycan can be separated from an unreacted Fc-containing molecule (acceptor molecule). Therefore, a highly pure Fc-containing molecule having a homogeneous glycan structure can be produced even if only a small amount of the glycan donor molecule is used in step 1. In this concept, step 2 needs to have high selectivity and rate of collection for producing an Fc-containing molecule having a glycan structure with a high rate of transglycosylation while reducing the amount of the glycan donor molecule used in step 1. Low selectivity cannot enhance the rate of transglycosylation because the target Fc-containing molecule with the glycan cannot be isolated from an unreacted Fc-containing molecule (acceptor molecule). A low rate of collection requires increasing the amount of the acceptor molecule added in step 1 in order to increase the yield of the Fc-containing molecule and therefore increases the amount of the glycan donor molecule used. In the case of applying the present invention to a method for producing a medical antibody at an industrial scale, the target product antibody needs to be able to be efficiently isolated, with desired biological activity maintained, in step 2.

Capillary gel electrophoresis (CE-SDS) which is an analysis technique for detecting an antibody without a glycan (without core GlcNAc) included in antibodies with an N297-linked glycan is known as a conventional isolation technique (Richard R. Rustandi et al., Electrophoresis. 2008 Sep; 29 (17): 3612-20). This technique can isolate a heavy chain of an antibody fragmented under reducing conditions according to the hydrokinetic size of the molecule. On the other hand, the antibody is denatured during the course of pretreatment and loses biological activity. In addition, the throughput thereof is limited to the order of several hundreds of μg because the antibody is isolated through a medium packed in fine capillaries. Also, a method using hydrophilic interaction chromatography (HILIC) is known as an analysis technique for isolating an antibody having core GlcNAc prepared using ENGase from an antibody having an N297-linked glycan before action of ENGase (Matthew A et al., Measuring the Glycan Occupancy of Intact mAbs using HILIC and Detection by Intrinsic Fluorescence). This technique does not require antibody fragmentation and can separate an antibody having core GlcNAc from an antibody having an N297-linked glycan. On the other hand, the antibody is denatured due to heating and exposure to an organic solvent and loses biological activity. In addition, the throughput thereof is limited to the order of several μg because the isolation requires an analytical column with a high theoretical plate number or an ultrahigh-performance liquid chromatography apparatus (UHPLC). Further, a method using cation exchange chromatography is known as an analysis

technique of isolating an antibody having core GlcNAc prepared using ENGase from an antibody having an N297-linked glycan before action of ENGase (Masaki Kurogochi et al., PLOS ONE|DOI:10.1371/journal.pone.0132848 July 22, 2015, and Shiyi Wang et al., Journal of Chromatography A, 1217 (2010) 6496-6502). This technique can separate an antibody having core GlcNAc from an antibody having an N297-linked glycan without denaturing the antibody. On the other hand, this technique has the disadvantages that, in order to achieve high-resolution separation, an analytical column with a high theoretical plate number is used and a gradient elution scheme of mixing two solutions by a complicated program is required; the throughput thereof is limited to the order of several hundreds of μg; and no satisfactory yield is obtained. Thus, any method for isolating an antibody according to the presence or absence of glycosylation, which can be used for the purpose of isolation or purification at an industrial scale, has not been reported before the present invention.

Under these circumstances, the present inventors have found that a target Fc-containing molecule with the glycan can be selectively isolated or purified at a high yield without being denatured, by a very simple method of contacting a reaction mixture with a cation exchange chromatography medium or a multimode chromatography medium under acidic conditions.

Step 2 can be carried out in accordance with a routine method of cation exchange chromatography or multimode chromatography. For example, the reaction mixture obtained in step 1 is mixed with an acidic buffer solution to obtain a load solution, and the load solution is injected into a column packed with a cation exchange chromatography medium or a multimode chromatography medium so that a flow-through molecule can be separated from a molecule adsorbed onto the column. Subsequently in step 2, a molecule non-specifically adsorbed to the column is washed off by the injection of an equilibration solution (or a washing solution), and a molecule specifically adsorbed to the column can be eluted by the injection of an eluate (or a regeneration solution). Then in step 2, a cleaning solution can be appropriately injected for the purpose of cleaning in place.

In step 2, the cation exchange chromatography or the multimode chromatography is carried out under acidic conditions. In one aspect of the present invention, the load solution (or the load solution and the equilibration solution) in step 2 has an acidic pH. In step 2, the load solution (or the load solution and the equilibration solution) having a lower pH has a higher improving effect on the rate of transglycosylation, and the improving effect on the rate of transglycosylation tends to be decreased with increases in pH. In one aspect of the present invention, "acidic conditions" can employ a given acidic pH (<7.0) so long as the intended isolation can be achieved. For example, a given pH selected from 2.5, 2.6, 2.7, 2.8, 2.9, 3.0, 3.1, 3.2, 3.3, 3.4, 3.5, 3.6, 3.7, 3.8, 3.9, and 4.0 can be adopted as the lower limit value (the lower limit value of the pH of the load solution and the equilibration solution), and a given pH selected from 5.0, 4.9, 4.8, 4.7, 4.6, 4.5, 4.4, 4.3, 4.2, 4.1, 4.0, 3.9, 3.8, 3.7, 3.6, and 3.5 can be adopted as the upper limit value (the upper limit value of the pH of the load solution and the equilibration solution). The pH range (the pH range of the load solution and the equilibration solution) under an "acidic condition" can be a given combination of the above-described upper limit and lower limit values or 2.5 to 5.0, preferably 3.0 to 4.8, further preferably 3.3 to 4.7, particularly preferably 3.4 to 4.6, and most preferably 3.5 to 4.5, though the pH is not limited thereto. Thus, in one aspect of the present invention, the acidic conditions in step 2 mean the range of pH 2.5 to 5.0, preferably pH 3.0 to 4.8, further preferably pH 3.3 to 4.7, particularly preferably pH 3.4 to 4.6, and most preferably pH 3.5 to 4.5.

In step 2, the reaction mixture obtained in step 1 is directly used as a load solution to be injected into a column, or a load solution can be prepared by appropriately adding a buffer solution to the reaction mixture. Any buffer solution can be used as the buffer solution for preparing the load solution so long as the load solution has an acidic pH. Examples thereof can include acetate buffer solutions, citrate buffer solutions, phosphate buffer solutions, glycine buffer solutions, and phthalate buffer solutions.

The load solution may be adjusted so as to have an intended salt concentration by the appropriate addition of a salt. The salt for preparing the load solution is not particularly limited as long as intended isolation can be achieved. Examples thereof can include sodium chloride, potassium chloride, and magnesium chloride. The salt concentration can be, for example, 30 to 2000 mM, preferably 150 to 1000 mM, more preferably 200 to 750 mM, further preferably 280 to 600 mM, and particularly preferably 310 to 500 mM. Note that the salt concentration of the load solution influences the yield or purity of a final target product and the optimum salt concentration varies depending on the type of the target product or the cation exchange chromatography medium or the multimode chromatography medium. It is therefore preferable to adopt the optimum salt concentration according to a given chromatography environment.

The concentration of the Fc-containing molecule in the load solution is not particularly limited so long as the intended isolation can be achieved. The concentration can be, for example, 0.1 to 100 mg/mL, preferably 0.5 to 50 mg/mL, more preferably 1.5 to 30 mg/mL, further preferably 2 to 20 mg/mL, and particularly preferably 3 to 10 mg/mL. The volume of the load solution itself to be injected into the medium can be appropriately determined according to the volume of the medium, etc. in accordance with a routine method.

In step 2, any material can be used as the equilibration solution as long as the solution equilibrates pH and a salt concentration in the medium before isolation and can wash off a molecule non-specifically adsorbed to the medium without substantially isolating a molecule adsorbed to the medium. For a buffer solution that can be used as the equilibration solution or a salt concentration in the equilibration solution, see the description about the load solution. The amount of the

equilibration solution injected can be appropriately determined according to the volume of the medium, etc. in accordance with a routine method and can be, for example, 1 to 100 Column Volume (CV), preferably 5 to 50 CV, more preferably 8 to 40 CV, further preferably 10 to 30 CV, and particularly preferably 12 to 20 CV, though the amount is not limited thereto.

In step 2, the eluate can elute a molecule adsorbed onto the medium, and any material can be used. Also, any material can be used as the cleaning solution so long as the cleaning in place (CIP) of the medium is attained. Thus, the eluate and the cleaning solution can be appropriately determined in accordance with a known method or by application of a known method.

In the production method according to the present invention, the rate of transglycosylation after completion of step 2 can be set to a given numeric value and can be set to, for example, 85% or more, 90% or more, or 95% or more. In one aspect of the present invention, the rate of transglycosylation in step 2 exceeds 90%, preferably exceeds 95%, and particularly preferably exceeds 99%. The process yield of step 2 can be also set to a given numeric value. A higher process yield is desirable because step 2 with a higher process yield can reduce the amount of the glycan donor molecule used in step 1, as described above. However, the process yield is not particularly limited as long as the intended isolation can be achieved. The process yield can be, for example, 1 to 99%, preferably 5 to 95%, more preferably 20 to 95%, and particularly preferably 40 to 90%.

In the present invention, the cation exchange chromatography medium means a medium having a cation exchange group. The cation exchange chromatography medium is classified into a strong acid type in which a sulfonic acid group ($R-SO_3H$) is bonded to the surface, and a weak acid type in which a carboxyl group (R-COOH), a phenolic hydroxy group (R-OH), a phosphonic acid group [$R-P(=O)(OH)_2$], or a phosphinic acid group [R-P(=O) (OH)-R] is bonded to the surface. Thus, in one aspect of the present invention, the cation exchange chromatography medium is a strong acid-type or weak acid-type medium and/or is a medium having a sulfonic acid group, a carboxyl group, a phenolic hydroxy group, a phosphonic acid group, or a phosphinic acid group as a cation exchange group. A specific form of the ion exchange chromatography medium may be any of particle, membrane, monolith, plate, chip, and fiber shapes, for example. From the viewpoint of the characteristics of the molecule to be adsorbed onto the medium, examples thereof include, but are not limited to, particle, membrane, and monolith shapes.

Examples of the particle-shaped cation exchange chromatography medium include, but are not limited to, SP Sepharose FF (Cytiva), SOURCE 15S (Cytiva), Capto S Impact (Cytiva), Eshmuno CP-FT (Merck), Exhmuno CPX (Merck), POROS HS (Thermo), POROS XS (Thermo), Nuvia HR-S (BiO-RAD) and Cellufine Max GS (JNC).

Examples of the membrane-shaped cation exchange chromatography medium include, but are not limited to, Sartobind S (Sartorius) and Mustang S (Pall).

Examples of the monolith-shaped cation exchange chromatography medium include, but are not limited to, CIM monolith SO3 (BIA Separation).

The cation exchange chromatography of step 2 can be carried out using a known apparatus. Examples of such an apparatus can include, but are not limited to, AKTA avant 25 (Cytiva). The flow rate of a mobile phase in the cation exchange chromatography of step 2 can be appropriately determined in accordance with a routine method.

In the present invention, the multimode chromatography medium contains a cation exchange group and at least one functional group capable of acting in a manner different from the cation exchange group. In one embodiment, the functional group capable of acting in a different manner is a functional group capable of having hydrophobic interaction. Examples of the cation exchange group include the same as those described about the cation exchange chromatography medium. Examples of the form of the multimode chromatography medium include the same as those described about the cation exchange chromatography medium. Examples of the multimode chromatography medium include, but are not limited to, Capto(TM) MMC and TOYOPEARL(TM) MX-TRP-650M. The multimode chromatography of step 2 can be carried out using a known apparatus. The flow rate of a mobile phase in the multimode chromatography of step 2 can be appropriately determined in accordance with a routine method.

Hereinbelow, the present invention will be described with reference to Examples. However, the invention is not limited to them.

## Examples

### Example 1: Synthesis of Glycan-Remodeled Antibody 1 Preparation of Modified Anti-EGFR Antibody 1-[SG-$(N_3)_2$]$_2$

[Synthesis Scheme]

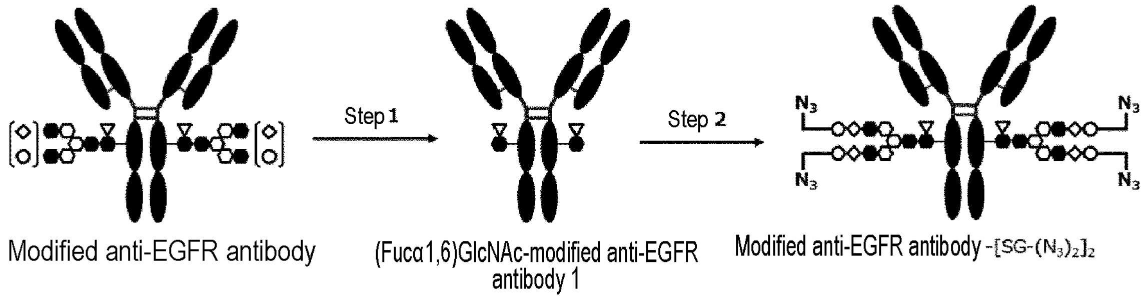


(Step 1)

Preparation of (Fucα1,6)GlcNAc-Modified Anti-EGFR Antibody 1

Phosphate-buffered saline (14.5 mL, 14.9 mg/mL, pH 6.0) containing modified anti-EGFR antibody 1 prepared according to Example 13 was mixed with wild-type EndoS-containing phosphate-buffered saline (0.140 mL, 7.70 mg/mL), and the mixture was shaken at 37°C for 4 h. The progress of the reaction was checked by Agilent 2100 bioanalyzer electrophoresis system (produced by Agilent Technologies). After completion of the reaction, purification by affinity chromatography and hydroxyapatite column chromatography were performed according to the following methods.

(1) Purification by Affinity Chromatography

Purification apparatus: AKTA avant 25 (produced by GE Healthcare)
Column: HiTrap rProtein A FF (5 mL) (produced by GE Healthcare)
Flow rate: 5 mL/min (1.25 mL/min at the time of charging)

At the time of column binding, the reaction mixture was added directly to the column, and the binding buffer (20 mM phosphate buffer (pH 6.0)) was made to flow at 1.25 mL/min for 2 CV, and then flowed at 5 mL/min for 5 CV. At the time of intermediate washing, 15 CV of washing solution (20 mM phosphate buffer (pH 7.0), 0.5 M sodium chloride solution) was flowed. In elution, 6 CV of elution buffer (IgG Elution buffer; produced by Thermo Scientific) was flowed. The eluate was immediately neutralized with 1 M Tris buffer (pH 9.0). Fractions containing the target product were subjected to buffer exchange to 5 mM phosphate buffer and 50 mM 2-morpholinoethanesulfonic acid (MES) solution (pH 6.8) according to the method described in common operation C. The antibody concentration in the resulting buffer solution was measured according to the method described in common operation B, and a crudely purified titled antibody solution (8.5 mL, 22.13 mg/mL) was thus obtained.

(2) Purification by Hydroxyapatite Chromatography Purification apparatus: AKTA avant 25

Column: Bio-Scale Mini CHT Type I cartridge (5 mL) (produced by BIO-RAD)
Flow rate: 5 mL/min (1.25 mL/min at the time of charging)

The solution obtained in (1) above was added to the column, and solution A (5 mM phosphate buffer, 50 mM MES solution (pH 6.8)) was made to flow at 1.25 mL/min for 2 CV, and then flowed at 5 mL/min for 3 CV. Subsequently, solution A and solution B (5 mM phosphate buffer, 50 mM MES solution (pH 6.8), 2 M sodium chloride solution) were used for elution. The elution conditions were solution A:solution B = 100:0 to 0:100 (5 CV). In addition, 5 CV of washing solution (500 mM phosphate buffer (pH 6.5)) was flowed. Fractions containing the target product were subjected to buffer exchange into 20 mM phosphate buffer (pH 6.0) according to the method described in common operation C. The antibody concentration in the resulting buffer solution was measured according to the method described in common operation B, and 20 mM phosphate buffer solution containing a titled antibody (14.5 mL, 12.93 mg/mL, pH 6.0) was thus obtained.

(Step 2)

Preparation of Modified Anti-EGFR Antibody 1-[SG-$(N_3)_2$]$_2$

[$N_3$-PEG(3)]$_2$-SG(10)Ox (44 mg) and EndoS (D233Q/Q303L)-containing phosphate-buffered saline (0.781 mL, 4.8 mg/mL) were added to 20 mM phosphate buffer solution (14.5 mL, 12.93 mg/mL, pH 6.0) containing the antibody obtained in step 1 , and the mixture was shaken at 30°C for 3 h. The progress of the reaction was checked by Agilent 2100 bioanalyzer electrophoresis system. [$N_3$-PEG(3)]$_2$-SG(10)Ox (4.2 mg) was further added thereto, and the mixture was shaken at 30°C for 1.5 h, followed by the addition of [$N_3$-PEG(3)]$_2$-SG(10)Ox (8.4 mg) again and shaking at 30°C for 2 h. After completion of the reaction, purification by affinity chromatography and hydroxyapatite column chromatography were performed, like in step 1. Fractions containing the target product were subjected to buffer exchange to phosphate buffered saline (pH 6.0) according to the method described in common operation C. The antibody concentration in the resulting buffer solution was measured according to the method described in common operation B, and phosphate-buffered saline containing a titled antibody (14.5 mL, 11.04 mg/mL, pH 6.0) was thus obtained.

Example 2: To Synthesize Glycan-Remodeled Antibody 2 Preparation of Modified Anti-EGFR Antibody 1-[MSG1-($N_3$)]$_2$

[Synthesis Scheme]

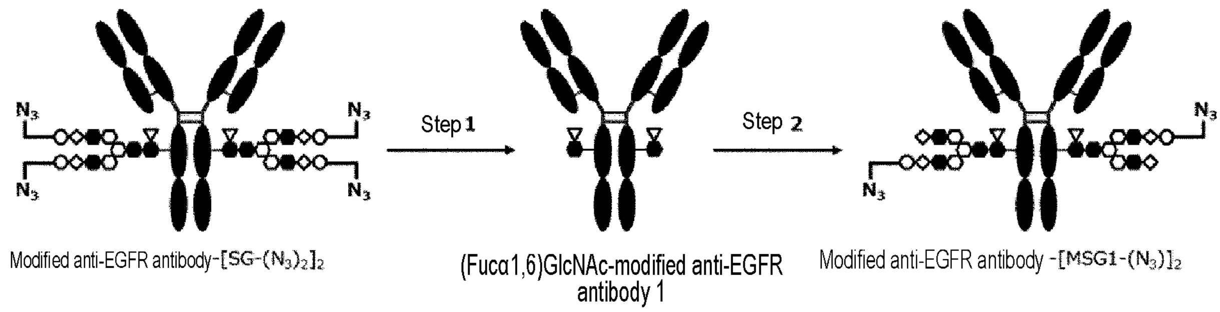


(Step 1)

Preparation of (Fucα1,6)GlcNAc-Modified Anti-EGFR Antibody 1

Using phosphate-buffered saline (9.0 mL, 11.04 mg/mL, pH 6.0) containing the glycan-remodeled antibody 1, substantially the same procedure as in step 1 of Example 1 was repeated. Then, the titled antibody-containing 20 mM phosphate buffer (9 mL, 9.11 mg/mL, pH 6.0) was obtained.

(Step 2)

Preparation of Modified Anti-EGFR Antibody 1-[MSG1-($N_3$)]$_2$

Using 20 mM phosphate buffer (9 mL, 9.11 mg/mL, pH 6.0) containing the antibody obtained in step 1 and water (0.130 mL) containing [$N_3$-PEG(3)]-MSG1(9)Ox (13 mg), substantially the same procedure as in step 2 of Example 1 was repeated. Then, the titled antibody-containing phosphate buffered saline (4 mL, 13.33 mg/mL, pH 6.0; 1 mL, 19.13 mg/mL, pH 6.0) was obtained.

Example 3: Synthesis of Glycan-Remodeled Antibody 3 Preparation of Modified Anti-EGFR Antibody 2-[SG-$(N_3)_2$]$_2$ [Synthesis Scheme]

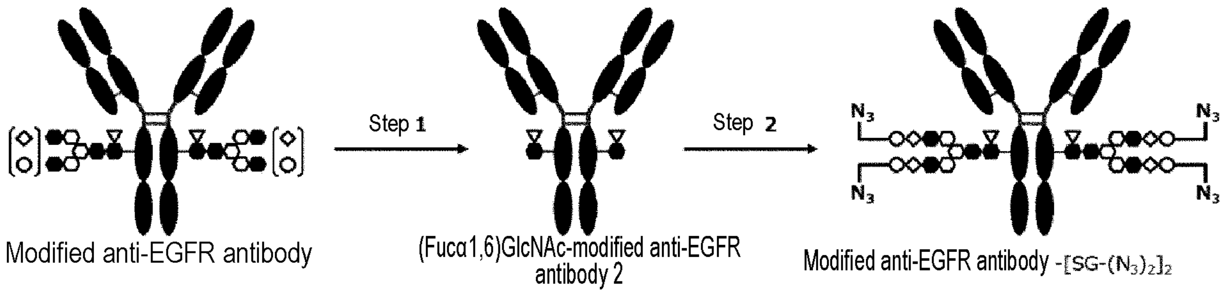


(Step 1)

Preparation of (Fucα1,6)GlcNAc-Modified Anti-EGFR Antibody 2

Using phosphate-buffered saline (20 mL, 11.25 mg/mL, pH 6.0) containing the modified anti-EGFR antibody 2 prepared according to Example 13, substantially the same procedure as in step 1 of Example 1 was repeated. Then, the titled antibody-containing 20 mM phosphate buffer (17 mL, 12.06 mg/mL, pH 6.0) was obtained.

(Step 2)

Preparation of Modified Anti-EGFR Antibody 2-[SG-$(N_3)_2$]$_2$

Using 20 mM phosphate buffer (8.5 mL, 12.06 mg/mL, pH 6.0) containing the antibody obtained in step 1 and [$N_3$-PEG(3)]$_2$-SG(10)Ox (40.8 mg), substantially the same procedure as in step 2 of Example 1 was repeated. Then, the titled antibody-containing phosphate buffered saline (8 mL, 11.09 mg/mL, pH 6.0) was obtained.

Example 4: Synthesis of Glycan-Remodeled Antibody 4 Preparation of Modified Anti-EGFR Antibody 2-[MSG1-($N_3$)]$_2$

[Synthesis Scheme]

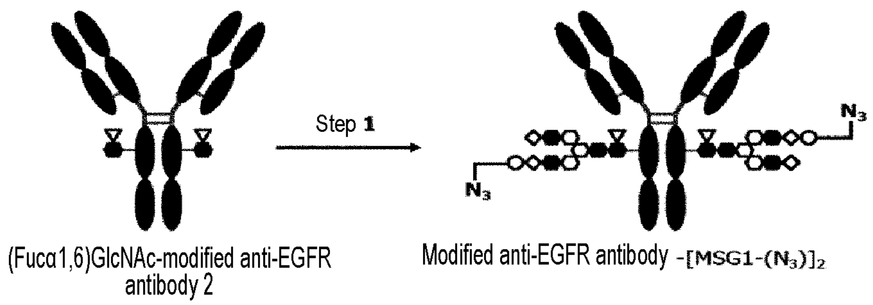


(Step 1)

Preparation of Modified Anti-EGFR Antibody 2-[MSG1-($N_3$)]$_2$

Using 20 mM phosphate buffer (8.5 mL, 12.06 mg/mL, pH 6.0) containing the antibody obtained in step 1 of Example 3 and water (0.190 mL) containing [$N_3$-PEG(3)]-MSG1(9)Ox (19 mg), substantially the same procedure as in step 2 of Example 1 was repeated. Then, the titled antibody-containing phosphate-buffered saline (8.5 mL, 10.58 mg/mL, pH 6.0) was obtained.

Example 5: Synthesis of Glycan-Remodeled Antibody 5 Preparation of Modified Anti-CDH6 Antibody 1-[SG-$(N_3)_2$]$_2$

[Synthesis Scheme]

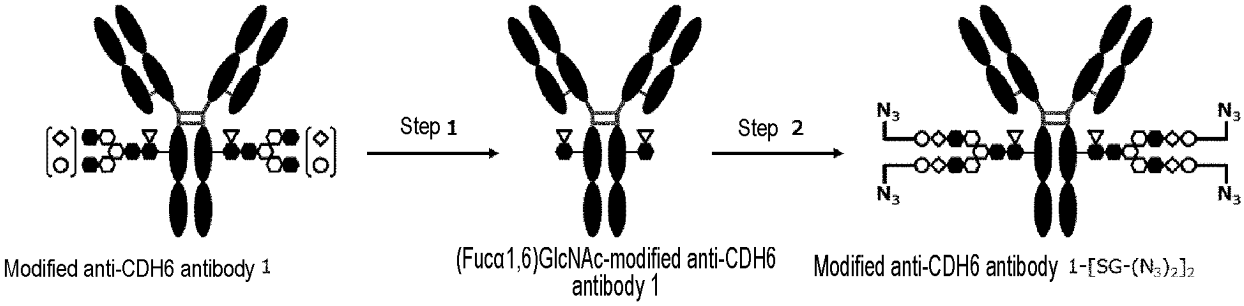


(Step 1)

Preparation of (Fucα1,6)GlcNAc-Modified Anti-CDH6 Antibody 1

Using phosphate-buffered saline (20.0 mL, 13.90 mg/mL, pH 6.0) containing the modified anti-CDH6 antibody 1 prepared according to Example 14, substantially the same procedure as in step 1 of Example 1 was repeated. Then, the titled antibody-containing 20 mM phosphate buffer (16 mL, 15.12 mg/mL, pH 6.0) was obtained.

(Step 2)

Preparation of Modified Anti-CDH6 Antibody 1-[SG-$(N_3)_2$]$_2$

Using 20 mM phosphate buffer (9.0 mL, 15.12 mg/mL, pH 6.0) containing the antibody obtained in step 1 and [$N_3$-PEG(3)]$_2$-SG(10)Ox (49.5 mg), substantially the same procedure as in step 2 of Example 1 was repeated. Then, the titled antibody-containing phosphate buffered saline (9 mL, 13.23 mg/mL, pH 6.0) was obtained.

Example 6: Synthesis of Glycan-Remodeled Antibody 6 Preparation of Modified Anti-CDH6 Antibody 1-[MSG1-$(N_3)$]$_2$

[Synthesis Scheme]

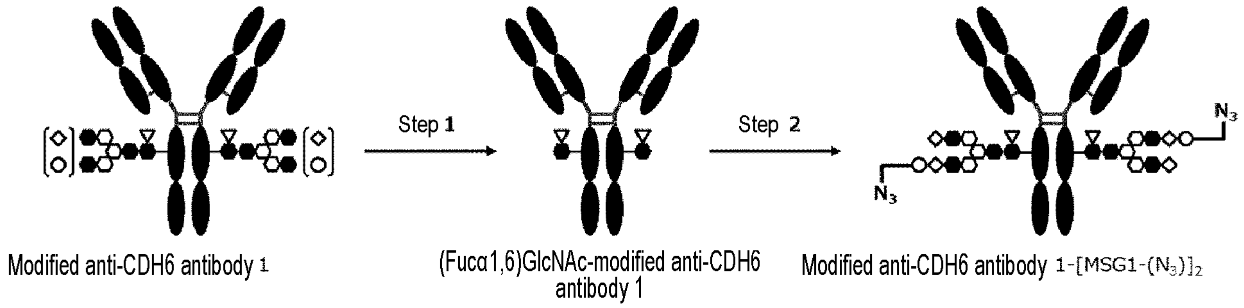


(Step 1)

Preparation of (Fucα1,6)GlcNAc-Modified Anti-CDH6 Antibody 1

Substantially the same procedure as in step 1 of Example 5 was repeated. Then, the titled antibody-containing phosphate buffered saline (17 mL, 14.85 mg/mL, pH 6.0) was obtained.

(Step 2)

Preparation of Modified Anti-CDH6 Antibody 1-[MSG1-$(N_3)$]$_2$

Using 20 mM phosphate buffer (2.7 mL, 14.85 mg/mL, pH 6.0) containing the antibody obtained in step 1 and water (0.075 mL) containing [$N_3$-PEG(3)]-MSG1(9)Ox (7.5 mg), substantially the same procedure as in step 2 of Example 1 was repeated. Then, the titled antibody-containing phosphate buffered saline (3 mL, 11.28 mg/mL, pH 6.0) was obtained.

Example 7: Synthesis of Antibody-Drug Conjugate 1 (Synthesis of Anti-EGFR Antibody 1-CDN Conjugate 1)

Glycan-remodeled antibody 1-containing phosphate-buffered saline (2.00 mL, 11.04 mg/mL, pH 6.0) was diluted with propylene glycol (1.00 mL). The resulting solution was mixed with a mixture of 10 mM dimethylsulfoxide solution of drug-linker 17a (bis(N,N-diethylethanaminium) N-[4-(11,12-didehydrodibenzo[b,f]azocin-5(6H)-yl)-4-oxobutanoyl]glycylglycyl-L-phenylalanyl-N-[(2-{9-[(5R,7R,8R,12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2$\lambda^5$,10$\lambda^5$-furo[3,2-l][1,3,6,9,11,2,10]pentaoxadiphosphacyclotetradecin-7-yl]-6-oxo-6,9-dihydro-1H-purin-1-yl}ethoxy)methyl]glycinamide; produced by the method described in WO2020/050406) (0.122 mL, 8 equivalents per antibody molecule) and propylene glycol (0.878 mL), and the reaction was carried out for 2 days at room temperature while using a tube rotator (MTR-103, AS ONE Corporation). The reaction mixture was subjected to purification by the method described in common operation D to give the target antibody-drug conjugatecontaining ABS solution (12.0 mL).

The following results were obtained by analysis according to the methods described in common operations E and G.

Antibody concentration: 1.38 mg/mL
Antibody yield: 16.58 mg (75%)
Average number of conjugated drugs: 3.7

Example 8: Synthesis of Antibody-Drug Conjugate 2 (Synthesis of Anti-EGFR Antibody 1-CDN Conjugate 2)

Using glycan-remodeled antibody 2-containing phosphate-buffered saline (1.00 mL, 13.33 mg/mL, pH 6.0) and dimethyl sulfoxide solution of drug-linker 17a (10 mM, 0.074 mL), procedures (reaction, purification, analysis) were performed according to the method described in Example 7 to give the target antibody-drug conjugate-containing ABS solution (6.5 mL). The following analysis results were obtained.

Antibody concentration: 1.57 mg/mL
Antibody yield: 10.23 mg (77%)
Average number of conjugated drugs: 1.9

Example 9: Synthesis of Antibody-Drug Conjugate 3 (Synthesis of Anti-EGFR Antibody 2-CDN Conjugate 1)

Using glycan-remodeled antibody 3-containing phosphate-buffered saline (2.00 mL, 11.09 mg/mL, pH 6.0) and dimethyl sulfoxide solution of drug-linker 17a (10 mM, 0.123 mL), procedures (reaction, purification, analysis) were performed according to the method described in Example 7 to give the target antibody-drug conjugate-containing ABS solution (12 mL). The following analysis results were obtained.

Antibody concentration: 1.40 mg/mL
Antibody yield: 16.77 mg (76%)
Average number of conjugated drugs: 3.8

Example 10: Synthesis of Antibody-Drug Conjugate 4 (Synthesis of Anti-EGFR Antibody 2-CDN Conjugate 2)

Using glycan-remodeled antibody 4-containing phosphate-buffered saline (2.00 mL, 10.58 mg/mL, pH 6.0) and dimethyl sulfoxide solution of drug-linker 17a (10 mM, 0.117 mL), procedures (reaction, purification, analysis) were performed according to the method described in Example 7 to give the target antibody-drug conjugate-containing ABS solution (12 mL). The following analysis results were obtained.

Antibody concentration: 1.45 mg/mL
Antibody yield: 17.46 mg (82%)
Average number of conjugated drugs: 1.9

Example 11: Synthesis of Antibody-Drug Conjugate 5 (Synthesis of Anti-CDH6 Antibody 1-CDN Conjugate 1)

Using glycan-remodeled antibody 5-containing phosphate-buffered saline (2.00 mL, 13.23 mg/mL, pH 6.0) and dimethyl sulfoxide solution of drug-linker 17a (10 mM, 0.146 mL), procedures (reaction, purification, analysis) were performed according to the method described in Example 7 to give antibody-drug conjugate-containing ABS solution (12 mL). The solution was purified by hydrophobic interaction chromatography. Fractions containing the target product were

subjected to buffer exchange into ABS using a NAP column and then concentrated according to the method described in common operation A to give the target antibody-drug conjugate-containing ABS solution (7.5 mL). The following analysis results were obtained.

Antibody concentration: 1.20 mg/mL
Antibody yield: 9.02 mg (34%)
Average number of conjugated drugs: 3.8

[Purification Conditions of Hydrophobic Interaction Chromatography]

Purification apparatus: AKTA avant 25
Column: HiTrap Butyl HP (5 mL) (produced by GE Healthcare)
Flow rate: 5 mL/min ((2.5 mL/min at the time of charging)
Mobile phase A: 1.5 M ammonium sulfate-containing 25 mM phosphate buffer (pH 7.0)
Mobile phase B: 25 mM phosphate buffer (pH 7.0)/isopropyl alcohol mixture (3:1)
Gradient program (mobile phase B): 0% (2 CV), 40% (3 CV), 50% (3 CV), 60% (3 CV), 100% (5 CV)

Example 12: Synthesis of Antibody-Drug Conjugate 6 (Synthesis of Anti-CDH6 Antibody 1-CDN Conjugate 2)

Using glycan-remodeled antibody 6-containing phosphate-buffered saline (2.00 mL, 11.71 mg/mL, pH 6.0) and dimethyl sulfoxide solution of drug-linker 17a (10 mM, 0.097 mL), procedures (reaction, purification, analysis) were performed according to the method described in Example 7 to give the target antibody-drug conjugate-containing ABS solution (12 mL). The following analysis results were obtained.

Antibody concentration: 1.56 mg/mL
Antibody yield: 18.68 mg (80%)
Average number of conjugated drugs: 1.8

Example 13: Production of Anti-EGFR Antibody A and Anti-EGFR Antibodies 1 to 3

Anti-EGFR antibody A and anti-EGFR antibodies 1 to 3 (herein, also referred to as modified anti-EGFR antibodies 1 to 3) were produced with reference to the Vectibix 100 mg Intravenous Infusion Review Results Report (March 5, 2010, Review and Administration Division, Pharmaceutical and Food Safety Bureau). The anti-EGFR antibody A used in the Examples is an IgG1 isotype. The anti-EGFR antibodies 1 and 3 were produced by introducing a LALA-DG mutation and a LALA mutation, respectively, into the anti-EGFR antibody A. The anti-EGFR antibody 2 is an IgG1 isotype with a LALA-DG mutation. Figs. 21-1, 21-2, and 21-3 show the light chain amino acid sequence (SEQ ID NO: 30) of anti-EGFR antibody A and anti-EGFR antibodies 1 to 3 and the heavy chain amino acid sequence (SEQ ID NO: 32) of anti-EGFR antibody 1, the heavy chain amino acid sequence (SEQ ID NO: 33) of anti-EGFR antibody 2, the heavy chain amino acid sequence (SEQ ID NO: 34) of anti-EGFR antibody A, and the heavy chain amino acid sequence (SEQ ID NO: 36) of anti-EGFR antibody 3, used in the Examples. Figure 20 shows the amino acid sequence of CDRL1 (SEQ ID NO: 23), the amino acid sequence of CDRL2 (SEQ ID NO: 24), the amino acid sequence of CDRL3 (SEQ ID NO: 25), the amino acid sequence of CDRH1 (SEQ ID NO: 26), the amino acid sequence of CDRH2 (SEQ ID NO: 27), and the amino acid sequence of CDRH3 (SEQ ID NO: 28) of anti-EGFR antibody A and anti-EGFR antibodies 1 to 3 used in the Examples.

Example 14: Production of Anti-CDH6 Antibody 1

Anti-CDH6 antibody 1 (herein, also referred to as modified anti-CDH6 antibody 1) was produced with reference to WO2018/212136. The anti-CDH6 antibody 1 used in the Examples is an IgG1 isotype with a LALA-DG mutation. Figure 23 shows the light chain amino acid sequence (SEQ ID NO: 44) and the heavy chain amino acid sequence (SEQ ID NO: 46) of anti-CDH6 antibody 1 used in the Examples. Figure 22 shows the amino acid sequence of CDRL1 (SEQ ID NO: 37), the amino acid sequence of CDRL2 (SEQ ID NO: 38), the amino acid sequence of CDRL3 (SEQ ID NO: 39), the amino acid sequence of CDRH1 (SEQ ID NO: 40), the amino acid sequence of CDRH2 (SEQ ID NO: 41), and the amino acid sequence of CDRH3 (SEQ ID NO: 42) of anti-CDH6 antibody 1 used in the Examples.

Reference Example 1: Synthesis of Glycan-Remodeled Antibody 7

Preparation of Anti-EGFR Antibody A-[SG-$(N_3)_2$]$_2$

[Synthesis Scheme]

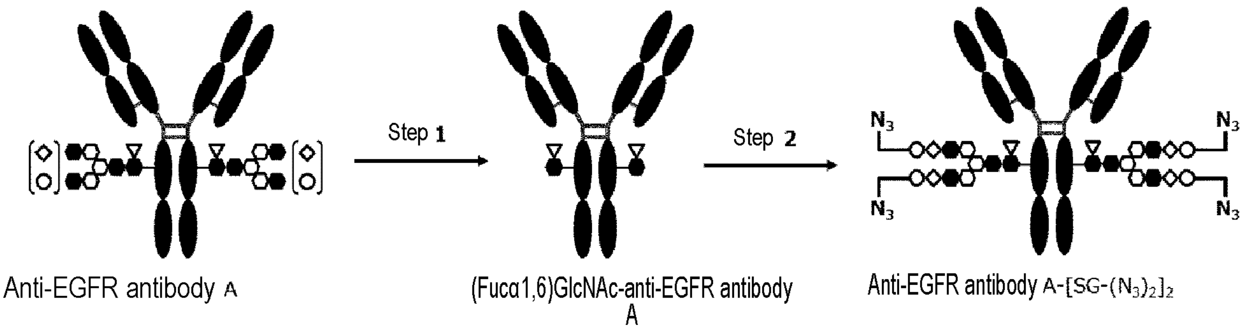


(Step 1)

Preparation of (Fucα1,6)GlcNAc-Anti-EGFR Antibody A

Using phosphate-buffered saline (9.5 mL, 14.1 mg/mL, pH 6.0) containing anti-EGFR antibody A prepared according to Example 13, substantially the same procedure as in step 1 of Example 1 was repeated. Then, the titled antibody-containing 20 mM phosphate buffer (7.5 mL, 15.93 mg/mL, pH 6.0) was obtained.

(Step 2)

Preparation of Anti-EGFR Antibody A-[SG-$(N_3)_2$]$_2$

¥using 20 mM phosphate buffer (7.5 mL, 15.93 mg/mL, pH 6.0) containing the antibody obtained in step 1 and [$N_3$-PEG(3)]$_2$-SG(10)Ox (30.4 mg), substantially the same procedure as in step 2 of Example 1 was repeated. Then, the titled antibody-containing phosphate buffered saline (3 mL, 11.10 mg/mL, pH 6.0) was obtained.

Reference Example 2: Synthesis of Glycan-Remodeled Antibody 8

Preparation of Anti-EGFR Antibody B-[MSG1-$(N_3)$]$_2$

[Synthesis Scheme] The following scheme shows IgG2.

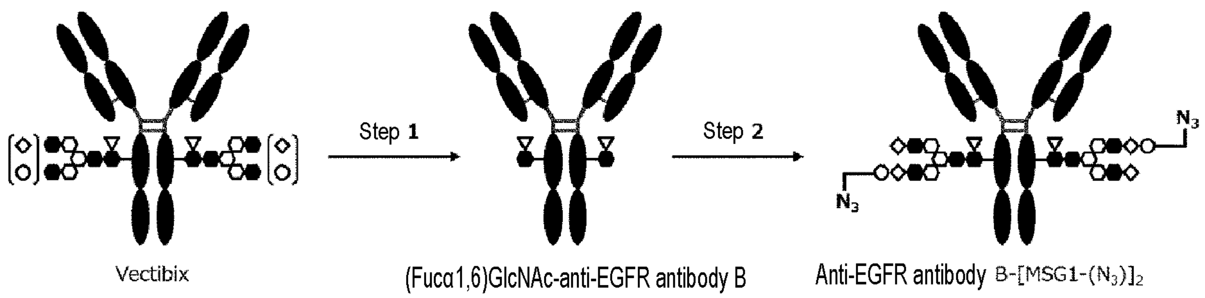


(Step 1)

Preparation of (Fucα1,6)GlcNAc-Anti-EGFR Antibody B

Commercially available Vectibix Intravenous Infusion (Takeda Pharmaceuticals) (100 mg/5 mL) was subjected

to buffer exchange using a NAP column (NAP-25 columns (produced by GE Healthcare)) to give antibody-containing phosphate-buffered saline (7.0 mL, 13.73 mg/mL, pH 6.0). Using this antibody solution (3.5 mL), substantially the same procedure as in step 1 of Example 1 was repeated. Then, the titled antibody-containing 20 mM phosphate buffer (5 mL, 8.49 mg/mL, pH 6.0) was obtained.

(Step 2)

Preparation of Anti-EGFR Antibody B-[MSG1-($N_3$)]$_2$

Using 20 mM phosphate buffer (4.5 mL, 8.49 mg/mL, pH 6.0) containing the antibody obtained in step 1 and water (0.076 mL) containing [$N_3$-PEG(3)]-MSG1(9)Ox (7.6 mg), substantially the same procedure as in step 2 of Example 1 was repeated. Then, the titled antibody-containing phosphate buffered saline (2.5 mL, 12.90 mg/mL, pH 6.0) was obtained.

Reference Example 3: Synthesis of Glycan-Remodeled Antibody 9

Preparation of Modified Anti-EGFR Antibody 3-[SG-($N_3$)$_2$]$_2$

[Synthesis Scheme]

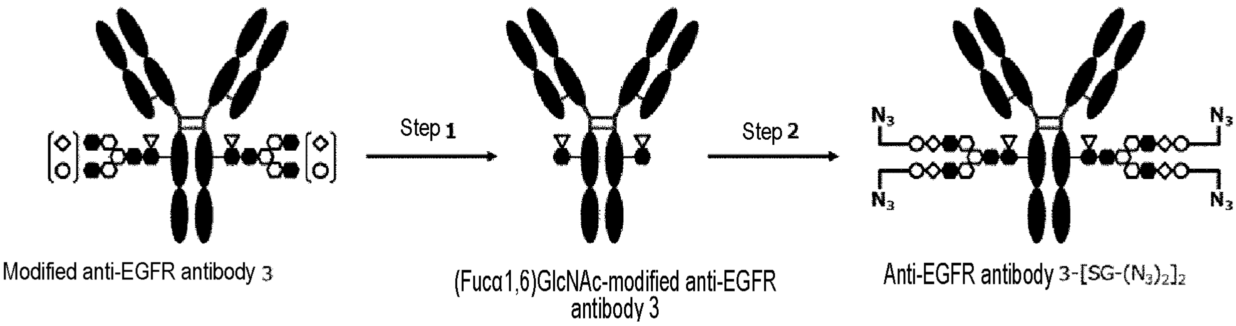


(Step 1)

Preparation of (Fucα1,6)GlcNAc-Modified Anti-EGFR Antibody 3

Using phosphate-buffered saline (10 mL, 14.00 mg/mL, pH 6.0) containing the modified anti-EGFR antibody 3 prepared according to Example 13, substantially the same procedure as in step 1 of Example 1 was repeated. Then, the titled antibody-containing 20 mM phosphate buffer (7.5 mL, 16.70 mg/mL, pH 6.0) was obtained.

(Step 2)

Preparation of Modified Anti-EGFR Antibody 3-[SG-($N_3$)$_2$]$_2$

Using 20 mM phosphate buffer (7.5 mL, 16.70 mg/mL, pH 6.0) containing the antibody obtained in step 1 and [$N_3$-PEG(3)]$_2$-SG(10)Ox (29 mg), substantially the same procedure as in step 2 of Example 1 was repeated. Then, the titled antibody-containing phosphate buffered saline (10 mL, 10.49 mg/mL, pH 6.0) was obtained.

Reference Example 4: Synthesis of Glycan-Remodeled Antibody 10

Preparation of Modified Anti-EGFR Antibody 3-[MSG1-($N_3$)]$_2$

[Synthesis Scheme]

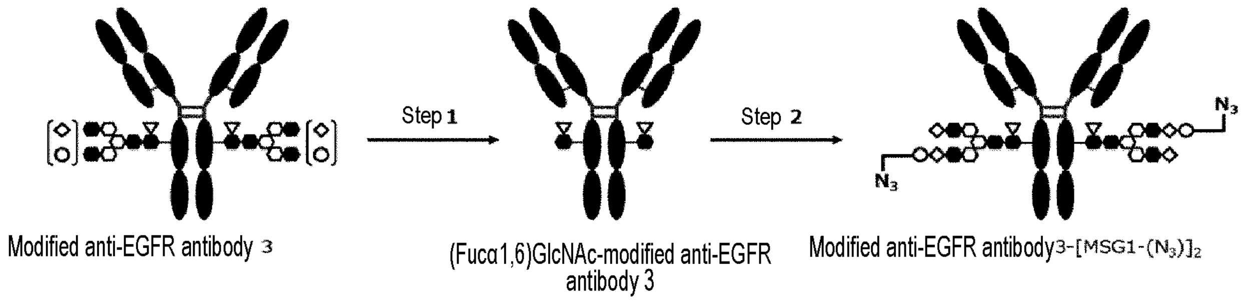


(Step 1)

Preparation of (Fucα1,6)GlcNAc-Modified Anti-EGFR Antibody 3

Substantially the same procedure as in step 1 of Reference Example 3 was repeated. Then, the titled antibody-containing 20 mM phosphate buffer (7.5 mL, 14.51 mg/mL, pH 6.0) was obtained.

(Step 2)

Preparation of Modified Anti-EGFR Antibody 3-[MSG1-$(N_3)]_2$

Using 20 mM phosphate buffer (7.5 mL, 14.51 mg/mL, pH 6.0) containing the antibody obtained in step 1 and water (0.173 mL) containing [$N_3$-PEG(3)]-MSG1(9)Ox (17.3 mg), substantially the same procedure as in step 2 of Example 1 was repeated. Then, the titled antibody-containing phosphate buffered saline (7.5 mL, 13.34 mg/mL, pH 6.0) was obtained.

Reference Example 5: Synthesis of Antibody-Drug Conjugate 7 (Synthesis of Anti-EGFR Antibody A-CDN Conjugate 1)

Using glycan-remodeled antibody 7-containing phosphate-buffered saline (2.00 mL, 11.10 mg/mL, pH 6.0) and dimethyl sulfoxide solution of drug-linker 17a (10 mM, 0.123 mL), procedures (reaction, purification, analysis) were performed according to the method described in Example 7 to give a target antibody-drug conjugate-containing ABS solution (12 mL). The following analysis results were obtained.

Antibody concentration: 1.39 mg/mL
Antibody yield: 16.70 mg (75%)
Average number of conjugated drugs: 3.7

Reference Example 6: Synthesis of Antibody-Drug Conjugate 8 (Synthesis of Anti-EGFR Antibody B-CDN Conjugate 1)

Using glycan-remodeled antibody 8-containing phosphate-buffered saline (0.50 mL, 12.90 mg/mL, pH 6.0) and dimethyl sulfoxide solution of drug-linker 17a (10 mM, 0.036 mL), procedures (reaction, purification, analysis) were performed according to the method described in Example 7 to give a target antibody-drug conjugate-containing PBS solution (3.5 mL, pH 7.4). The following analysis results were obtained.

Antibody concentration: 1.06 mg/mL
Antibody yield: 3.72 mg (58%)
Average number of conjugated drugs: 1.9

Reference Example 7: Synthesis of Antibody-Drug Conjugate 9 (Synthesis of Anti-EGFR Antibody 3-CDN Conjugate 1)

Using glycan-remodeled antibody 9-containing phosphate-buffered saline (1.00 mL, 10.49 mg/mL, pH 6.0) and dimethyl sulfoxide solution of drug-linker 17a (10 mM, 0.058 mL), procedures (reaction, purification, analysis) were performed according to the method described in Example 7 to give a target antibody-drug conjugate-containing ABS solution (6.5 mL). The following analysis results were obtained.

Antibody concentration: 1.12 mg/mL
Antibody yield: 7.26 mg (69%)
Average number of conjugated drugs: 3.5

Reference Example 8: Synthesis of Antibody-Drug Conjugate 10 (Synthesis of Anti-EGFR Antibody 3-CDN Conjugate 2)

Using glycan-remodeled antibody 10-containing phosphate-buffered saline (1.00 mL, 13.34 mg/mL, pH 6.0) and dimethyl sulfoxide solution of drug-linker 17a (10 mM, 0.074 mL), procedures (reaction, purification, analysis) were performed according to the method described in Example 7 to give a target antibody-drug conjugate-containing ABS solution (6.5 mL). The following analysis results were obtained.

Antibody concentration: 1.68 mg/mL
Antibody yield: 10.91 mg (82%)
Average number of conjugated drugs: 1.9

(Reference Example 9: Synthesis of ML-RR-CDA·$2Na^+$)

The ML-RR-CDA·$2Na^+$ used as a reference compound herein was synthesized according to the procedure described in Patent Literature 10 (WO2014/189805).

(Reference Example 10: Synthesis of 2',3'-cGAMP)

2',3'-cGAMP used as a reference compound herein was synthesized enzymatically from ATP and GTP using cGAS. The preparation of cGAS and the enzymatic reaction were carried out by modifying, if appropriate, the procedures described in the literatures (Immunity, 2013, 39, 1019-1031; Cell Rep. 2014, 6, 421-430). Purification was performed by column chromatography using a weakly basic anion exchange resin (DIAION WA10) and a synthetic adsorbent (SEPABEADS SP207SS).

(Test Example 1) Evaluation of STING Agonist Activity by Using Reporter Cells

<Reporter Gene Assay>

Human STING agonist activity was evaluated using THP1-Dual™ cells (HAQ mutant) (InvivoGen, CA, US), in which activation of the interferon regulatory factor-3 (IRF3) pathway, which is downstream of the STING pathway, could be checked.

The assay was conducted as follows. Each test compound was dispensed onto a 96-well plate. Then, reporter cells were added thereto to start stimulation. The cells were cultured for 24 h under conditions at 37°C and 5% $CO_2$, and then centrifuged to collect the supernatant. Next, the collected supernatant was added to a 384-well plate, and QUANTI-Luc (InvivoGen) solution was added. After mixing well, the luminescence was measured using a plate reader. The value for the maximum count in cells treated with 1.37 to 100 μM ML-RR-CDA·$2Na^+$ (Compound 21 in WO2014/189805) was set to 100% and the count in cells treated with PBS was set to 0%. Then, the concentration of each test compound required to obtain 50% count was calculated as the value for EC50 (μM). Table 5 shows the structural formula and NMR data of the test compound, and Table 4 shows the results of the assay for the human STING agonist activity. Note that the test compound can be produced according to the above <3. Production Method> or WO2020/050406.

[Table 3]

| Compound No. | THP1-Dual cell IRF EC50 (μM) |
|---|---|
| 6a | 0.6 |
| 6b | 2.1 |
| 33a | 0.18 |
| 34a | 0.20 |
| 34b | 0.55 |
| 39a | 0.19 |
| 39b | 0.41 |

(continued)

| Compound No. | THP1-Dual cell IRF EC50 (μM) |
|---|---|
| 44a | 0.21 |
| 44b | 0.35 |
| 48a | 0.035 |
| 48b | 0.039 |
| 52a | 0.31 |
| 52b | 0.46 |
| 53a | 6.0 |
| 54 | 4.0 |
| ML-RR-CDA·2Na$^+$ | 4.2 |
| 2' 3' -cGAMP | 21.8 |

[Table 4]

| Compound No. Structural formula | $^1$H-NMR ($CD_3OD$) |
|---|---|
| 6 a 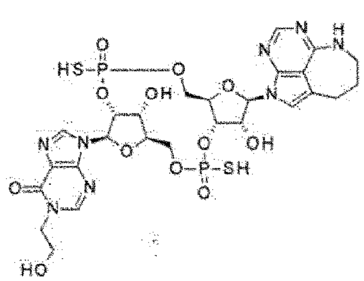 | δ : 8.63 (1H, s), 8.22 (1H, s ), 8.02 (1H, s), 7.11 (1H, s ), 6.30-6.24 (2H, m), 5.46 -5.37 (1H, m), 5. 23-5.15 ( 1H, m), 4. 83-4.79(1H, m), 4.78-4.74 (1H, m), 4. 53-4 .42(2H, m), 4. 35-4.16(3H , m), 4. 16-3.97 (3H, m), 3. 83-3.78 (2H, m), 3. 52-3. 4 7 (2H, m), 2.88-2.81(2H, m ), 2.03-1.95(2H, m) |
| 6 b 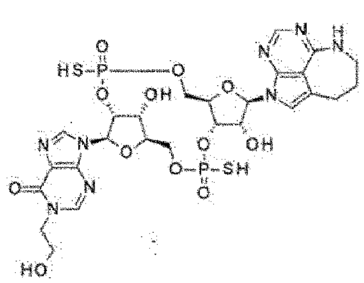 | δ : 8.72(1H, s), 8.23 (1H, s ), 8.02 (1H, s), 7.11 (1H, s ), 6.30 (2H, dd, J=13.6, 7. 6Hz), 5.48-5.39 (2H, m), 4 . 78(1H, dd, J=6. 7, 4. 2Hz) , 4.51-4.28 (5H, m), 4. 26-4.13(2H, m), 4.06-4.00(1 H, m), 3.93-3.86(1H, m), 3 . 85-3.80 (2H, m), 3.52-3. 47(2H, m), 2.94-2.88 (2H, m), 2.05-1.97 (2H, m) |
| 3 3 a 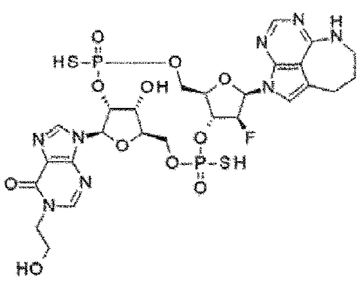 | δ : 8.67(1H, s), 8.24 (1H, s ), 8.04 (1H, s), 7.05 (1H, s ), 6.73 (1H, dd, J=23. 9, 2. 7Hz), 6.28 (1H, d, J=8.5Hz ), 5.43-5.24(3H, m), 4.77 -4.72(1H, m), 4.51-4.32 ( 4H, m), 4.26-4.12(2H, m), 4.06-3.91(2H, m), 3.82(2 H, t, J=5.1Hz), 3.50(2H, t , J=5.1Hz), 2. 92-2. 85 (2H , m), 2. 06-1. 97 (2H, m) |

(continued)

| Compound No. Structural formula | $^1$H-NMR ($CD_3OD$) |
|---|---|
| 3 4 a 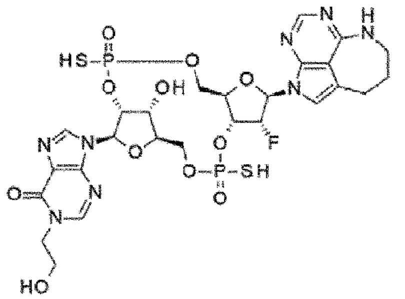 | δ : 8. 58 (1H, m), 8.11(1H, m ), 8.03 (1H, s), 7.11 (1H, s ), 6.47 (1H, d, J=17.5Hz), 6.26 (1H, d, J=8.5Hz), 5. 5 3-5.36(2H, m), 5.29-5.17 (1H, m), 4. 77 (1H, d, J=4. 2 Hz),4. 54-4.46(1H, m), 4. 44-4.38(1H, m), 4. 35-4.3 2 (1H, m), 4.30-4.25(2H, m ), 4.25-4.1.6 (1H, m), 4. 06 -3.99 (1H, m), 3.96-3.85 ( 1H, m), 3.82-3.71(2H, m), 3. 54-3. 42 (2H, m), 2. 77-2 .68 (1H, m), 2.66-2.55(1H , m), 2.02-1.81(2H, m) |
| 3 4 b 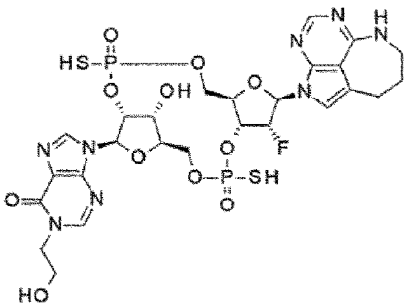 | δ : 8.61(1H, m), 8. 16 (1H, m ), 8.02(1H, m), 7.36 (1H, s ), 6.49 (1H, dd, J=16.0, 2. 1Hz), 6. 28 (1H, d, J=8.5Hz ), 5.56-5.33(3H, m), 4.58 -4.49 (2H, m), 4.45-4.37 ( 2H, m), 4.31-4.27(1H, m), 4.25-4.16 (1H, m), 4.10-3 . 98(3H, m), 3.80 (2H, t, J = 5.1Hz), 3. 48 (2H, dd, J=6. 7, 3. 6Hz), 2.90-2.72(2H, m), 2.00-1.90 (2H, m) |
| 3 9 a 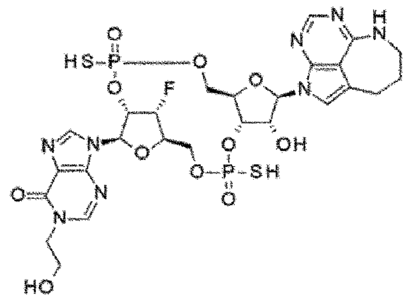 | δ : 8.66 (1H, m), 8. 26(1H, s ), 8.02 (1H, s), 7.09 (1H, s ), 6.29-6.24(2H, m), 5.68 -5.45(2H, m), 5.26-5.18 ( 1H, m), 4.78-4.73(1H, m), 4.62-4.42(3H, m), 4.26-3 .98(5H, m), 3. 8 5-3.78 (2H , m), 3.53-3.47 (2H, m), 2. 91-2. 84 (2H, m), 2. 04-1. 9 6 (2H, m) |
| 3 9 b 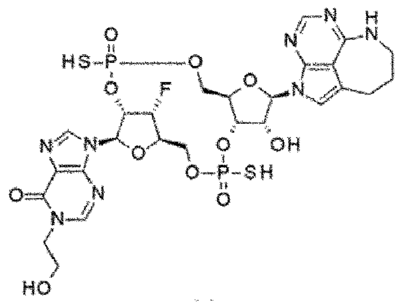 | δ : 8.73 (1H, m), 8.25 (1H, s ), 8.02(1H, s), 7. 10 (1H, b r s), 6.33(1H, d, J=6.7Hz) ,6.28 (1H, d, J=9. 1Hz),5. 65-5.50 (1H, m), 5.49-5.4 3 (1H, m), 5. 31 (1H, d d, J=5 4. 4, 3. 6Hz),4. 79 (1H, dd, J=6. 3, 4. 5Hz),4. 6 2-4. 34 (4H, m), 4.27-4.14(2H, m) , 4. 08-4.01(1H, m), 3. 93-3.87 (1H, m), 3.86-3.80(2 H, m), 3.53-3.47(2H, m), 2 .95-2.89(2H, m), 2.06-1. 97(2H, m) |

(continued)

| Compound No. Structural formula | $^1$H-NMR ($CD_3OD$) |
|---|---|
| 4 4 a 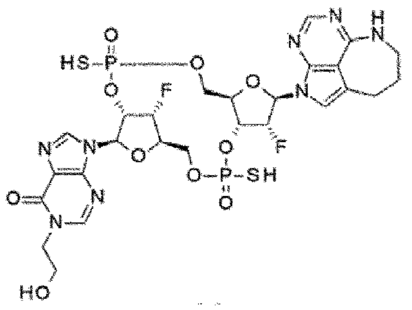 | δ : 8.63 (1H, s), 8.16 (1H, s ), 8.03 (1H, s), 7.08 (1H, s ), 6.47 (1H, dd, J=17.5, 1. 8Hz), 6.27 (1H, d, *J*=7.9Hz ), 5.64-5.36 (3H, m), 5.31 -5. 20 (1H, m), 4.62-4.50 ( 2H, m), 4.44-4.39 (1H, m), 4. 32-4. 12 (3H, m), 4. 10-4 . 03 (1H, m), 3.99-3.91 (1H ,m), 3.83-3.72 (2H, m), 3. 52-3.46(2H, m), 2.78-2.7 2 (2H, m), 2.04-1.85(2H, m ) |
| 4 4 b 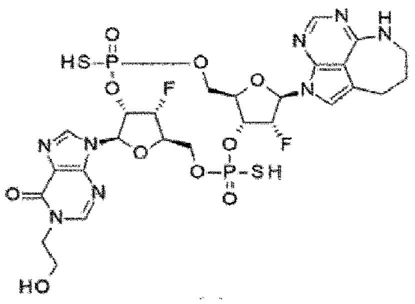 | δ : 8. 6 9 (1H, s), 8.21(1H, s ), 8. 0 3 (1H, s), 7.25 (1H, s ), 6.49(1H, dd, J=15.1, 3. 0Hz), 6.28(1H, d, J=9. 1Hz ), 5. 64-5.28(4H, m), 4.61 -4.39(4H, m), 4. 26-4.17 ( 1H, m), 4.13-3.95(3H, m), 3.84-3.77(2H, m), 3.53-3 .45 (2H, m), 2.92-2.77 (2H , m), 2. 02-1.92 (2H, m) |
| 4 8 a 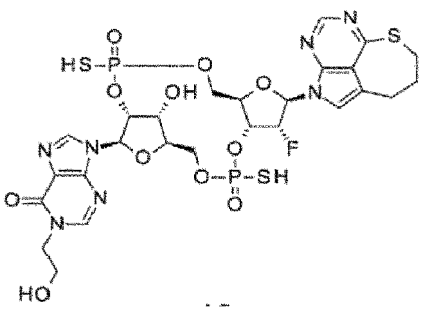 | δ : 8. 55 (1H, brs), 8. 41 (1H , s), 8. 09 (1H, brs), 7. 51( 1H, s), 6. 58 (1H, d, J=16.9 Hz), 6. 25(1H, d, J=7.9Hz) , 5. 55-5.34 (2H, m), 5. 30-5. 17 (1H, m), 4. 74 (1H, d, J =4. 2Hz), 4.55-4. 47 (1H, m ), 4. 4 6 - 4. 40(1H, m), 4. 37 -4. 30(2H, m), 4. 28-4.16 ( 2H, m), 4. 05-3.99 (1H, m), 3. 90-3. 70(3H, m), 3.28-3 . 20(1H, m), 3.18-3.10 (1H , m), 2.91-2.82 (1H, m), 2. 76-2. 64 (1H, m), 2.33-2.2 2 (1H, m), 2.21-2.09(1H, m ) |
| 4 8 b 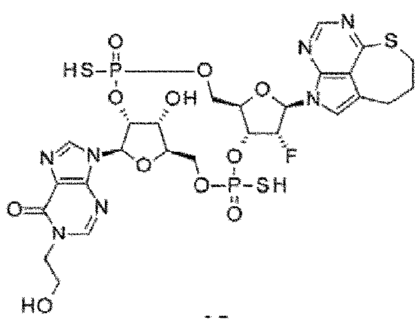 | δ : 8. 60(1H, s), 8. 41 (1H, s ), 8. 16(1H, s), 7. 72 (1H, s ), 6. 60(1H, d, J=15.7Hz), 6. 28(1H, d, J=7.9Hz), 5. 6 1-5.33(3H, m), 4. 59-4.49 (2H, m), 4.48-4.39 (2H, m) , 4. 34-4.27 (1H, m), 4. 25-4. 16(1H, m), 4. 11-3.9 9 (3 H, m), 3. 86-3.75(2H, m), 3 . 25-3.11(2H, m), 3. 05-2. 90(2H, m), 2. 35-2.17 (2H, m) |

(continued)

| Compound No. Structural formula | $^1$H-NMR ($CD_3OD$) |
|---|---|
| 52a 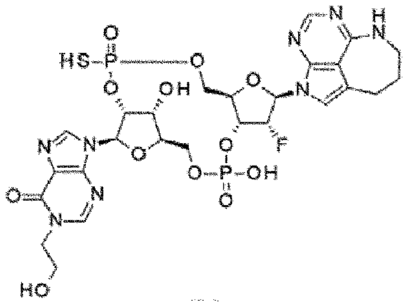 | δ : 8. 55(1H, m), 8. 15 (1H, m ), 8. 03(1H, s), 7. 14(1H, d , J=4. 8Hz), 6. 46 (1H, d, J= 18.1Hz), 6. 28 (1H, d, J=7. 9Hz), 5. 50-5.29(2H, m), 5 . 16-5.04(1H, m), 4. 74-4. 69 (1H, m), 4. 40-4. 18 (6H, m), 4. 13-4.07(1H, m), 4. 0 2-3. 91 (1H, m), 3. 84-3. 74 (2H, m), 3. 53-3.43 (2H, m) , 2. 81-2.63(2H, m), 2. 02-1. 85(2H, m) |
| 52b 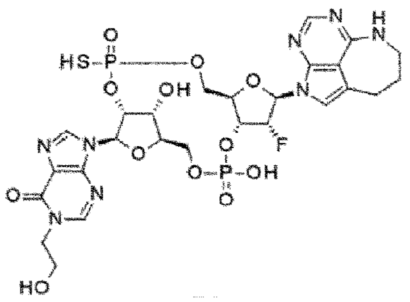 | δ : 8. 58(1H, d, J=3.0Hz), 8 . 19(1H, d, J=2.4Hz), 8.03 (1H, s), 7. 40(1H, s), 6. 49 (1H, dd, J=16. 3, 1. 8Hz), 6. 29 (1H, d, J=8. 5Hz), 5. 5 1-5. 22(3H, m), 4. 59-4.55 (1H, m), 4. 43-4.17 (5H, m) , 4. 14-4.01 (3H, m), 3. 85-3. 78 (2H, m), 3. 51-3.44 (2 H, m), 2. 90-2.75 (2H, m), 1 . 99-1.90(2H, m) |
| 53a 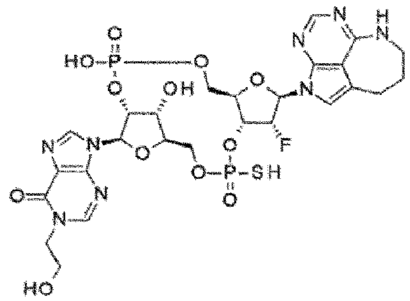 | δ : 8. 58(1H, s), 8. 12(1H, s ), 8. 02(1H, s), 7. 13 (1H, s ), 6. 47 (1H, d, J=18.1Hz), 6. 27 (1H, d, J=8. 5Hz), 5. 4 1 (1H, dd, J=51.7, 3. 9Hz), 5. 29-5.16 (2H, m), 4. 58-4 .52(2H, m), 4.36-4.1 7 (5H , m), 4. 05-3.90 (2H, m), 3. 82-3.71 (2H, m), 3. 52-3.4 3 (2H, m), 2.76-2.63(2H, m ), 2.02-1.85(2H, m) |
| 54 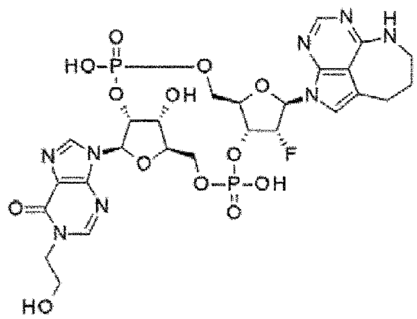 | δ : 8. 56(1H, s), 8. 19(1H, s ), 8. 03(1H, s), 7. 10(1H, s ), 6. 46 (1H, d, J=18.7Hz), 6. 31(1H, d, J=8. 5Hz), 5. 5 1-5. 32(1H, m), 5. 24-5. 01 (2H, m), 4. 61-4.56(1H, m) , 4. 41-4.21(5H, m), 4. 21-3. 97(3H, m), 3. 87-3.75(2 H, m), 3. 54-3.41(2H, m), 2 . 83-2.67 (2H, m), 2. 02-1. 85 (2H, m) |

$^1$H-NMR spectra were measured using JEOL ECS-400 (400 MHz), Varian 400-MR (400 MHz), or Varian Unity Inova 500 (500 MHz).

The results have revealed that each compound used in an antibody-drug conjugate of the present invention has agonist activity against human STING. It has also been found that the agonist activity against mouse STING was equivalent to or higher than that of existing CDNs (WO2020/050406).

(Test Example 2) Thermal Shift Assay Using Recombinant STING C-Terminal Binding Domain Protein

(i) Construction of Various Expression Plasmids

<Construction of Human TMEM173-Expressing Plasmid>

The plasmid for expressing human STING (sometimes, herein referred to as human TMEM173) in mammalian cells was a human TMEM173 cDNA clone (a plasmid for expressing Accession NM_198282.3, H232 (REF) mutant STING) (GeneCopoeia, MD, US) in which arginine (R) at position 232 was mutated to histidine (H) (herein, referred to as H232 mutant or REF mutant), and was purchased. The human H232 (REF) mutant STING amino acid sequence is provided as SEQ ID NO: 3 and the nucleotide sequence is provided as SEQ ID NO: 4. In addition, the H232 mutant STING expression plasmid was used as a template for site-directed mutagenesis based on the Inverse PCR method. The expression plasmid for each of wild-type STING and HAQ (R71H, G230A and R293Q) mutant STING was constructed. The amino acid sequence of human wild-type STING is shown in SEQ ID NO: 1 and the nucleotide sequence is shown in SEQ ID NO: 2. The amino acid sequence of human HAQ mutant STING is shown in SEQ ID NO: 5 and the nucleotide sequence is shown in SEQ ID NO: 6. The amino acid sequences of human wild-type STING, REF mutant STING, and HAQ mutant STING are shown in Figure 21.

<Construction of Expression Plasmid for Recombinant STING C-Terminal Binding Domain Protein and Others>

The cDNA for human STING C-terminal binding domain (aa. 139 to 342) protein (UniProt entry Q86WV6) was prepared by PCR from the full-length human TMEM173 cDNA clone-expressing plasmid (wild-type, H232 mutant, and HAQ mutant) using two different primers (5'-ACCTGTATTTTCAGGGCCTGGCCCCAGCTGAGATCTCTG-3 '(hST Fw_v2) (SEQ ID NO: 21) and 5'-CAGAATTCGCAAGCTTTTAAGTAACCTCTTCCTTTTCCTCCTGC-3' (hST Rv_V3) (SEQ ID NO: 22)). Each PCR product was inserted into the *E. coli* expression vector, pET15b, using an In-Fusion HD Cloning Kit (Takara Bio) such that the N-terminus contained a 6xHis tag consisting of six histidine residues, an Avidin tag and a TEV protease cleavage site. Expression plasmids: pET15b-HisAviTEV- hSTING(139-342) human wild type, pET15b-HisAviTEV hSTING(139-342) human REF mutant and pET15b-HisAviTEV-hSTING(139-342) human AQ mutant were constructed.

The cDNA used for expressing mouse STING C-terminal domain (aa. 138-341) protein (UniProt entry Q3TBT3) was an artificially synthesized cDNA (eurofins Genomics) corresponding to amino acids at positions 138 to 341 of the mouse TMEM173 cDNA sequence. The amino acid sequence of mouse STING is shown in SEQ ID NO: 7 and the nucleotide sequence is shown in SEQ ID NO: 8. The synthesized cDNA was inserted into the *E. coli* expression vector, pET15b, using an In-Fusion HD Cloning Kit such that the N-terminus contained a 6xHis tag consisting of six histidine residues, an Avidin tag, and a TEV protease cleavage site. The expression plasmid of pET15b-HisAviTEV-mSTING(138-341) mouse wild type was constructed.

The pCDF_Duet-1 BirA(1-321) expression plasmid was constructed by inserting an artificially synthesized *E. coli* BirA (UniProt entry P06709) cDNA into the pCDF_Duet-1 vector.

(ii) Preparation of STING C-Terminal Binding Domain Protein

Each of the prepared expression plasmid pET15b-HisAviTEV-hSTING(139-342) (human wild-type (Hu-WT), human REF mutant (Hu-REF) or human AQ mutant (Hu-AQ) STING C-terminal binding domain protein), and expression plasmid pET15b-HisAviTEV-mSTING(138-341) (mouse wild type (Ms-WT) STING C-terminal binding domain protein) was co-transformed with pCDF_Duet-1 BirA(1-321) expression plasmid into Competent E.coli Rosetta 2 (DE3) (Merck Millipore, MA, US) to prepare each HisAviTEV-STING expression strain. Each expression strain was added to TB broth containing 100 μg/mL ampicillin, 50 μg/mL streptomycin, and 30 μg/mL kanamycin, and cultured at 37°C. Expression was then induced with 100 μM IPTG, and the cells were further cultured at 16°C.

The culture broth was centrifuged, and the obtained bacteria were frozen and thawed. The proteins were extracted by sonication, and the supernatant was collected by centrifugation. The resulting supernatant was purified through a HisTrap FF column (GE Healthcare) using an AKTAexpress chromatography system (GE Healthcare, IL, US), made to pass through a Superdex200 16/60 column (GE Healthcare), and eluted. Fractions containing proteins with the target molecular weight were collected by SEC as His-Avi-TEV-hSTING(139-342) human wild-type protein, HisAviTEV-hSTING(139-342) human REF mutant protein, HisAviTEV-hSTING(139-342) human AQ mutant protein, or His-Avi-TEV-mSTING(138-341) mouse wild-type protein.

The amino acid sequence of the HisAviTEV-hSTING(139-342) human wild-type protein is set forth in SEQ ID NO: 9, the amino acid sequence of the HisAviTEV-hSTING(139-342) human REF mutant protein is set forth in SEQ ID NO: 10, the amino acid sequence of the HisAviTEV-hSTING(139-342) human AQ mutant protein is set forth in SEQ ID NO: 11, and the amino acid sequence of the His-Avi-TEV-mSTING(138-341) mouse wild-type protein is set forth in SEQ ID NO: 12.

(iii) STING Binding Assay

The binding of a compound to each STING C-terminal binding domain protein was determined by a thermal shift assay, which uses the increase in the thermal denaturation temperature of the protein as an indicator.

Specifically, each test compound (final concentration 0.5 mM), SYPRO Orange (final concentration: 20 × concentration), and the STING protein were mixed in a 384-well real-time PCR plate using a plate shaker. A real-time PCR system was used to increase the temperature from 25°C to 95°C at a rate of 0.03°C per second, and the thermal denaturation temperature of the protein was measured using the fluorescence emitted by SYPRO Orange as an indicator. Tm (the midpoint of the unfolding transition) (°C) was determined as the temperature at which the rate of increase in fluorescence intensity reached its maximum value. The shift of Tm by the test compound was calculated as ΔTm (°C) by subtracting, from the Tm value of each compound, the Tm value of the well without the compound. The results of the binding assay for each STING protein are shown in Table 6.

[Table 5]

| Compound No. | ΔTm(°C) | | | |
|---|---|---|---|---|
| | Hu-WT | Hu-REF | Hu--AQ | Ms-WT |
| 6a | 10.6 | 5. 7 | 14. 8 | 15. 7 |
| 6b | 8.4 | 3. 4 | 11. 3 | 12. 9 |
| 34a | 14.7 | 8. 8 | 18. 9 | 20. 3 |
| 34b | 12.0 | 6. 5 | 14.0 | 16. 7 |
| 39a | 10.4 | 5. 7 | 12.9 | 15. 0 |
| 39b | 8.9 | 3. 7 | 10.5 | 13. 1 |
| 44a | 1.3.4 | 8. 7 | 18. 2 | 19. 3 |
| 44b | 11.4 | 6. 2 | 14. 3 | 17. 0 |
| 48a | 15.9 | 9. 3 | 22. 2 | 16. 0 |
| 48b | 14. 0 | 7. 3 | 17. 8 | 15. 8 |
| 52a | 10.9 | 5. 6 | 13.5 | 14. 3 |
| 52b | 9. 2 | 4. 4 | 10. 8 | 12. 3 |
| 53a | 9. 6 | 5. 4 | 11.6 | 14. 3 |
| 54 | 8. 3 | 3. 4 | 9. 8 | 12.1 |
| ML-RR-CDA·2Na$^+$ | 7. | 2. 7 | 12. 7 | 15. 2 |
| 2' 3'-cGAMP | 0 13.7 | 4. 1 | 24. 3 | 25. 8 |

The results have revealed that each compound used in an antibody-drug conjugate of the present invention has binding activity toward human wild-type STING or mutant STING, and mouse wild-type STING.

(Test Example 3) Anti-Tumor Test (1)

CT26.WT, a mouse colon cancer cell line purchased from American Type Culture Collection, was transduced with the human mouse chimeric EGFR gene with an epitope site for anti-EGFR antibody substituted with a human type to establish CT26.WT-chimeraEGFR cells.

CT26.WT-chimeraEGFR cells were transplanted subcutaneously into the right axillary region of each BALB/c mouse (Day 0), and random grouping was conducted after 7 days. The anti-EGFR antibody-CDN conjugate was administered once into the tail vein at a dose of 1.0 mg/kg on Day 7. The number of mice in each group was 8.

The results are shown in Figure 4. In the graph, the anti-EGFR antibody 1-CDN conjugate 1 has a LALA-DG mutation in the Fc region and the anti-EGFR antibody 3-CDN conjugate 1 has a LALA mutation in the Fc region. In both of the antibody-CDN conjugates, the average number of conjugated drugs is about 4. In the graph, the line (black squares) denotes the average tumor volume of the vehicle group; the line (black triangles) denotes the average tumor volume of the anti-EGFR antibody 3-CDN conjugate 1 administration group; and the line (black circles) denotes the average tumor volume of the anti-EGFR antibody 1-CDN conjugate 1 administration group. The vertical axis represents the tumor volume

(mm$^3$) and the horizontal axis represents the number of days after tumor implantation. Tumor growth progressed in the vehicle group. In contrast, the tumor growth was markedly inhibited in both of the anti-EGFR antibody-CDN conjugate administration groups.

In conclusion, both of the anti-EGFR antibody-CDN conjugates, in which the average number of conjugated drugs is about 4, and have each mutation in the Fc region, have been demonstrated to have a potent anti-tumor effect. Their anti-tumor effects were equivalent.

(Test Example 4) Anti-Tumor Test (2)

CT26.WT-chimeraEGFR cells were transplanted subcutaneously into the right axillary region of each BALB/c mouse (Day 0), and random grouping was conducted after 7 days. The anti-EGFR antibody-CDN conjugate was administered once into the tail vein at a dose of 1.0 mg/kg on Day 7. The number of mice in each group was 6.

The results are shown in Figure 5. In the graph, the anti-EGFR antibody 2-CDN conjugate 1 has a LALA-DG mutation in an Fc region. In this antibody-CDN conjugate, the average number of conjugated drugs is about 4. In the graph, the line (black squares) denotes the average tumor volume of the vehicle group; and the line (black circles) denotes the average tumor volume of the anti-EGFR antibody 2-CDN conjugate 1 administration group. The vertical axis represents the tumor volume (mm$^3$) and the horizontal axis represents the number of days after tumor implantation. Tumor growth progressed in the vehicle group. In contrast, the tumor growth was markedly inhibited in the anti-EGFR antibody-CDN conjugate administration group.

In conclusion, the anti-EGFR antibody-CDN conjugate in which the average number of conjugated drugs is about 4 and the Fc region has a LALA-DG mutation has been demonstrated to have a potent anti-tumor effect.

(Test Example 5) Anti-Tumor Test (3)

CT26.WT-chimeraEGFR cells were transplanted subcutaneously into the right axillary region of each BALB/c mouse (Day 0), and random grouping was conducted after 7 days. The anti-EGFR antibody-CDN conjugate was administered once into the tail vein at a dose of 2.0 mg/kg on Day **7.** The number of mice in each group was 6 or **8.**

The results are shown in Figure **6.** In the graph, the anti-EGFR antibody 1-CDN conjugate 2 has a LALA-DG mutation in the Fc region, and the anti-EGFR antibody 3-CDN conjugate 2 has a LALA mutation in the Fc region. In both of the antibody-CDN conjugates, the average number of conjugated drugs is about 2. In the graph, the line (black squares) denotes the average tumor volume of the vehicle group; the line (black triangles) denotes the average tumor volume of the anti-EGFR antibody 3-CDN conjugate 2 administration group; and the line (black circles) denotes the average tumor volume of the anti-EGFR antibody 1-CDN conjugate 2 administration group. The vertical axis represents the tumor volume (mm$^3$) and the horizontal axis represents the number of days after tumor implantation. Tumor growth progressed in the vehicle group. In contrast, the tumor growth was markedly inhibited in both of the anti-EGFR antibody-CDN conjugate administration groups.

In conclusion, both of the anti-EGFR antibody-CDN conjugates, in which the average number of conjugated drugs is about 2 and have each mutation in the Fc region, have been demonstrated to have a potent anti-tumor effect. Their anti-tumor effects were equivalent.

(Test Example 6) Anti-Tumor Test (4)

CT26.WT-chimeraEGFR cells were transplanted subcutaneously into the right axillary region of each BALB/c mouse (Day 0), and random grouping was conducted after 7 days. The anti-EGFR antibody-CDN conjugate was administered once into the tail vein at a dose of 2.0 mg/kg on Day 7. The number of mice in each group was 6.

The results are shown in Figure 7. In the graph, the anti-EGFR antibody 2-CDN conjugate 2 has a LALA-DG mutation in the Fc region. In this antibody-CDN conjugate, the average number of conjugated drugs is about **2.** In the graph, the line (black squares) denotes the average tumor volume of the vehicle group; and the line (black circles) denotes the average tumor volume of the anti-EGFR antibody 2-CDN conjugate 2 administration group. The vertical axis represents the tumor volume (mm$^3$) and the horizontal axis represents the number of days after tumor implantation. Tumor growth progressed in the vehicle group. In contrast, the tumor growth was markedly inhibited in the anti-EGFR antibody-CDN conjugate administration group.

In conclusion, the anti-EGFR antibody-CDN conjugate, in which the average number of conjugated drugs is about 2 and the Fc region has a LALA-DG mutation, has been demonstrated to have a potent anti-tumor effect.

(Test Example 7) Anti-Tumor Test (5)

PC-9 cells, a human lung cancer cell line having an EGFR exon 19 deletion mutation purchased from European

Collection of Authenticated Cell Cultures, were transplanted subcutaneously into the right axillary region of each BALB/c-nu mouse (Day 0), and random grouping was conducted after 7 days. The anti-EGFR antibody-CDN conjugate was administered once into the tail vein at a dose of 0.5 mg/kg on Day 8. The number of mice in each group was 6.

The results are shown in Figure 8. In the graph, the anti-EGFR antibody 2-CDN conjugate 1 has a LALA-DG mutation in the Fc region. In this antibody-CDN conjugate, the average number of conjugated drugs is about 4. In the graph, the line (black squares) denotes the average tumor volume of the vehicle group; and the line (black circles) denotes the average tumor volume of the anti-EGFR antibody 2-CDN conjugate 1 administration group. The vertical axis represents the tumor volume ($mm^3$) and the horizontal axis represents the number of days after tumor implantation. Tumor growth progressed in the vehicle group. In contrast, the tumor growth was markedly inhibited in the anti-EGFR antibody 2-CDN conjugate 1 administration group.

In conclusion, the anti-EGFR antibody-CDN conjugate, in which the average number of conjugated drugs is about 4 and the Fc region has a LALA-DG mutation, has been demonstrated to have a potent anti-tumor effect on lung cancer tumor having an EGFR exon 19 deletion mutation.

(Test Example 8) Anti-Tumor Test (6)

NCI-H358 cells, a KRAS G12C mutation-positive human lung cancer cell line purchased from American Type Culture Collection, were transplanted subcutaneously into the right axillary region of each BALB/c-nu mouse (Day 0), and random grouping was conducted after 22 days. The anti-EGFR antibody 2-CDN conjugate 1 was administered once into the tail vein at a dose of 0.5 mg/kg on Day 22. The number of mice in each group was **6.**

The results are shown in Figure **9.** In the graph, the anti-EGFR antibody 2-CDN conjugate 1 has a LALA-DG mutation in the Fc region. In this antibody-CDN conjugate, the average number of conjugated drugs is about **4.** In the graph, the line (black squares) denotes the average tumor volume of the vehicle group; and the line (black circles) denotes the average tumor volume of the anti-EGFR antibody 2-CDN conjugate 1 administration group. The vertical axis represents the tumor volume ($mm^3$) and the horizontal axis represents the number of days after tumor implantation. Tumor growth progressed in the vehicle group. In contrast, the tumor growth was markedly inhibited in the anti-EGFR antibody 2-CDN conjugate 1 administration group.

In conclusion, the anti-EGFR antibody-CDN conjugate, in which the average number of conjugated drugs is about 4 and the Fc region has a LALA-DG mutation, has been demonstrated to have a potent anti-tumor effect on a KRAS G12C mutation-positive lung cancer tumor.

(Test Example 9) Anti-Tumor Test (7)

CT26.WT was transduced with the human CDH6 gene to produce CT26.WT-hCDH6 cells.

CT26.WT-hCDH6 cells were transplanted subcutaneously into the right axillary region of each BALB/c mouse (Day 0), and random grouping was conducted after 8 days. The anti-CDH6 antibody 1-CDN conjugate 1 was administered at a dose of 1.2 mg/kg and the anti-CDH6 antibody 1-CDN conjugate 2 was administered once into the tail vein at a dose of 2.4 mg/kg on Day **8.** The number of mice in each group was 8.

The results are shown in Figure 10. In the graph, the anti-CDH6 antibody 1-CDN conjugate 2 is an antibody-CDN conjugate in which the average number of conjugated drugs is about **2.** In the graph, the anti-CDH6 antibody 1-CDN conjugate 1 is an antibody-CDN conjugate in which the average number of conjugated drugs is about **4.** Both the antibody-CDN conjugates have a LALA-DG mutation in the Fc region. In the graph, the line (black squares) denotes the average tumor volume of the vehicle group; the line (white circles) denotes the average tumor volume of the anti-CDH6 antibody 1-CDN conjugate 1 administration group; and the line (inverted white triangles) denotes the average tumor volume of the anti-CDH6 antibody 1-CDN conjugate 2 administration group. The vertical axis represents the tumor volume ($mm^3$) and the horizontal axis represents the number of days after tumor implantation. Tumor growth progressed in the vehicle group. In contrast, the tumor growth was markedly inhibited in both the anti-CDH6 antibody 1-CDN conjugate 1 and the anti-CDH6 antibody 1-CDN conjugate 2 administration groups.

In conclusion, the anti-CDH6 antibody-CDN conjugates, in which the average number of conjugated drugs is about 4 or about 2, and the Fc region has a LALA-DG mutation, have been demonstrated to have a potent anti-tumor effect.

(Test Example 10) Evaluation of Activity of Antibody-CDN Conjugate Using Reporter Cell - (1)

The evaluation was carried out using HCC827 cells, a human lung cancer cell line purchased from American Type Culture Collection, and THP1-Dual(TM) cells (InvivoGen, CA, US).

The assay was conducted as follows. HCC827 cells were seeded onto a 96-well plate and cultured for 24 h under conditions at 37°C and 5% $CO_2$. Then, THP1-Dual reporter cells and each test compound were added thereto to start stimulation. After 48 h, the plate was centrifuged to collect the supernatant. Then, the luminescence was measured using

QUANTI-Luc (InvivoGen).

The results are shown in Figure 11. In the graph, the line (black triangles) denotes the average count value of the anti-EGFR antibody 3-CDN conjugate addition group; and the line (black circles) denotes the average count value of the anti-EGFR antibody 1-CDN conjugate addition group. The broken line denotes the CDN conjugate 2 with an average drug conjugation number of about 2; and the solid line denotes the CDN conjugate 1 with an average drug conjugation number of about 4. The vertical axis represents the count value and the horizontal axis represents the concentration. All the anti-EGFR antibody-CDN conjugate addition groups markedly induced reporter activity.

In conclusion, all of the anti-EGFR antibody-CDN conjugates having a LALA-DG mutation in the Fc region have been demonstrated to have reporter activity.

(Test Example 11) Evaluation of Activity of Antibody-CDN Conjugate Using Reporter Cell - (2)

The evaluation was carried out using OVCAR4 cells, a human ovarian cancer cell line purchased from National Cancer Institute, and THP1-Dual(TM) cells (InvivoGen, CA).

The assay was conducted as follows. OVCAR4 cells were seeded onto a 96-well plate and cultured for 24 h under conditions at 37°C and 5% $CO_2$. Then, THP1-Dual reporter cells and each test compound were added thereto to start stimulation. After 48 h, the plate was centrifuged to collect the supernatant. Then, the luminescence was measured using QUANTI-Luc (InvivoGen).

The results are shown in Figure 12. In the graph, the line (black triangles) denotes the anti-CDH6 antibody 1-CDN conjugate addition group. The solid line denotes the average count value of the CDN conjugate 1 with an average drug conjugation number of about 4; and the broken line denotes the average count value of the CDN conjugate 2 with an average drug conjugation number of about 2. The vertical axis represents the count value and the horizontal axis represents the concentration. All of the anti-CDH6 antibody-CDN conjugate addition groups markedly induced reporter activity.

In conclusion, all of the anti-CDH6 antibody-CDN conjugates having a LALA-DG mutation in an Fc region have been demonstrated to have reporter activity.

(Test Example 12) Tumor Rechallenge Test (1)

Substantially the same anti-tumor test as in (Test Example 3) Anti-Tumor Test (1) was conducted. The anti-EGFR antibody 1-CDN conjugate 1 was administered once into the tail vein at a dose of 1.0 mg/kg on Day 7. On Day 80, CT26.WT cells were transplanted subcutaneously into the left axillary region of each BALB/c mouse which showed complete tumor regression.

The results of Day 99 are shown in Figure 13. The vertical axis represents the tumor volume ($mm^3$). Each dot denotes the tumor volume of each individual and the horizontal line in the graph represents the median value. Tumor growth progressed in the age-matched untreated control group. In contrast, the tumor growth was markedly inhibited in the anti-EGFR antibody 1-CDN conjugate 1 administration group.

In conclusion, the anti-EGFR antibody 1-CDN conjugate 1, in which the average number of conjugated drugs is about 4 and the Fc region has a LALA-DG mutation, has been demonstrated to establish long-term anti-tumor immunity.

(Test Example 13) Tumor Rechallenge Test (2)

Substantially the same anti-tumor test as in (Test Example 5) Anti-Tumor Test (3) was conducted. The anti-EGFR antibody 1-CDN conjugate 2 was administered once into the tail vein at a dose of 1.0 mg/kg or 2.0 mg/kg on Day 7. On day 80, CT26.WT cells were transplanted subcutaneously into the left axillary region of each BALB/c mouse which showed complete tumor regression.

The results of Day 100 are shown in Figure 14. The vertical axis represents the tumor volume ($mm^3$). Each dot denotes the tumor volume of each individual and the horizontal line in the graph represents the median value. Tumor growth progressed in the age-matched control group. In contrast, the tumor growth was markedly inhibited in the anti-EGFR antibody 1-CDN conjugate 2 administration group.

In conclusion, the anti-EGFR antibody 1-CDN conjugate 2, in which the average number of conjugated drugs is about 2 and the Fc has a LALA-DG mutation, has been demonstrated to establish long-term anti-tumor immunity.

(Test Example 14) Cytokine Production Test Using Human Whole Blood

Fresh human peripheral blood was collected and added to a 96-well plate. Then, each test compound was added at a concentration of 10 μg/mL when the compound had an average drug conjugation number of about 4 and at a concentration of 20 μg/mL when the compound had an average drug conjugation number of about 2. The blood was

incubated for 24 h under conditions at 37°C and 5% $CO_2$. After centrifugation, plasma was collected and its IL-6 concentration was measured using AlphaLISA (PerkinElmer).

Figure 15A shows the results of calculating numeric values using the standard line of Log Scale (Logarithmic Scale), and Figure 15B shows the results of calculating numeric values using the standard line of Linear Scale. The numeric values exhibit a similar tendency between the Logarithmic Scale and the Linear Scale. The numeric values calculated at the Linear Scale indicate more accurate concentrations. The vertical axis represents the IL-6 concentration (pg/mL). The horizontal axis represents the mutation in the Fc region of each antibody-CDN conjugate. WT indicates that the Fc region has no mutation. The anti-EGFR antibody B-CDN conjugate is an antibody-CDN conjugate obtained using an IgG2 antibody, and the other antibody-CDN conjugates were obtained using an IgG1 antibody. Cytokine production was markedly inhibited in the antibody 1-CDN conjugate addition group having an IgG1 antibody and a LALA-DG mutation compared to the antibody-CDN conjugate addition groups having an IgG2 or IgG1 antibody and no mutation (WT) or a LALA mutation in the Fc region. This result was substantially the same between the anti-EGFR antibody 1-CDN conjugate with an average drug conjugation number of about 2 and the anti-EGFR antibody 1-CDN conjugate with an average drug conjugation number of about 4. As for the anti-CDH6 antibody 1-CDN conjugate, cytokine production was also markedly inhibited in the antibody-CDN conjugate addition group with an average drug conjugation number of about 2 or about 4 having an IgG1 antibody and a LALA-DG mutation.

In conclusion, use of an IgG1 antibody and a LALA-DG mutation has been demonstrated to be excellent for the purpose of inhibiting cytokine production when each antibody-CDN conjugate is administered. The cytokine production in this test correlated with FcγR-binding activity shown in Figure 17.

On the other hand, in the case of testing Naked IgG1 antibody (without a CDN conjugate) having a mutation in the Fc region in substantially the same manner, no clear difference was found in cytokine production (data not shown). Accordingly, it has been revealed that the conjugation of the antibody permits sensitive evaluation of the FcγR-binding activity of the antibody. A safe antibody-drug conjugate having decreased antibody effector functions has been successfully obtained using this evaluation system.

(Test Example 15) Evaluation of Human FcγRI Binding Activity of Anti-EGFR Antibody with Introduced Effector-Less Mutation

Anti-EGFR antibodies with various introduced effector-less mutations were evaluated for their binding activity against human FcγRI using Biacore T200 (Cytiva). The evaluation employed anti-EGFR antibody (anti-EGFR antibody B) which is an IgG2-type antibody, an Fc substitution variant thereof (IgG1 WT) (anti-EGFR antibody A) obtained by replacing the Fc region with an IgG1 type, a mutant (modified anti-EGFR antibody 3) obtained by introducing a L234A/L235A (LALA) mutation into the Fc region of IgG1 **WT,** and a mutant (modified anti-EGFR antibody 1) obtained by introducing a L234A/L235A/D265G (LALA-DG) mutation thereinto. The anti-EGFR antibody B used in the Test Example was obtained as HBS-EP+ solution by subjecting commercially available Vectibix Intravenous Infusion (Takeda Pharmaceuticals) (100 mg/5 mL) to buffer exchange using Amicon Ultra-15 Centrifugal Filter Unit (50K, produced by Merck). The binding activity was determined by allowing His-tagged human FcγRI to be captured as a ligand onto an anti-His antibody-immobilized sensor chip, then adding each anti-EGFR antibody as an analyte to the sensor chip, and measuring the interaction between the ligand and the analyte by Surface Plasmon Resonance (SPR). Anti-6X His tag antibody [AD1.1.10] (abcam) was immobilized onto Sensor Chip CM5 (Cytiva) according to the instructions of the kit. Recombinant Human Fc gamma RI/CD64 Protein (R&D systems) prepared at 10 μg/mL with HBS-EP+ (GE Healthcare) was contacted with the sensor chip for 60 sec at 10 μL/min and thereby captured. Then, the anti-EGFR antibody prepared at each concentration with HBS-EP+ was added to the sensor chip for 120 sec at a flow rate of 30 μL/min. Figure 16A shows a graph of the value of Resonance Unit (RU) plotted at the antibody concentration (0.3, 0.4, 0.6, 0.9, 1.3, 2.0, 3.1, 4.7 μM). The graph of Figure 17A shows the RU value obtained at the antibody concentration of 4.7 μM.

The RU value indicating binding to human FcγRI was IgG1 WT >> LALA >> IgG2 > LALA-DG.

(Test Example 16) Evaluation of Human FcγRIIa Binding Activity of Anti-EGFR Antibody with Introduced Effector-Less Mutation

The anti-EGFR antibodies described in Test Example 15 were evaluated for their binding activity against human FcγRIIa using Biacore T200. The binding activity was determined by directly immobilizing human FcγR as a ligand onto a sensor chip, then adding each anti-EGFR antibody as an analyte to the sensor chip, and measuring the interaction between the ligand and the analyte by SPR. Recombinant Human Fc gammaRIIA/CD32a (R167) Protein (R&D systems) was immobilized as human FcγRIIa onto Sensor Chip CM5 according to the instructions of the kit. Then, the anti-EGFR antibody prepared at each concentration with HBS-EP+ was added to the sensor chip for 120 sec at a flow rate of 30 μL/min. Figure 16B shows a graph of the RU value plotted at each antibody concentration (0.9, 1.3, 2.0, 3.1, 4.7 μM) when human FcγRIIa was used as a ligand. The graph of Figure 17B shows the RU value obtained at the antibody concentration

of 4.7 μM.

The RU value indicating binding to human FcγRIIa was IgG1 WT > IgG2 >> LALA > LALA-DG.

(Test Example 17) Evaluation of Human FcγRIIb/c Binding Activity of Anti-EGFR Antibody with Introduced Effector-Less Mutation

In substantially the same manner as in Test Example 16, the anti-EGFR antibodies described in Test Example 15 were evaluated for their binding activity against human FcγRIIb/c. Recombinant Human Fc gamma RIIB/C (CD32b/c) Protein (R&D systems) was used as human FcγRIIb/c. Figure 16C shows a graph of the RU value plotted at each antibody concentration (0.9, 1.3, 2.0, 3.1, 4.7 μM) when human FcγRIIb/c was used as a ligand. The graph of Figure 17C shows the RU value obtained at the antibody concentration of 4.7 μM.

The RU value indicating binding to human FcγRIIb/c was IgG1 WT > IgG2 > LALA > LALA-DG.

(Test Example 18) Evaluation of Human FcγRIIIa Binding Activity of Anti-EGFR Antibody with Introduced Effector-Less Mutation

In substantially the same manner as in Test Example 16, the anti-EGFR antibodies described in Test Example 15 were evaluated for their binding activity against human FcγRIIIa. Recombinant Human Fc gamma RIIIA/CD16a protein (R&D systems) was used as human FcγRIIIa. Figure 16D shows a graph of the RU value plotted at each antibody concentration (0.9, 1.3, 2.0, 3.1, 4.7 μM) when human FcγRIIIa was used as a ligand. The graph of Figure 17D shows the RU value obtained at the antibody concentration of 4.7 μM.

The RU value indicating binding to human FcγRIIIa was IgG1 WT > LALA > IgG2 > LALA-DG.

(Test Example 19) Evaluation of Human FcγRIIIb Binding Activity of Anti-EGFR Antibody with Introduced Effector-Less Mutation

In substantially the same manner as in Test Example 16, the anti-EGFR antibodies described in Test Example 15 were evaluated for their binding activity against human FcγRIIIb. Recombinant Human Fc gamma RIIIB/CD16b Protein (R&D systems) was used as human FcγRIIIb. Figure 16E shows a graph of the RU value plotted at each antibody concentration (0.9, 1.3, 2.0, 3.1, 4.7 μM) when human FcγRIIIb was used as a ligand. The graph of Figure 17E shows the RU value obtained at the antibody concentration of 4.7 μM.

The RU value indicating binding to human FcγRIIIb was IgG1 WT > LALA ≈ IgG2 ≈ LALA-DG.

(Test Example 20) Evaluation of Each Human FcγR Binding Activity of Conjugate of Anti-EGFR Antibody with Introduced Effector-Less Mutation and CDN

In substantially the same manner as in Test Examples 15 to 19, conjugates of the anti-EGFR antibodies described in Test Example 15 and CDN (DAR2: anti-EGFR antibody B-CDN conjugate 2, anti-EGFR antibody 3-CDN conjugate 2, anti-EGFR antibody 1-CDN conjugate 2, or DAR4: anti-EGFR antibody A-CDN conjugate 1, anti-EGFR antibody 3-CDN conjugate 1, anti-EGFR antibody 1-CDN conjugate 1) were evaluated for their binding activity against each human FcγR. The graphs of Figs. 17A to 17E show the RU value obtained at the antibody concentration of 4.7 μM when each human FcγR was used as a ligand.

Neither CDN conjugation nor an increase in DAR influenced the rank order of the amplitude of the RU value among the effector-less mutants. This suggested that the human FcgR-binding activity of the antibody-CDN conjugate basically depends on the human FcgR-binding activity of the parent antibody.

The antibody-drug conjugate comprising the antibody IgG1 WT or the antibody having a LALA mutation in the Fc region tended to have higher FcγRI-binding activity than that of the parent antibody and the FcγRI-binding activity tended to be further increased with an increase in DAR. On the other hand, the antibody-drug conjugate having a LALA-DG mutation in the Fc region maintained low FcγRI-binding activity similar to that of the parent antibody (Figure 17A). In conclusion, it is suggested that a LALA-DG mutant is preferable for the purpose of obtaining an antibody-drug conjugate having decreased antibody effector functions to the same level as that of a parent antibody.

(Test Example 21) Evaluation of Thermal Stability of Conjugate of Anti-EGFR Antibody with Introduced Effector-Less Technique and CDN

Conjugates of an anti-EGFR antibody and a CDN (anti-EGFR antibody A-CDN conjugate 1, anti-EGFR antibody 3-CDN conjugate 1, anti-EGFR antibody 1-CDN conjugate 1) were evaluated for their thermal stability using MicroCal VP-Capillary DSC System (Malvern). Each anti-EGFR antibody-CDN conjugate 1, prepared at an antibody concentration of

0.5 mg/mL using 25 mM Histidine, 5 w/v% Sorbitol (pH 5.5), was examined for a change in heat capacity $C_p$ (kcal/mol/°C) when the temperature was increased from 20°C to 120°C at a heating rate of 60°C/hr. A peak in the curve that shows such change in heat capacity indicates the thermal denaturation of each antibody domain, and thermal denaturation peaks are generally observed in the order of CH2, Fab, and CH3 (Biochem. Biophys. Res. Commun. 355, 751-757 (2007)). The table of Figure 18 shows the value of peak temperature ($T_m$) of CH2 in these antibodies and the amount of change in $T_m$ value ($\Delta T_m$) from the anti-EGFR antibody A.

The anti-EGFR antibody A-CDN conjugate 1 and the anti-EGFR antibody 3-CDN conjugate 1 had the same level of thermal stability. On the other hand, the thermal stability of the anti-EGFR antibody 1-CDN conjugate 1 exhibited a $T_m$ value higher by 4°C or more than that of the other antibodies.

(Test Example 22) Tumor Rechallenge Test (3)

Substantially the same anti-tumor test as in (Test Example 9) Anti-Tumor Test (7) was conducted. The anti-CDH6 antibody 1-CDN conjugate 1 and the anti-CDH6 antibody 1-CDN conjugate 2 were administered once into the tail vein at a dose of 1.2 mg/kg and 2.4 mg/kg, respectively, on Day 7. CT26.WT-hCDH6 cells were transplanted subcutaneously into the left axillary region of each mouse having complete tumor regression among these groups on Day 84. Age-matched untreated BALB/c mice were used as the control group. The results of Day 98 for the anti-CDH6 antibody 1-CDN conjugate 1 and the anti-CDH6 antibody 1-CDN conjugate 2 are shown in Figure 24. The vertical axis represents the tumor volume ($mm^3$). Each dot denotes the tumor volume of each individual and the horizontal line in the graph represents the median value. Tumor growth progressed in the control group. In contrast, the tumor growth was markedly inhibited in the anti-CDH6 antibody 1-CDN conjugate 1 and the anti-CDH6 antibody 1-CDN conjugate 2 administration groups.

In conclusion, both of the anti-CDH6 antibody-CDN conjugates, in which the average number of conjugated drugs is about 2 or 4, having mutations in the Fc region, have been demonstrated to establish long-term anti-tumor immunity.

(Test Example 23) Anti-Tumor Test (8)

A498 cells, a human kidney cancer cell line purchased from ATCC, were transplanted subcutaneously into the right axillary region of each BALB/c-nu mouse (Day 0), and random grouping was conducted after 20 days. Each anti-CDH6 antibody-CDN conjugate was administered once into the tail vein at a dose of 0.3 mg/kg or 0.6 mg/kg, when the average number of conjugated drugs was 4 or 2, respectively. The number of mice in each group was 6.

The results are shown in Figure 25. Both of the antibodies in the graph have a LALA-DG mutation in the Fc region In the anti-CDH6 antibody 1-CDN conjugate 1 and the anti-CDH6 antibody 1-CDN conjugate 2, the average number of conjugated drugs is 4 and 2, respectively. In the graph, the line (black squares) denotes the vehicle group; the line (black circles) denotes the anti-CDH6 antibody 1-CDN conjugate 1 administration group; and the line (black triangles) denotes the anti-CDH6 antibody 1-CDN conjugate 2 administration group. The vertical axis represents the tumor volume ($mm^3$) and the horizontal axis represents the number of days after tumor implantation. Tumor growth progressed in the vehicle group. In contrast, the tumor growth was markedly inhibited in both the anti-CDH6 antibody 1-CDN conjugate administration groups.

In conclusion, both of the anti-CDH6 antibody-CDN conjugates, in which the average number of conjugated drugs is about 2 or 4, and having mutations in the Fc region, have been demonstrated to have a potent anti-tumor effect.

(Test Example 24) Anti-Tumor Test (9)

786-O cells, a human kidney cancer cell line purchased from ATCC, were transplanted subcutaneously into the right axillary region of each BALB/c-nu mouse (Day 0), and random grouping was conducted after 28 days. Each anti-CDH6 antibody-CDN conjugate was administered once into the tail vein at a dose of 0.1 mg/kg, irrespective of the average number of conjugated drugs . The number of mice in each group was **6.**

The results are shown in Figs. 26 and 27. Both of the antibodies in the graphs have a LALA-DG mutation in the Fc region. In anti-CDH6 antibody 1-CDN conjugate 1 and anti-CDH6 antibody 1-CDN conjugate 2, the average number of conjugated drugs is 4 and 2, respectively. In the graphs, the line (black squares) denotes the vehicle group; the line (black circles) denotes the anti-CDH6 antibody 1-CDN conjugate 1 administration group; and the line (black triangles) denotes the anti-CDH6 antibody 1-CDN conjugate 2 administration group. The vertical axis represents the tumor volume ($mm^3$) and the horizontal axis represents the number of days after tumor implantation. Tumor growth progressed in the vehicle group. In contrast, the tumor growth was markedly inhibited in both of the anti-CDH6 antibody 1-CDN conjugate administration groups.

In conclusion, both of the anti-CDH6 antibody-CDN conjugates, in which the average number of drugs conjugated is about 2 or 4, and having mutations in the Fc region, have been demonstrated to have a potent anti-tumor

effect.

(Test Example 25) Anti-Tumor Test (10)

OV-90 cells, a human ovarian cancer cell line purchased from ATCC, were transplanted subcutaneously into the right axillary region of each BALB/c-nu mouse (Day 0), and random grouping was conducted after 7 or 15 days. Anti-CDH6 antibody 1-CDN conjugate 1, in which the average number of conjugated drug is about 4, was administered at a dose of 0.3 mg/kg, and anti-CDH6 antibody 1-CDN conjugate 2, in which the average number of conjugated drug is about 2, was administered at a dose of 0.6 mg/kg, once into the tail vein. The number of mice in each group was 6.

The results are shown in Figs. 28 and 29. Both of the antibodies in the graphs have a LALA-DG mutation in the Fc region. In anti-CDH6 antibody 1-CDN conjugate 1 and anti-CDH6 antibody 1-CDN conjugate 2, the average number of conjugated drugs is 4 and 2, respectively. In the graphs, the line (black squares) denotes the vehicle group; the line (black circles) denotes the anti-CDH6 antibody 1-CDN conjugate 1 administration group; and the line (black triangles) denotes the anti-CDH6 antibody 1-CDN conjugate 2 administration group. The vertical axis represents the tumor volume ($mm^3$) and the horizontal axis represents the number of days after tumor implantation. Tumor growth progressed in the vehicle group. In contrast, the tumor growth was markedly inhibited in both of the anti-CDH6 antibody 1-CDN conjugate administration groups.

In conclusion, both of the anti-CDH6 antibody-CDN conjugates in which the average number of drugs conjugated is about 2 or 4, having mutations in the Fc region, have been demonstrated to have a potent anti-tumor effect.

Example 15: Method for Producing Modified Anti-EGFR Antibody 2-[SG-$(N_3)_2$]$_2$ or Modified Anti-CDH6 Antibody 1-[SG-$(N_3)_2$]$_2$ by One-Pot Method

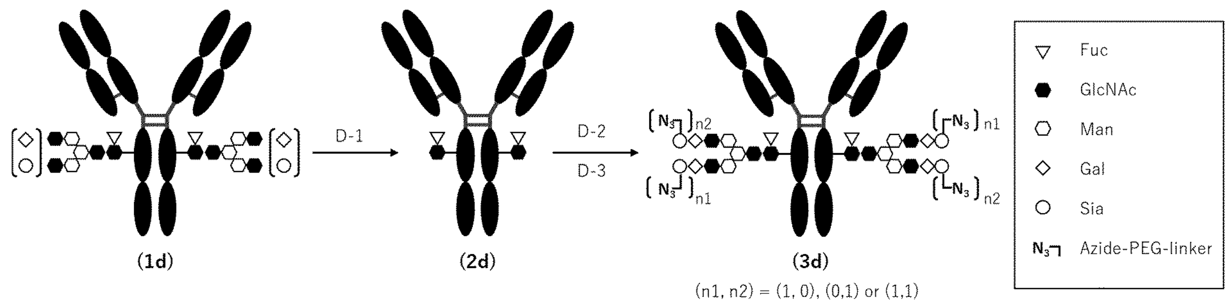


A "one-pot method" (Patent Literature: WO2018003983, WO2022050300, Non-Patent Literature: PLoS ONE 2018,13, e0193534), which is characterized by concurrently using two types of Endo enzymes to directly subject an N297 glycan of an antibody to transglycosylation with a glycan donor with an unactivated reducing terminal, was carried out. This method may be adopted as (Step D-3) of "Method D: Production of Glycan-Remodeled Antibody". Specifically, the target product (3d) can be prepared by providing a reaction mixture containing (2d) to (3d) using the one-pot method, followed by purification by a suitable method.

In general, the one-pot method can employ given enzymes as described in the above literature and the glycan donor molecule in an appropriate combination. In the following Examples, each enzyme was selected as one representative example from among the enzymes described in the above literature. (Fucα1,6)GlcNAc-modified anti-EGFR antibody 2 and (Fucα1,6)GlcNAc-modified anti-CDH6 antibody 1 were selected as representative examples of the acceptor molecule. A glycan donor molecule (G-10), which is not described in the above literature, was selected as the glycan donor molecule (Table 7).

[Table 6]

Table 7 Enzyme solution, acceptor solution, and glycan donor molecule used in one-pot reaction

| **Enzyme** | **Amino acid sequence** | **Preparation example** |
|---|---|---|
| E n do - S i enzyme (D241M/ Q311L) | | Endo-Si enzyme is produced with reference to the method described in WO 2022/050300 to prepare a solution (PBS(-) (FUJIFILM Wako Pure Chemical Corp.)) with an enzyme concentration of 3 mg/mL to 10 mg/mL. |
| Endo - R p enzyme (N 1 7 2 H) | | Endo-Rp enzyme is produced with reference to the method described in WO 2018/101454 to prepare a solution (50 mM Tris, 150 mM NaCl, pH 8.0) with an enzyme concentration of 3 mg/mL to 6 mg/mL. |
| **Acceptor molecule** | **Amino acid sequence** | **Preparation example** |
| (Fucα1,6)GlcNAc -modified anti-EGFR antibody 2 | SEQ ID NO:30(light chain) SEQ ID NO:33 (heavy chain) | A solution that can be produced according to the method of step 1 of Example 3 is subjected to buffer exchange using an ultrafiltration membrane to prepare 50 mM Tris-HCl buffer (pH 7.25) solution with an antibody concentration of 90 mg/mL or higher. |
| (Fucα1,6)GlcNAc -modified anti-CDH6 antibody 1 | SEQ ID NO:4 4 (light chain) SEQ ID NO:4 6 (heavy chain) | A solution that can be produced according to the method of step 1 of Example 5 is subjected to buffer exchange using an ultrafiltration membrane to prepare 50 mM Tris-HCl buffer (pH 7.25) solution with an antibody concentration of 90 mg/mL or higher. |
| **Glycan donor molecule** | **Glycan structure** | **Exemplary synthesis method** |
| G-10 | O-linked glycan | Described in Example 15-1 |

Example 15-1: Synthesis of Glycan Donor Molecule (G-10)

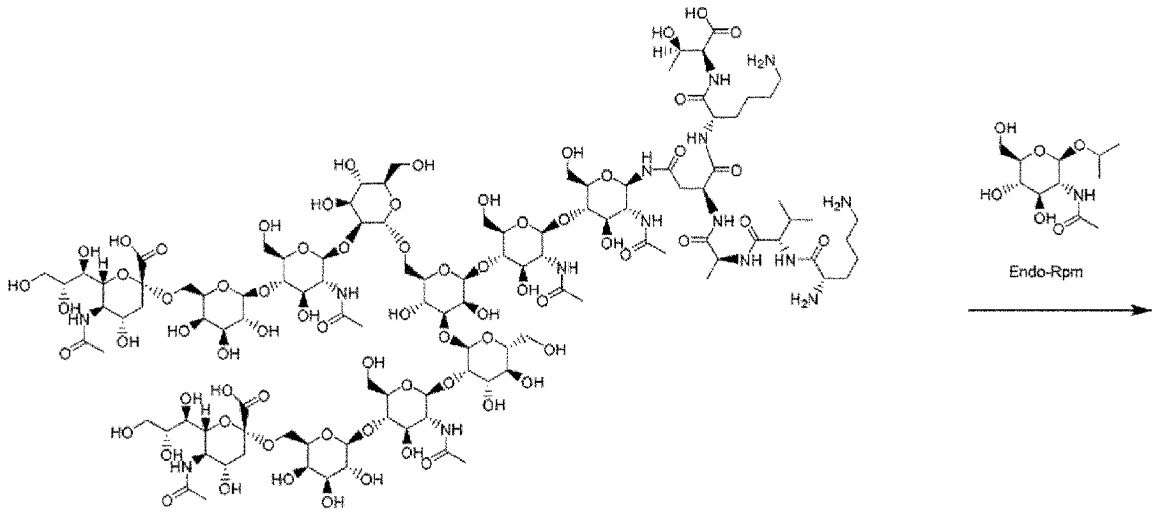


SGP

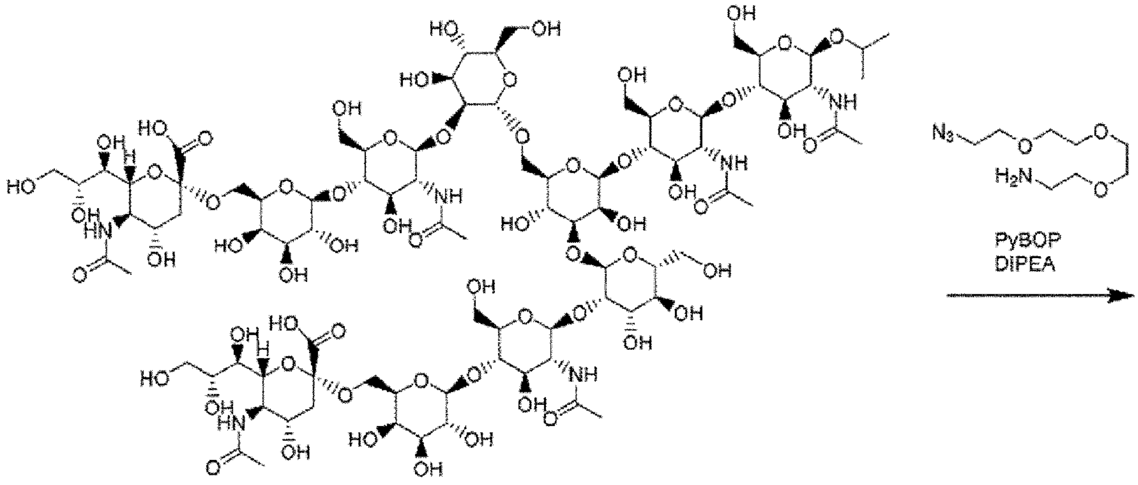


4

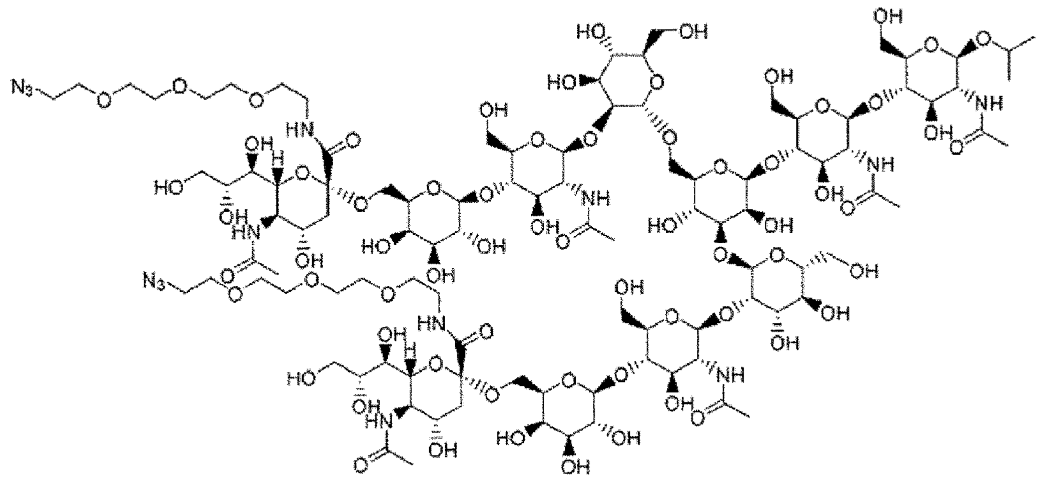

G-10

(15-1a) [Glycan exchange reaction]

SGP (20.0 g) and 1-Methylethyl 2-(acetylamino)-2-deoxy-β-D-glucopyranoside (36.8 g, 20 equiv) were dissolved in a phosphate buffer solution (280 mL, pH 5.5, 0.2 mol/L) and then warmed to 50°C. The resulting mixture was mixed with Endo-Rp N172H (9.1 mg), and the mixture was stirred for 72 h with the temperature unchanged.

[Purification]

After completion of the reaction, the reaction mixture was filtered at room temperature. A portion of the resulting filtrate was purified by Sartocon Hydrosart-2kDa (Sartorius) and subsequently Amberlite(R) IR120 H form (Merck), and then freeze-dried to give solid product 4 (3.72 g from SGP 5.6 g).

(15-1b) [Condensation reaction]

Solid product 4 (3.0 g) obtained in step (15-1a) was dissolved using DMF (59.1 mL) and purified water (0.9 mL) and then mixed with N,N-diisopropylethylamine (1.03 g, 7.94 mmol) and 11-azido-3,6,9-trioxaundecan-1-amine (867 mg, 3.97 mmol). 1H-benzotriazol-1-yloxytripyrrolidinophosphonium hexafluorophosphate (6.20 g, 11.9 mmol) was added in three divided portions at 20°C to the resulting solution, and the mixture was then stirred for 23 h with the temperature unchanged.

[Purification]

After reaction, the reaction mixture was diluted with purified water, then purified by Sartocon Hydrosart-2kDa (Sartorius), and concentrated. The resulting concentrate was subjected to separation and purification using acetonitrile and water as the eluent and using YMC-Actus Triart Prep C18-S (produced by YMC Co., Ltd.) as the column. A main peak detected by UV detection (210 nm) was collected and concentrated under reduced pressure to give the target compound G-10 (2.77 g). ESI-MS: Calcd for C103H176N14066: $[M+2H]^{2+}$ 1333.5496 (most abundant mass), Found 1333.5450

Example 15-2: Preparation of Reaction Mixture Containing Modified Anti-EGFR Antibody 2-[SG-$(N_3)_2$]$_2$ or Modified Anti-CDH6 Antibody 1-[SG-$(N_3)_2$]$_2$

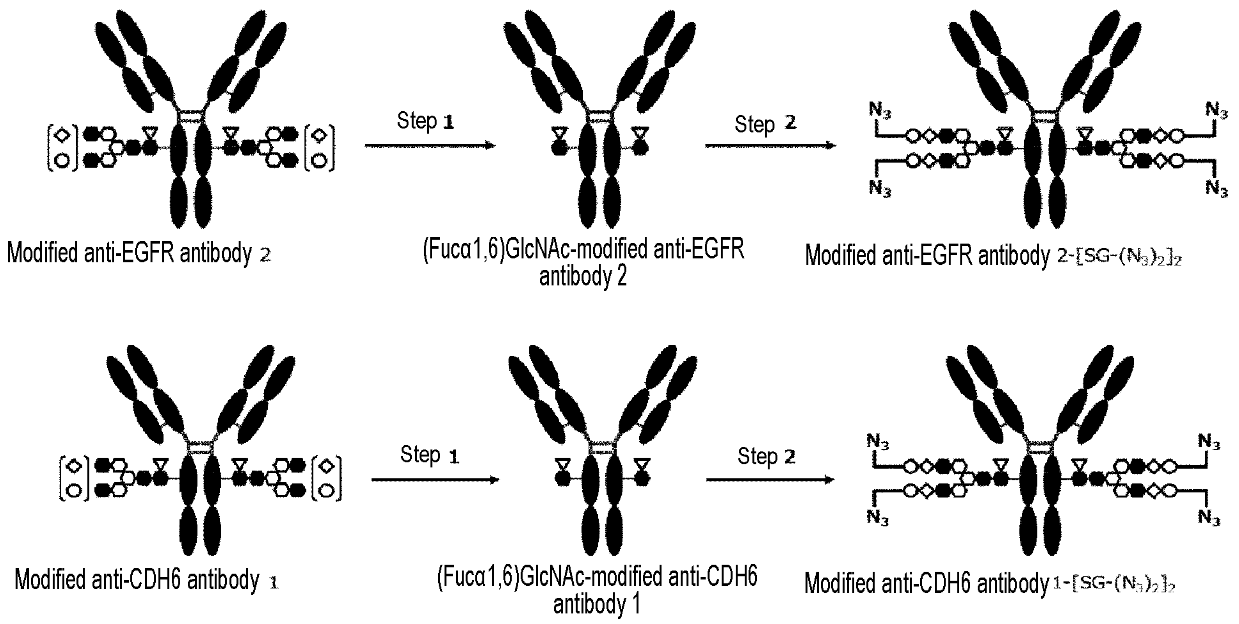


(15-2a) [Transglycosylation Reaction]

The transglycosylation reaction was carried out by each of the protocols given below. In the case of adding an additive-containing solution, it should be noted that the concentration of the additive was set to not cause precipitation of an antibody or an enzyme by the addition of the additive; an aqueous solution of the additive prepared beforehand so as not to change the pH of a buffer solution was added.

[Transglycosylation Reaction Standard Protocol 1]

(Fucα1,6)GlcNAc-modified anti-EGFR antibody 2 (3 mg), glycan donor molecule G-10 (20 equivalents), Endo-Si

mutant (hereinafter, also referred to as "Endo-Sim"; 1.6% w/w), Endo-Rp mutant (hereinafter, also referred to as "Endo-Rpm"; 0.2% w/w), and an appropriate amount of 50 mM Tris-HCl buffer (pH 7.25) were mixed. If necessary, an appropriate amount of an additive-containing solution with the content shown in Table 8 was added thereto to bring the total liquid volume to 50 μL, followed by reaction at 30°C for 28 h.

Sampling was performed 16 h, 18 h, 20 h, 22 h, and 24 h after the start of the reaction. For the sampling, a portion of the reaction mixture was extracted, diluted with purified water so as to obtain a concentration of the antibody contained therein of 1 mg/mL, and cryopreserved until the start of LabChip analysis.

[Transglycosylation Reaction Standard Protocol 2]

(Fucα1,6)GlcNAc-modified anti-CDH6 antibody 1 (15 mg), glycan donor molecule G-10 (25 equivalents), Endo-Sim (1.6% w/w), Endo-Rpm (0.2% w/w), and an appropriate amount of 50 mM Tris-HCl buffer (pH 7.25) were mixed. If necessary, an appropriate amount of an additive-containing solution with the content shown in Table 9 was added thereto to bring the total liquid volume to 300 μL, followed by reaction at 30°C for 20 h.

Sampling was performed 16 h, 18 h, and 20 h after the start of the reaction. For the sampling, a portion of the reaction mixture was extracted and cryopreserved until start of LabChip analysis.

(15-2b) [Measurement of Rate of Transglycosylation Using LabChip Analysis]

The progress of the transglycosylation reaction was checked using gel electrophoresis of protein (WO2013/120066, Huang W, et al., J Am Chem Soc. 2012, 134, 12308-12318). The fully automated protein electrophoresis system used LabChip GX II (produced by PerkinElmer) as the apparatus and Protein Express Assay LabChip and Protein Express Reagent Kit (produced by PerkinElmer) as reagents.

When the sampled solution is analyzed by LabChip according to the above [Transglycosylation Reaction Standard Protocol], an unreacted material and a transglycosylation product are confirmed as separate peaks from the electropherogram. The rate of transglycosylation was calculated from the peak area ratio between the unreacted material and the transglycosylation product according to the following expression.

Rate of transglycosylation (%) = [[H chain peak area derived from the transglycosylated antibody] / {[H chain peak area derived from the unreacted material] + [H chain peak area derived from the transglycosylated antibody]}] × 100

The rate of transglycosylation at each reaction time was calculated under the respective reaction conditions used for (Fucα1,6)GlcNAc-modified anti-EGFR antibody 2 or (Fucα1,6)GlcNAc-modified anti-CDH6 antibody 1 (Table 8).

[Table 7]

Table 8

| Example/ Reference Example | Antibody | Additive (concentration within system) | Rate of transglycosylation (%) | | | | |
|---|---|---|---|---|---|---|---|
| | | | 16h | 18h | 20h | 22h | 24h |
| 15-2-1 | (Fucα1,6) GlcNAc-modified anti-EGFR antibody 2 | - | 75. 6 | 76.6 | 77.0 | 77.5 | 77.6 |
| 15-2-2 | | NaCl(0. 5M) | 83.2 | 85. 2 | 86. 8 | 86.9 | 83. 8 |
| 15-2-3 | | $CH_3COOK$(0. 5M) | 85.1 | 84.3 | 82.9 | 82.8 | 82.2 |
| 15 2 4 | | $CH_3COONH_4$(0. 5M) | 81.0 | 83.1 | 84. 1 | 81. 4 | 85.1 |
| 10-1 | | $CaCl_2/2H_2O$(0.04M) | 31.0 | 34. 3 | 38.0 | 41. 6 | 45.1 |
| 15-2-5 | | LiCl(0. 1M) | 78.6 | 79.9 | 80. 2 | 81.0 | 80.9 |
| 15-2-6 | | $CH_3COONa$ (0. 04M) | 77.4 | 79.0 | 79. 5 | 80. 4 | 79.5 |
| 10-2 | | $MgCl_2/6H_2O$(0.04M) | 62. 6 | 67. 1 | 70. 6 | 74. 3 | 76.6 |
| 10-3 | | $MgSO_4$(0. 04M) | 63.5 | 69. 0 | 71. 2 | 73.5 | 75. 7 |
| 10-4 | | $MnSO_4/5H_2O$(0.04M) | 20.6 | 22. 8 | 24.6 | 31.2 | 30. 4 |
| 15-2-7 | | $NH_4SO_4$(0. 04M) | 75. 1 | 77.3 | 77. 9 | 73.4 | 77. 5 |
| 10-5 | | Glucose (0. 04M) | 32. 7 | 33. 4 | 33. 9 | 34.3 | 34. 5 |
| 15-2-8 | | Sorbitol(0. 04M) | 76.0 | 77. 2 | 78. 5 | 78.2 | 79.4 |
| 15-2-9 | | Trehalose/$2H_2O$(0.04M) | 75. 9 | 77.2 | 78. 8 | 78.6 | 79.1 |
| 15-2-10 | | Arg-HCl(0.04M) | 77. 4 | 79.9 | 80. 7 | 81.5 | 82.0 |
| 15-2-11 | | His-HCl /$H_2O$(0.04M) | 77. 9 | 79.4 | 79.4 | 79.4 | 79.8 |
| 15-2-12 | | DMSO(1%v/v) | 77. 5 | 78.5 | 79. 3 | 79.2 | 79.7 |
| 15-2-13 | | PEG6000(1%v/v) | 80. 1 | 79.9 | 80. 4 | 80.2 | 80.5 |
| 15-2-14 | | Tween20(1%v/v) | 77. 4 | 78.9 | 76. 9 | 78.8 | 78.8 |
| 15-2-15 | | EtOH(1%v/v) | 77. 1 | 77.8 | 78. 6 | 78.9 | 78.5 |

[Table 8]

Table 9

| Example | Antibody | Additive (concentration within system) | Rate of transglycosylation (%) | | |
|---|---|---|---|---|---|
| | | | 16h | 18h | 20h |
| 15 2-16 | (Fucα1,6)GlcNAc-modified anti-CDH6 antibody 1 | - | 80.5 | 81.6 | 82.8 |
| 15-2-17 | | NaCl (0.3M) | 87.4 | 88.0 | 88.2 |

The presence of a molecular species (hereinafter, referred to as an additive) suitable for the antibody or the glycan donor molecule used is expected to positively or negatively influence the rate of transglycosylation in the one-pot reaction compared to the absence of addition of the additive, owing to electrostatic interaction, hydrophobic interaction, a solvent effect, or the like applied thereto.

Any given molecule can be added so long as it is an additive having an improving effect on reaction efficiency. Here, inorganic salts (sodium chloride, lithium chloride, calcium chloride, magnesium sulfate, ammonium sulfate, magnesium chloride), organic salts (ammonium acetate, sodium acetate, potassium acetate), organic solvents (ethanol, dimethyl sulfoxide), surfactants (polysorbate 20, PEG6000), saccharides (glucose, sorbitol, trehalose), amino acids (arginine, histidine), and the like were used as representative examples thereof and examined for the presence or absence of an effect brought about by the addition thereof (Tables 8 and 9).

It has been confirmed that the addition of the additive may drastically improve the rate of transglycosylation. In particular, a monovalent alkali metal salt, particularly, sodium chloride, ammonium acetate, or potassium acetate, is highly effective in this respect.

From Reference Examples 10-1 and 10-4, a divalent metal ion is considered to become a factor that affects an enzyme structure and negatively influences its reaction activity, and to inhibit the intended progression of the reaction.

From Reference Example 10-5, glucose might serve as an acceptor of a glycan activated by the Endo-Rp enzyme and is therefore considered to inhibit the progression of the reaction. On the other hand, trehalose, which contains α-glucose in a disaccharide structure, or sorbitol, which is completely linearized by the reduction of glucose, does not inhibit the progression of the reaction and, instead, improved the rate of transglycosylation (Examples 15-2-8 and 15-2-9).

Example 15-3: Method for Setting Concentration of Additive Used in One-Pot Method

The concentration of the additive can be arbitrarily set to not precipitate the antibody or the enzymes upon the addition of the additive, namely so long as the reactivity of the one-pot reaction remains. Here, sodium chloride was used as a representative example of the additive, and a specific setting method is shown as one example.

[Transglycosylation Reaction Standard Protocol 3]

(Fucα1,6)GlcNAc-modified anti-EGFR antibody 2 (3 mg), glycan donor molecule G-10 (25 equivalents), Endo-Sim (1.6% w/w), Endo-Rpm (0.2% w/w), and an appropriate amount of 50 mM Tris-HCl buffer (pH 7.25) were mixed. If necessary, an appropriate amount of an additive-containing solution with the content shown in Table 10 was added thereto to bring the total liquid volume to 50 μL, followed by reaction at 30°C for 24 h.

Sampling was performed 18 h, 20 h, and 24 h after the start of the reaction. For the sampling, a portion of the reaction mixture was extracted, diluted with purified water so as to obtain a concentration of the antibody therein of 1 mg/mL, and cryopreserved until the start of LabChip analysis. After completion of the reaction, the rate of transglycosylation at each reaction time was calculated under the respective reaction conditions used for (Fucα1,6)GlcNAc-modified anti-EGFR antibody 2 according to the method described in (15-2b) (Table 10).

[Table 9]

Table 10

| | Antibody | Glycan donor molecule (equivalents) | Additive (concentration within system) | Rate of transglycosylation (%) | | |
|---|---|---|---|---|---|---|
| | | | | 18h | 22h | 24h |
| 15-3-1 | (Fucα1,6) GlcNAc-modified anti-EGFR antibody 2 | G-10(25eq.) | - | 81.3 | 81.3 | 81.8 |
| 15-3-2 | | G-10(25eq.) | NaCl (0.15M) | 86.6 | 86.8 | 87.3 |
| 153-3 | | G-10(25eq.) | NaCl (0.30M) | 88.0 | 88.3 | 88.9 |
| 15-3-4 | | G-10(25eq.) | NaCl (0.45M) | 89.1 | 89.9 | 90.2 |
| 15-3-5 | | G-10(25eq.) | NaCl (0.60M) | 90.5 | 91.1 | 91.5 |

From the results of Example 15-3, sodium chloride selected as the additive has been confirmed to improve the rate of transglycosylation, as its salt concentration within the reaction system was higher. Likewise, the optimum salt concentration of the additive used can be set according to the type of the additive to be used.

Thus, a person skilled in the art can structurally identify an additive having a characteristic of improving the rate of transglycosylation reaction with reference to the present specification. Thus, any additive not described in Example 15-2 and Example 15-3 may be used at the optimum concentration in the one-pot method to prepare a reaction mixture containing the target product modified anti-EGFR antibody 2-[SG-$(N_3)_2$]$_2$ or modified anti-CDH6 antibody 1-[SG-$(N_3)_2$]$_2$, which can be then purified by a suitable method.

Industrial Applicability

The present invention provides an antibody-drug conjugate containing a CDN derivative with strong STING agonist activity and potent anti-tumor effects. This antibody-drug conjugate is useful as a therapeutic agent for diseases (e.g., cancer) associated with STING agonist activity. The present invention also provides a novel method for producing an Fc-containing molecule having an N297-linked glycan including a glycan donor molecule-derived glycan.

Sequence Listing

SEQ ID NO: 1: the amino acid sequence of human wild-type STING
SEQ ID NO: 2: the nucleic acid sequence for human wild-type STING
SEQ ID NO: 3: the amino acid sequence of human H232 (REF) mutant STING
SEQ ID NO: 4: the nucleic acid sequence for human H232 (REF) mutant STING
SEQ ID NO: 5: the amino acid sequence of human HAQ mutant STING
SEQ ID NO: 6: the nucleic acid sequence for human HAQ mutant STING
SEQ ID NO: 7: the amino acid sequence of mouse STING
SEQ ID NO: 8: the nucleic acid sequence for mouse STING
SEQ ID NO: 9: the amino acid sequence of HisAviTEV-hSTING(139-342)human wild-type protein
SEQ ID NO: 10: the amino acid sequence of HisAviTEV-hSTING(139-342)human REF mutant protein
SEQ ID NO: 11: the amino acid sequence of HisAviTEV-hSTING(139-342)human AQ mutant protein
SEQ ID NO: 12: the amino acid sequence of His-Avi-TEV-mSTING(138-341)mouse wild-type protein
SEQ ID NOs: 13-22: primer sequences
SEQ ID NO: 23: the amino acid sequence of CDRL1 of anti-EGFR antibodies (modified anti-EGFR antibodies 1 to 3)
SEQ ID NO: 24: the amino acid sequence of CDRL2 of anti-EGFR antibodies (modified anti-EGFR antibodies 1 to 3)
SEQ ID NO: 25: the amino acid sequence of CDRL3 of anti-EGFR antibodies (modified anti-EGFR antibodies 1 to 3)
EQ ID NO: 26: the amino acid sequence of CDRH1 of anti-EGFR antibodies (modified anti-EGFR antibodies 1 to 3)
SEQ ID NO: 27: the amino acid sequence of CDRH2 of anti-EGFR antibodies (modified anti-EGFR antibodies 1 to 3)
SEQ ID NO: 28: the amino acid sequence of CDRH3 of anti-EGFR antibodies (modified anti-EGFR antibodies 1 to 3)
SEQ ID NO: 29: the amino acid sequence of the light chain variable region of anti-EGFR antibodies (modified anti-EGFR antibodies 1 to 3)
SEQ ID NO: 30: the amino acid sequence of the light chain of anti-EGFR antibodies (modified anti-EGFR antibodies 1 to 3)
SEQ ID NO: 31: the amino acid sequence of the heavy chain variable region of anti-EGFR antibodies (modified anti-EGFR antibodies 1 to 3)
SEQ ID NO: 32: the amino acid sequence of the heavy chain of a LALA-DG mutant anti-EGFR antibody (modified anti-EGFR antibody 1)
SEQ ID NO: 33: the amino acid sequence of the heavy chain of a LALA-DG mutant anti-EGFR antibody (modified anti-EGFR antibody 2)
SEQ ID NO: 34: the amino acid sequence of the heavy chain of anti-EGFR antibody A
SEQ ID NO: 35: the amino acid sequence of the heavy chain of anti-EGFR antibody B (Vectibix)
SEQ ID NO: 36: the amino acid sequence of the heavy chain of a LALA-DG mutant anti-EGFR antibody (modified anti-EGFR antibody 3)
SEQ ID NO: 37: the amino acid sequence of CDRL1 of an anti-CDH6 antibody (modified anti-CDH6 antibody 1)
SEQ ID NO: 38: the amino acid sequence of CDRL2 of an anti-CDH6 antibody (modified anti-CDH6 antibody 1)
SEQ ID NO: 39: the amino acid sequence of CDRL3 of an anti-CDH6 antibody (modified anti-CDH6 antibody 1)
SEQ ID NO: 40: the amino acid sequence of CDRH1 of an anti-CDH6 antibody (modified anti-CDH6 antibody 1)
SEQ ID NO: 41: the amino acid sequence of CDRH2 of an anti-CDH6 antibody (modified anti-CDH6 antibody 1)
SEQ ID NO: 42: the amino acid sequence of CDRH3 of an anti-CDH6 antibody (modified anti-CDH6 antibody 1)
SEQ ID NO: 43: the amino acid sequence of the light chain variable region of an anti-CDH6 antibody (modified anti-CDH6 antibody 1)
SEQ ID NO: 44: the amino acid sequence of the light chain of an anti-CDH6 antibody (modified anti-CDH6 antibody 1)
SEQ ID NO: 45: the amino acid sequence of the heavy chain variable region of an anti-CDH6 antibody (modified anti-CDH6 antibody 1)
SEQ ID NO: 46: the amino acid sequence of the heavy chain of a LALA-DG mutant anti-CDH6 antibody (modified anti-CDH6 antibody 1)
SEQ ID NO: 47: the amino acid sequence of wild-type Endo-S
SEQ ID NO: 48: the amino acid sequence of wild-type Endo-S2
SEQ ID NO: 49: the amino acid sequence of wild-type Endo-Si
SEQ ID NO: 50: the amino acid sequence of wild-type Endo-M
SEQ ID NO: 51: the amino acid sequence of wild-type Endo-Rp
SEQ ID NO: 52: the amino acid sequence of wild-type Endo-CC
SEQ ID NO: 53: the amino acid sequence of wild-type Endo-Om
SEQ ID NO: 54: the amino acid sequence of wild-type Endo-Sd
SEQ ID NO: 55: the amino acid sequence of wild-type Endo-Sz
SEQ ID NO: 56: the amino acid sequence of wild-type Endo-Se

**Claims**

1. An antibody-drug conjugate represented by the following formula (II):

$$Ab-\left[-L-D\right]_{m^1} \qquad (II)$$

wherein $m^1$ ranges from 1 to 10;
Ab represents an antibody or a functional fragment of the antibody, wherein the antibody or the functional fragment of the antibody optionally has a remodeled glycan, and the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody;
L represents a linker linking Ab and D, wherein
Ab may directly bond to L from an amino acid residue of Ab or may bond to L via the glycan or remodeled glycan of Ab;
D represents a compound represented by the following formula (I) :

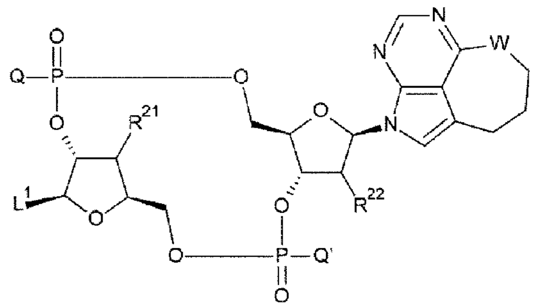

(I)

wherein
L bonds to a hydroxy group included in $L^1$,
$L^1$ represents a group having the following formula:

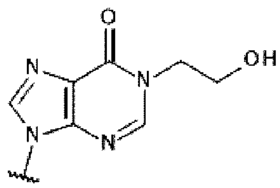

wherein the wavy line represents a position of substitution,
Q and Q' each independently represent a hydroxy group or a thiol group,
$R^{21}$ and $R^{22}$ each independently represent a hydroxy group or a fluorine atom, and
W represents -NH- or a sulfur atom.

2. The antibody-drug conjugate according to claim 1, wherein D is represented by any one of the following two formulae:

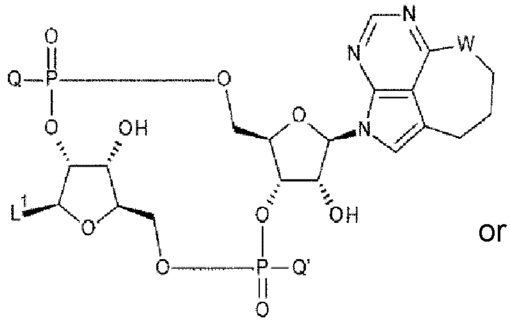

or

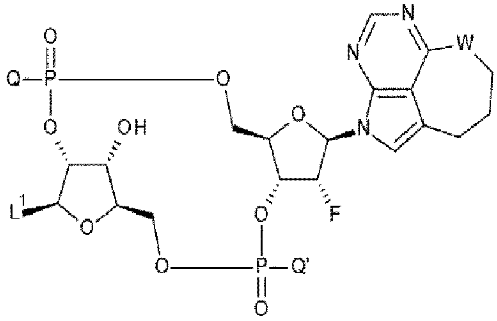

wherein $L^1$, Q, Q', and W are as defined above.

**3.** The antibody-drug conjugate according to claim 1 or 2, wherein D is represented by any one of the following two formulae:

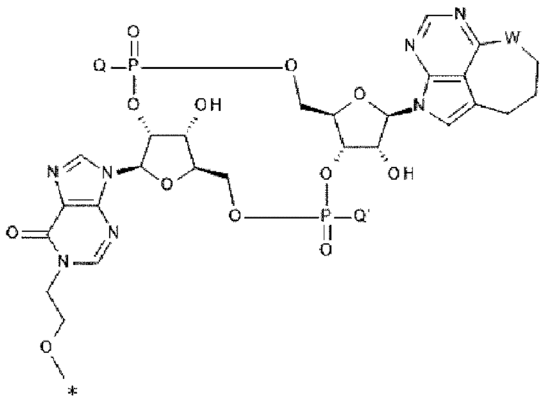

or

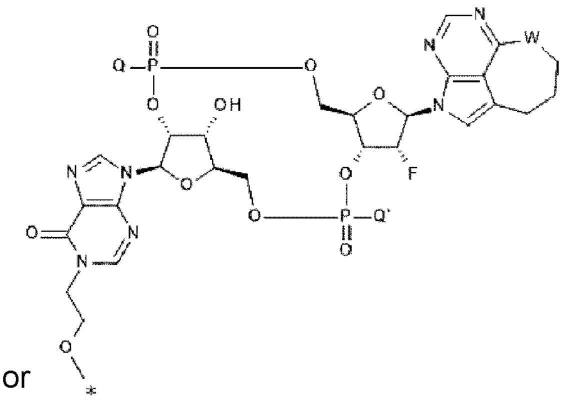

wherein the asterisk represents bonding to L, and Q, Q', and W are as defined above.

**4.** The antibody-drug conjugate according to any one of claims 1 to 3, wherein D is represented by any one of the following three formulae:

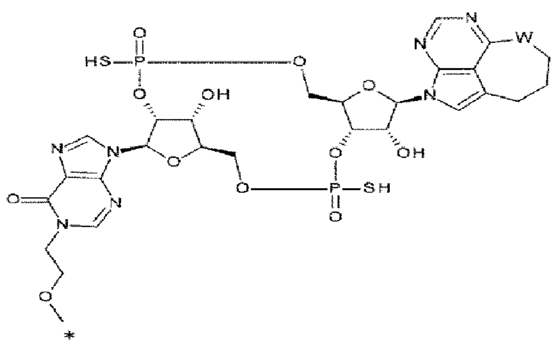

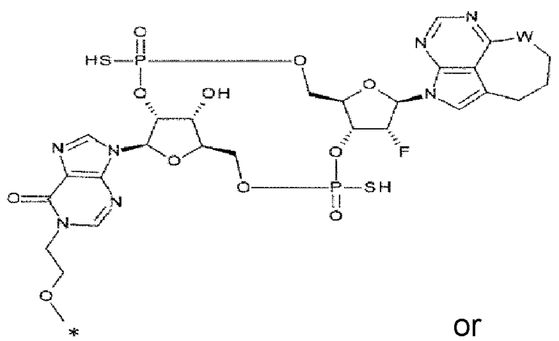

or

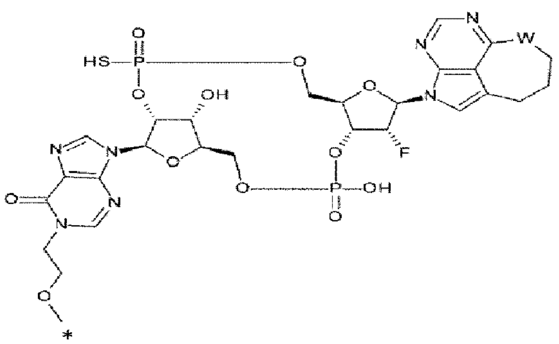

wherein the asterisk represents bonding to L, and W is as defined above.

**5.** The antibody-drug conjugate according to any one of claims 1 to 4, wherein D is represented by any one of the following three formulae:

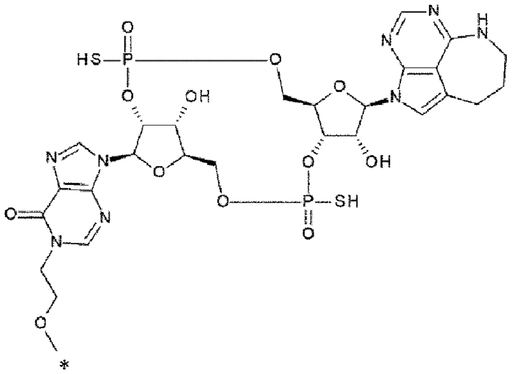

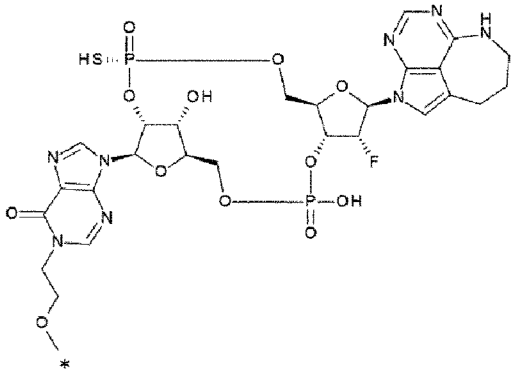

or

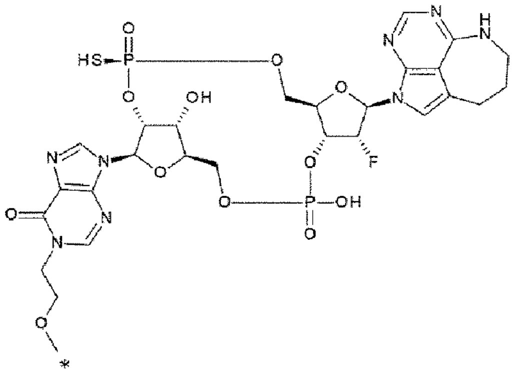

wherein the asterisk represents bonding to L.

**6.** The antibody-drug conjugate according to any one of claims 1 to 4, wherein D is represented by any one of the following four formulae:

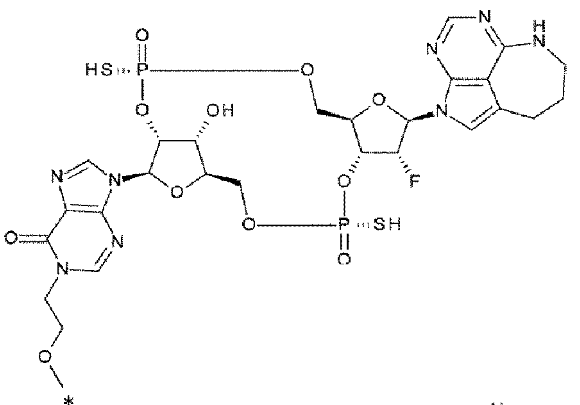

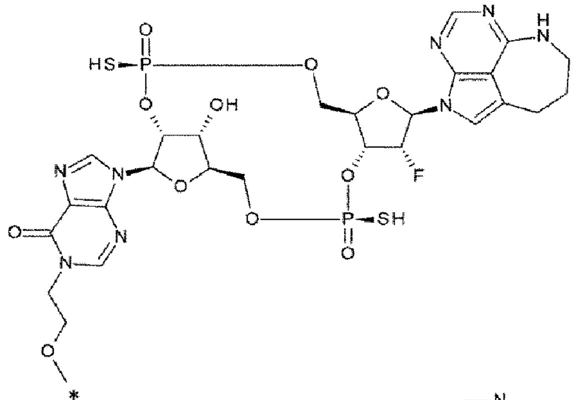

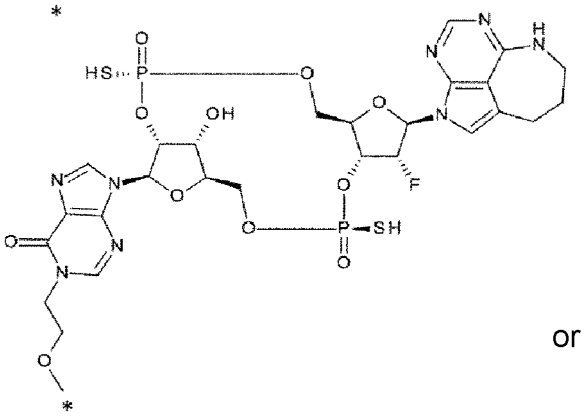

or

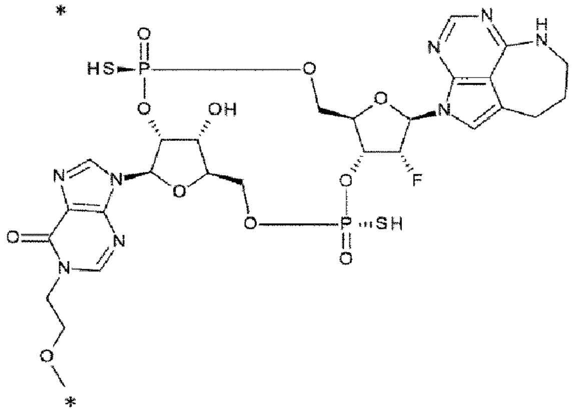

wherein the asterisk represents bonding to L.

**7.** The antibody-drug conjugate according to any one of claims 1 to 4 and 6, wherein D is represented by the following formula:

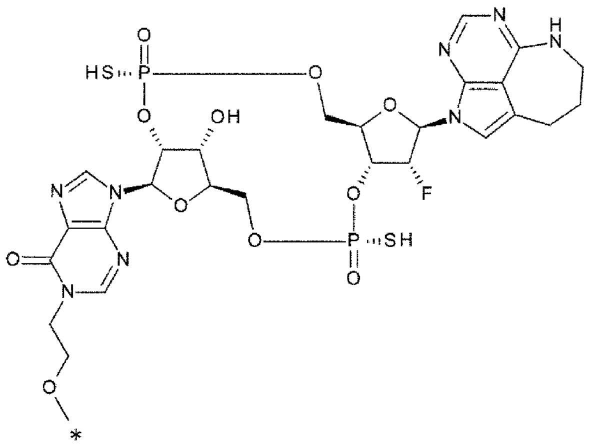

wherein the asterisk represents bonding to L.

**8.** The antibody-drug conjugate according to any one of claims 1 to 7, wherein the linker L is represented by -Lb-La-Lp-Lc-*,

wherein the asterisk represents bonding to the drug D;
Lp represents a linker consisting of an amino acid sequence cleavable in a target cell or is absent;
La represents any one selected from the group consisting of the following:

$-C(=O)-(CH_2CH_2)n^2-C(=O)-$,

$-C(=O)-(CH_2CH_2)n^2-CH_2-C(=O)-$,

$-C(=O)-(CH_2CH_2)n^2-C(=O)-NH-(CH_2CH_2)n^3-C(=O)-$,

$-C(=O)-(CH_2CH_2)n^2-C(=O)-NH-(CH_2CH_2)n^3-CH_2-C(=O)-$,

$-C(=O)-(CH_2CH_2)n^2-C(=O)-NH-(CH_2CH_2O)n^3-CH_2-C(=O)-$,

$-(CH_2)n^4-O-C(=O)-$,

and

$-(CH_2)n^9-C(=O)-$,

where $n^2$ represents an integer of 1 to 3, $n^3$ represents an integer of 1 to 5, $n^4$ represents an integer of 0 to 2, and $n^9$ represents an integer of 2 to 7;
Lb represents a spacer for bonding La to the glycan or remodeled glycan of Ab or a spacer for bonding La to a cysteine residue of Ab; and
Lc represents $-NH-CH_2-$, $-NH-phenyl-CH_2-O(C=O)-$, or $-NH-heteroaryl-CH_2-O(C=O)-$, or is absent.

**9.** The antibody-drug conjugate according to claim 8, wherein Lc is $-NH-CH_2-$.

**10.** The antibody-drug conjugate according to claim 8 or 9, wherein Lp is any one of -GGFG-, -GGPI-, -GGVA-, -GGFM-, -GGVCit-, -GGFCit-, -GGICit-, -GGPL-, -GGAQ-, or -GGPP-.

**11.** The antibody-drug conjugate according to claim 10, wherein Lp is -GGFG- or -GGPI-.

**12.** The antibody-drug conjugate according to any one of claims 8 to 11, wherein La represents any one selected from the group consisting of the following:

$-C(=O)-CH_2CH_2-C(=O)-$,

$-C(=O)-CH_2CH_2-C(=O)-NH-(CH_2CH_2O)_3-CH_2-C(=O)-$,

$-C(=O)-CH_2CH_2-C(=O)-NH-(CH_2CH_2O)_4-CH_2-C(=O)-$,

and

$-(CH_2)_5-C(=O)-$.

**13.** The antibody-drug conjugate according to any one of claims 8 to 12, wherein Lb is represented by any one of the following formulae:

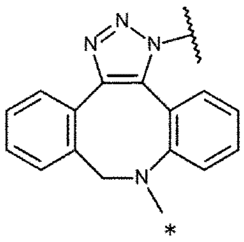

or

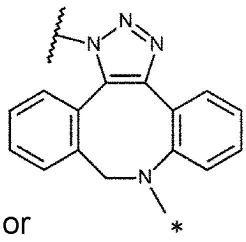

or

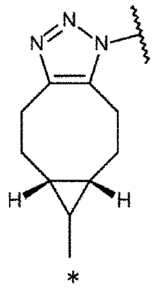

or

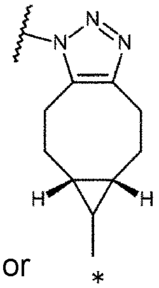

wherein, in the structural formulae of Lb shown above, the asterisk represents bonding to La, and the wavy line represents bonding to the glycan or remodeled glycan of Ab.

**14.** The antibody-drug conjugate according to any one of claims 8 to 12, wherein Lb represents -(succinimid-3-yl-N)-, wherein - (succinimid-3-yl-N)- is represented by the following structural formula:

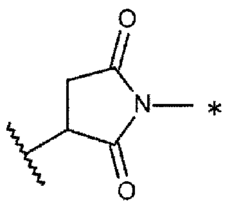

wherein the asterisk represents bonding to La, and the wavy line represents bonding to a side chain of a cysteine residue of the antibody by forming a thioether.

**15.** The antibody-drug conjugate according to any one of claims 8 to 13, wherein the linker L is represented by -Lb-La-Lp-Lc-*

wherein the asterisk represents bonding to the drug D;
Lp is -GGFG- or -GGPI-;
La represents $-C(=O)-CH_2CH_2-C(=O)-$;
Lb represents the following formula:

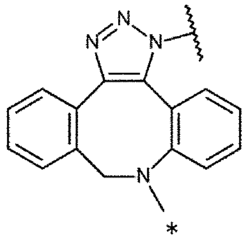

or

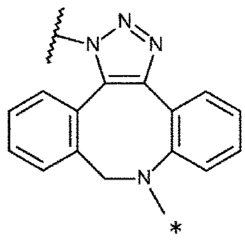

wherein, in the structural formulae of Lb shown above, the asterisk represents bonding to La, and the wavy line represents bonding to the glycan or remodeled glycan of Ab; and
Lc represents $-NH-CH_2-$.

**16.** The antibody-drug conjugate according to any one of claims 1 to 15, wherein the average number of the conjugated drug molecules per antibody molecule in the antibody-drug conjugate ranges from 1 to 10.

**17.** The antibody-drug conjugate according to claim 16, wherein the average number of the conjugated drug molecules per antibody molecule in the antibody-drug conjugate ranges from 1 to 5.

**18.** The antibody-drug conjugate according to claim 17, wherein the average number of the conjugated drug molecules per antibody molecule in the antibody-drug conjugate ranges from 1 to 3 or from 3 to 5.

**19.** The antibody-drug conjugate according to any one of claims 1 to 18, wherein the antibody bonds to L via a glycan bonding to Asn297 of the antibody (N297 glycan).

**20.** The antibody-drug conjugate according to claim 19, wherein the N297 glycan is a remodeled glycan.

**21.** The antibody-drug conjugate according to claim 19 or 20, wherein the N297 glycan is N297-(Fuc)MSG1 or N297-(Fuc)SG having a structure represented by the following formula:

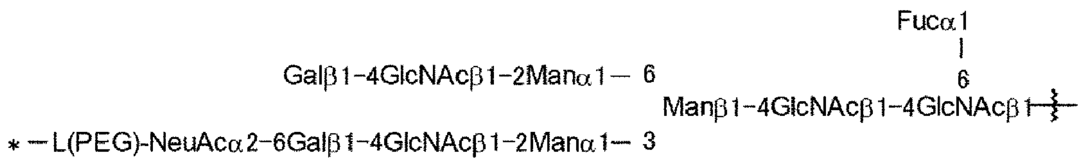

[N297-(Fuc)MSG1]

wherein the wavy line represents bonding to Asn297 of the antibody,
L(PEG) represents $-(CH_2-CH_2-O)n^5-CH_2-CH_2-NH-$,
wherein the amino group at the right end of the L(PEG) is bound via an amide bond to a carboxyl group at the 2-position of a sialic acid at the non-reducing terminal of the 1-3 branched chain of β-Man in the N297 glycan, the asterisk represents bonding to a nitrogen atom at the 1- or 3-position of the 1,2,3-triazole ring of Lb in the linker L, and
$n^5$ is an integer of 2 to 5;

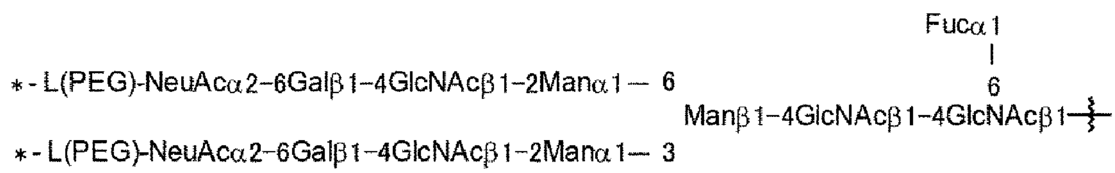

[N297-(Fuc)SG]

wherein the wavy line represents bonding to Asn297 of the antibody,
L(PEG) represents $-(CH_2-CH_2-O)n^5-CH_2-CH_2-NH-$,
wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at 1- or 3-position on the 1,2,3-triazole ring of Lb in the linker L, and
$n^5$ is an integer of 2 to 5.

**22.** The antibody-drug conjugate according to any one of claims 19 to 21, wherein the antibody-drug conjugate is represented by the following formula:

$$Ab\left[(\text{N297 glycan})\left[L — D\right]_{m^2}\right]_2$$

wherein $m^2$ represents an integer of 1 or 2,
L is a linker linking N297 glycan and D, as defined above, Ab represents an antibody or a functional fragment of the antibody, wherein the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody,
N297 glycan is represented by N297-(Fuc)MSG1 or N297-(Fuc)SG having a structure represented by the following formula:

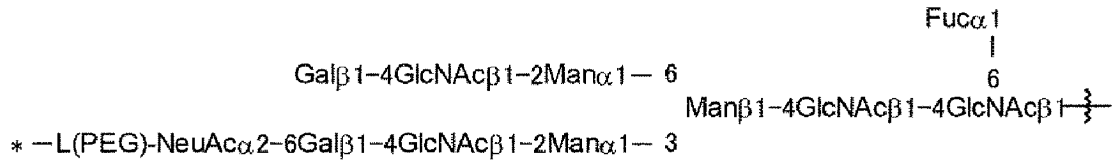

[N297-(Fuc)MSG1]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of the 1-3 branched chain of β-Man in the N297 glycan,
the asterisk represents bonding to a nitrogen atom at the 1- or 3-position of the 1,2,3-triazole ring of Lb in the linker L, and
$n^5$ represents an integer of 2 to 5; or

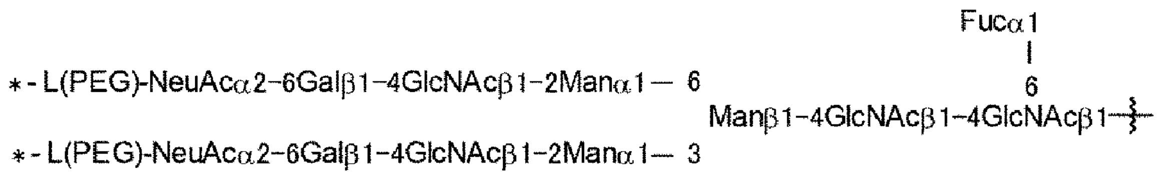

[N297-(Fuc)SG]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L, and
$n^5$ represents an integer of 2 to 5,
D is represented by any one of the following four formulae:

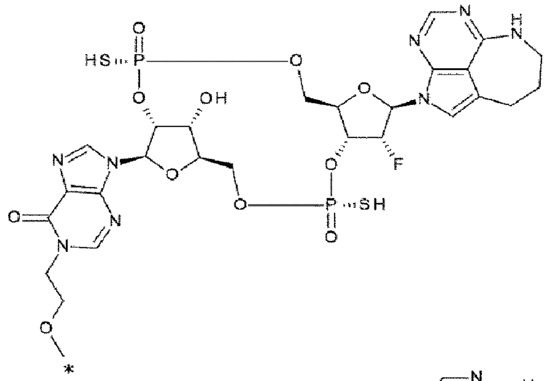

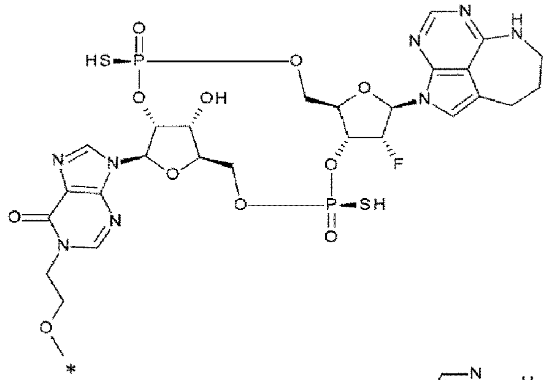

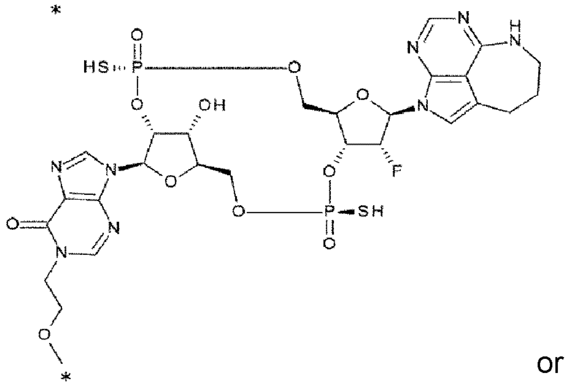

or

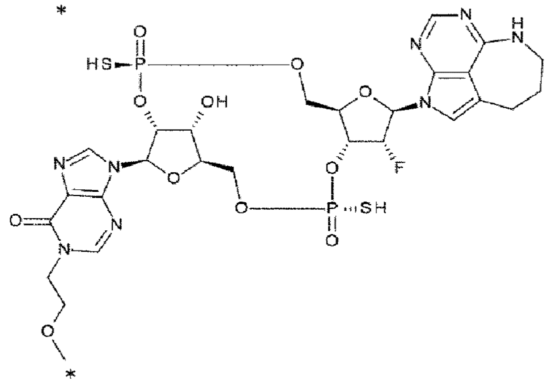

wherein the asterisk represents bonding to L.

**23.** The antibody-drug conjugate according to claim 22, wherein the antibody-drug conjugate is selected from the following formulae:

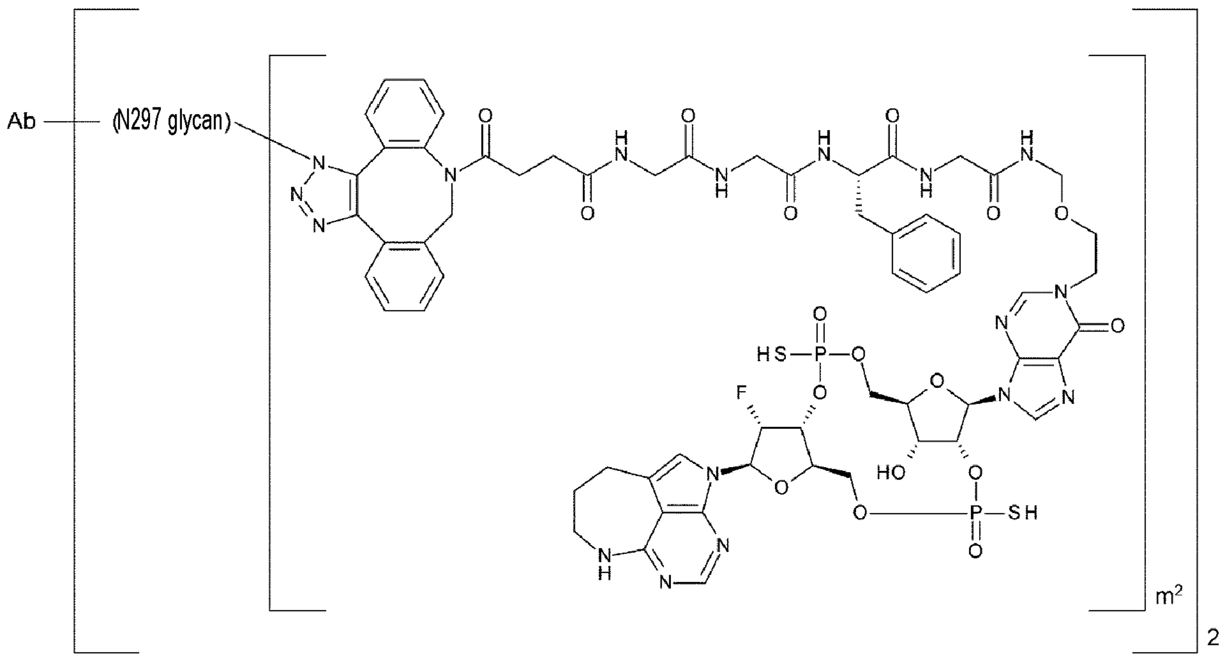

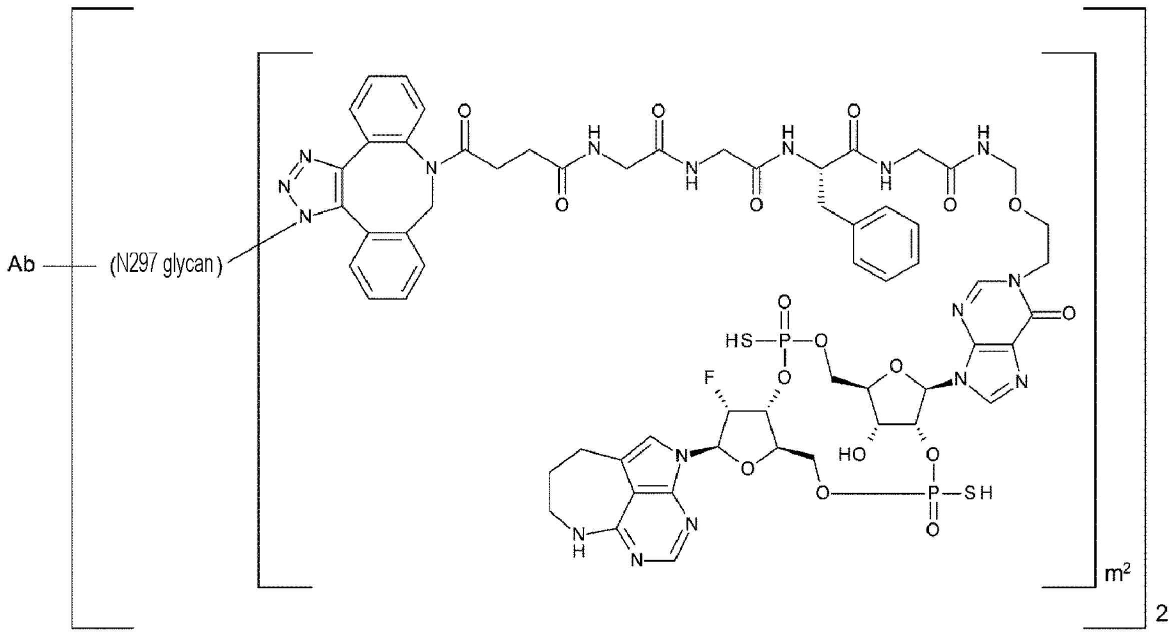

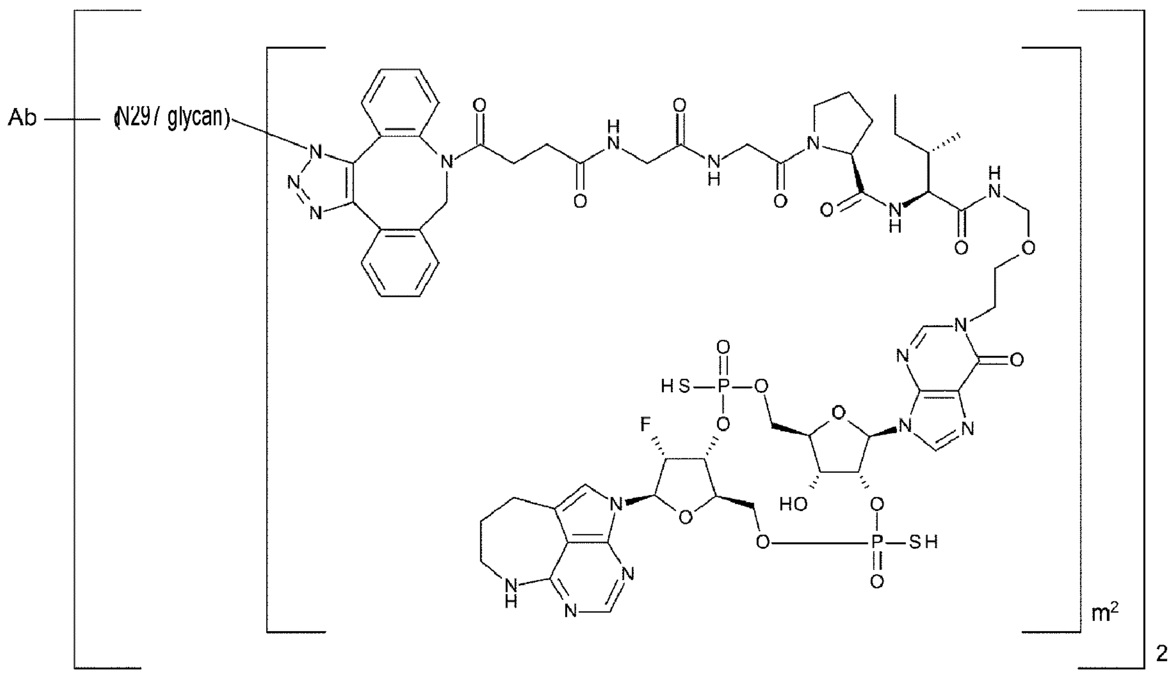

or

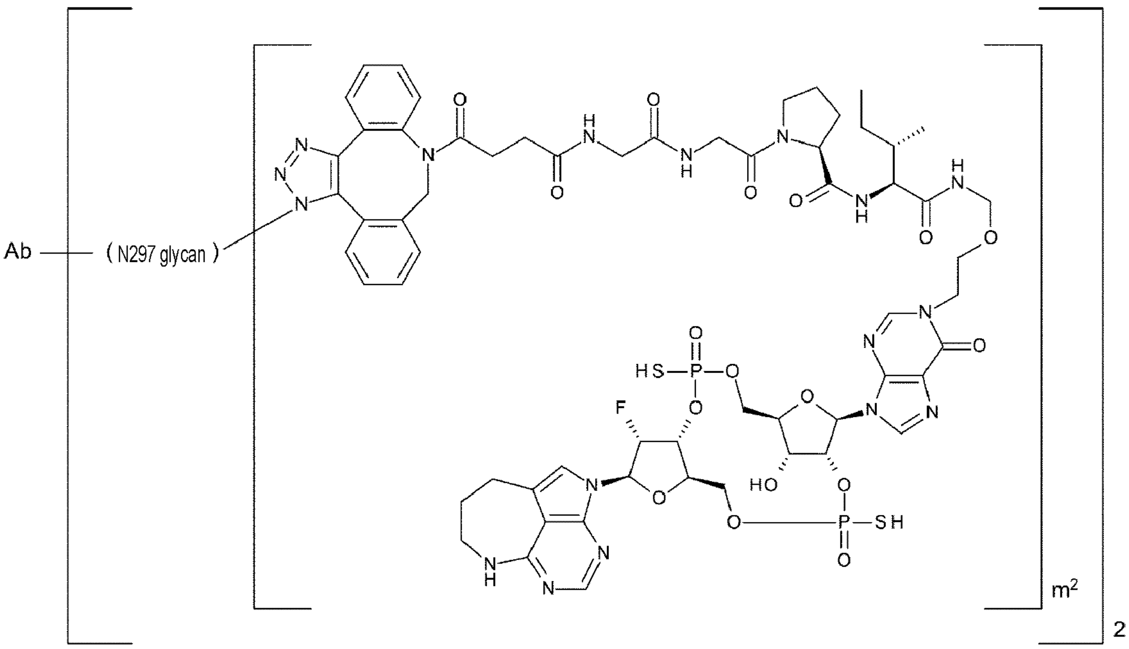

wherein, in each structural formula shown above, $m^2$ is an integer of 1 or 2,

Ab represents an antibody or a functional fragment of the antibody, wherein the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody,

N297 glycan is represented by any one of N297-(Fuc)MSG1 or N297-(Fuc)SG having a structure represented by the following formula:

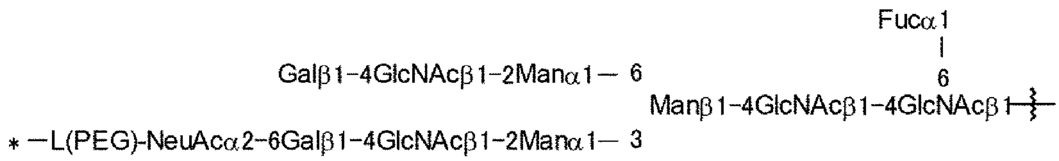

[N297-(Fuc)MSG1]

wherein the wavy line represents bonding to Asn297 of the antibody,

L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of the 1-3 branched chain of β-Man in the N297 glycan,

the asterisk represents bonding to a nitrogen atom at the 1- or 3-position of a 1,2,3-triazole ring of Lb in the linker L, and

$n^5$ represents an integer of 2 to 5; or

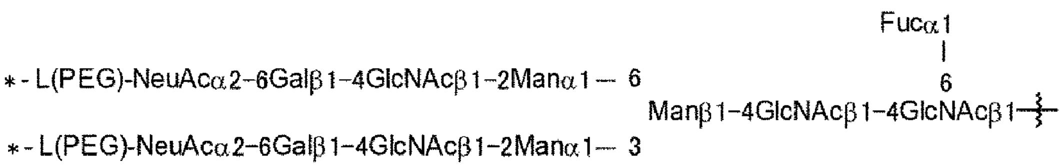

[N297-(Fuc)SG]

wherein the wavy line represents bonding to Asn297 of the antibody,

L(PEG) represents $-(CH_2-CH_2-O)n^5-CH_2-CH_2-NH-$, wherein the amino groups at the right end of the L(PEG) is bound via an amide bond to a carboxyl group at the 2-position of a sialic acid at the non-reducing terminal of each of the 1-3 and 1-6 branched chains of β-Man in the N297 glycan,

the asterisk represents bonding to a nitrogen atom at the 1- or 3-position of the 1,2,3-triazole ring of Lb in the linker L, and
$n^5$ represents an integer of 2 to 5.

**24.** The antibody-drug conjugate according to claim 23, wherein the antibody-drug conjugate is selected from the following formulae:

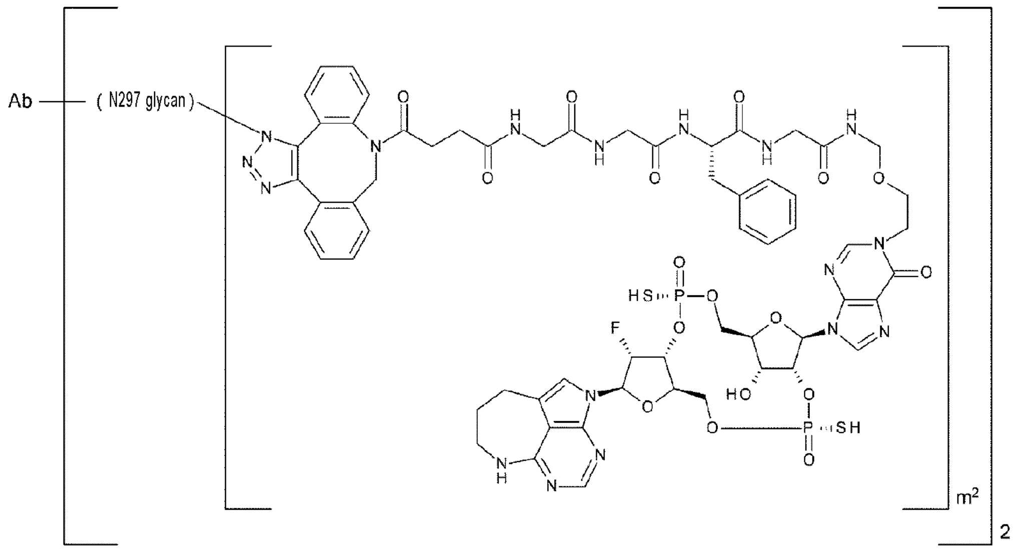

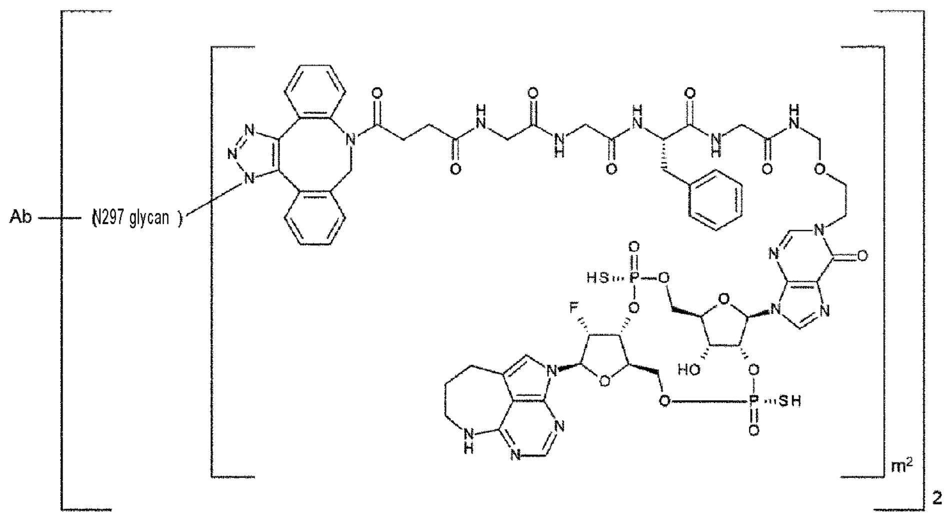

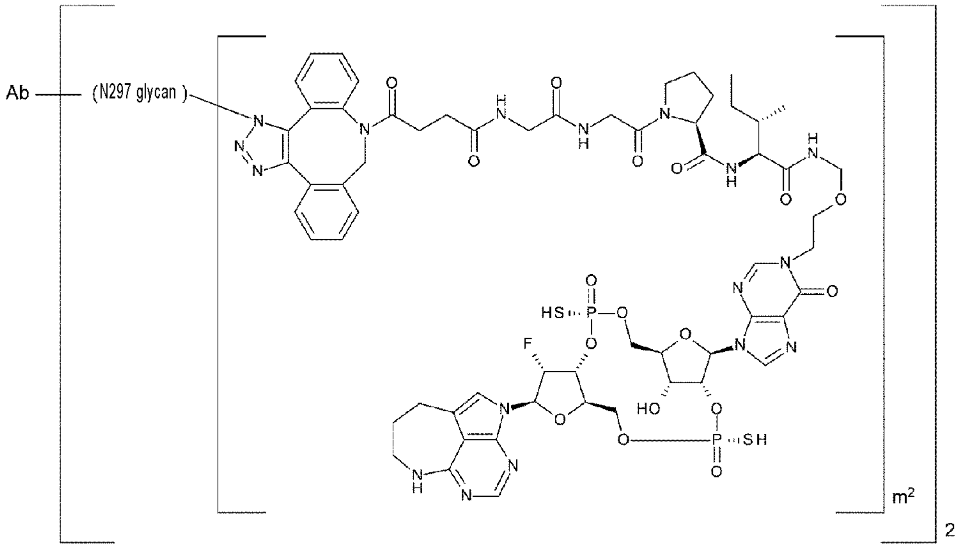

or

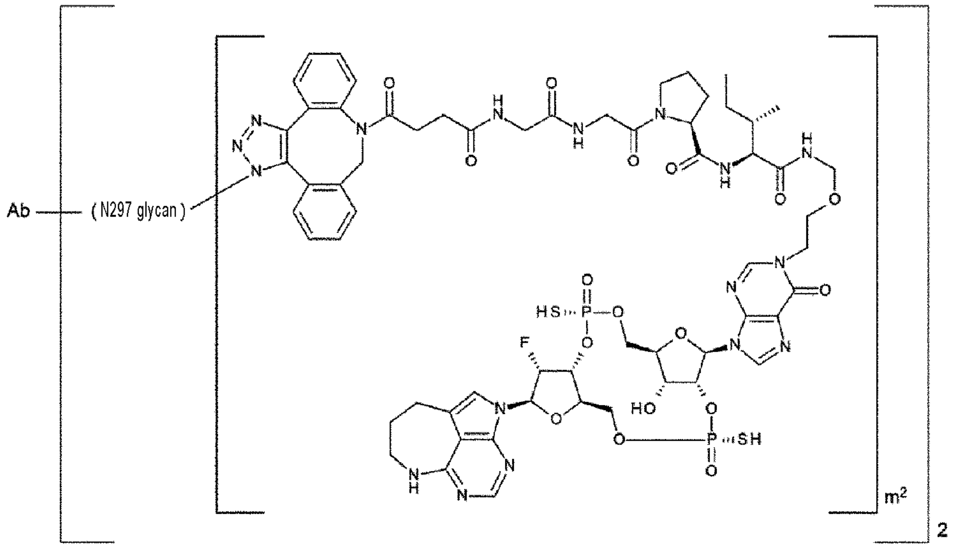

wherein, in each structural formula shown above, $m^2$ is an integer of 1 or 2,
Ab represents an antibody or a functional fragment of the antibody, wherein the amino acid residues at positions 234, 235, and 265 (all according to EU numbering) of the Fc region of the antibody or the functional fragment of the antibody are Ala, Ala, and Gly, respectively, wherein the antibody is an anti-EGFR antibody or an anti-CDH6 antibody,
N297 glycan is represented by any one of N297-(Fuc)MSG1 or N297-(Fuc)SG having a structure represented by the following formula:

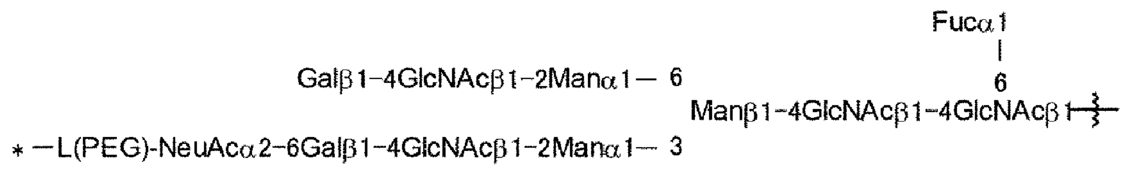

[N297-(Fuc)MSG1]

wherein the wavy line represents bonding to Asn297 of the antibody,
L (PEG) represents $-(CH_2-CH_2-O)n^5-CH_2-CH_2-NH-$, wherein the amino group at the right end of the L(PEG) is

bound via an amide bond to a carboxyl group at the 2-position of a sialic acid at the non-reducing terminal of the 1-3 branched chain of β-Man in the N297 glycan,
the asterisk represents bonding to a nitrogen atom at the 1- or 3-position of the 1,2,3-triazole ring of Lb in the linker L, and
$n^5$ represents an integer of 2 to 5; or

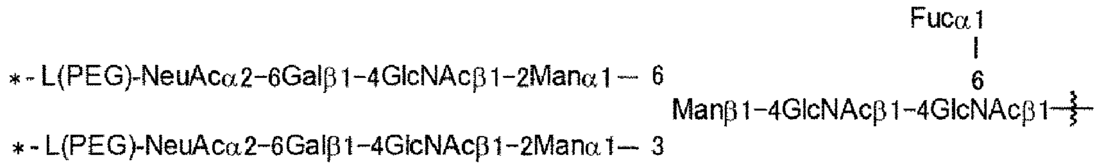

[N297-(Fuc)SG]

wherein the wavy line represents bonding to Asn297 of the antibody,
L(PEG) represents $-(CH_2-CH_2-O)n^5-CH_2-CH_2-NH-$, wherein the amino groups at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L, and
$n^5$ represents an integer of 2 to 5.

**25.** The antibody-drug conjugate according to any one of claims 1 to 24, wherein the antibody is an anti-EGFR antibody.

**26.** The antibody-drug conjugate according to any one of claims 1 to 24, wherein the antibody is an anti-CDH6 antibody.

**27.** The antibody-drug conjugate according to claim 25, wherein the antibody is an antibody comprising a light chain consisting of an amino acid sequence set forth in SEQ ID NO: 30 and a heavy chain consisting of an amino acid sequence set forth in SEQ ID NO: 32, or an antibody comprising a light chain consisting of an amino acid sequence set forth in SEQ ID NO: 30 and a heavy chain consisting of an amino acid sequence set forth in SEQ ID NO: 33, or any one of these antibodies comprising a heavy chain in which one or several amino acid residues are deleted at the carboxyl terminus thereof.

**28.** The antibody-drug conjugate according to claim 26, wherein the antibody is an antibody comprising a light chain consisting of an amino acid sequence set forth in SEQ ID NO: 44 and a heavy chain consisting of an amino acid sequence set forth in SEQ ID NO: 46, or any one of these antibodies comprising a heavy chain in which one or several amino acid residues are deleted at the carboxyl terminus thereof.

**29.** The antibody-drug conjugate according to claim 25, wherein the antibody is an antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, wherein the amino acid residues at positions 234, 235, and 265 (all according to EU numbering) of the Fc region of the antibody are Ala, Ala, and Gly, respectively.

**30.** The antibody-drug conjugate according to claim 26, wherein the antibody is an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein the amino acid residues at positions 234, 235, and 265 (all according to EU numbering) of the Fc region of the antibody are Ala, Ala, and Gly, respectively.

**31.** The antibody-drug conjugate according to claim 25, wherein the antibody is an antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, wherein the amino acid residues at positions 234, 235, and 265 (all according to EU numbering) of the Fc region of the antibody are Ala, Ala, and Gly, respectively.

**32.** The antibody-drug conjugate according to claim 26, wherein the antibody is an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein the amino acid residues at positions 234, 235, and 265 (all according to EU numbering) of the Fc region of the antibody are Ala, Ala, and Gly, respectively.

**33.** An antibody-drug conjugate represented by the following formula:

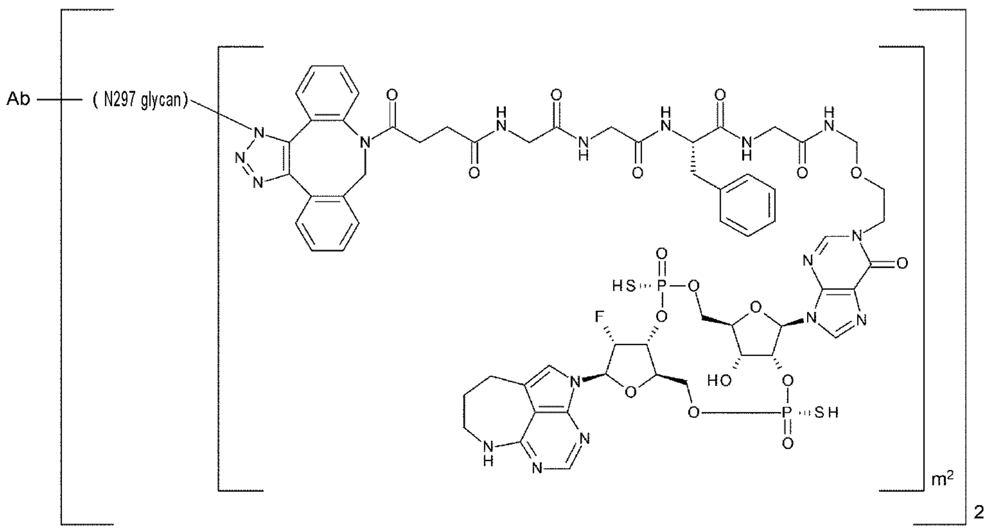

or

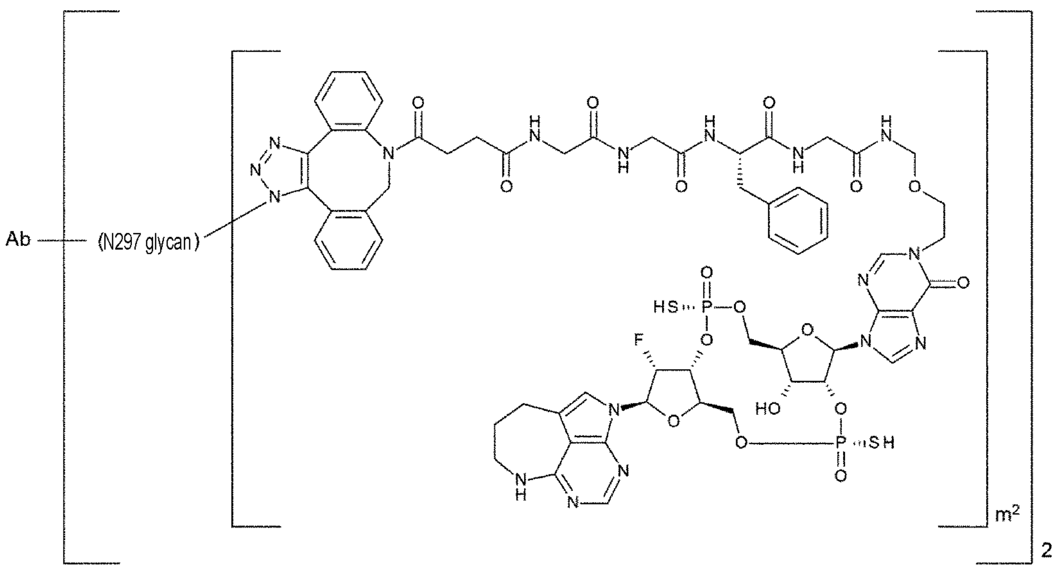

wherein Ab represents any one selected from the group consisting of the following:

an antibody comprising a light chain consisting of an amino acid sequence set forth in SEQ ID NO: 30 and a heavy chain consisting of an amino acid sequence set forth in SEQ ID NO: 32,
an antibody comprising a light chain consisting of an amino acid sequence set forth in SEQ ID NO: 30 and a heavy chain consisting of an amino acid sequence set forth in SEQ ID NO: 33,
an antibody comprising a light chain consisting of an amino acid sequence set forth in SEQ ID NO: 44 and a heavy chain consisting of an amino acid sequence set forth in SEQ ID NO: 46, and

any one of these antibodies comprising a heavy chain in which one or several amino acid residues are deleted at the carboxyl terminus thereof,
wherein N297 glycan is represented by the following formula:

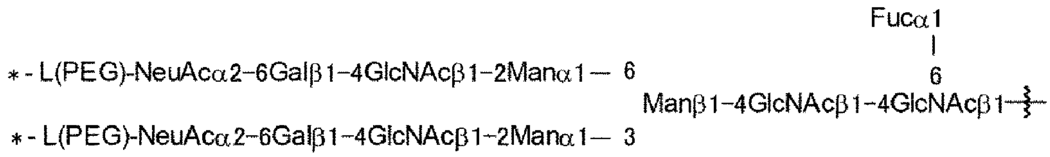

[N297-(Fuc)SG]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan
the asterisk represents bonding to a nitrogen atom at the 1- or 3-position of a 1,2,3-triazole ring of Lb in the linker L,
$n^5$ is 3, and
$m^2$ is 2.

**34.** An antibody-drug conjugate represented by the following formula:

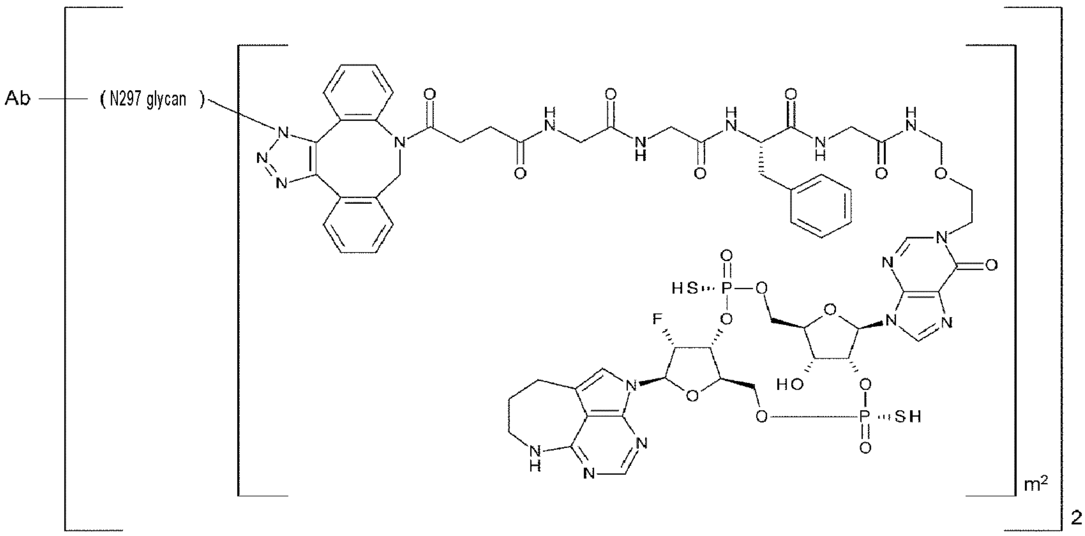

or

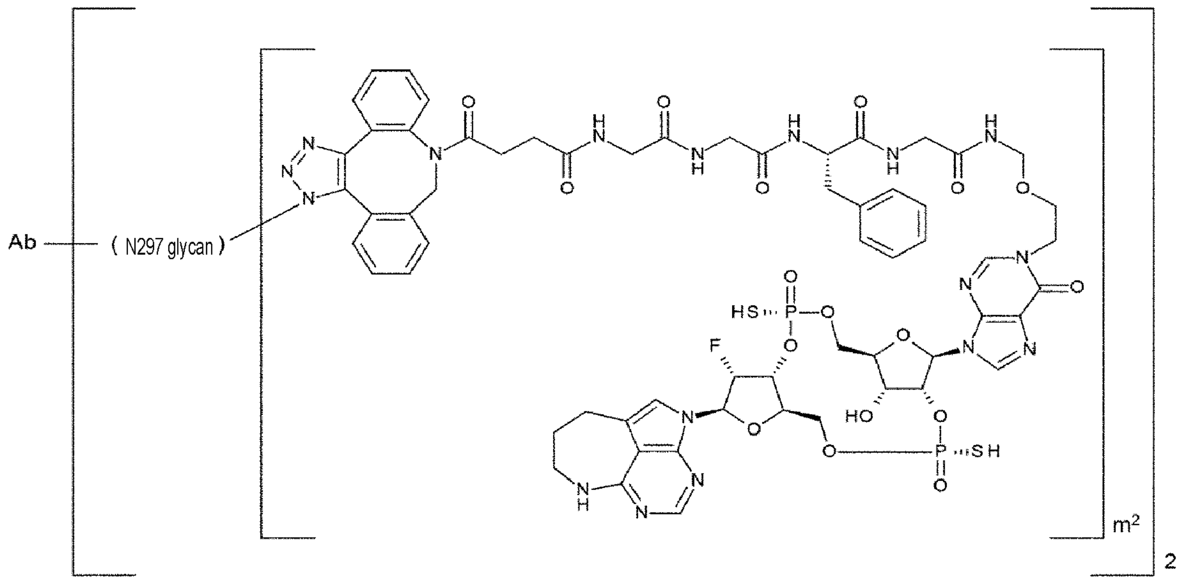

wherein Ab represents any one selected from the group consisting of the following:

an antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, and

an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein

the amino acid residues at positions 234, 235, and 265 (all according to EU numbering) of the Fc region of the antibody are Ala, Ala, and Gly, respectively,

wherein N297 glycan is represented by the following formula:

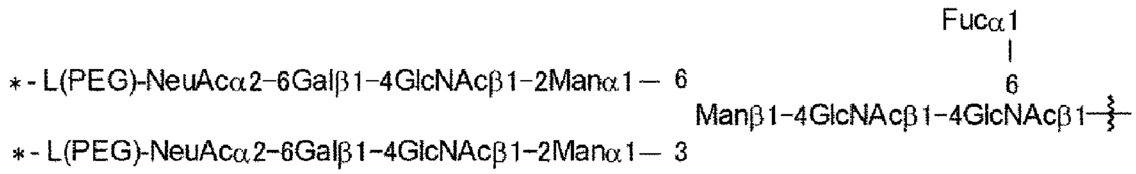

[N297-(Fuc)SG]

wherein the wavy line represents bonding to Asn297 of the antibody,

L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,

the asterisk represents bonding to a nitrogen atom at the 1- or 3-position of the 1,2,3-triazole ring of Lb in the linker L,

$n^5$ is 3, and

$m^2$ is 2.

**35.** An antibody-drug conjugate represented by the following formula:

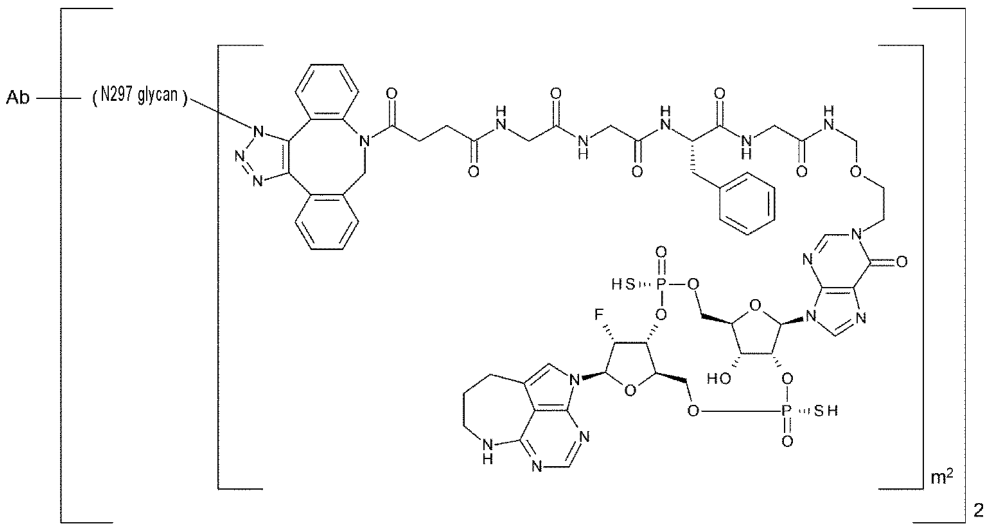

or

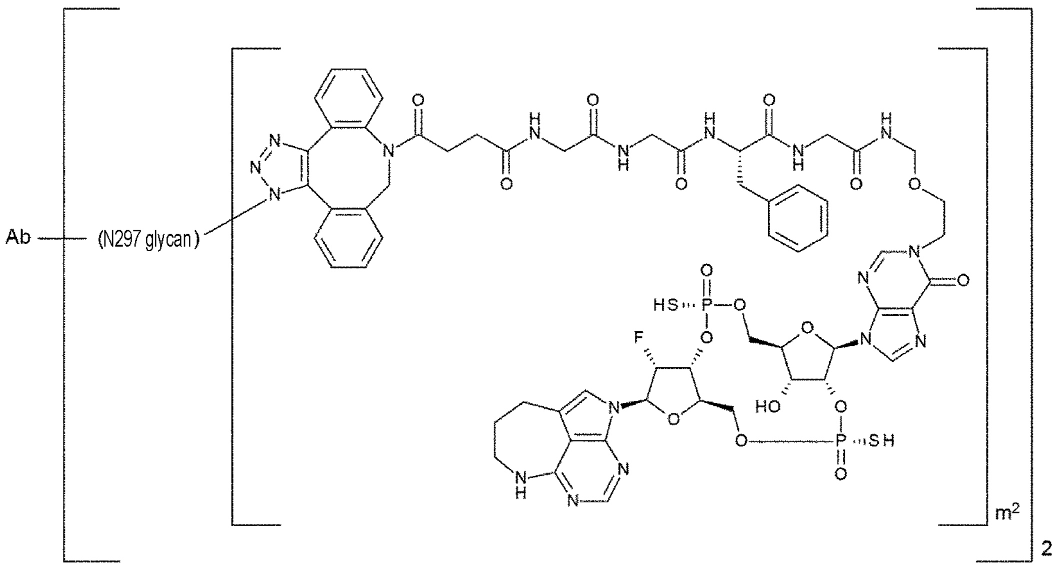

wherein Ab represents any one selected from the group consisting of the following:

an antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, and

an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein

the amino acid residues at positions 234, 235, and 265 (all according to EU numbering) of the Fc region of the antibody are Ala, Ala, and Gly, respectively,

wherein N297 glycan is represented by the following formula:

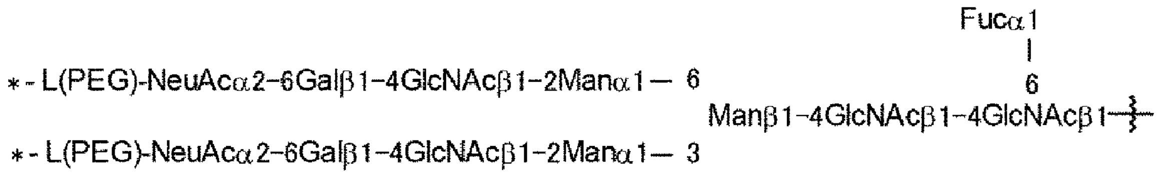

[N297-(Fuc)SG]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position on the 1,2,3-triazole ring of Lb in the linker L,
$n^5$ is 3, and
$m^2$ is 2.

**36.** An antibody-drug conjugate represented by the following formula:

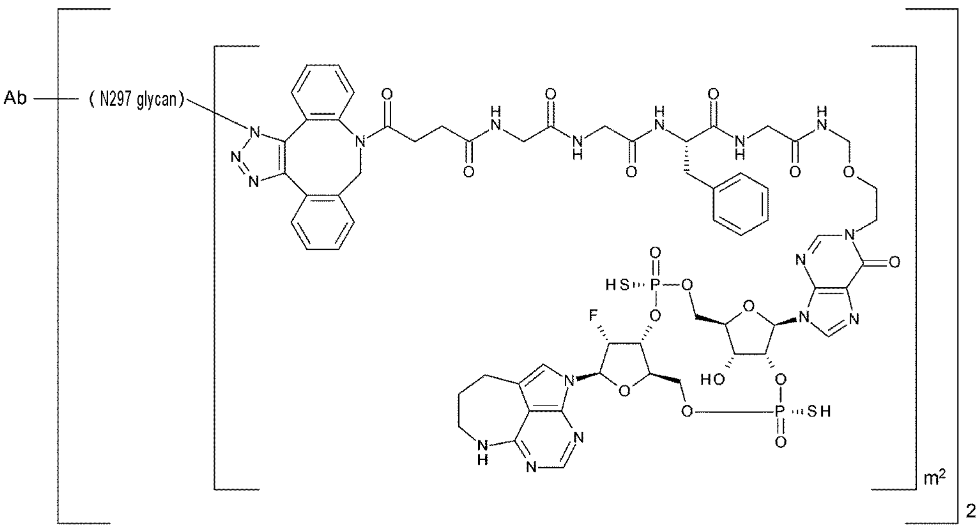

or

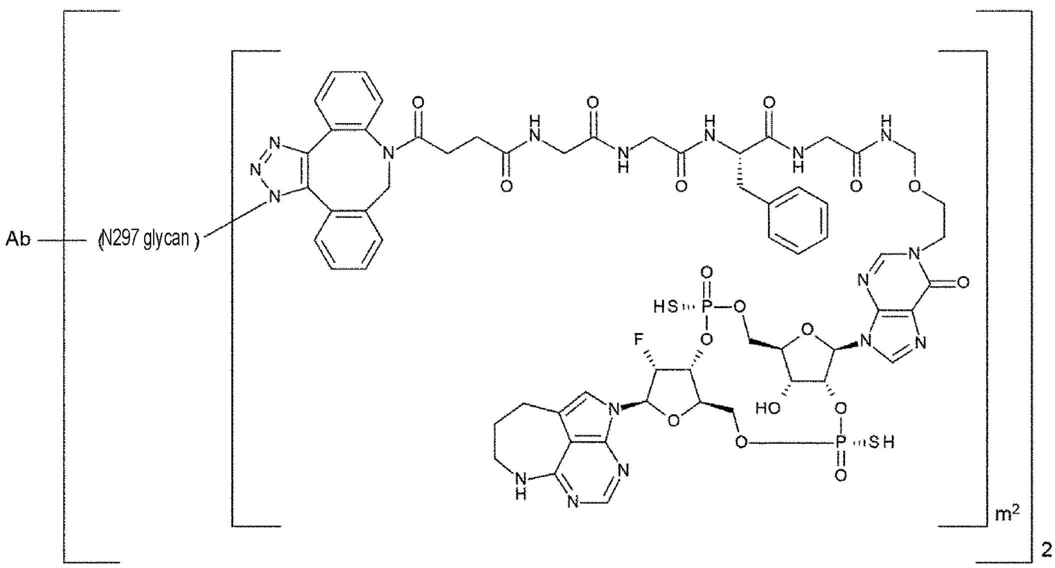

wherein Ab represents any one selected from the group consisting of the following:

an antibody comprising a light chain consisting of an amino acid sequence set forth in SEQ ID NO: 30 and a heavy chain consisting of an amino acid sequence set forth in SEQ ID NO: 32,
an antibody comprising a light chain consisting of an amino acid sequence set forth in SEQ ID NO: 30 and a heavy chain consisting of an amino acid sequence set forth in SEQ ID NO: 33,
an antibody comprising a light chain consisting of an amino acid sequence set forth in SEQ ID NO: 44 and a heavy chain consisting of an amino acid sequence set forth in SEQ ID NO: 46, and
any one of these antibodies comprising a heavy chain in which one or several amino acid residues are deleted at the carboxyl terminus thereof,
wherein N297 glycan is represented by the following formula:

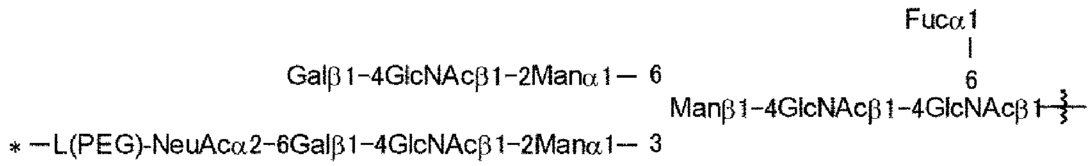

[N297-(Fuc)MSG1]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L,
$n^5$ is 3, and
$m^2$ is 1.

**37.** An antibody-drug conjugate represented by the following formula:

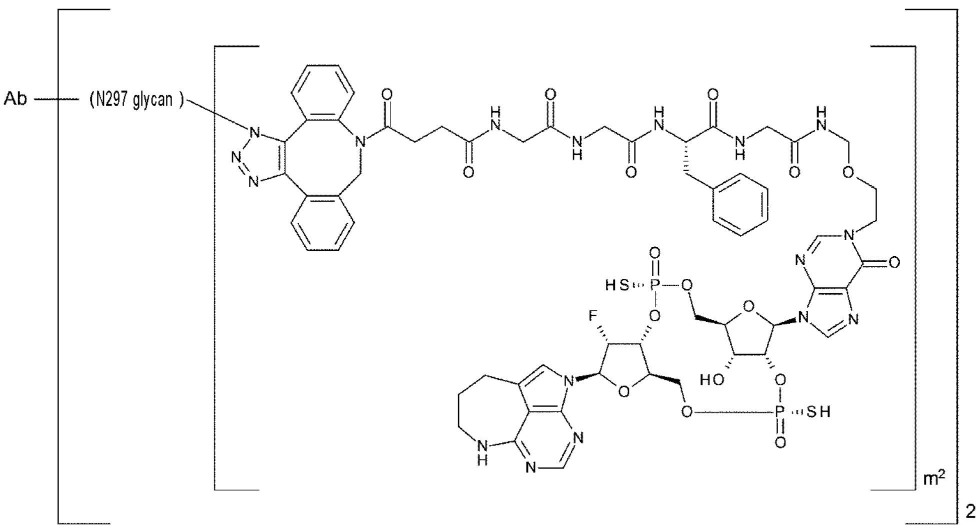

or

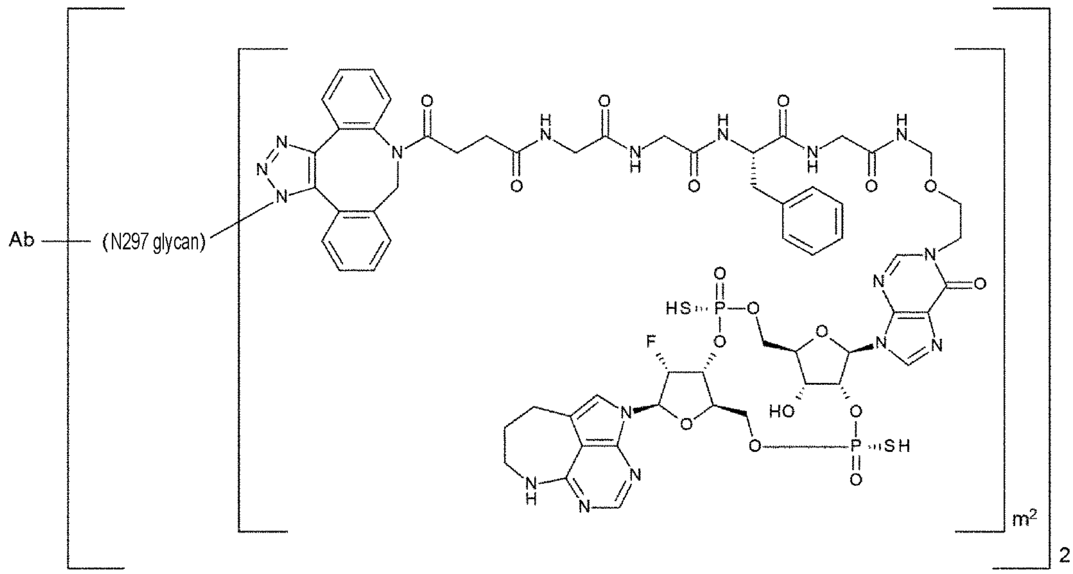

wherein Ab represents any one selected from the group consisting of the following:

an antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, and
an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein
the amino acid residues at positions 234, 235, and 265 (all according to EU numbering) of the Fc region of the antibody are Ala, Ala, and Gly, respectively, wherein
N297 glycan is represented by the following formula:

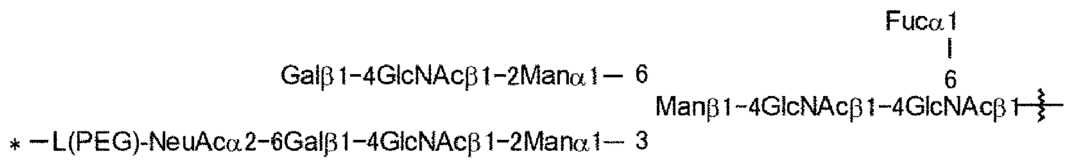

[N297-(Fuc)MSG1]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal of each of the 1-3 and 1-6 branched chains of β-Man in the N297 glycan,
the asterisk represents bonding to a nitrogen atom at the 1- or 3-position of the 1,2,3-triazole ring of Lb in the linker L,
$n^5$ is 3, and
$m^2$ is 1.

**38.** An antibody-drug conjugate represented by the following formula:

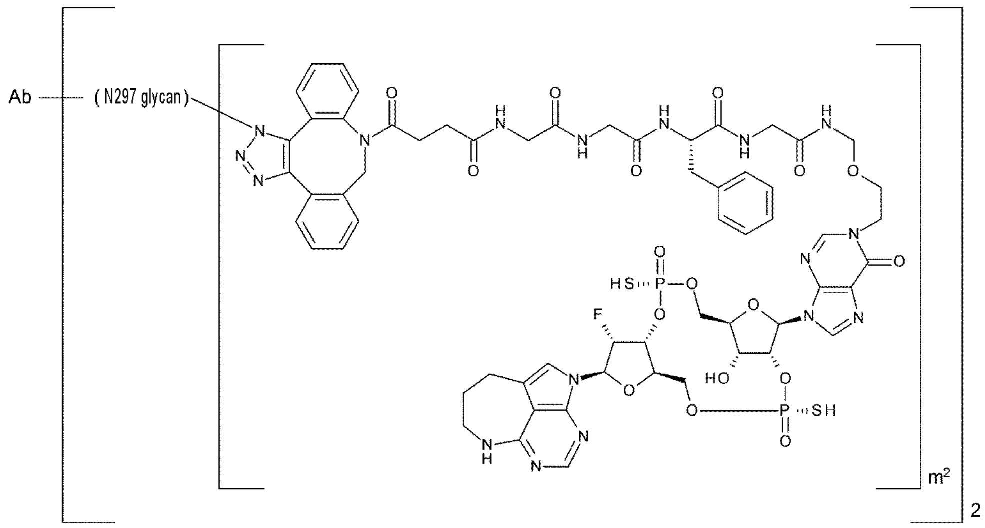

or

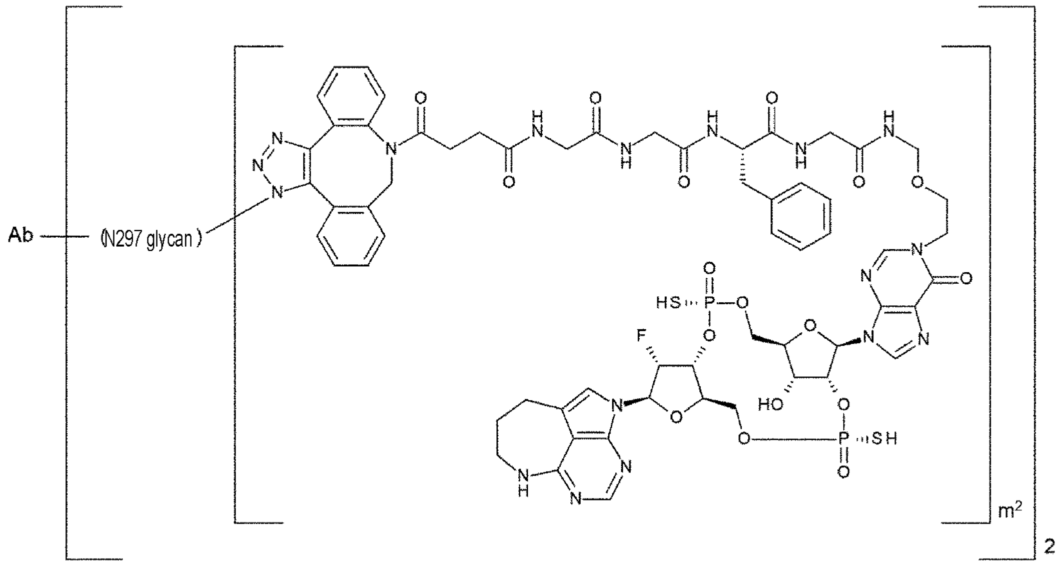

wherein Ab represents any one selected from the group consisting of the following:

an antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, and

an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein

the amino acid residues at positions 234, 235, and 265 (all according to EU numbering) of the Fc region of the antibody are Ala, Ala, and Gly, respectively,

wherein N297 glycan is represented by the following formula:

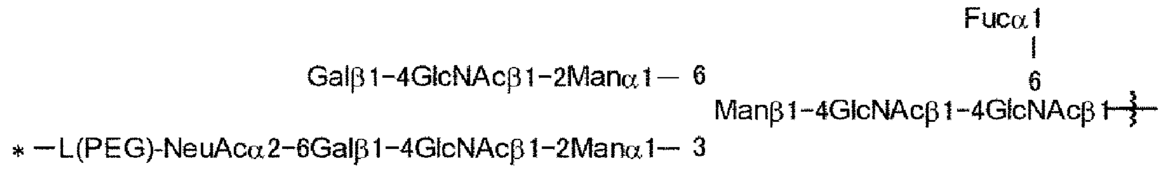

[N297-(Fuc)MSG1]

wherein the wavy line represents bonding to Asn297 of the antibody,
L(PEG) represents $-(CH_2-CH_2-O)n^5-CH_2-CH_2-NH-$, wherein the amino group at the right end of the L(PEG) is bound via an amide bond to a carboxyl group at the 2-position of a sialic acid at the non-reducing terminal on each of the 1-3 and 1-6 branched chains of β-Man in the N297 glycan,
the asterisk represents bonding to a nitrogen atom at the 1- or 3-position of the 1,2,3-triazole ring of Lb in the linker L,
$n^5$ is 3, and
$m^2$ is 1.

**39.** A STING agonist comprising the antibody-drug conjugate according to any one of claims 1 to 38.

**40.** A pharmaceutical composition comprising the antibody-drug conjugate according to any one of claims 1 to 38.

**41.** An anti-tumor agent comprising the antibody-drug conjugate according to any one of claims 1 to 38.

**42.** The anti-tumor agent according to claim 41, wherein the tumor is lung cancer, kidney cancer, urothelial cancer, colorectal cancer, prostate cancer, glioblastoma multiforme, ovarian cancer, pancreatic cancer, breast cancer, melanoma, liver cancer, hepatocellular carcinoma, bladder cancer, gastric cancer, esophageal cancer, endometrial cancer, testicular cancer, cervical cancer, placental choriocarcinoma, brain tumor, head and neck cancer, thyroid cancer, mesothelioma, gastrointestinal stromal tumor (GIST), gallbladder cancer, bile duct cancer, adrenal cancer, squamous-cell carcinoma, pharyngeal cancer, tongue cancer, auditory organ cancer, thymus cancer, small intestine cancer, leukemia, malignant lymphoma, plasmacytoma, myeloma, sarcoma, EGFR mutation-positive cancer, or cancer associated with a mutation in an EGFR signaling molecule.

**43.** A method for treating cancer, comprising administering any one selected from the group consisting of the antibody-drug conjugate according to any one of claims 1 to 38, the STING agonist according to claim 39, the pharmaceutical composition according to claim 40, and the anti-tumor agent according to claim 41 or 42.

**44.** The method according to claim 43, wherein the cancer is lung cancer, kidney cancer, urothelial cancer, colorectal cancer, prostate cancer, glioblastoma multiforme, ovarian cancer, pancreatic cancer, breast cancer, melanoma, liver cancer, hepatocellular carcinoma, bladder cancer, gastric cancer, esophageal cancer, endometrial cancer, testicular cancer, cervical cancer, placental choriocarcinoma, brain tumor, head and neck cancer, thyroid cancer, mesothelioma, gastrointestinal stromal tumor (GIST), gallbladder cancer, bile duct cancer, adrenal cancer, squamous-cell carcinoma, pharyngeal cancer, tongue cancer, auditory organ cancer, thymus cancer, small intestine cancer, leukemia, malignant lymphoma, plasmacytoma, myeloma, sarcoma, EGFR mutation-positive cancer, or cancer associated with a mutation in an EGFR signaling molecule.

**45.** (A) An antibody comprising a light chain comprising CDRL1 consisting of an amino acid sequence set forth in SEQ ID NO: 23, CDRL2 consisting of an amino acid sequence set forth in SEQ ID NO: 24, and CDRL3 consisting of an amino acid sequence set forth in SEQ ID NO: 25 and a heavy chain comprising CDRH1 consisting of an amino acid sequence set forth in SEQ ID NO: 26, CDRH2 consisting of an amino acid sequence set forth in SEQ ID NO: 27, and CDRH3 consisting of an amino acid sequence set forth in SEQ ID NO: 28, wherein the amino acid residues at positions 234, 235, and 265 (all according to EU numbering) of the Fc region of the antibody are Ala, Ala, and Gly, respectively; or (B) an antibody comprising a light chain comprising CDRL1 consisting of an amino acid sequence set forth in SEQ ID NO: 37, CDRL2 consisting of an amino acid sequence set forth in SEQ ID NO: 38, and CDRL3 consisting of an amino acid sequence set forth in SEQ ID NO: 39 and a heavy chain comprising CDRH1 consisting of an amino acid sequence set forth in SEQ ID NO: 40, CDRH2 consisting of an amino acid sequence set forth in SEQ ID NO: 41, and CDRH3 consisting of an amino acid sequence set forth in SEQ ID NO: 42, wherein the amino acid residues at positions 234, 235, and 265 (all according to EU numbering) of the Fc region of the antibody are Ala, Ala, and Gly, respectively.

**46.** (A) An antibody comprising a light chain comprising a light chain variable region consisting of an amino acid sequence set forth in SEQ ID NO: 29 and a heavy chain comprising a heavy chain variable region consisting of an amino acid sequence set forth in SEQ ID NO: 31, wherein the amino acid residues at positions 234, 235, and 265 (all according to EU numbering) of the Fc region of the antibody are Ala, Ala, and Gly, respectively; or
(B) an antibody comprising a light chain comprising a light chain variable region consisting of an amino acid sequence set forth in SEQ ID NO: 43 and a heavy chain comprising a heavy chain variable region consisting of an amino acid sequence set forth in SEQ ID NO: 45, wherein the amino acid residues at positions 234, 235, and 265 (all according to EU numbering) of the Fc region of the antibody are Ala, Ala, and Gly, respectively.

**47.** (A) An antibody comprising a light chain consisting of an amino acid sequence set forth in SEQ ID NO: 30 and a heavy chain consisting of an amino acid sequence set forth in SEQ ID NO: 32, or an antibody comprising a light chain consisting of an amino acid sequence set forth in SEQ ID NO: 30 and a heavy chain consisting of an amino acid sequence set forth in SEQ ID NO: 33;

(B) an antibody comprising a light chain consisting of an amino acid sequence set forth in SEQ ID NO: 44 and a heavy chain consisting of an amino acid sequence set forth in SEQ ID NO: 46; or
(C) an antibody comprising a heavy chain in which one or several amino acid residues are deleted at a carboxyl terminus in either one of the antibodies (A) and (B).

**48.** The pharmaceutical composition according to claim 40 or the anti-tumor agent according to claim 41 or 42, wherein the pharmaceutical composition or the anti-tumor agent is administered in combination with an additional medicine.

**49.** The pharmaceutical composition according to claim 40 or the anti-tumor agent according to claim 41 or 42, wherein the pharmaceutical composition or the anti-tumor agent comprises an additional medicine.

**50.** The method according to claim 43 or 44, wherein any one selected from the group consisting of the antibody-drug conjugate according to any one of claims 1 to 38, the STING agonist according to claim 39, the pharmaceutical composition according to claim 40, and the anti-tumor agent according to claim 41 or 42 is administered in combination with an additional medicine.

Fig. 1

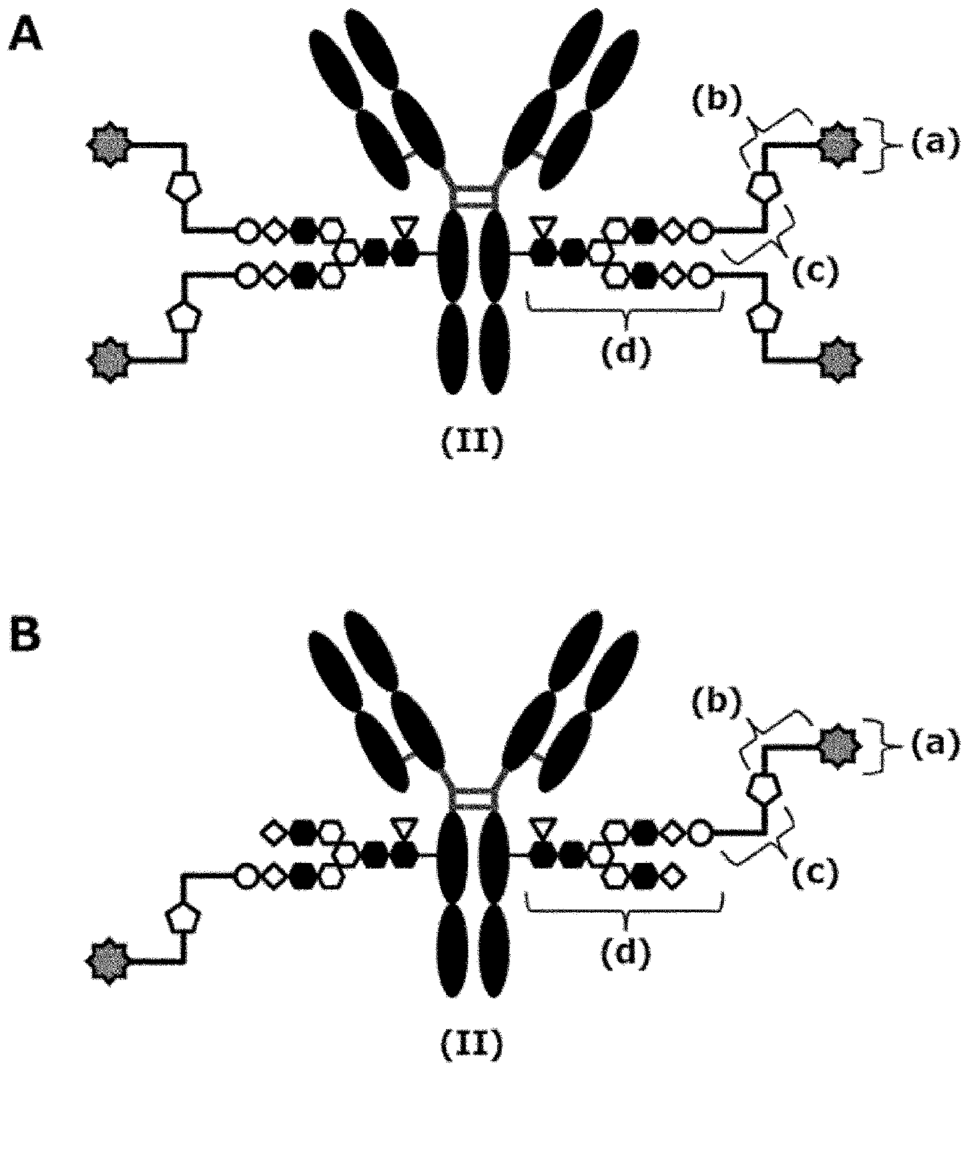

Fig. 2

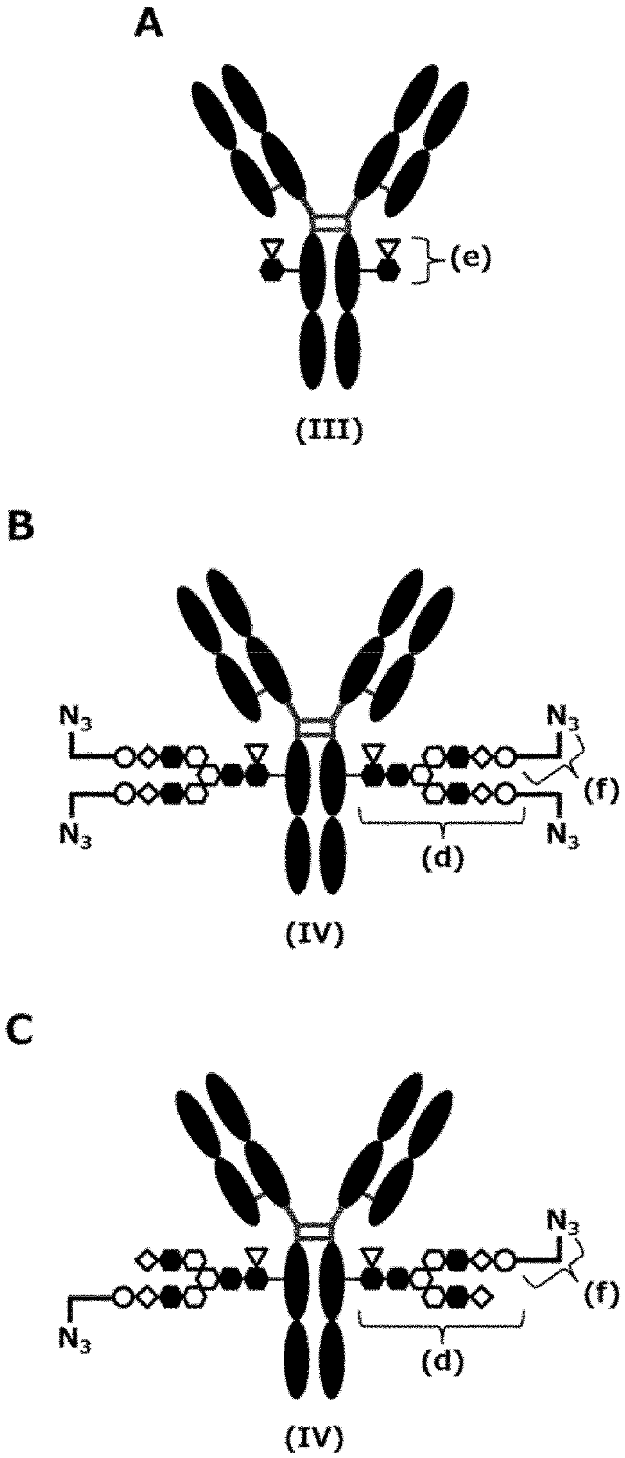

Fig. 3

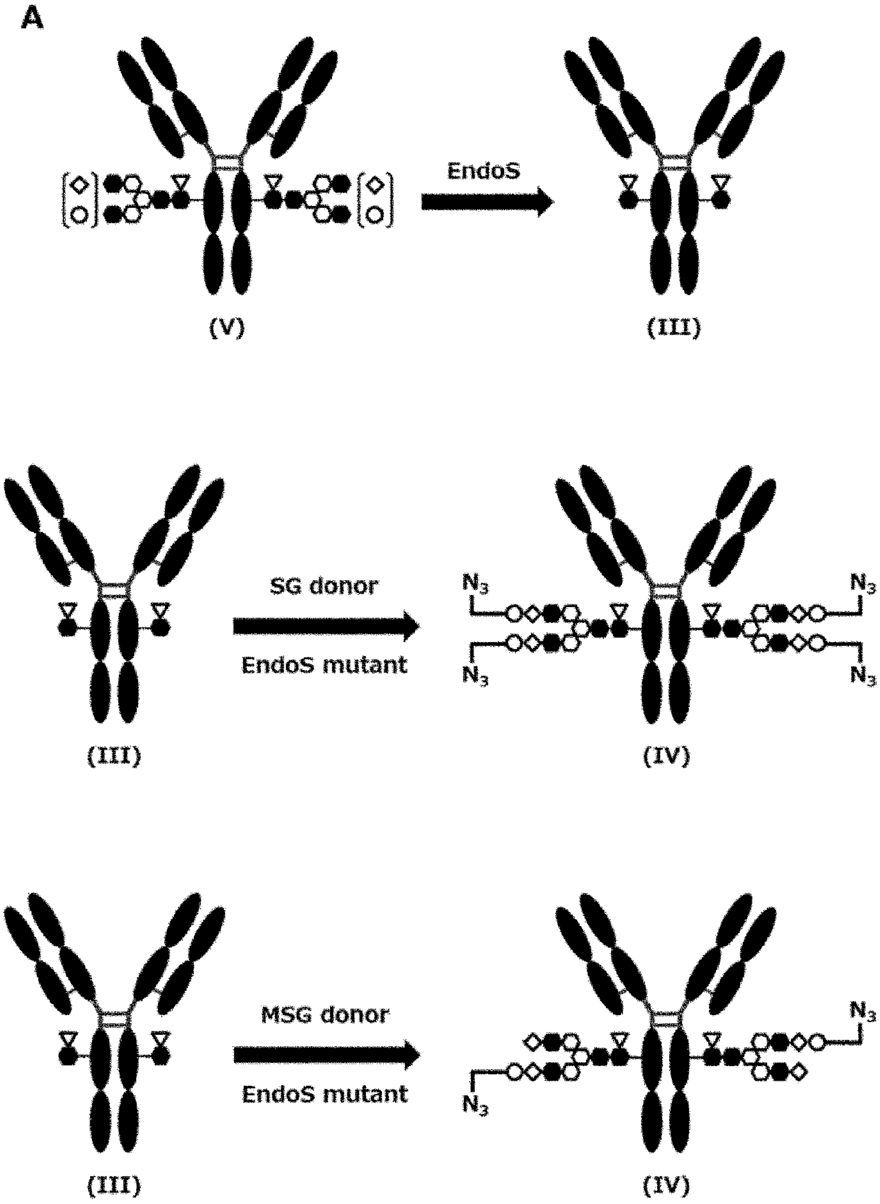
A
EndoS
(V)
(III)
B
SG donor
EndoS mutant
(III)
(IV)
C
MSG donor
EndoS mutant
(III)
(IV)

Fig. 4

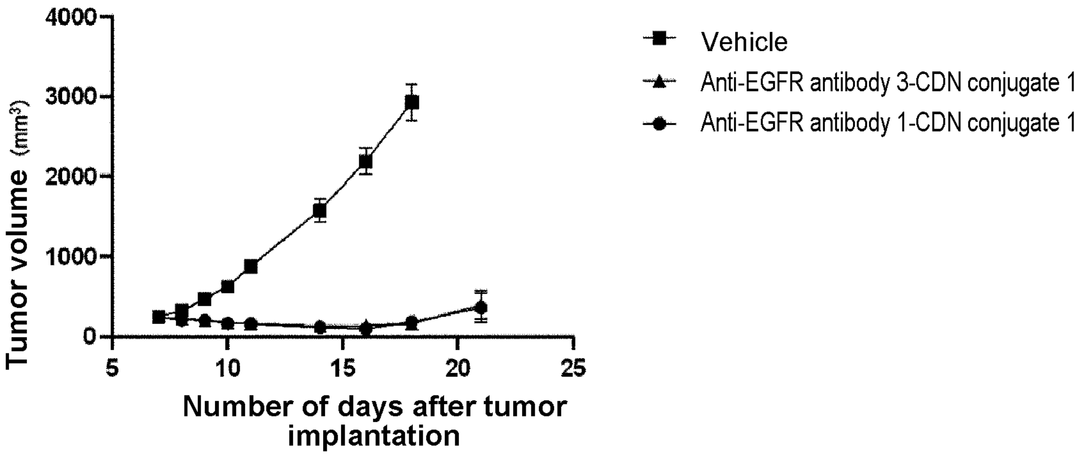
Tumor volume (mm3)
4000
3000
2000
1000
0
5
10
15
20
25
Number of days after tumor implantation
Vehicle
Anti-EGFR antibody 3-CDN conjugate 1
Anti-EGFR antibody 1-CDN conjugate 1

Fig. 5

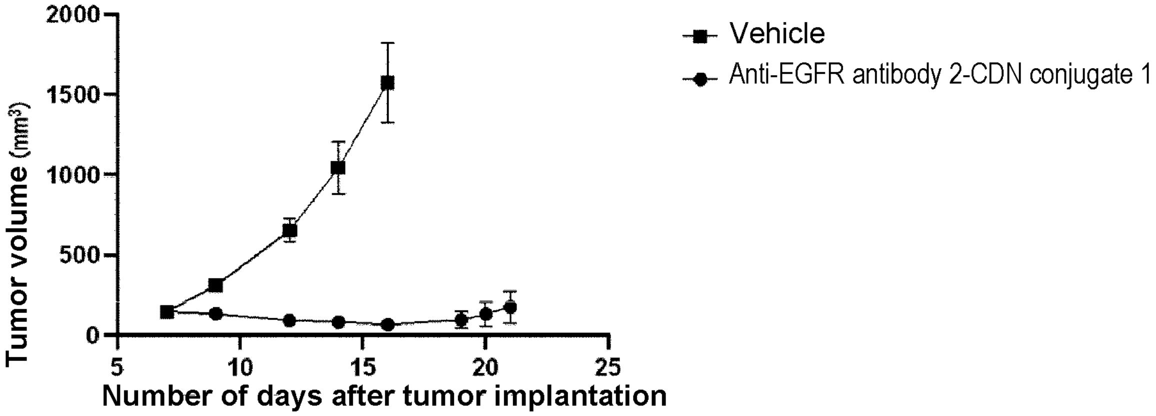


Fig. 6

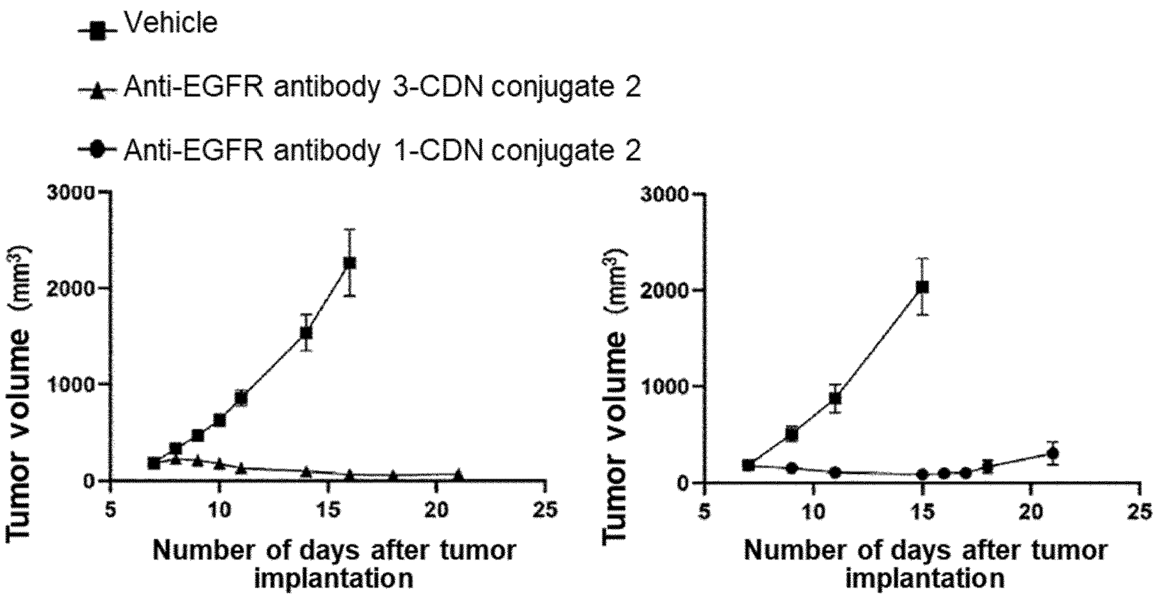


Fig. 7

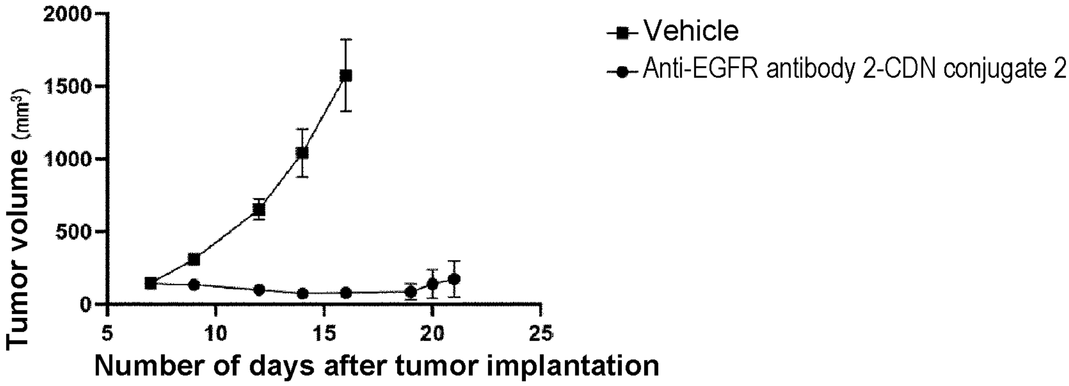

Fig. 8

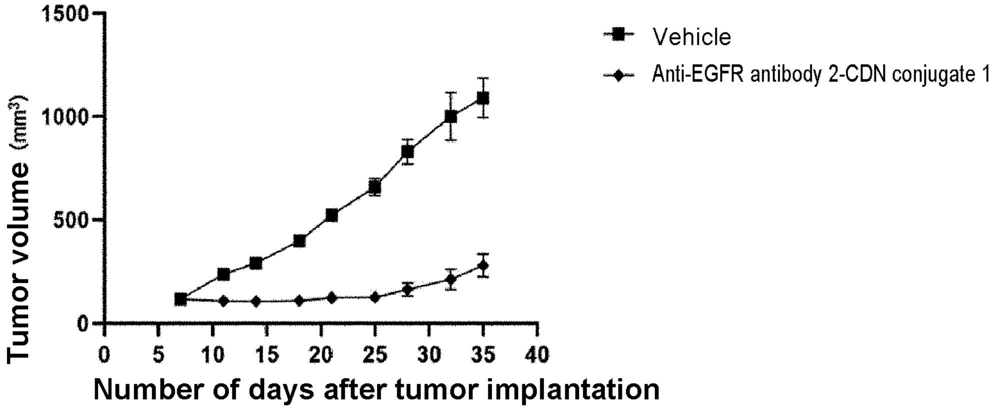

Vehicle
Anti-EGFR antibody 2-CDN conjugate 1
Tumor volume (mm3)
1500
1000
500
0
0 5 10 15 20 25 30 35 40
Number of days after tumor implantation

Fig. 9

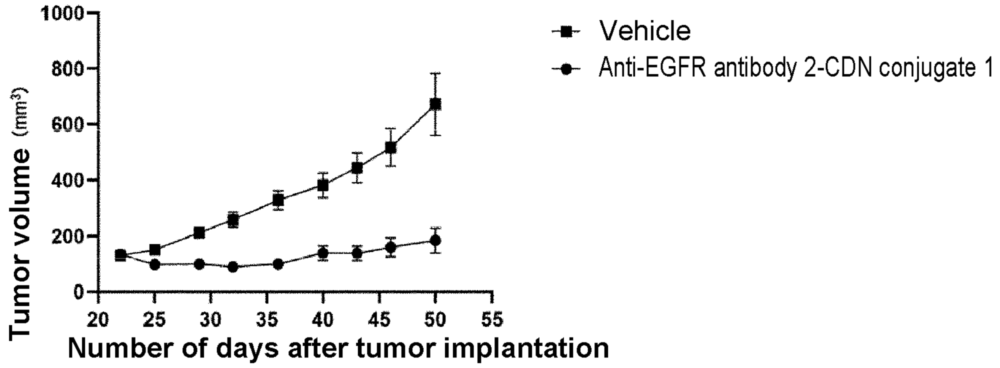


Fig. 10

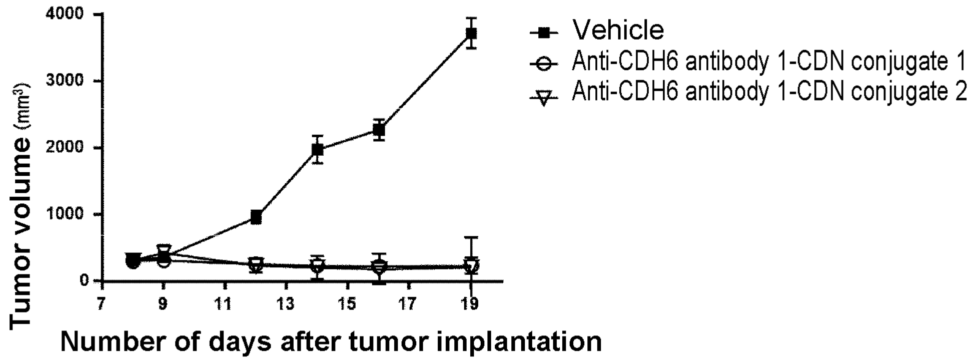


Fig. 11

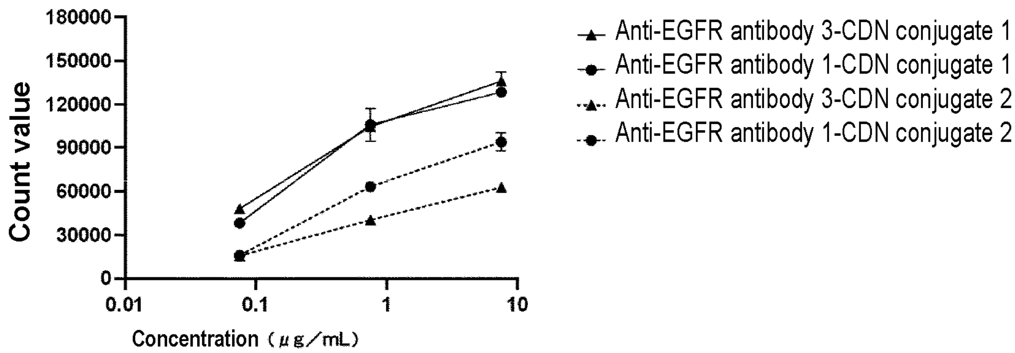


Fig. 12

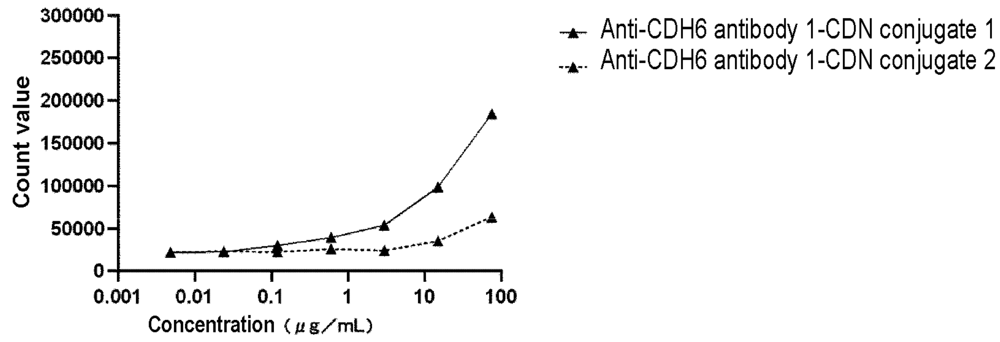

Fig. 13

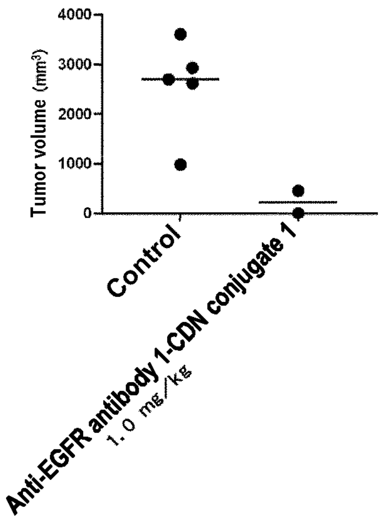


Fig. 14

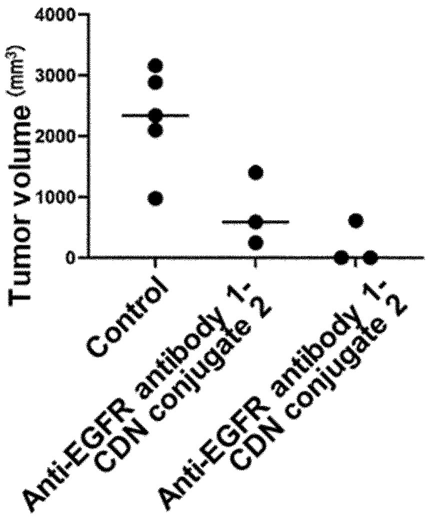


Fig. 15A

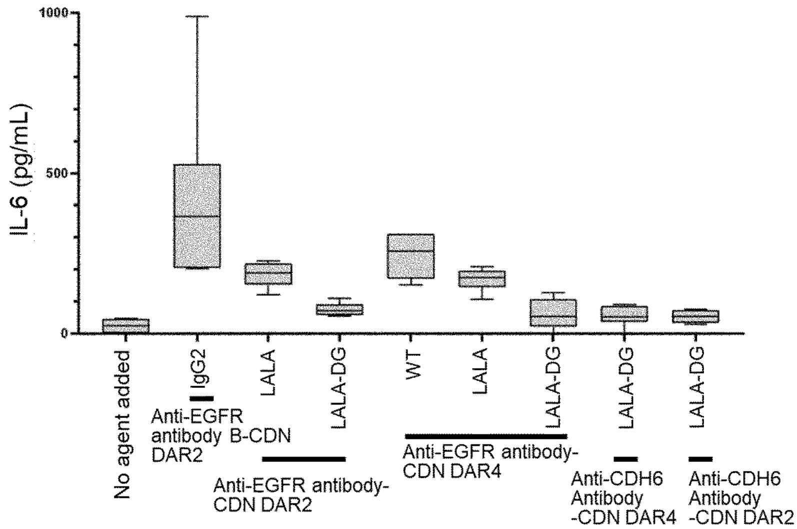

Fig. 15B

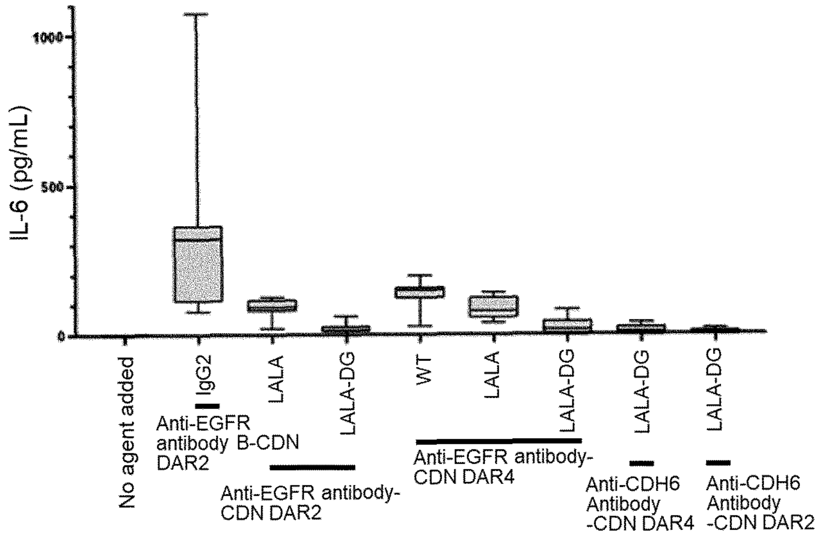


Fig. 16A

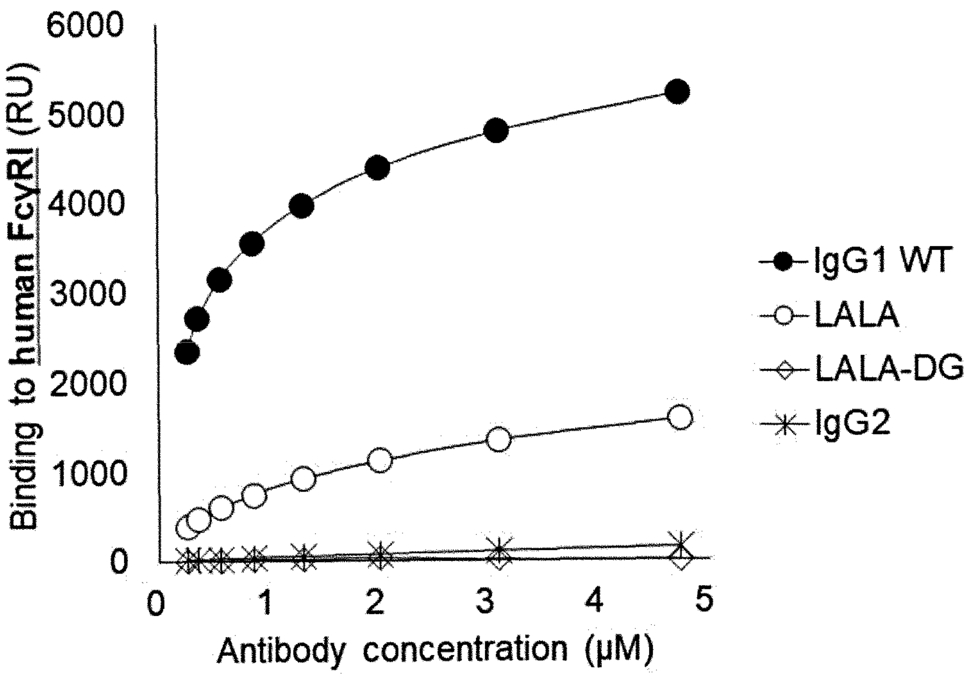

Fig. 16B

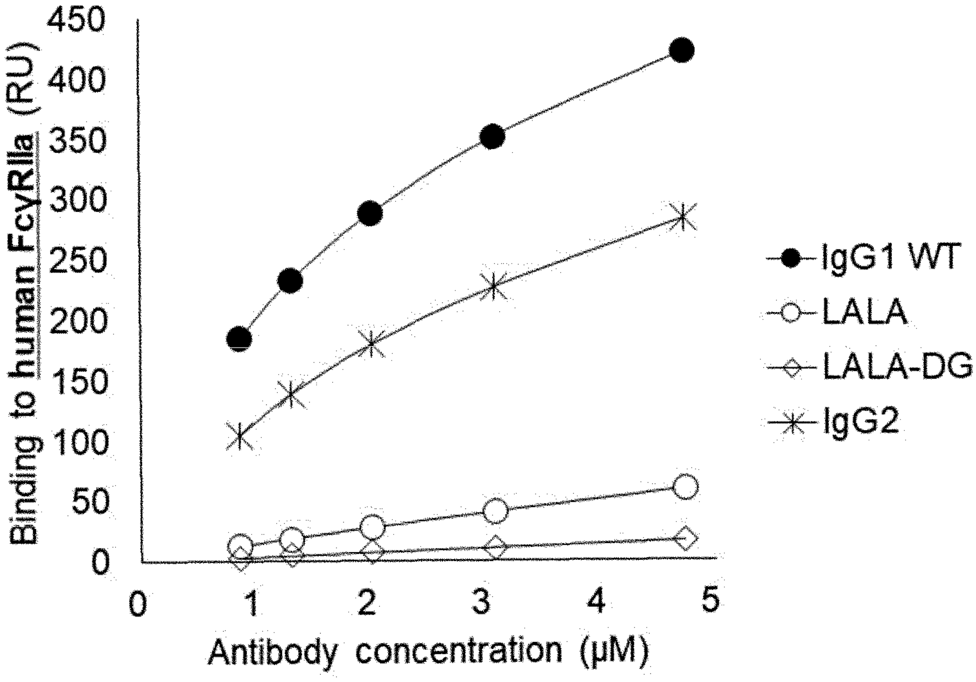


Fig. 16C

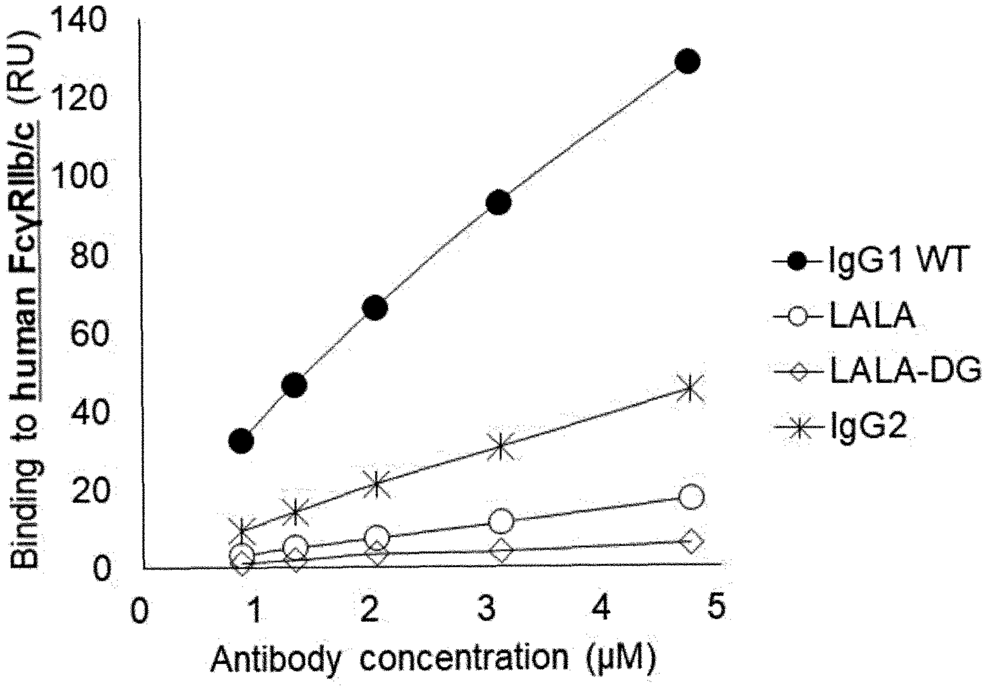

Fig. 16D

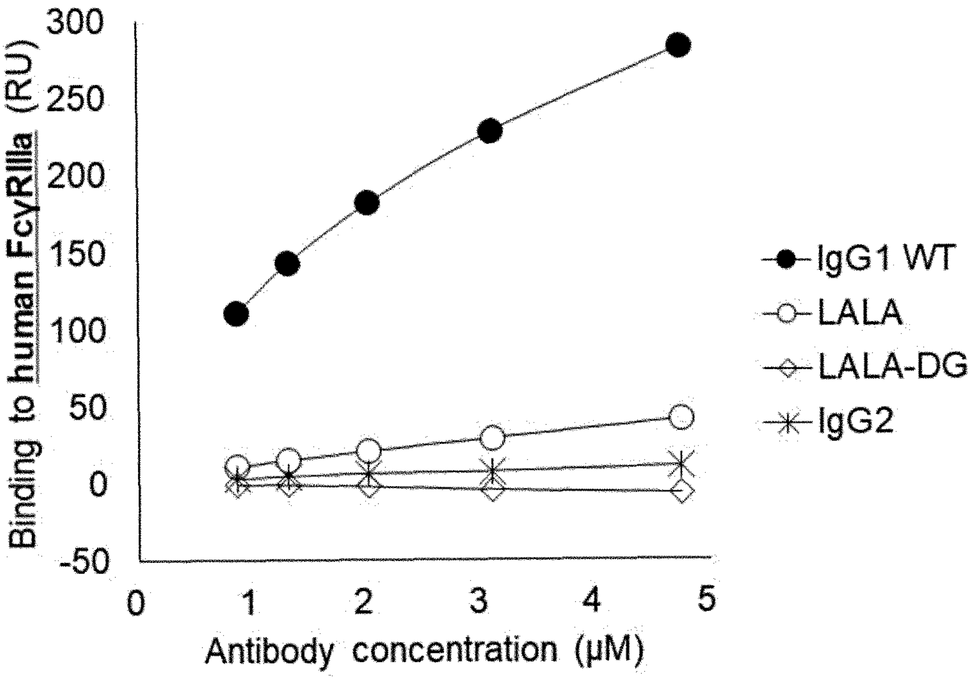


Fig. 16E

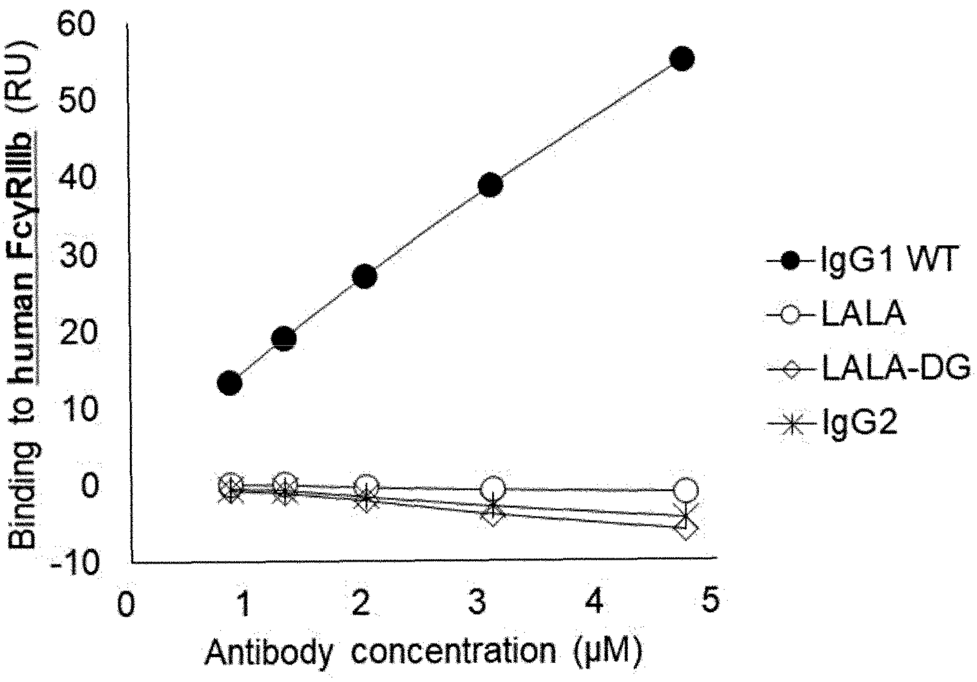

Fig. 17A

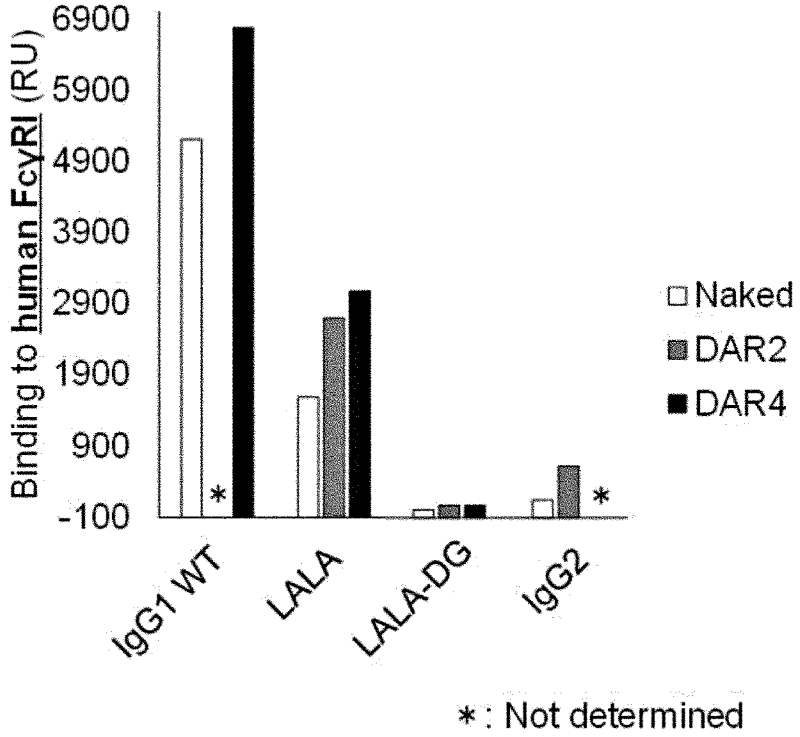


Fig. 17B

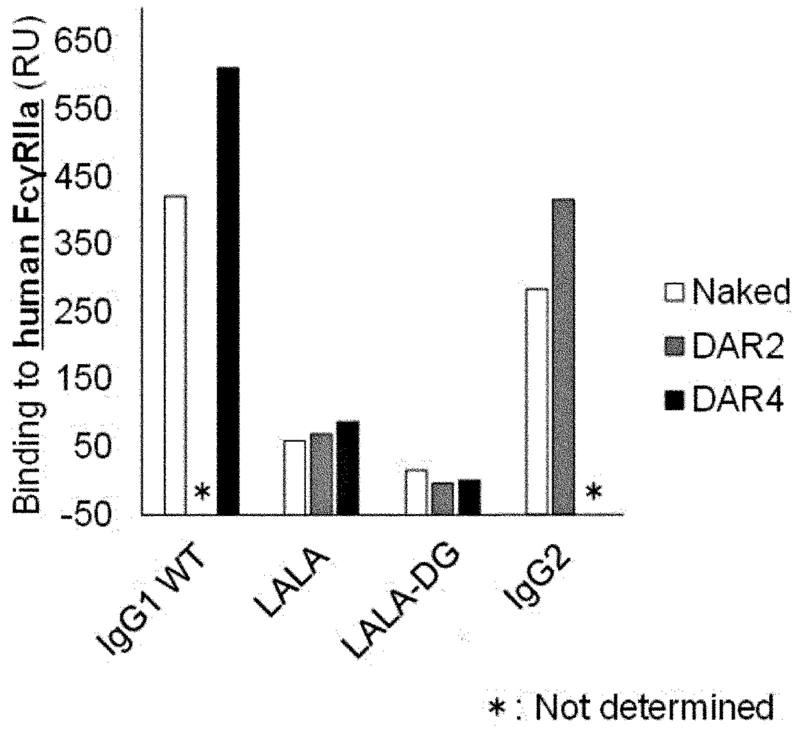

Fig. 17C

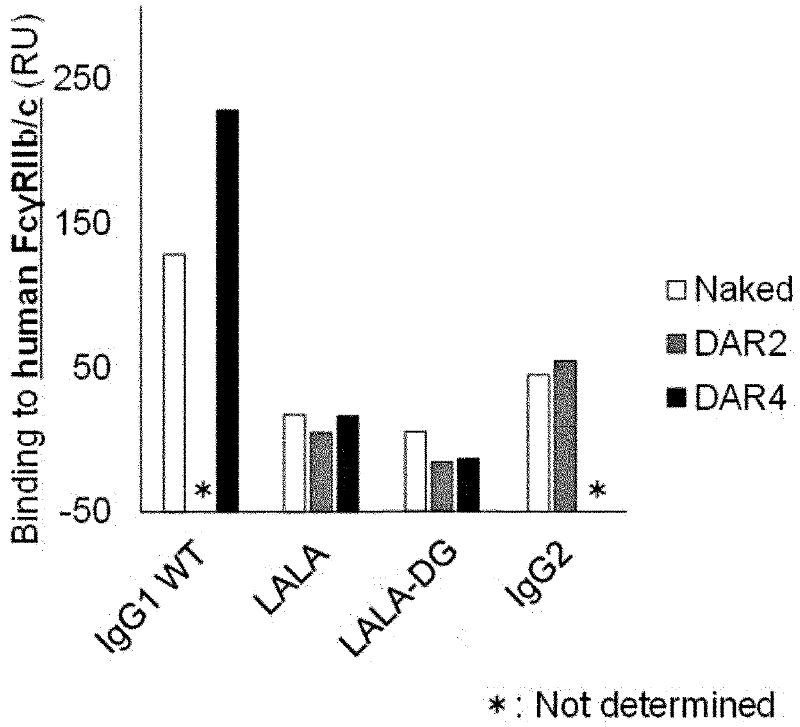


Fig. 17D

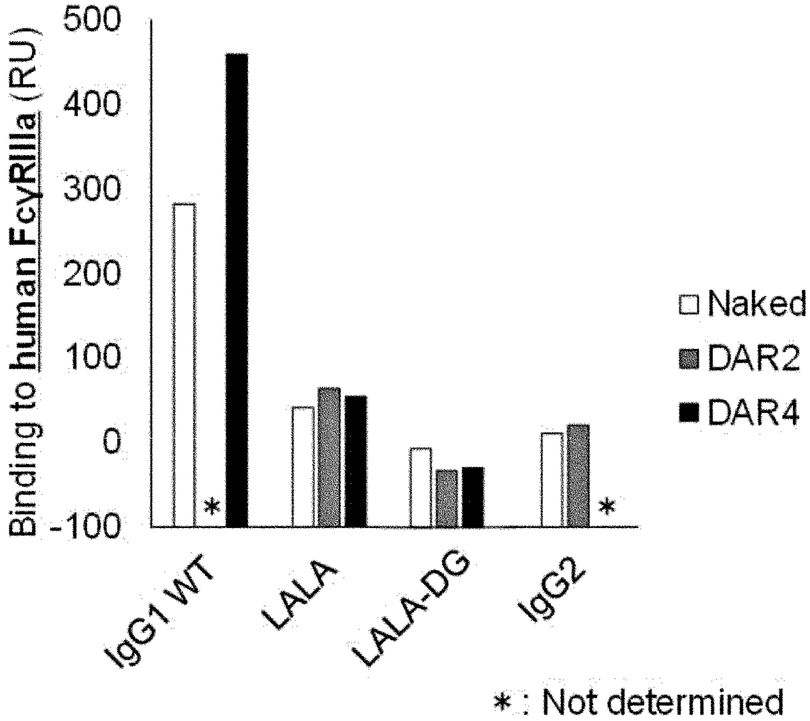

Fig. 17E

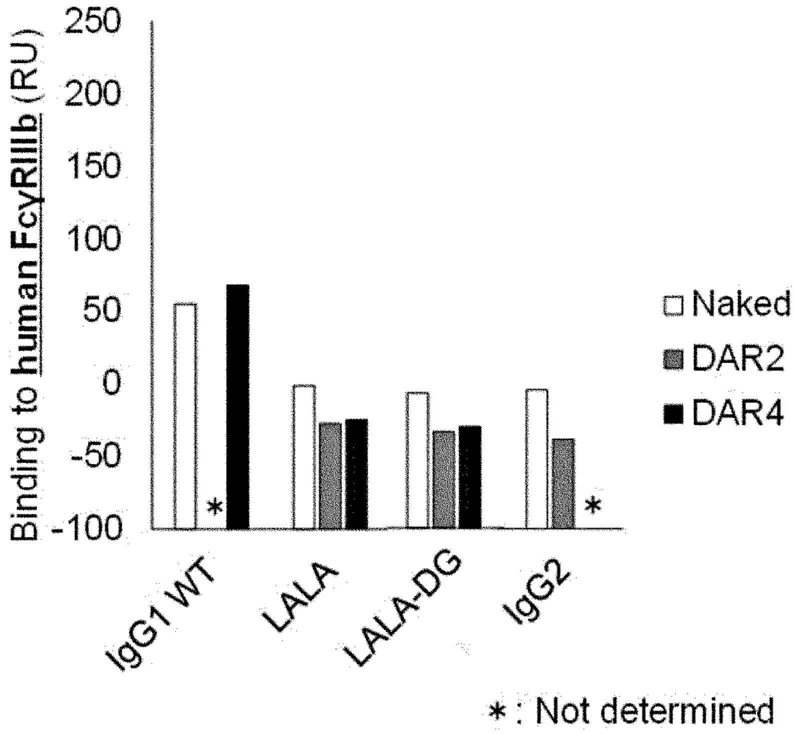


Fig. 18

| | CH2 $T_m$ (℃) | $\Delta T_m$ (℃) |
|---|---|---|
| IgG1 WT | 70.6 | — |
| LALA | 71.0 | + 0.4 |
| LALA-DG | 75.1 | + 4.5 |

# Fig. 19

(a) The amino acid sequence of human wild-type STING (SEQ ID NO: 1)

MPHSSLHPSIPCPRGHGAQKAALVLLSACLVTLWGLGEPPEHTLRYLVLHLASLQLGLLLNGVCSLAEELRHIHS
RYRGSYWRTVRACLGCPLRRGALLLLSIYFYYSLPNAVGPPFTWMLALLGLSQALNILLGLKGLAPAEISAVCEK
GNFNVAHGLAWSYYIGYLRLILPELQARIRTYNQHYNNLLRGAVSQRLYILLPLDCGVPDNLSMADPNIRFLDKL
PQQTGDRAGIKDRVYSNSIYELLENGQRAGTCVLEYATPLQTLFAMSQYSQAGFSREDRLEQAKLFCRTLEDILA
DAPESQNNCRLIAYQEPADDSSFSLSQEVLRHLRQEEKEEVTVGSLKTSAVPSTSTMSQEPELLISGMEKPLPLR
TDFS

(b) The amino acid sequence of human STING REF mutant (R232H) (SEQ ID NO: 3)

MPHSSLHPSIPCPRGHGAQKAALVLLSACLVTLWGLGEPPEHTLRYLVLHLASLQLGLLLNGVCSLAEELRHIHS
RYRGSYWRTVRACLGCPLRRGALLLLSIYFYYSLPNAVGPPFTWMLALLGLSQALNILLGLKGLAPAEISAVCEK
GNFNVAHGLAWSYYIGYLRLILPELQARIRTYNQHYNNLLRGAVSQRLYILLPLDCGVPDNLSMADPNIRFLDKL
PQQTGDHAGIKDRVYSNSIYELLENGQRAGTCVLEYATPLQTLFAMSQYSQAGFSREDRLEQAKLFCRTLEDILA
DAPESQNNCRLIAYQEPADDSSFSLSQEVLRHLRQEEKEEVTVGSLKTSAVPSTSTMSQEPELLISGMEKPLPLR
TDFS

(The site of R232H mutation is underlined.)

(c) The amino acid sequence of human STING HAQ mutant (R71H, G230A, R293Q)

MPHSSLHPSIPCPRGHGAQKAALVLLSACLVTLWGLGEPPEHTLRYLVLHLASLQLGLLLNGVCSLAEELHHIHS
RYRGSYWRTVRACLGCPLRRGALLLLSIYFYYSLPNAVGPPFTWMLALLGLSQALNILLGLKGLAPAEISAVCEK
GNFNVAHGLAWSYYIGYLRLILPELQARIRTYNQHYNNLLRGAVSQRLYILLPLDCGVPDNLSMADPNIRFLDKL
PQQTADRAGIKDRVYSNSIYELLENGQRAGTCVLEYATPLQTLFAMSQYSQAGFSREDRLEQAKLFCQTLEDILA
DAPESQNNCRLIAYQEPADDSSFSLSQEVLRHLRQEEKEEVTVGSLKTSAVPSTSTMSQEPELLISGMEKPLPLR
TDFS

(The three underlines indicate the sites of R71H, G230A, and R293Q mutations, respectively.)

Fig. 20

The amino acid sequence of CDRL1 of anti-EGFR antibodies (modified anti-EGFR antibodies 1 to 3) (SEQ ID NO: 23)

QASQDISNYLN

The amino acid sequence of CDRL2 of anti-EGFR antibodies (modified anti-EGFR antibodies 1 to 3) (SEQ ID NO: 24)

DASNLET

The amino acid sequence of CDRL3 of anti-EGFR antibodies (modified anti-EGFR antibodies 1 to 3) (SEQ ID NO: 25)

QHFDHLPLA

The amino acid sequence of CDRH1 of anti-EGFR antibodies (modified anti-EGFR antibodies 1 to 3) (SEQ ID NO: 26)

SGDYYWT

The amino acid sequence of CDRH2 of anti-EGFR antibodies (modified anti-EGFR antibodies 1 to 3) (SEQ ID NO: 27)

HIYYSGNTNYNPSLKS

The amino acid sequence of CDRH3 of anti-EGFR antibodies (modified anti-EGFR antibodies 1 to 3) (SEQ ID NO: 28)

DRVTGAFDI

# Fig. 21-1

The amino acid sequence of the light chain variable region of anti-EGFR antibodies (modified anti-EGFR antibodies 1 to 3) (SEQ ID NO: 29)

DIQMTQSPSSLSASVGDRVTITCQASQDISNYLNWYQQKPGKAPKLLIYDASNLETGVPS

RFSGSGSGTDFTFTISSLQPEDIATYFCQHFDHLPLAFGGGTKVEIKR

The amino acid sequence of the light chain of anti-EGFR antibodies (modified anti-EGFR antibodies 1 to 3) (SEQ ID NO: 30)

DIQMTQSPSSLSASVGDRVTITCQASQDISNYLNWYQQKPGKAPKLLIYDASNLETGVPS

RFSGSGSGTDFTFTISSLQPEDIATYFCQHFDHLPLAFGGGTKVEIKRTVAAPSVFIFPP

SDEQLKSGTASVVCLLNNFYPREAKVQWKVDNALQSGNSQESVTEQDSKDSTYSLSSTLT

LSKADYEKHKVYACEVTHQGLSSPVTKSFNRGEC

The amino acid sequence of the heavy chain variable region of anti-EGFR antibodies (modified anti-EGFR antibodies 1 to 3) (SEQ ID NO: 31)

QVQLQESGPGLVKPSETLSLTCTVSGGSVSSGDYYWTWIRQSPGKGLEWIGHIYYSGNTN

YNPSLKSRLTISIDTSKTQFSLKLSSVTAADTAIYYCVRDRVTGAFDIWGQGTMVTVSS

# Fig. 21-2

The amino acid sequence of the heavy chain of a LALA-DG mutant anti-EGFR antibody (modified anti-EGFR antibody 1) (SEQ ID NO: 32)

QVQLQESGPGLVKPSETLSLTCTVSGGSVSSGDYYWTWIRQSPGKGLEWIGHIYYSGNTN
YNPSLKSRLTISIDTSKTQFSLKLSSVTAADTAIYYCVRDRVTGAFDIWGQGTMVTVSSA
STKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKRVEPKSCDKTHTCPPCPAPEAAGGP
SVFLFPPKPKDTLMISRTPEVTCVVVGVSHEDPEVKFNWYVDGVEVHNAKTKPREEQYNS
TYRVVSVLTVLHQDWLNGKEYKCKVSNKALPAPIEKTISKAKGQPREPQVYTLPPSREEM
TKNQVSLTCLVKGFYPSDIAVEWESNGQPENNYKTTPPVLDSDGSFFLYSKLTVDKSRWQ
QGNVFSCSVMHEALHNHYTQKSLSLSPGK

The amino acid sequence of the heavy chain of a LALA-DG mutant anti-EGFR antibody (modified anti-EGFR antibody 2) (SEQ ID NO: 33)

QVQLQESGPGLVKPSETLSLTCTVSGGSVSSGDYYWTWIRQSPGKGLEWIGHIYYSGNTN
YNPSLKSRLTISIDTSKTQFSLKLSSVTAADTAIYYCVRDRVTGAFDIWGQGTMVTVSSA
STKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPEAAGGP
SVFLFPPKPKDTLMISRTPEVTCVVVGVSHEDPEVKFNWYVDGVEVHNAKTKPREEQYNS
TYRVVSVLTVLHQDWLNGKEYKCKVSNKALPAPIEKTISKAKGQPREPQVYTLPPSREEM
TKNQVSLTCLVKGFYPSDIAVEWESNGQPENNYKTTPPVLDSDGSFFLYSKLTVDKSRWQ
QGNVFSCSVMHEALHNHYTQKSLSLSPGK

The amino acid sequence of the heavy chain of anti-EGFR antibody A (SEQ ID NO: 34)

QVQLQESGPGLVKPSETLSLTCTVSGGSVSSGDYYWTWIRQSPGKGLEWIGHIYYSGNTN
YNPSLKSRLTISIDTSKTQFSLKLSSVTAADTAIYYCVRDRVTGAFDIWGQGTMVTVSS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK

# Fig. 21-3

The amino acid sequence of the heavy chain of anti-EGFR antibody B (Vectibix) (SEQ ID NO: 35)

QVQLQESGPGLVKPSETLSLTCTVSGGSVSSGDYYWTWIRQSPGKGLEWIGHIYYSGNTN
YNPSLKSRLTISIDTSKTQFSLKLSSVTAADTAIYYCVRDRVTGAFDIWGQGTMVTVSSA
STKGPSVFPLAPCSRSTSESTAALGCLVKDYFPEPVTVSWNSGALTSGVHTFPAVLQSSG
LYSLSSVVTVPSSNFGTQTYTCNVDHKPSNTKVDKTVERKCCVECPPCPAPPVAGPSVFL
FPPKPKDTLMISRTPEVTCVVVDVSHEDPEVQFNWYVDGVEVHNAKTKPREEQFNSTFRV
VSVLTVVHQDWLNGKEYKCKVSNKGLPAPIEKTISKTKGQPREPQVYTLPPSREEMTKNQ
VSLTCLVKGFYPSDIAVEWESNGQPENNYKTTPPMLDSDGSFFLYSKLTVDKSRWQQGNV
FSCSVMHEALHNHYTQKSLSLSPGK

The amino acid sequence of the heavy chain of a LALA mutant anti-EGFR antibody (modified anti-EGFR antibody 3) (SEQ ID NO: 36)

QVQLQESGPGLVKPSETLSLTCTVSGGSVSSGDYYWTWIRQSPGKGLEWIGHIYYSGNTN
YNPSLKSRLTISIDTSKTQFSLKLSSVTAADTAIYYCVRDRVTGAFDIWGQGTMVTVSSA
STKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKRVEPKSCDKTHTCPPCPAPEAAGGP
SVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEM
TKNQVSLTCLVKGFYPSDIAVEWESNGQPENNYKTTPPVLDSDGSFFLYSKLTVDKSRWQ
QGNVFSCSVMHEALHNHYTQKSLSLSPGK

Fig. 22

The amino acid sequence of CDRL1 of anti-CDH6 antibody (modified anti-CDH6 antibody 1) (SEQ ID NO: 37)

KASQNIYKNLA

The amino acid sequence of CDRL2 of anti-CDH6 antibody (modified anti-CDH6 antibody 1) (SEQ ID NO: 38)

DANTLQT

The amino acid sequence of CDRL3 of anti-CDH6 antibody (modified anti-CDH6 antibody 1) (SEQ ID NO: 39)

QQYYSGWA

The amino acid sequence of CDRH1 of anti-CDH6 antibody (modified anti-CDH6 antibody 1) (SEQ ID NO: 40)

RNFMH

The amino acid sequence of CDRH2 of anti-CDH6 antibody (modified anti-CDH6 antibody 1) (SEQ ID NO: 41)

WIYPGDGETEYAQKFQG

The amino acid sequence of CDRH3 of anti-CDH6 antibody (modified anti-CDH6 antibody 1) (SEQ ID NO: 42)

GVYGGFAGGYFDF

# Fig. 23

The amino acid sequence of the light chain variable region of anti-CDH6 antibody (modified anti-CDH6 antibody 1) (SEQ ID NO: 43)

DIQMTQSPSSLSASVGDRVTITCKASQNIYKNLAWYQQKPGKAPKLLIYDANTLQTGVPS
RFSGSGSGSDFTLTISSLQPEDFATYFCQQYYSGWAFGQGTKVEIKR

The amino acid sequence of the light chain of anti-CDH6 antibody (modified anti-CDH6 antibody 1) (SEQ ID NO: 44)

DIQMTQSPSSLSASVGDRVTITCKASQNIYKNLAWYQQKPGKAPKLLIYDANTLQTGVPS
RFSGSGSGSDFTLTISSLQPEDFATYFCQQYYSGWAFGQGTKVEIKRTVAAPSVFIFPPS
DEQLKSGTASVVCLLNNFYPREAKVQWKVDNALQSGNSQESVTEQDSKDSTYSLSSTLTL
SKADYEKHKVYACEVTHQGLSSPVTKSFNRGEC

The amino acid sequence of the heavy chain variable region of anti-CDH6 antibody (modified anti-CDH6 antibody 1) (SEQ ID NO: 45)

EVQLVQSGAEVKKPGASVKVSCKASGYTFTRNFMHWVRQAPGQGLEWMGWIYPGDGETEY
AQKFQGRVTITADTSTSTAYMELSSLRSEDTAVYYCARGVYGGFAGGYFDFWGQGTLVTV
SS

The amino acid sequence of the heavy chain of a LALA-DG mutant anti-CDH6 antibody (modified anti-CDH6 antibody 1) (SEQ ID NO: 46)

EVQLVQSGAEVKKPGASVKVSCKASGYTFTRNFMHWVRQAPGQGLEWMGWIYPGDGETEY
AQKFQGRVTITADTSTSTAYMELSSLRSEDTAVYYCARGVYGGFAGGYFDFWGQGTLVTV
SSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAA
GGPSVFLFPPKPKDTLMISRTPEVTCVVVGVSHEDPEVKFNWYVDGVEVHNAKTKPREEQ
YNSTYRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKLTVDKS
RWQQGNVFSCSVMHEALHNHYTQKSLSLSPGK

Fig. 24

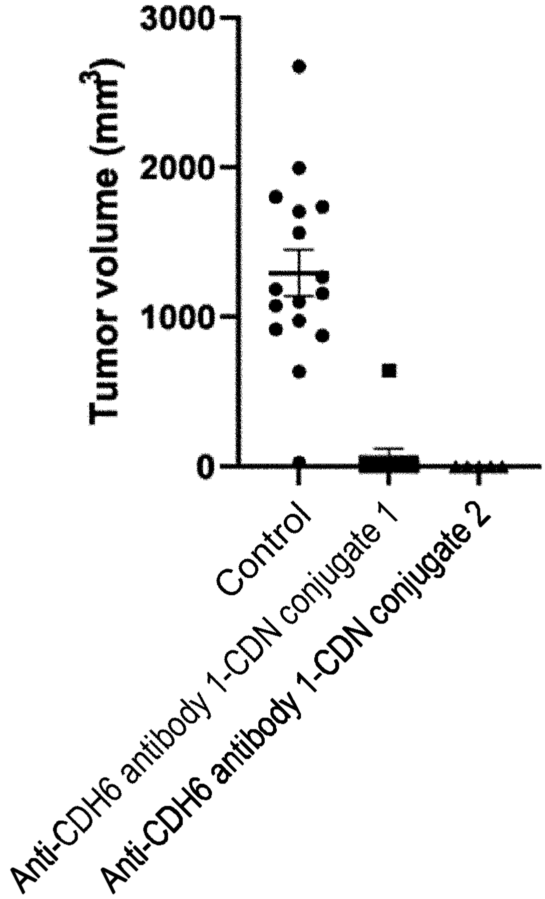
Tumor volume (mm$^3$)
3000
2000
1000
0
Control
Anti-CDH6 antibody 1-CDN conjugate 1
Anti-CDH6 antibody 1-CDN conjugate 2

Fig. 25

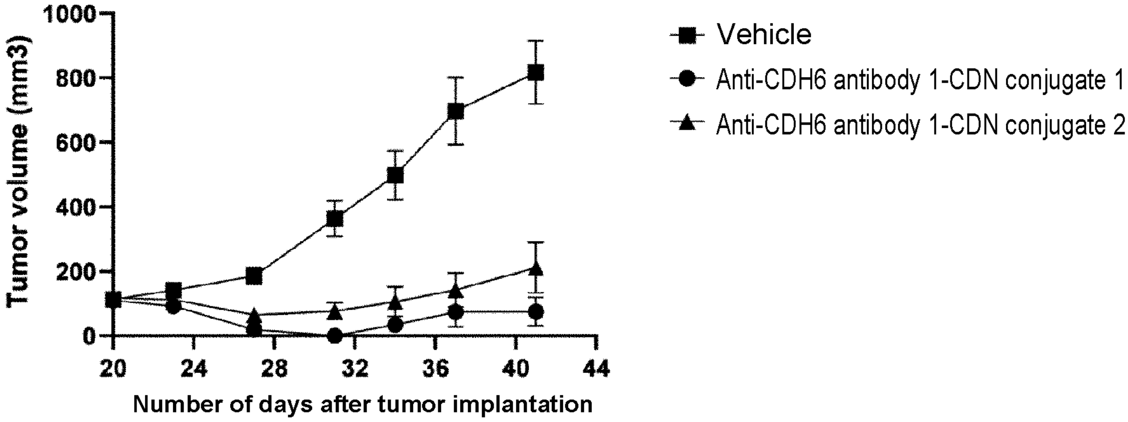
Tumor volume (mm3)
1000
800
600
400
200
0
20
24
28
32
36
40
44
Number of days after tumor implantation
Vehicle
Anti-CDH6 antibody 1-CDN conjugate 1
Anti-CDH6 antibody 1-CDN conjugate 2

Fig. 26

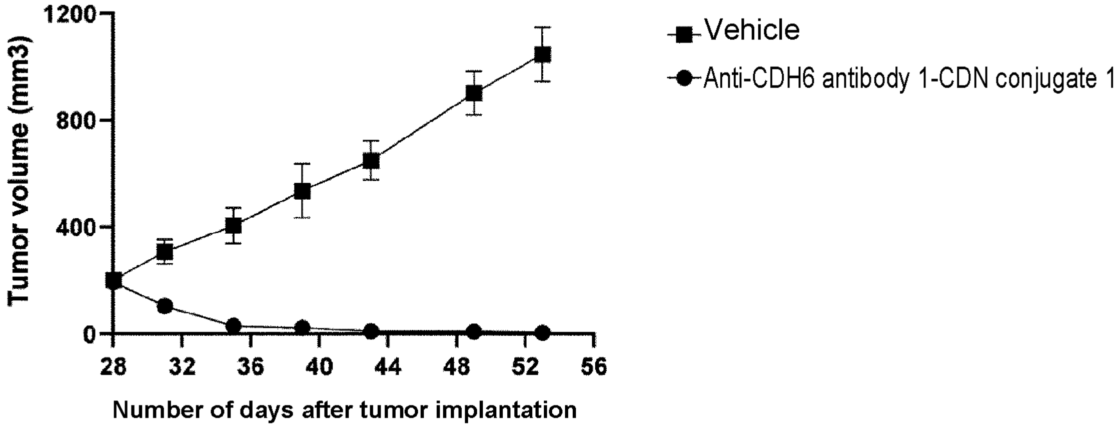


Fig. 27

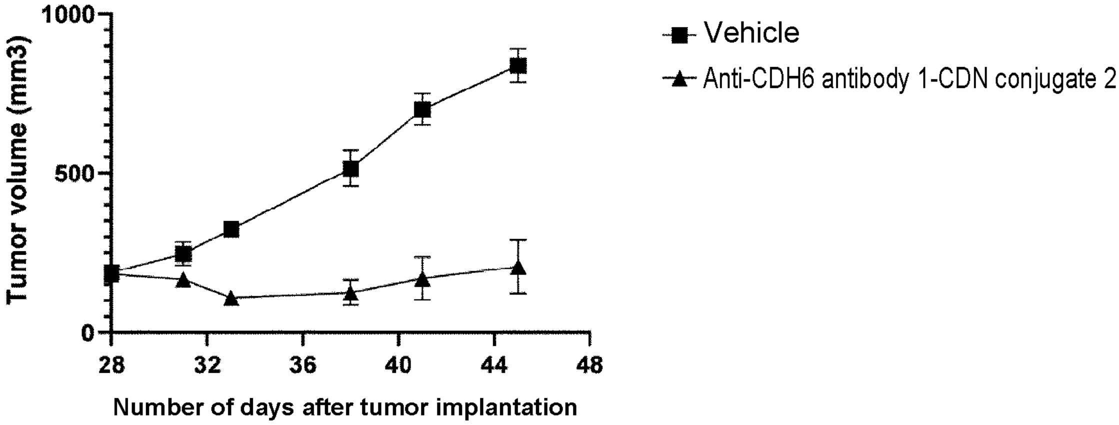


Fig. 28

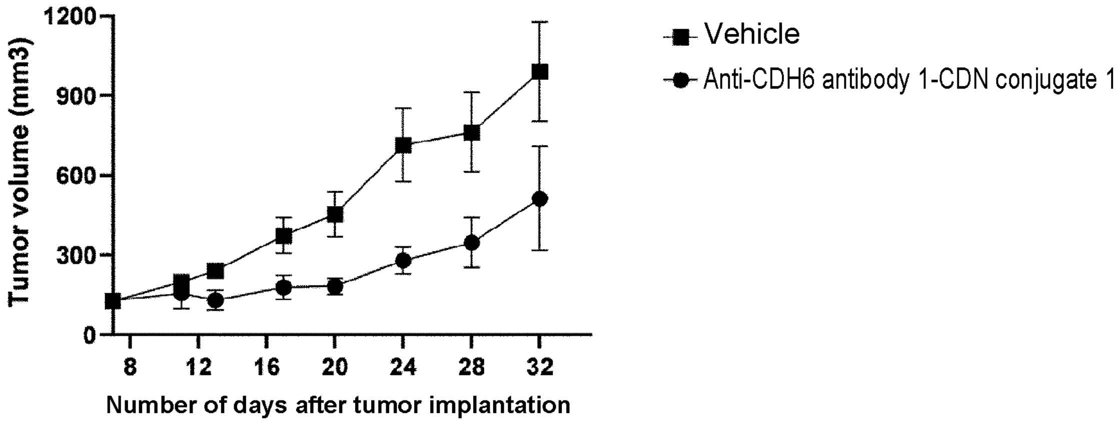

Fig. 29

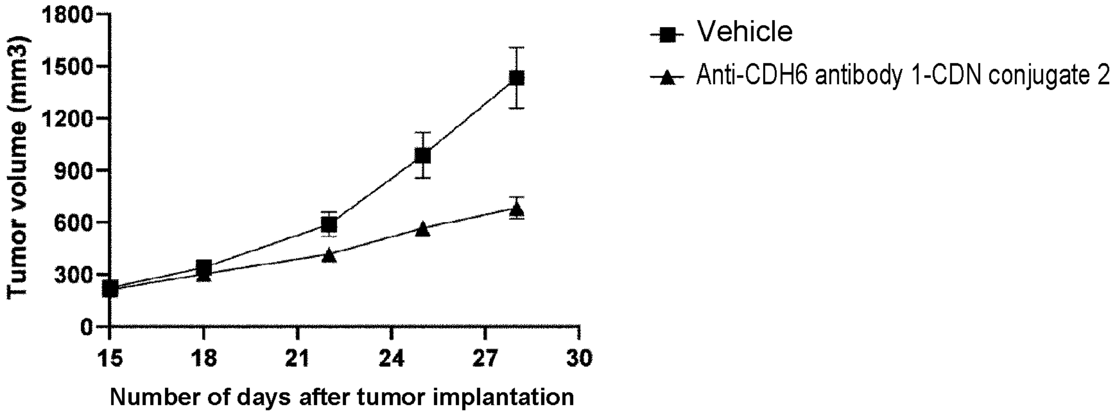


Fig. 30

SEQ ID NO: 47 the amino acid sequence of wild-type Endo-S **NCBI Accession number: AAK34539.1**

1 MDKHLLVKRT LGCVCAATLM GAALATHHDS LNTVKAEEKT VQVQKGLPSI 50
51 DSLHYLSENS KKEFKEELSK AGQESQKVKE ILAKAQQADK QAQELAKMKI 100
101 PEKIPMKPLH GPLYGGYFRT WHDKTSDPTE KDKVNSMGEL PKEVDLAFIF 150
151 HDWTKDYSLF WKELATKHVP KLNKQGTRVI R**T**IPWRFLAG GDNSGIAEDT 200
201 SKYPNTPEGN KALAKAIVDE YVYKYNLDGL **D**V**D**VEHDSIP KVDKKEDTAG 250
251 VERSIQVFEE IGKLIGPKGV DKSRLFIM**DS** **T**YMADKNPLI ERGAPYINLL 300
301 LV**Q**VYGSQGE KGGWEPVSNR PEKTMEERWQ GYSKYIRPEQ YMIGFSFYE**E** 350
351 NAQEGNLWYD INSRKDEDKA NGINTDITGT RAERYARWQP KTGGVKGGIF 400
401 SYAIDRDGVA HQPKKYAKQK EFKDATDNIF HSDYSVSKAL KTVMLKDKSY 450
451 DLIDEKDFPD KALREAVMAQ VGTRKGDLER FNGTLRLDNP AIQSLEGLNK 500
501 FKKLAQLDLI GLSRITKLDR SVLPANMKPG KDTLETVLET YKKDNKEEPA 550
551 TIPPVSLKVS GLTGLKELDL SGFDRETLAG LDAATLTSLE KVDISGNKLD 600
601 LAPGTENRQI FDTMLSTISN HVGSNEQTVK FDKQKPTGHY PDTYGKTSLR 650
651 LPVANEKVDL QSQLLFGTVT NQGTLINSEA DYKAYQNHKI AGRSFVDSNY 700
701 HYNNFKVSYE NYTVKVTDST LGTTTDKTLA TDKEETYKVD FFSPADKTKA 750
751 VHTAKVIVGD EKTMMVNLAE GATVIGGSAD PVNARKVFDG QLGSETDNIS 800
801 LGWDSKQSII FKLKEDGLIK HWRFFNDSAR NPETTNKPIQ EASLQIFNIK 850
851 DYNLDNLLEN PNKFDDEKYW ITVDTYSAQG ERATAFSNTL NNITSKYWRV 900
901 VFDTKGDRYS SPVVPELQIL GYPLPNADTI MKTVTTAKEL SQQKDKFSQK 950
951 MLDELKIKEM ALETSLNSKI FDVTAINANA GVLKDCIEKR QLLKK 995

# Fig. 31

SEQ ID NO: 48 the amino acid sequence of wild-type Endo-S2 **NCBI Accession number: ACI61688.1**

1 MDKHLLVKRT LGCVCAATLM GAALATHHDS LNTVKAEEKT VQTGKTDQQV 50
51 GAKLVQEIRE GKRGPLYAGY FRTWHDRAST GIDGKQQHPE NTMAEVPKEV 100
101 DILFVFHDHT ASDSPFWSEL KDSYVHKLHQ QGTALVQ**T**IG VNELNGRTGL 150
151 SKDYPDTPEG NKALAAAIVK AFVTDRGVDG L**D**I**D**IEHEFT NKRTPEEDAR 200
201 ALNVFKEIAQ LIGKNGSDKS KLLIM**DTT**LS VENNPIFKGI AEDLDYLLR**Q** 250
251 YYGSQGGEAE VDTINSDWNQ YQNYIDASQF MIGFSFFE**E**S ASKGNLWFDV 300
301 NEYDPNNPEK GKDIEGTRAK KYAEWQPSTG GLKAGIFSYA IDRDGVAHVP 350
351 STYKNRTSTN LQRHEVDNIS HTDYTVSRKL KTLMTEDKRY DVIDQKDIPD 400
401 PALREQIIQQ VGQYKGDLER YNKTLVLTGD KIQNLKGLEK LSKLQKLELR 450
451 QLSNVKEITP ELLPESMKKD AELVMVGMTG LEKLNLSGLN RQTLDGIDVN 500
501 SITHLTSFDI SHNSLDLSEK SEDRKLLMTL MEQVSNHQKI TVKNTAFENQ 550
551 KPKGYYPQTY DTKEGHYDVD NAEHDILTDF VFGTVTKRNT FIGDEEAFAI 600
601 YKEGAVDGRQ YVSKDYTYEA FRKDYKGYKV HLTASNLGET VTSKVTATTD 650
651 ETYLVDVSDG EKVVHHMKLN IGSGAIMMEN LAKGAKVIGT SGDFEQAKKI 700
701 FDGEKSDRFF TWGQTNWIAF DLGEINLAKE WRLFNAETNT EIKTDSSLNV 750
751 AKGRLQILKD TTIDLEKMDI KNRKEYLSND ENWTDVAQMD DAKAIFNSKL 800
801 SNVLSRYWRF CVDGGASSYY PQYTELQILG QRLSNDVANT LKD 843

# Fig. 32

SEQ ID NO: 49 the amino acid sequence of wild-type Endo-Si **NCBI Accession number: EKB52062.1**

1 MNKRLLVKRT FGCVCAAAIL GVAPLSHPTI VEAREELKMP NGLEQSIADV 50
51 EAKIDALTYL SKNSKDEFKH SMYEIPSNRE HKPVSPKQAL QNAKKADAQA 100
101 ERLAKMTIPK KEELKALEGP LYGGYFRTWQ DKTSDPTETN KVNSFGELPK 150
151 EVDLAFVFHD YTKDYSLFWE ELATKQVPKL NKQGTRVIR**T** IPWRFLSGAD 200
201 HSDISADKEK FPNTEAGNKA LAKAIVDEYV YKYNLDGL**D**I **D**IERDSVPKV 250
251 NDKEDPEALA RTVEVFKEIG KLIGANGADK SRLLIM**DTT**Y TAEENPLIKE 300
301 TAQYLNLLLV **Q**VYGFSGENG NYLHHKNILD ETSSMEGRWQ GYSKYIRPEQ 350
351 YMVGFSFYE**E** KDFNNRWKDI NEEDPSDPHI GEKIQGTRAE RYAKWQPKTG 400
401 GLKGGLFSYA IDRDGVAQPK QKTEHPELDK IVKSEYKVSK ALKKLMMTDD 450
451 QYQPIDQSDF PDKALRESII KQVGTRRGDL ERFKGTLRLD NPEIKDLTGL 500
501 NKLKRVAKLE LINLPKITKI DKDDLPQNLK PLTDNQKSNL EIKGTYDDSK 550
551 LYKDIPAFDL VISGLSGLES LDISGHQRDT LSGIDASTLP SLKAINISDN 600
601 HFDLAQGTEN RHILDTILAT LAKNGASTAS FDKQKPKGLY PESYSTAPLH 650
651 LQVGQGKINV IDDLIFGTRT NQNTLINTEN DFEAYKEQTI QGKPFIAPDY 700
701 LYDNFKVSYK EYSASIVDST LAETTDKTID TAKAETYQVT VSNKDGKTVH 750
751 SVKVIVGDEK PMMVNLAQDA KIIGTDNMTQ SAKVFDGQKD QFLLSWNKDS 800
801 SVIFELKTPG TAKHWRFFDD GKNDSVTLSV FKGDASNFET EKDKAENWVE 850
851 ITKDSRKNDD KVFSSPLEVD NAKYLKVTIK KEAKYIYFNE LQILGYPGVV 900
901 AKKTADDLRP TEADKSDDKS DKNDTEAK 928

# Fig. 33

SEQ ID NO: 50 the amino acid sequence of wild-type Endo-M **NCBI Accession number: BAB43869.1**

```
1   MPSLQLQPDD KLAPVSFALK SMNELRDWTP DEKIKFNVSS VALQPRVKNA 50
51  LKPQLLLTHD MAGGYKEDKN IQGNNYKDIY NIQYWHLADT FVYFSHERVS 100
101 IPPVNWTNAC HRNGVKCLGT FLVEGNNQMH EMEALLHGPP LLNNTDDPMR 150
151 LWSPYYADQL VAIAKHYGFD GWLFNIECEF FPFPTNPKFK AEELAKFLHY 200
201 FKEKLHNEIP GSQLIWYDSM TNEGEIHWQN QLTWKNELFF KNTDGIFLNY 250
251 WWKKEYPEMA RRVAEGIGRS GLEVYFGTDV WGRHTYGGGG FKSYKGVKTA 300
301 YSAMTSSALF GMAWTYEHFE KSEFEKMDRL FWCGGKYSDY PPPPPKNPDD 350
351 EKEVESDDSE DELMYGHKKG IADTVESIPV PGTDWFVTNF DRGFGNRFYY 400
401 RGKRLLSQPW SHLSHQAILP NKSYRNPEIY PTDQNIKITS SLDCDHGAFL 450
451 GGTSLIIKGQ RFNHRESHDV ETEISIPLYK LSLDASKGCS LRYIYRTLLM 500
501 KDVKLTVACH FSLKTNDSVN FFKVWQPDEN FSFEYDDGMR ATVTTENSTE 550
551 SRCFLLRTTE EDTGENDWIT KTINVPAVPE GSQLYITRLE VSVVLDTAGL 600
601 VGLVNQVIAC LGYISIIPTI NSGIKTDSSR IIQDLFWKDQ KYTKIGKESL 650
651 DDIAQEEVHR YYGTLNWENT ANVVNAWEEI DYYNVFYKES DDSATRIFLG 700
701 TAFCNQFRVS GLDIILSKLP KIVIEAVNKE GYISSSGSID LSLN       744
```

# Fig. 34

SEQ ID NO: 51 the amino acid sequence of wild-type Endo-Rp **NCBI Accession number:** unregistered *

```
1   MPSLELQQAA DTRLFESMPL QTMNELGSWE PSNASRANIA TIPLHQRSNL 50
51  DPAEPRLIVT HDMAGGYKED SNIQGNTYDT IYSCQYWQYV DTFIYFSHHR 100
101 VTIPPVNWIN ACHRNGVKTL GTFIVEGAAG MFALERFVYG PEPGQRNSWS 150
151 PYYADKLVDI AEFYGFDGWL LNIESDFFPL YRNPSLKAIH LAKLLRYLKN 200
201 AMHARVPGSE IIWYDSMTTN GSVQWQNNIT PKNSIFFEAA DGIFLNYWWN 250
251 ATVPPLALQV AHRLGRQGSD VYFGTDVWGR GTFGGGGFDS YLAVGTARAF 300
301 KTSSALFGTA WIYEHFGKKD FELMDRLLWL GGDQSEYPAQ EGEQNRTVKV 350
351 TSHLGRHPGI ADVSPVRSAP GKTWFATWFD RGYGTGFYYQ GKKLLSQPWS 400
401 HLSHQSIPPN LIARLQREEN HGLSYFLADD DAYIGGTSLL IAAEITQERQ 450
451 LPLYQLEYDV TEGCEVQFIY KSPEPDMQGK IDIYLNLQVT DILPDELAFY 500
501 WQDVTDASSQ ADATTAMRLY LNENTVIYLK PSRKQELAEG WLLCSVRVPP 550
551 TYPLGIATIK ELGIHVDGKE TVLFRLGLLT IIPLGDAPSA LSRITQVQLQ 600
601 RDEDIHSKCP SSSCELWATL SWMMEHNSKE DWDQVDHYMI FFKNVDSKAE 650
651 PIFLGTSFST EYRISGLEIK KHGNSIEIWA VNRLGTVIAR QDIDIQ      696
```

# Fig. 35

SEQ ID NO: 52 the amino acid sequence of wild-type Endo-CC **NCBI Accession number: EAU82416.1**

| | | | | | | |
|---|---|---|---|---|---|---|
| 1 | MPIAGKKFHP | RALPEFWRTF | REMDEWRATQ | TGPQARPAEG | ILKYVPRKIR | 50 |
| 51 | PADIAGKGRL | LVSHDYKGGY | VEDPFSKSYS | FNWWFSTDSF | NYFAHHRITI | 100 |
| 101 | PPPEWINAAH | RQGVPILGTI | IFEGGSDEDI | LRMVIGKTPG | STSNFHAERN | 150 |
| 151 | AEYTVPVSSY | YAELFADLAV | ERGFDGWLL**N** | VEIGLQGGSE | QARGLAAWVA | 200 |
| 201 | LLQQEVLKKV | GPHGLVIW**Y**D | **S**VTVRGDLWW | QDRLNAFNLP | FFLNSSGIFT | 250 |
| 251 | **N**YWWYNDAPQ | KQIDFLSRVD | PNLTGQTAEP | HQYNLQKTIQ | DIYIGV**D**V**W**G | 300 |
| 301 | RGSHGGGGFG | AYKAIEHADP | KGLGFSVA**LF** | AQ**G**WTWETEE | EKPGWNWAQF | 350 |
| 351 | WDYDSKLWVG | PPGVVEAPDH | TVKPGEYPCV | HGPFQPISSF | FLTYPPPDPL | 400 |
| 401 | DLPFYTNFCP | GIGDAWFVEG | KEVFRSETGW | TDMDKQTTVG | DLVWPRPKIY | 450 |
| 451 | DLPSQNASQA | TLNAAFNFND | AWNGGNSLQI | NLTVPGGATT | YGAYWVPIQT | 500 |
| 501 | FTFSSRRQYE | ASIVYKPGLS | GKTRFDAKYE | VGIRTITGED | QGKIISNTTT | 550 |
| 551 | EVGNGWRKVH | ILFEIETPVE | GGSIIVPSSI | GLVIAVSNVS | TTEQFEFPFL | 600 |
| 601 | VGQITIHPHL | PDRYKEFKPA | LLWLLFTPSA | GTNSLDGTLT | WDVVAAIERP | 650 |
| 651 | PPVEINNPDD | AQIPWNLQPT | KQEWFPDFLY | FNVYVLELLD | GGGQGPPQWI | 700 |
| 701 | GTTGYDGEKK | RFFIYDESLP | PTSGLRRFTF | QIEGVLETGE | STHWYDAPAA | 750 |
| 751 | PSATAGGEQK | RTRRTSLKSV | LSPLRRKKSK | GDISVAK | | 787 |

# Fig. 36

SEQ ID NO: 53 the amino acid sequence of wild-type Endo-Om **NCBI Accession number: BAN20080.1**

| | | | | | | |
|---|---|---|---|---|---|---|
| 1 | MAQSQLLGGA | VRPVFFDKLE | ELRRWHTQSA | NLSRESELDS | LNVATEPFSS | 50 |
| 51 | YERAQTGSGS | RSSEPVPGDK | EDPPIKLMVC | HDFKGGYQDY | EDAQPLGYFP | 100 |
| 101 | HPTGSRYFLQ | YPQLIDQFVY | FSHHRVTVPP | VNWINFCHRN | GIKCFGTVIF | 150 |
| 151 | EGNASKDFEE | LDRLVSRDEK | GDFVFVDALI | KLAAHYGFDG | YLL**N**IETTFS | 200 |
| 201 | NTKIAADLEP | FAEQLKSGLH | CLDSKNELIW | **Y**D**S**YVFPANK | VSYTNGVTES | 250 |
| 251 | NYNFFSLSDA | FFS**N**YWWNIK | NLQENIKNVG | VLGVQKKIYV | GY**D**V**W**GRGTL | 300 |
| 301 | VGKGGFDSSL | ACKMIAKFKS | NVA**LF**AP**A**WT | YESLGPKDFN | QNDARFWIGL | 350 |
| 351 | FENESSISST | VPPHSSAVYK | INESSFIFYT | NFSSGEGNRF | FSKGSEVYRK | 400 |
| 401 | NWVNGSLQFD | LPIDLHRKDK | NGLQWALDKS | DAFHGGACLE | IKYSEIKDEN | 450 |
| 451 | GYQIFNNQMV | SDFTLFNFTK | ECHFPTVNVK | VTYKLNHKTK | STFKIKIKYI | 500 |
| 501 | IERRFRSVQT | VRTGYLTIPL | LSTSGKWFTV | EESFQINLQT | SHEYIVLESA | 550 |
| 551 | HVTYDEDRSA | DSFFRSYIVE | DSAITSVIDN | EEYEKLINSE | IYNDDEDEDW | 600 |
| 601 | ILVPSDVSIS | SSESQSNDSK | TQYLGRKLFG | NKSTPKTRTL | EGTAPLLRIG | 650 |
| 651 | EFAIISANNY | PSSNFLAVTS | VKSIESSRLE | GDSLVLLNWQ | VGEGHQKGVC | 700 |
| 701 | YYIIYVNGAV | VGLSVAPKFI | YQDTELASEN | SASARSNYKK | SGLGSSSDRK | 750 |
| 751 | SKVRVDSVDK | LGNVFTGSEV | WV | | | 772 |

# Fig. 37

**SEQ ID NO: 54 the amino acid sequence of wild-type Endo-Sd GenBank Accession number: ANI26082**

| | | | | | | |
|---|---|---|---|---|---|---|
| 1 | MDKRLLVKRT | LGCVCSATLM | GTILGTHHDS | LISVKAEEKI | TQVSQTSTSI | 50 |
| 51 | DDLHYLSENS | KKEFKEELSK | EKVPEKVKEI | LSKAQQANKQ | AQELAEMKVP | 100 |
| 101 | DKIPMKPLNG | PLYGGYFRTW | HDKTSDPLEK | DKVNSMGELP | KEVDLAFVFH | 150 |
| 151 | DWTKDYSLFW | KELATKHVPK | LNKQGTRVIR | **T**IPWRFLAGG | DNSGIAEDAS | 200 |
| 201 | KYPNTPEGNK | ALAKAIVDEY | VYKYNLDGL**D** | V**D**IEHDSIPK | VNGEASDENL | 250 |
| 251 | KRSIDVFEEI | GKLIGPKGAD | KSRLFIM**DST** | YMADKNPLIE | RGAPYIDLLL | 300 |
| 301 | V**Q**VYGSQGEQ | GEFQNDTKSV | TKTPEERWQG | YSKYIRPEQY | MIGFSFYE**E**K | 350 |
| 351 | AGSGNLWYDI | NARKDEDTAN | GINDDITGTR | AERYARWQPK | TGGVKGGIFS | 400 |
| 401 | YAIDRDGVAH | QPKQIAEKDK | QSVKNNRPLI | SEITDNIFHS | NYSVSKTLKT | 450 |
| 451 | VMLKDKAYDL | IDEKDFPDKA | LREAVMAQVG | TRKGDLERFN | GTLRLDNPAI | 500 |
| 501 | QSLEGLNKFK | KLAQLDLIGL | SRIIKLDQSV | LPANMKPGKD | PLETVLETYK | 550 |
| 551 | KNGKEEPAII | PPVSLTVSGL | TGLKELDLSG | FDRETLAGID | AATLTSLEKV | 600 |
| 601 | DISDNKLDLA | PKTENRQIFD | VMLSTVNNNA | GISEQSIKFD | NQKPAGNYPQ | 650 |
| 651 | TYGATNLQLP | VRQEKIDLQH | QLLFGTITNQ | GTLINSEADY | KTYRNQKIAG | 700 |
| 701 | RNFVDPDYPY | NNFKVSHDNY | TVKVTDSTLG | TTTDKMLATD | KEETYKVDFF | 750 |
| 751 | SPTDKTKAVH | TAKVIVGDEK | TMMVNLAEGA | TVIKSENDEN | AQKVFNGIME | 800 |
| 801 | YNPLSFNNKS | SIIFEIKDPS | LAKYWRLFND | SSKDKKDYIK | EAKLEVFTGQ | 850 |
| 851 | LNAEADVKTI | LEKPDNWVTV | STYSGEEKVF | SHSLDNISAK | YWRVTVDNKK | 900 |
| 901 | DQYGYVSLPE | LQILGYPLPN | ADTIMKTVTV | AKELSQQKDK | FPQQLLDEST | 950 |
| 951 | AKEAVVEASL | NSKLFDTGVI | NTNVEALKNV | VDECLAYEKN | KETAFKATED | 1000 |
| 1001 | YRAAVNGVKA | ESVTVEEMAQ | LKDLIGKAAH | LNSKIDAKLA | DREYDKDLLG | 1050 |
| 1051 | LIGELTNITR | TVKSFVK | | | | 1067 |

# Fig. 38

**SEQ ID NO: 55 the amino acid sequence of wild-type Endo-Sz GenBank Accession number: KIS14581.1**

| | | | | | | |
|---|---|---|---|---|---|---|
| 1 | MEKQVLVKKT | LKCVCAAALM | VAILAAQHDS | LIRVKAEDKL | VQTSPSVSAI | 50 |
| 51 | DALHYLSENS | KKEFKEELSK | VEKAQPEKLK | EIVSKAQQAD | KQAKTLAEMK | 100 |
| 101 | VPEKIPMKPL | KGPLYGGYFR | TWHDKTSDPA | EKDKVNSMGE | LPKEVDLAFV | 150 |
| 151 | FHDWTKDYSL | FWQELATKHV | PTLNKQGTRV | IR**T**IPWRFLA | GGDHSGIAED | 200 |
| 201 | AQKYPNTPEG | NKALAKAIVD | EYVYKYNLDG | L**D**V**D**IERDSI | PKVNKEESKE | 250 |
| 251 | GIERSIQVFE | EIGKLIGPKG | ADKSRLFIM**D** | **ST**YMADKNPL | IERGAPYIDL | 300 |
| 301 | LLV**Q**VYGTQG | EKGGFDNANH | KAVDTMEERW | ESYSKYIRPE | QYMVGFSFYE | 350 |
| 351 | **E**KANSGNLWY | DVNVEDDTNP | NIGSEIKGTR | AERYAKWQPK | TGGVKGGIFS | 400 |
| 401 | YGIDRDGVAH | PKKNGPKTPD | LDKIVKSDYK | VSKALKKVME | NDKSYELIDQ | 450 |
| 451 | KDFPDKALRE | AVIAQVGSRR | GNLERFNGTL | RLDNPDIKSL | EGLNKLKKLA | 500 |
| 501 | KLELIGLSQI | TKLDSSVLPE | NIKPTKDTLV | SVLETYKNDD | RKEEAKAIPQ | 550 |
| 551 | VALTISGLTG | LKELNLAGFD | RDSLAGIDAA | SLTSLEKVDL | SSNKLDLAAG | 600 |
| 601 | TENRQILDTM | LATVTKHGGV | SEKTFVFDHQ | KPTGLYPDTY | GTKSLQLPVA | 650 |
| 651 | NDTIDLQAKL | LFGTVTNQGT | LINSEADYKA | YQEQEIAGHR | FVDSSYDYKA | 700 |
| 701 | FAVTYKDYKI | KVTDSTLGVT | DHKDLSTSKE | ETYKVEFFSP | INSTKPVHEA | 750 |
| 751 | KIVVGEEKTM | MVNLAEGATI | IGGDADPTNA | KKVFDGLLNN | DTTTLSTSNK | 800 |
| 801 | ASIIFELKEP | GLVKHWRFFN | DSKISKADYI | KEAKLEAFVG | HLEDSSKVKD | 850 |
| 851 | SLEKSTEWVT | VSDYSGEAQE | FSQPLNNIGA | KYWRITIDNK | KSQYGYVSLP | 900 |
| 901 | ELQIIGHRLP | EAATVMTTMA | AAEELSQQKD | KFSQEQLKEL | EVKVAALKAA | 950 |
| 951 | LDNKMFNADT | INASFADVKA | YIDKLLADAA | GKKTLGKATK | EAQPVATDAK | 1000 |
| 1001 | EKAESENPKA | D | | | | 1011 |

# Fig. 39

**SEQ ID NO: 56 the amino acid sequence of wild-type Endo-Se** **GenBank Accession number: CAW92602.1**

| | | | | | | |
|---|---|---|---|---|---|---|
| 1 | MEKQVLVKKT | LKCVCAAALM | VAILAAQHDS | LIRVKAEDKV | VQTSPSVSAI | 50 |
| 51 | DDLHYLSENS | KKEFKEGLSK | AGEVPEKLKD | ILSKAQQADK | QAKVLAEMKV | 100 |
| 101 | PEKIAMKPLK | GPLYGGYFRT | WHDKTSDPAE | KDKVNSMGEL | PKEVDLAFVF | 150 |
| 151 | HDWTKDYSFF | WQELATKHVP | TLNKQGTRVI | R**T**IPWRFLAG | GDHSGIAEDT | 200 |
| 201 | QKYPNTPEGN | KALAKAIVDE | YVYKYNLDGL | **D**V**D**IERDSIP | KVNGKESNEN | 250 |
| 251 | IQRSIAVFEE | IGKLIGPKGA | DKSRLFIM**DS** | **T**YMADKNPLI | ERGAQYIDLL | 300 |
| 301 | LV**Q**VYGTQGE | KGDWDPVARK | PEKTMEERWE | SYSKYIRPEQ | YMVGFSFYE**E** | 350 |
| 351 | NAGSGNLWYD | INERKDDHNP | LNSEIAGTRA | ERYAKWQPKT | GGVKGGIFSY | 400 |
| 401 | AIDRDGVAHQ | PKKVSDDEKR | TNKAIKDITD | GIVKSDYKVS | KALKKVMEND | 450 |
| 451 | KSYELIDQKD | FPDKALREAV | IAQVGSRRGD | LERFNGTLRL | DNPDIKSLEG | 500 |
| 501 | LNKLKKLAKL | ELIGLSQITK | LDSLVLPANA | KPTKDTLANV | LEAYDSAKKE | 550 |
| 551 | ETKAIPQVAL | TISGLTGLKE | LNLAGFDRDS | LAGIDAASLT | SLEKVDLSSN | 600 |
| 601 | KLDLAAGTEN | RQILDTMLAT | VTKHGGVSEK | TFVFDHQKPT | GLYPDTYGTK | 650 |
| 651 | SLQLPVANDT | IDLQAKLLFG | TVTNQGTLIN | SEADYKAYQE | QEIAGHRFVD | 700 |
| 701 | SSYDYKAFAV | TYKDYKIKVT | DSTLGVTDHK | DLSTSKEETY | KVEFFSPTNS | 750 |
| 751 | TKPVHEAKVV | VGAEKTMMVN | LAEGATVIGG | DADPTNAKKV | FDGLLNNDTT | 800 |
| 801 | ILSTSNKASI | IFELKEPGLV | KYWRFFNDSK | ISKADCIKEA | KLEAFVGHLE | 850 |
| 851 | AGSKVKDSLE | KSSKWVTVSD | YSGEDQEFSQ | PLNNIGAKYW | RITVDTKGGR | 900 |
| 901 | YNWPSLPELQ | IIGYQLPAAD | LVMAMLATAE | ELSQQKDKFS | QEQLKELEVK | 950 |
| 951 | IAALKAALDS | KMFNADAINA | STADLKAYVD | KLLADRTDQE | KVAKAAKVEQ | 1000 |
| 1001 | PVATDIKENT | EPENPKTD | | | | 1018 |

## INTERNATIONAL SEARCH REPORT

International application No.
**PCT/JP2023/030872**

**A. CLASSIFICATION OF SUBJECT MATTER**

***A61K 47/68***(2017.01)i; ***A61K 31/7064***(2006.01)i; ***A61K 31/708***(2006.01)i; ***A61K 39/395***(2006.01)i; ***A61K 45/00***(2006.01)i; ***A61P 35/00***(2006.01)i; ***A61P 43/00***(2006.01)i; ***C07K 16/28***(2006.01)i; ***C12N 15/13***(2006.01)i
FI: A61K47/68; A61K31/7064; A61K31/708; A61K39/395 N; A61K39/395 T; A61K39/395 L; A61P35/00; A61P43/00 121; A61K45/00; C12N15/13; C07K16/28 ZNA

According to International Patent Classification (IPC) or to both national classification and IPC

**B. FIELDS SEARCHED**

Minimum documentation searched (classification system followed by classification symbols)

A61K47/68; A61K31/7064; A61K31/708; A61K39/395; A61K45/00; A61P35/00; A61P43/00; C07K16/28; C12N15/13

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/050406 A1 (DAIICHI SANKYO CO., LTD.) 12 March 2020 (2020-03-12) claims, paragraph [0182], examples | 1-50 |
| Y | WO 2021/177438 A1 (DAIICHI SANKYO CO., LTD.) 10 September 2021 (2021-09-10) claims, examples | 1-50 |
| Y | JP 2016-508153 A (GENMAB A/S) 17 March 2016 (2016-03-17) claims, paragraphs [0051], [0119], examples | 1-50 |
| P, Y | WO 2023/028715 A1 (ZYMEWORKS BC INC.) 09 March 2023 (2023-03-09) claims, examples | 1-50 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 September 2023** | **14 November 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/030872**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   "In the form of an Annex C/ST.25 text file" above should be understood as "in ST. 26 format".

Form PCT/ISA/210 (continuation of first sheet) (January 2015)

**INTERNATIONAL SEARCH REPORT**
**Information on patent family members**

International application No.
**PCT/JP2023/030872**

| Patent document cited in search report | Publication date (day/month/year) | Patent family member(s) | Publication date (day/month/year) |
|---|---|---|---|
| WO 2020/050406 A1 | 12 March 2020 | US 2022/0008549 A1<br>claims, paragraph [0358], examples<br>EP 3848054 A1<br>CN 112714649 A<br>KR 10-2021-0057066 A | |
| WO 2021/177438 A1 | 10 September 2021 | EP 4115909 A1<br>claims, examples<br>CN 115209921 A<br>KR 10-2022-0151630 A | |
| JP 2016-508153 A | 17 March 2016 | WO 2014/108483 A1<br>claims, p. 13, lines 3-28, p. 26, lines 3-8, examples<br>EP 2869845 A1<br>KR 10-2016-0027177 A<br>CN 105722529 A | |
| WO 2023/028715 A1 | 09 March 2023 | CA 3177101 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 198807089 A
- WO 199951642 A
- WO 200042072 A
- WO 2013092001 A
- WO 2015109131 A
- WO 2020086776 A
- WO 2013118858 A
- WO 2014099824 A
- WO 2014179335 A
- WO 2014189805 A
- WO 2014189806 A
- WO 2015074145 A
- WO 2015185565 A
- WO 2016096714 A
- WO 2016012305 A
- WO 2016145102 A
- WO 2017027646 A
- WO 2017027645 A
- WO 2017075477 A
- WO 2017093933 A
- WO 2017100305 A
- WO 2017123669 A
- WO 2017161349 A
- WO 2017175147 A
- WO 2017175156 A
- WO 2018009466 A
- WO 2018045204 A
- WO 2018060323 A
- WO 2018067423 A
- WO 2018065360 A
- WO 2014093936 A
- WO 2018009648 A
- WO 2018100558 A
- WO 2021202984 A
- WO 2022097117 A
- WO 2019084060 A
- WO 2020050406 A
- WO 2021177438 A
- WO 2018003983 A
- WO 2014057687 A
- WO 8807089 A
- WO 9428027 A
- WO 9429351 A
- WO 9007861 A
- WO 1998050433 A
- WO 2002092771 A
- WO 2018212136 A
- US 2016361436 A
- WO 2011027868 A
- WO 2022050300 A
- WO 2019065964 A
- WO 2022163846 A
- WO 2018101454 A
- WO 2017137459 A
- WO 2013120066 A


### Non-patent literature cited in the description


- *J Immnotoxicol*, 2008, vol. 5 (1), 11-5
- *Protein Eng Des Sel.*, 2016, vol. 29 (10), 457-66
- *J Biol Chem*, 2017, vol. 292 (5), 1865-75
- *Nature Cancer*, 2021, vol. 2, 18-33
- **SEAN W. SMITH**. SBT6050, a HER2-Directed TLR8 ImmunoTAC™ Therapeutic, is a Potent Human Myeloid Cell Agonist with Tumor-Localized Activity. *World ADC 2020*
- *Nature*, 2008, vol. 455, 674-678
- *Mol. Cell*, 2013, vol. 51, 226-235
- *Science*, 2015, vol. 347, 2630
- *J. Virol.*, 2014, vol. 88, 5328-5341
- *Immunity*, 2014, vol. 41, 830-842
- *Immunity*, 2014, vol. 41, 843-852
- *Nat. Med.*, 2015, vol. 21, 1209-1215
- *J. Immunol.*, 1994, vol. 153, 4684-4693
- *J. Clin. Oncol.*, 2011, vol. 29, 2965-2971
- *J. Immunol.*, 2013, vol. 190, 5216-5225
- *Sci. Rep.*, 2016, vol. 6, 19049
- *Mol. Cell*, 2015, vol. 59, 891-903
- *Cell Rep.*, 2015, vol. 11, 1018-1030
- *AACR Tumor Immunology and Immunotherapy*, 2017
- *Biochem. Biophys. Res. Commun.*, 2007, vol. 355, 751-757
- *PLoS One*, 21 October 2013, vol. 8 (10), e77846
- *Genes and Immunity*, 2011, vol. 12, 263-269
- *Protein Cell*, 2018, vol. 9 (1), 33-46
- *Pharm Res*, 2015, vol. 32, 3526-3540
- *Int. J. Mol. Sci*, 2016, vol. 17, 561

- *Proceedings of the National Academy of Sciences of the United States of America*, 15 May 1969, vol. 63 (1), 78-85
- *Front. Immunol.*, 2014, vol. 5, 520
- **KABAT et al.** Sequences of Proteins of Immunological Interest. Service National Institutes of Health, 1991
- *Cell Death and Differentiation*, 2008, vol. 15, 751-761
- *Molecular Biology of the Cell*, December 2004, vol. 15, 5268-5282
- *Bio Techniques*, January 2000, vol. 28, 162-165
- **KOHLER** ; **MILSTEIN**. *Nature*, 1975, vol. 256, 495-497
- Monoclonal Antibodies. Plenum Press, 1980, 365-367
- *Proc. Natl. Acad. Sci. U.S.A.*, 1984, vol. 81, 6851-6855
- *Nature*, 1986, vol. 321, 522-525
- *Journal of Chromatography A*, 1995, vol. 705, 129-134
- *Analytical Biochemistry*, 2007, vol. 360, 75-83
- *Cell*, 1981, vol. 23, 175-182
- *Proc. Natl. Acad. Sci. U.S.A.*, 1980, vol. 77, 4126-4220
- *ACS Chem. Biol.*, 2012, vol. 7, 110-122
- *ACS Med. Chem. Lett.*, 2016, vol. 7, 1005-1008
- *Bioconjugate Chem.*, 2015, vol. 26, 2233-2242
- *Angew. Chem. Int. Ed.*, 2016, vol. 55, 2361-2367
- **EON-DUVAL, A. et al.** *Biotechnol. Prog.*, 2012, vol. 28, 608-622
- **SANGLIER-CIANFERANI, S.** *Anal. Chem.*, 2013, vol. 85, 715-736
- **EDELMAN et al.** *Proc. Natl. Acad. Sci. U.S.A.*, 1969, vol. 63, 78-85
- **UMEKAWA et al.** *Biochim. Biophys. Acta*, 2010, vol. 1800, 1203-1209
- *Org. Lett.*, 2010, vol. 12, 3269-3271
- *PLos ONE*, 2018, vol. 13, e0193534
- *J. Am. Chem. Soc.*, 2004, vol. 126, 15046-15047
- *Protein Science*, 1995, vol. 4, 2411-2423
- *Bioconjugate Chem.*, 2015, vol. 26, 2198-2215
- *Expert Opin Ther Pat.*, 2018, vol. 28, 251-276
- **COLLIN M** ; **OLSEN A**. *EMBO J.*, 2001, vol. 20, 3046-3055
- **GOODFELLOW JJ et al.** *J Am Chem Soc.*, 2012, vol. 134, 8030-8033
- **SJOGREN J et al.** *Biochem J.*, 2013, vol. 455, 107-118
- **SHIVATARE SS et al.** *Chem Commun*, 2018, vol. 54, 6161-6164
- **HUANG W et al.** *Chembiochem*, 2011, vol. 12, 932-941
- **GIDDENS JP et al.** *J Biol Chem.*, 2016, vol. 291, 9356-9370
- **PIER GB** ; **MADIN SH.** *Int J Syst Bacteriol.*, 1976, vol. 26, 545-553
- **VINCENT P. RICHARD et al.** *Genome Biol. Evol.*, 2014, vol. 6, 741-753
- **B. TRASTOY et al.** *PNAS*, 2014, vol. 111 (18), 6714-6719
- **YAMAMOTO K et al.** *Biochem Biophys Res Commun.*, 1994, vol. 203, 244-252
- **UMEKAWA M et al.** *J. Biol Chem.*, 2021, vol. 285, 511-521
- **ESHIMA Y et al.** *PLoS One.*, 2015, vol. 10, 0132859
- **MURAKAMI S et al.** *Glycobiology*, 2013, vol. 23, 736-744
- **JOAQUIM et al.** *Processes*, 2022, vol. 10, 494
- **F GRUENINGER-LEITCH et al.** *Protein Sci.*, 1996, vol. 12, 2617-22
- **EMILY MK et al.** *Protein Eng Des Sel.*, 2005, vol. 10, 497-501
- **CHEN et al.** *Adv Drug Deliv Rev.*, 15 October 2013, vol. 65 (10), 1357-1369
- **RICHARD R. RUSTANDI et al.** *Electrophoresis*, September 2008, vol. 29 (17), 3612-20
- **MASAKI KUROGOCHI et al.** *PLOS ONE*, 22 July 2015
- **SHIYI WANG et al.** *Journal of Chromatography A*, 2010, vol. 1217, 6496-6502
- *Immunity*, 2013, vol. 39, 1019-1031
- *Cell Rep.*, 2014, vol. 6, 421-430
- *PLoS ONE*, 2018, vol. 13, e0193534
- **HUANG W et al.** *J Am Chem Soc.*, 2012, vol. 134, 12308-12318